# EUROPEAN PATENT APPLICATION

(11) **EP 2 623 104 A1**
(43) Date of publication of application: **07.08.2013**
(21) Application number: 13163748.0
(22) Date of filing: 19.03.2010
(51) Int. Cl.: A61K 31/7064, A61K 31/7052, A61K 31/7068, A61K 31/7076, A61K 31/7042, A61P 31/12, C07H 19/06, C07H 19/12, C07H 19/14, C07H 19/16, C07H 19/20

(54) **Substituted nucleoside and nucleotide analogs**

(30) Priority: 20.03.2009 US 162198 P; 10.07.2009 US 224815 P; 14.08.2009 US 234169 P
(62) Divisional of application: 10754203.7
(71) Applicant: Alios Biopharma, Inc., South San Francisco, CA 94080 (US)
(72) Inventor: Beigelman, Leonid, San Mateo, CA 94402 (US); Blatt, Lawrence, San Francisco, CA 94121 (US); Wang, Guangyi, Carlsbad, CA 92008 (US)
(74) Representative: Stenbäck, Maria Elisabeth

(57) **Abstract**

Disclosed herein are nucleotide analogs with protected phosphates, methods of synthesizing nucleotide analogs with protected phosphates and methods of treating diseases and/or conditions such as viral infections, cancer, and/or parasitic diseases with the nucleotide analogs with protected phosphates.

## Description

### BACKGROUND

### Field

The present application relates to the fields of chemistry, biochemistry and medicine. More particularly, disclosed herein are nucleotide analogs with protected phosphates, pharmaceutical compositions that include one or more nucleotide analogs with protected phosphates and methods of synthesizing the same. Also disclosed herein are methods of treating diseases and/or conditions with the nucleotide analogs with protected phosphates.

### Description of the Related Art

Nucleoside analogs are a class of compounds that have been shown to exert antiviral and anticancer activity both in vitro and in vivo, and thus, have been the subject of widespread research for the treatment of viral infections and cancer. Nucleoside analogs are therapeutically inactive compounds that are converted by host or viral enzymes to their respective active anti-metabolites, which, in turn, inhibit polymerases involved in viral or cell proliferation. The activation occurs by a variety of mechanisms, such as the addition of one or more phosphate groups and, or in combination with, other metabolic processes.

### SUMMARY

An embodiment disclosed herein relates to a compound of Formula (I), or a pharmaceutically acceptable salt, prodrug or prodrug ester thereof.

Another embodiment disclosed herein relates to a compound of Formula (II), or a pharmaceutically acceptable salt, prodrug or prodrug ester thereof.

Some embodiments disclosed herein relate to methods of synthesizing a compound of Formula (I).

Other embodiments disclosed herein relate to methods of synthesizing a compound of Formula (II).

An embodiment disclosed herein relates to pharmaceutical compositions that can include one or more compounds of Formulae (I) and (II), or a pharmaceutically acceptable carrier, diluent, excipient or combination thereof. The pharmaceutical compositions of the compounds of Formulae (I) and (II) can be used in the manufacture of a medicament for treating an individual suffering from a neoplastic disease, a viral infection, or a parasitic disease. The pharmaceutical compositions of the compounds of Formulae (I) and (II) can be used for treating a neoplastic disease, a viral infection, or a parasitic disease.

Some embodiments disclosed herein relate to methods of ameliorating or treating a neoplastic disease that can include administering to a subject suffering from the neoplastic disease a therapeutically effective amount of one or more compounds of Formulae (I) and (II), or a pharmaceutical composition that includes one or more compounds of Formulae (I) and (II). The compounds of Formulae (I) and (II) can be used in the manufacture of a medicament for treating an individual suffering from a neoplastic disease. The compounds of Formulae (I) and (II) can be used for treating a neoplastic disease.

Other embodiments disclosed herein relate to methods of inhibiting the growth of a tumor that can include administering to a subject having a tumor a therapeutically effective amount of one or more compounds of Formulae (I) and (II), or a pharmaceutical composition that includes one or more compounds of Formulae (I) and (II).

Still other embodiments disclosed herein relate to methods of ameliorating or treating a viral infection that can include administering to a subject suffering from the viral infection a therapeutically effective amount of one or more compounds of Formulae (I) and (II), or a pharmaceutical composition that includes one or more compounds of Formulae (I) and (II). The compounds of Formulae (I) and (II) can be used in the manufacture of a medicament for treating an individual suffering from a viral infection. The compounds of Formulae (I) and (II) can be used for treating a viral infection.

Yet still other embodiments disclosed herein relate to methods of ameliorating or treating a parasitic disease that can include administering to a subject suffering from the parasitic disease a therapeutically effective amount of one or more compounds of Formulae (I) and (II), or a pharmaceutical composition that includes one or more compounds of Formulae (I) and (II). The compounds of Formulae (I) and (II) can be used in the manufacture of a medicament for treating an individual suffering from a parasitic disease. The compounds of Formulae (I) and (II) can be used for treating a parasitic disease.

### BRIEF DESCRIPTION OF THE DRAWINGS

Figure 1 shows one method for preparing 2',5'-dimethyl nucleosides and nucleotides in which the base is uracil or guanine.

Figure 2 shows one method for preparing 2',5'-dimethyl nucleosides and nucleotides in which the base is cytosine, uracil, adenine or guanine.

Figure 3 shows one method for preparing 2',5'-dimethyl-adenosine phosphoramidate.

### DETAILED DESCRIPTION

Unless defined otherwise, all technical and scientific terms used herein have the same meaning as is commonly understood by one of ordinary skill in the art. All patents, applications, published applications and other publications referenced herein are incorporated by reference in their entirety unless stated otherwise. In the event that there are a plurality of definitions for a term herein, those in this section prevail unless stated otherwise.

As used herein, any "R" group(s) such as, without limitation, R¹, R^{1a} and R^{1b}, represent substituents that can be attached to the indicated atom. A non-limiting list of R groups include, but are not limited to, hydrogen, alkyl, alkenyl, alkynyl, cycloalkyl, cycloalkenyl, cycloalkynyl, aryl, heteroaryl, heteroalicyclyl, aralkyl, heteroaralkyl, (heteroalicyclyl)alkyl, hydroxy, protected hydroxy, alkoxy, aryloxy, acyl, ester, mercapto, cyano, halogen, thiocarbonyl, O-carbamyl, N-carbamyl, O-thiocarbamyl, N-thiocarbamyl, C-amido, N-amido, S-sulfonamido, N-sulfonamido, C-carboxy, protected C-carboxy, O-carboxy, isocyanato, thiocyanato, isothiocyanato, nitro, silyl, sulfenyl, sulfinyl, sulfonyl, haloalkyl, haloalkoxy, trihalomethanesulfonyl, trihalomethanesulfonamido, and amino, including mono- and di-substituted amino groups, and the protected derivatives thereof. An R group may be substituted or unsubstituted. If two "R" groups are covalently bonded to the same atom or to adjacent atoms, then they may be "taken together" as defined herein to form a cycloalkyl, cycloalkenyl, cycloalkynyl, aryl, heteroaryl or heteroalicyclyl group. For example, without limitation, if R' and R" of an NR'R" group are indicated to be "taken together", it means that they are covalently bonded to one another at their terminal atoms to form a ring that includes the nitrogen:

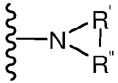

Whenever a group is described as being "optionally substituted" that group may be unsubstituted or substituted with one or more of the indicated substituents. Likewise, when a group is described as being "unsubstituted or substituted" if substituted, the substituent may be selected from one or more the indicated substituents. If no substituents are indicated, it is meant that the indicated "optionally substituted" or "substituted" group may be substituted with one or more group(s) individually and independently selected from alkyl, alkenyl, alkynyl, cycloalkyl, cycloalkenyl, cycloalkynyl, aryl, heteroaryl, heteroalicyclyl, aralkyl, heteroaralkyl, (heteroalicyclyl)alkyl, hydroxy, protected hydroxyl, alkoxy, aryloxy, acyl, ester, mercapto, alkylthio, arylthio, cyano, halogen, thiocarbonyl, O-carbamyl, N-carbamyl, O-thiocarbamyl, N-thiocarbamyl, C-amido, N-amido, S-sulfonamido, N-sulfonamido, C-carboxy, protected C-carboxy, O-carboxy, isocyanato, thiocyanato, isothiocyanato, nitro, silyl, sulfenyl, sulfinyl, sulfonyl, haloalkyl, haloalkoxy, trihalomethanesulfonyl, trihalomethanesulfonamido, and amino, including mono- and di-substituted amino groups, and the protected derivatives thereof. Each of these substituents can be further substituted.

As used herein, "Cₐ to C_{b}" in which "a" and "b" are integers refer to the number of carbon atoms in an alkyl, alkenyl or alkynyl group, or the number of carbon atoms in the ring of a cycloalkyl, cycloalkenyl, cycloalkynyl, aryl, heteroaryl or heteroalicyclyl group. That is, the alkyl, alkenyl, alkynyl, ring of the cycloalkyl, ring of the cycloalkenyl, ring of the cycloalkynyl, ring of the aryl, ring of the heteroaryl or ring of the heteroalicyclyl can contain from "a" to "b", inclusive, carbon atoms. Thus, for example, a "C₁ to C₄ alkyl" group refers to all alkyl groups having from 1 to 4 carbons, that is, CH₃-, CH₃CH₂-, CH₃CH₂CH₂-, (CH₃)₂CH-, CH₃CH₂CH₂CH₂-, CH₃CH₂CH(CH₃)-and (CH₃)₃C-. If no "a" and "b" are designated with regard to an alkyl, alkenyl, alkynyl, cycloalkyl cycloalkenyl, cycloalkynyl, aryl, heteroaryl or heteroalicyclyl group, the broadest range described in these definitions is to be assumed.

As used herein, "alkyl" refers to a straight or branched hydrocarbon chain that comprises a fully saturated (no double or triple bonds) hydrocarbon group. The alkyl group may have 1 to 20 carbon atoms (whenever it appears herein, a numerical range such as "1 to 20" refers to each integer in the given range; e.g., "1 to 20 carbon atoms" means that the alkyl group may consist of 1 carbon atom, 2 carbon atoms, 3 carbon atoms, *etc.,* up to and including 20 carbon atoms, although the present definition also covers the occurrence of the term "alkyl" where no numerical range is designated). The alkyl group may also be a medium size alkyl having 1 to 10 carbon atoms. The alkyl group could also be a lower alkyl having 1 to 6 carbon atoms. The alkyl group of the compounds may be designated as "C₁-C₆ alkyl" or similar designations. By way of example only, "C₁-C₄ alkyl" indicates that there are one to four carbon atoms in the alkyl chain, i.e., the alkyl chain is selected from methyl, ethyl, propyl, iso-propyl, n-butyl, iso-butyl, sec-butyl, and t-butyl. By way of example only, "C₁-C₆ alkyl" indicates that there are one to six carbon atoms in the alkyl chain. Typical alkyl groups include, but are in no way limited to, methyl, ethyl, propyl, isopropyl, butyl, isobutyl, tertiary butyl, pentyl, hexyl, and the like. The alkyl group may be substituted or unsubstituted.

As used herein, "alkenyl" refers to an alkyl group that contains in the straight or branched hydrocarbon chain one or more double bonds. An alkenyl group may be unsubstituted or substituted.

As used herein, "alkynyl" refers to an alkyl group that contains in the straight or branched hydrocarbon chain one or more triple bonds. An alkynyl group may be unsubstituted or substituted.

As used herein, "cycloalkyl" refers to a completely saturated (no double or triple bonds) mono- or multi- cyclic hydrocarbon ring system. When composed of two or more rings, the rings may be joined together in a fused fashion. Cycloalkyl groups can contain 3 to 10 atoms in the ring(s) or 3 to 8 atoms in the ring(s). A cycloalkyl group may be unsubstituted or substituted. Typical cycloalkyl groups include, but are in no way limited to, cyclopropyl, cyclobutyl, cyclopentyl, cyclohexyl, and the like.

As used herein, "cycloalkenyl" refers to a mono- or multi- cyclic hydrocarbon ring system that contains one or more double bonds in at least one ring; although, if there is more than one, the double bonds cannot form a fully delocalized pi-electron system throughout all the rings (otherwise the group would be "aryl," as defined herein). When composed of two or more rings, the rings may be connected together in a fused fashion. A cycloalkenyl group may be unsubstituted or substituted.

As used herein, "cycloalkynyl" refers to a mono- or multi- cyclic hydrocarbon ring system that contains one or more triple bonds in at least one ring. If there is more than one triple bond, the triple bonds cannot form a fully delocalized pi-electron system throughout all the rings. When composed of two or more rings, the rings may be joined together in a fused fashion. A cycloalkynyl group may be unsubstituted or substituted.

As used herein, "aryl" refers to a carbocyclic (all carbon) monocyclic or multicyclic aromatic ring system (including fused ring systems where two carbocyclic rings share a chemical bond) that has a fully delocalized pi-electron system throughout all the rings. The number of carbon atoms in an aryl group can vary. For example, the aryl group can be a C₆-C₁₄ aryl group, a C₆-C₁₀ aryl group, or a C₆ aryl group. Examples of aryl groups include, but are not limited to, benzene, naphthalene and azulene. An aryl group may be substituted or unsubstituted.

As used herein, "heteroaryl" refers to a monocyclic or multicyclic aromatic ring system (a ring system with fully delocalized pi-electron system) that contain(s) one or more heteroatoms, that is, an element other than carbon, including but not limited to, nitrogen, oxygen and sulfur. The number of atoms in the ring(s) of a heteroaryl group can vary. For example, the heteroaryl group can contain 4 to 14 atoms in the ring(s), 5 to 10 atoms in the ring(s) or 5 to 6 atoms in the ring(s). Furthermore, the term "heteroaryl" includes fused ring systems where two rings, such as at least one aryl ring and at least one heteroaryl ring, or at least two heteroaryl rings, share at least one chemical bond. Examples of heteroaryl rings include, but are not limited to, furan, furazan, thiophene, benzothiophene, phthalazine, pyrrole, oxazole, benzoxazole, 1,2,3-oxadiazole, 1,2,4-oxadiazole, thiazole, 1,2,3-thiadiazole, 1,2,4-thiadiazole, benzothiazole, imidazole, benzimidazole, indole, indazole, pyrazole, benzopyrazole, isoxazole, benzoisoxazole, isothiazole, triazole, benzotriazole, thiadiazole, tetrazole, pyridine, pyridazine, pyrimidine, pyrazine, purine, pteridine, quinoline, isoquinoline, quinazoline, quinoxaline, cinnoline, and triazine. A heteroaryl group may be substituted or unsubstituted.

As used herein, "heteroalicyclic" or "heteroalicyclyl" refers to three-, four-, five-, six-, seven-, eight-, nine-, ten-, up to 18-membered monocyclic, bicyclic, and tricyclic ring system wherein carbon atoms together with from 1 to 5 heteroatoms constitute said ring system. A heterocycle may optionally contain one or more unsaturated bonds situated in such a way, however, that a fully delocalized pi-electron system does not occur throughout all the rings. The heteroatoms are independently selected from oxygen, sulfur, and nitrogen. A heterocycle may further contain one or more carbonyl or thiocarbonyl functionalities, so as to make the definition include oxo-systems and thio-systems such as lactams, lactones, cyclic imides, cyclic thioimides, cyclic carbamates, and the like. When composed of two or more rings, the rings may be joined together in a fused fashion. Additionally, any nitrogens in a heteroalicyclic may be quaternized. Heteroalicyclyl or heteroalicyclic groups may be unsubstituted or substituted. Examples of such "heteroalicyclic" or "heteroalicyclyl" groups include but are not limited to, 1,3-dioxin, 1,3-dioxane, 1,4-dioxane, 1,2-dioxolane, 1,3-dioxolane, 1,4-dioxolane, 1,3-oxathiane, 1,4-oxathiin, 1,3-oxathiolane, 1,3-dithiole, 1,3-dithiolane, 1,4-oxathiane, tetrahydro-1,4-thiazine, 2H-1,2-oxazine, maleimide, succinimide, barbituric acid, thiobarbituric acid, dioxopiperazine, hydantoin, dihydrouracil, trioxane, hexahydro-1,3,5-triazine, imidazoline, imidazolidine, isoxazoline, isoxazolidine, oxazoline, oxazolidine, oxazolidinone, thiazoline, thiazolidine, morpholine, oxirane, piperidine *N*-Oxide, piperidine, piperazine, pyrrolidine, pyrrolidone, pyrrolidione, 4-piperidone, pyrazoline, pyrazolidine, 2-oxopyrrolidine, tetrahydropyran, 4H-pyran, tetrahydrothiopyran, thiamorpholine, thiamorpholine sulfoxide, thiamorpholine sulfone, and their benzo-fused analogs (e.g., benzimidazolidinone, tetrahydroquinoline, 3,4-methylenedioxyphenyl).

An "aralkyl" is an aryl group connected, as a substituent, via a lower alkylene group. The lower alkylene and aryl group of an aralkyl may be substituted or unsubstituted. Examples include but are not limited to benzyl, substituted benzyl, 2-phenylalkyl, 3-phenylalkyl, and naphtylalkyl.

A "heteroaralkyl" is heteroaryl group connected, as a substituent, via a lower alkylene group. The lower alkylene and heteroaryl group of heteroaralkyl may be substituted or unsubstituted. Examples include but are not limited to 2-thienylalkyl, 3-thienylalkyl, furylalkyl, thienylalkyl, pyrrolylalkyl, pyridylalkyl, isoxazolylalkyl, and imidazolylalkyl, and their substituted as well as benzo-fused analogs.

A "(heteroalicyclyl)alkyl" is a heterocyclic or a heteroalicyclylic group connected, as a substituent, via a lower alkylene group. The lower alkylene and heterocyclic or a heterocyclyl of a (heteroalicyclyl)alkyl may be substituted or unsubstituted. Examples include but are not limited tetrahydro-2H-pyran-4-yl)methyl, (piperidin-4-yl)ethyl, (piperidin-4-yl)propyl, (tetrahydro-2H-thiopyran-4-yl)methyl, and (1,3-thiazinan-4-yl)methyl.

"Lower alkylene groups" are straight-chained tethering groups, forming bonds to connect molecular fragments via their terminal carbon atoms. Examples include but are not limited to methylene (-CH₂-), ethylene (-CH₂CH₂-), propylene (-CH₂CH₂CH₂-), and butylene (-CH₂CH₂CH₂CH₂-). A lower alkylene group may be substituted or unsubstituted.

As used herein, "alkoxy" refers to the formula -OR wherein R is an alkyl, alkenyl, alkynyl, cycloalkyl, cycloalkenyl, cycloalkynyl, aryl, heteroaryl, heteroalicyclyl, aralkyl, or (heteroalicyclyl)alkyl is defined as above. Examples of include methoxy, ethoxy, n-propoxy, 1-methylethoxy (isopropoxy), n-butoxy, iso-butoxy, sec-butoxy, tert-butoxy, phenoxy and the like. An alkoxy may be substituted or unsubstituted.

As used herein, "acyl" refers to a hydrogen, alkyl, alkenyl, alkynyl, or aryl connected, as substituents, via a carbonyl group. Examples include formyl, acetyl, propanoyl, benzoyl, and acryl. An acyl may be substituted or unsubstituted.

As used herein, "hydroxyalkyl" refers to an alkyl group in which one or more of the hydrogen atoms are replaced by hydroxy group. Examples of hydroxyalkyl groups include but are not limited to, 2-hydroxyethyl, 3-hydroxypropyl, 2-hydroxypropyl, and 2,2-dihydroxyethyl. A hydroxyalkyl may be substituted or unsubstituted.

As used herein, "haloalkyl" refers to an alkyl group in which one or more of the hydrogen atoms are replaced by halogen (e.g., mono-haloalkyl, di-haloalkyl and tri-haloalkyl). Such groups include but are not limited to, chloromethyl, fluoromethyl, difluoromethyl, trifluoromethyl and 1-chloro-2-fluoromethyl, 2-fluoroisobutyl. A haloalkyl may be substituted or unsubstituted.

As used herein, "haloalkoxy" refers to an alkoxy group in which one or more of the hydrogen atoms are replaced by halogen (e.g., mono-haloalkoxy, di-haloalkoxy and tri- haloalkoxy). Such groups include but are not limited to, chloromethoxy, fluoromethoxy, difluoromethoxy, trifluoromethoxy and 1-chloro-2-fluoromethoxy, 2-fluoroisobutoxy. A haloalkoxy may be substituted or unsubstituted.

A "sulfenyl" group refers to an "-SR" group in which R can be hydrogen, alkyl, alkenyl, alkynyl, cycloalkyl, cycloalkenyl, cycloalkynyl, aryl, heteroaryl, heteroalicyclyl, aralkyl, or (heteroalicyclyl)alkyl. A sulfenyl may be substituted or unsubstituted.

A "sulfinyl" group refers to an "-S(=O)-R" group in which R can be the same as defined with respect to sulfenyl. A sulfinyl may be substituted or unsubstituted.

A "sulfonyl" group refers to an "SO₂R" group in which R can be the same as defined with respect to sulfenyl. A sulfonyl may be substituted or unsubstituted.

An "O-carboxy" group refers to a "RC(=O)O-" group in which R can be hydrogen, alkyl, alkenyl, alkynyl, cycloalkyl, cycloalkenyl, cycloalkynyl, aryl, heteroaryl, heteroalicyclyl, aralkyl, or (heteroalicyclyl)alkyl, as defined herein. An O-carboxy may be substituted or unsubstituted.

The terms "ester" and "C-carboxy" refer to a "-C(=O)OR" group in which R can be the same as defined with respect to O-carboxy. An ester and C-carboxy may be substituted or unsubstituted.

A "thiocarbonyl" group refers to a "-C(=S)R" group in which R can be the same as defined with respect to O-carboxy. A thiocarbonyl may be substituted or unsubstituted.

A "trihalomethanesulfonyl" group refers to an "X₃CSO₂-" group wherein X is a halogen.

A "trihalomethanesulfonamido" group refers to an "X₃CS(O)₂RN-" group wherein X is a halogen and R defined with respect to O-carboxy.

The term "amino" as used herein refers to a -NH₂ group.

As used herein, the term "hydroxy" refers to a -OH group.

A "cyano" group refers to a "-CN" group.

The term "azido" as used herein refers to a -N₃ group.

An "isocyanato" group refers to a "-NCO" group.

A "thiocyanato" group refers to a "-CNS" group.

An "isothiocyanato" group refers to an " -NCS" group.

A "mercapto" group refers to an "-SH" group.

A "carbonyl" group refers to a C=O group.

An "S-sulfonamido" group refers to a "-SO₂NR_{A}R_{B}" group in which R_{A} and R_{B} can be the same as R defined with respect to O-carboxy. An S-sulfonamido may be substituted or unsubstituted.

An "N-sulfonamido" group refers to a "R_{B}SO₂N(R_{A})-" group in which R_{A} and R_{B} can be the same as R defined with respect to O-carboxy. A N-sulfonamido may be substituted or unsubstituted.

An "O-carbamyl" group refers to a "-OC(=O)NR_{A}R_{B}" group in which R_{A} and R_{B} can be the same as R defined with respect to O-carboxy. An O-carbamyl may be substituted or unsubstituted.

An "N-carbamyl" group refers to an "R_{B}OC(=O)NR_{A} -" group in which R_{A} and R_{B} can be the same as R defined with respect to O-carboxy. An N-carbamyl may be substituted or unsubstituted.

An "O-thiocarbamyl" group refers to a "-OC(=S)-NR_{A}R_{B}" group in which R_{A} and R_{B} can be the same as R defined with respect to O-carboxy. An O-thiocarbamyl may be substituted or unsubstituted.

An "N-thiocarbamyl" group refers to an "R_{B}OC(=S)NR_{A}-" group in which R_{A} and R_{B} can be the same as R defined with respect to O-carboxy. An N-thiocarbamyl may be substituted or unsubstituted.

A "C-amido" group refers to a "-C(=O)NR_{A}R_{B}" group in which R_{A} and R_{B} can be the same as R defined with respect to O-carboxy. A C-amido may be substituted or unsubstituted.

An "N-amido" group refers to a "R_{B}C(=O)NR_{A}-" group in which R_{A} and R_{B} can be the same as R defined with respect to O-carboxy. An N-amido may be substituted or unsubstituted.

As used herein, "organylcarbonyl" refers to a group of the formula - C(=O)R' wherein R' can be alkyl, alkenyl, alkynyl, cycloalkyl, cycloalkenyl, cycloalkynyl, aryl, heteroaryl, heteroalicyclyl, aralkyl, or (heteroalicyclyl)alkyl. An organylcarbonyl can be substituted or unsubstituted.

The term "alkoxycarbonyl" as used herein refers to a group of the formula -C(=O)OR' wherein R' can be the same as defined with respect to organylcarbonyl. An alkoxycarbonyl can be substituted or unsubstituted.

As used herein, "organylaminocarbonyl" refers to a group of the formula C(=O)NR'R" wherein R' and R" can each be independently selected from the same substituents as defined with respect to organylcarbonyl. An organylaminocarbonyl can be substituted or unsubstituted.

As used herein, the term "levulinoyl" refers to a - C(=O)CH₂CH₂C(=O)CH₃ group.

The term "halogen atom," as used herein, means any one of the radio-stable atoms of column 7 of the Periodic Table of the Elements, i.e., fluorine, chlorine, bromine, or iodine, with fluorine and chlorine being preferred.

Where the numbers of substituents is not specified (e.g. haloalkyl), there may be one or more substituents present. For example "haloalkyl" may include one or more of the same or different halogens. As another example, "C₁-C₃ alkoxyphenyl" may include one or more of the same or different alkoxy groups containing one, two or three atoms.

As used herein, the term "nucleoside" refers to a compound composed of any pentose or modified pentose moiety attached to a specific portion of a heterocyclic base, tautomer, or derivative thereof such as the 9-position of a purine, 1-position of a pyrimidine, or an equivalent position of a heterocyclic base derivative. Examples include, but are not limited to, a ribonucleoside comprising a ribose moiety and a deoxyribonucleoside comprising a deoxyribose moiety. In some instances, the nucleoside can be a nucleoside drug analog.

As used herein, the term "nucleoside drug analog" refers to a compound composed of a nucleoside that has therapeutic activity, such as antiviral, antineoplastic, anti-parasitic and/or antibacterial activity.

As used herein, the term "nucleotide" refers to a nucleoside having a phosphate ester substituted on the 5'-position or an equivalent position of a nucleoside derivative.

As used herein, the term "heterocyclic base" refers to a purine, a pyrimidine and derivatives thereof. The term "purine" refers to a substituted purine, its tautomers and analogs thereof. Similarly, the term "pyrimidine" refers to a substituted pyrimidine, its tautomers and analogs thereof. Examples of purines include, but are not limited to, purine, adenine, guanine, hypoxanthine, xanthine, theobromine, caffeine, uric acid and isoguanine. Examples of pyrimidines include, but are not limited to, cytosine, thymine, uracil, and derivatives thereof. An example of an analog of a purine is 1,2,4-triazole-3-carboxamide.

Other non-limiting examples of heterocyclic bases include diaminopurine, 8-oxo-N⁶-methyladenine, 7-deazaxanthine, 7-deazaguanine, N⁴,N⁴-ethanocytosin, N⁶,N⁶-ethano-2,6-diaminopurine, 5-methylcytosine, 5-fluorouracil, 5-bromouracil, pseudoisocytosine, isocytosine, isoguanine, and other heterocyclic bases described in U.S. Patent Nos. 5,432,272 and 7,125,855, which are incorporated herein by reference for the limited purpose of disclosing additional heterocyclic bases.

The term "-O-linked amino acid" refers to an amino acid that is attached to the indicated moiety via its main-chain carboxyl function group. When the amino acid is attached, the hydrogen that is part of the -OH portion of the carboxyl function group is not present and the amino acid is attached via the remaining oxygen. An -O-linked amino acid can be protected at any nitrogen group that is present on the amino acid. For example, an -O-linked amino acid can contain an amide or a carbamate group. Suitable amino acid protecting groups include, but are not limited to, carbobenzyloxy (Cbz), p-methoxybenzyl carbonyl (Moz or MeOZ), tert-butyloxycarbonyl (BOC), 9-fluorenylmethyloxycarbonyl (FMOC), benzyl (Bn), p-methoxybenzyl (PMB), 3,4-dimethoxybenzyl (DMPM), and tosyl (Ts) groups. The term "-N-linked amino acid" refers to an amino acid that is attached to the indicated moiety via its main-chain amino or mono-substituted amino group. When the amino acid is attached in an -N-linked amino acid, one of the hydrogens that is part of the main-chain amino or mono-substituted amino group is not present and the amino acid is attached via the nitrogen. An -N-linked amino acid can be protected at any hydroxyl or carboxyl group that is present on the amino acid. For example, an -N-linked amino acid can contain an ester or an ether group. Suitable amino acid protecting groups include, but are not limited to, methyl esters, ethyl esters, propyl esters, benzyl esters, tert-butyl esters, silyl esters, orthoesters, and oxazoline. As used herein, the term "amino acid" refers to any amino acid (both standard and non-standard amino acids), including, but limited to, α₋amino acids β-amino, acids, γ-amino acids and δ-amino acids. Examples of suitable amino acids, include, but are not limited to, alanine, asparagine, aspartate, cysteine, glutamate, glutamine, glycine, proline, serine, tyrosine, arginine, histidine, isoleucine, leucine, lysine, methionine, phenylalanine, threonine, tryptophan and valine.

The terms "derivative," "variant," or other similar terms refer to a compound that is an analog of the other compound.

The terms "protecting group" and "protecting groups" as used herein refer to any atom or group of atoms that is added to a molecule in order to prevent existing groups in the molecule from undergoing unwanted chemical reactions. Examples of protecting group moieties are described in T. W. Greene and P. G. M. Wuts, Protective Groups in Organic Synthesis, 3. Ed. John Wiley & Sons (1999), and in J.F.W. McOmie, Protective Groups in Organic Chemistry Plenum Press (1973), both of which are hereby incorporated by reference for the limited purpose of disclosing suitable protecting groups. The protecting group moiety may be chosen in such a way, that they are stable to certain reaction conditions and readily removed at a convenient stage using methodology known from the art. A non-limiting list of protecting groups include benzyl; substituted benzyl; alkylcarbonyls (e.g., t-butoxycarbonyl (BOC)); arylalkylcarbonyls (e.g., benzyloxycarbonyl, benzoyl); substituted methyl ether (e.g. methoxymethyl ether); substituted ethyl ether; a substituted benzyl ether; tetrahydropyranyl ether; silyl ethers (e.g., trimethylsilyl, triethylsilyl, triisopropylsilyl, t-butyldimethylsilyl, or t-butyldiphenylsilyl); esters (e.g. benzoate ester); carbonates (e.g. methoxymethylcarbonate); sulfonates (e.g. tosylate, mesylate); acyclic ketal (e.g. dimethyl acetal); cyclic ketals (e.g., 1,3-dioxane or 1,3-dioxolanes); acyclic acetal; cyclic acetal; acyclic hemiacetal; cyclic hemiacetal; and cyclic dithioketals (e.g., 1,3-dithiane or 1,3-dithiolane).

"Leaving group" as used herein refers to any atom or moiety that is capable of being displaced by another atom or moiety in a chemical reaction. More specifically, in some embodiments, "leaving group" refers to the atom or moiety that is displaced in a nucleophilic substitution reaction. In some embodiments, "leaving groups" are any atoms or moieties that are conjugate bases of strong acids. Examples of suitable leaving groups include, but are not limited to, tosylates and halogens. Non-limiting characteristics and examples of leaving groups can be found, for example in Organic Chemistry, 2d ed., Francis Carey (1992), pages 328-331; Introduction to Organic Chemistry, 2d ed., Andrew Streitwieser and Clayton Heathcock (1981), pages 169-171; and Organic Chemistry, 5th ed., John McMurry (2000), pages 398 and 408; all of which are incorporated herein by reference for the limited purpose of disclosing characteristics and examples of leaving groups.

As used herein, the abbreviations for any protective groups, amino acids and other compounds, are, unless indicated otherwise, in accord with their common usage, recognized abbreviations, or the IUPAC-IUB Commission on Biochemical Nomenclature (See, Biochem. 1972 11:942-944).

A "prodrug" refers to an agent that is converted into the parent drug *in vivo.* Prodrugs are often useful because, in some situations, they may be easier to administer than the parent drug. They may, for instance, be bioavailable by oral administration whereas the parent is not. The prodrug may also have improved solubility in pharmaceutical compositions over the parent drug. Examples of prodrugs include compounds that have one or more biologically labile groups attached to the parent drug (e.g., a compound of Formula I and/or a compound of Formula II). For example, one or more biologically labile groups can be attached to a functional group of the parent drug (for example, by attaching one or more biologically labile groups to a phosphate). When more than one biologically labile groups is attached, the biologically labile groups can be the same or different. The biologically labile group(s) can be linked (for example, through a covalent bond), to an oxygen or a heteroatom, such as a phosphorus of a monophosphate, diphosphate, triphosphate, and/or a stabilized phosphate analog containing carbon, nitrogen or sulfur (referred to hereinafter in the present paragraph as "phosphate"). In instances where the prodrug is form by attaching one or more biologically labile groups to the phosphate, removal of the biologically labile group in the host produces a phosphate. The removal of the biologically labile group(s) that forms the prodrug can be accomplished by a variety of methods, including, but not limited to, oxidation, reduction, amination, deamination, hydroxylation, dehydroxylation, hydrolysis, dehydrolysis, alkylation, dealkylation, acylation, deacylation, phosphorylation, dephosphorylation, hydration and/or dehydration. An example, without limitation, of a prodrug would be a compound which is administered as an ester (the "prodrug") to facilitate transmittal across a cell membrane where water solubility is detrimental to mobility but which then is metabolically hydrolyzed to the carboxylic acid, the active entity, once inside the cell where water-solubility is beneficial. A further example of a prodrug might comprise a short peptide (polyaminoacid) bonded to an acid group where the peptide is metabolized or cleaved to reveal the active moiety. Additional examples of prodrug moieties include the following: R^{*}, R^{*}C(=O)OCH₂-, R^{*}C(=O)SCH₂CH₂-, R^{*}C(=O)SCHR'NH-, phenyl-O-, N-linked amino acids, O-linked amino acids, peptides, carbohydrates, and lipids, wherein each R^{*} can be independently selected from an alkyl, an alkenyl, an alkynyl, an aryl, an aralkyl, acyl, sulfonate ester, a lipid, an -N-linked amino acid, an -O-linked amino acid, a peptide and a cholesterol. The prodrug can be a carbonate. The carbonate can be a cyclic carbonate. The cyclic carbonate can contain a carbonyl group between two hydroxyl groups that results in the formation of a five or six memebered ring. Conventional procedures for the selection and preparation of suitable prodrug derivatives are described, for example, in Design of Prodrugs, (ed. H. Bundgaard, Elsevier, 1985), which is hereby incorporated herein by reference for the limited purpose of describing procedures and preparation of suitable prodrug derivatives.

The term "pro-drug ester" refers to derivatives of the compounds disclosed herein formed by the addition of any of several ester-forming groups that are hydrolyzed under physiological conditions. Examples of pro-drug ester groups include pivaloyloxymethyl, acetoxymethyl, phthalidyl, indanyl and methoxymethyl, as well as other such groups known in the art, including a (5-R-2-oxo-1,3-dioxolen-4-yl)methyl group. Other examples of pro-drug ester groups can be found in, for example, T. Higuchi and V. Stella, in "Pro-drugs as Novel Delivery Systems", Vol. 14, A.C.S. Symposium Series, American Chemical Society (1975); and "Bioreversible Carriers in Drug Design: Theory and Application", edited by E. B. Roche, Pergamon Press: New York, 14-21 (1987) (providing examples of esters useful as prodrugs for compounds containing carboxyl groups). Each of the above-mentioned references is herein incorporated by reference for the limited purpose of disclosing ester-forming groups that can form prodrug esters.

The term "pharmaceutically acceptable salt" refers to a salt of a compound that does not cause significant irritation to an organism to which it is administered and does not abrogate the biological activity and properties of the compound. In some embodiments, the salt is an acid addition salt of the compound. Pharmaceutical salts can be obtained by reacting a compound with inorganic acids such as hydrohalic acid (e.g., hydrochloric acid or hydrobromic acid), sulfuric acid, nitric acid, phosphoric acid and the like. Pharmaceutical salts can also be obtained by reacting a compound with an organic acid such as aliphatic or aromatic carboxylic or sulfonic acids, for example acetic, succinic, lactic, malic, tartaric, citric, ascorbic, nicotinic, methanesulfonic, ethanesulfonic, p-toluensulfonic, salicylic or naphthalenesulfonic acid. Pharmaceutical salts can also be obtained by reacting a compound with a base to form a salt such as an ammonium salt, an alkali metal salt, such as a sodium or a potassium salt, an alkaline earth metal salt, such as a calcium or a magnesium salt, a salt of organic bases such as dicyclohexylamine, N-methyl-D-glucamine, tris(hydroxymethyl)methylamine, C₁-C₇ alkylamine, cyclohexylamine, triethanolamine, ethylenediamine, and salts with amino acids such as arginine, lysine, and the like.

It is understood that, in any compound described herein having one or more chiral centers, if an absolute stereochemistry is not expressly indicated, then each center may independently be of R-configuration or S-configuration or a mixture thereof. Thus, the compounds provided herein may be enatiomerically pure or be stereoisomeric mixtures. In addition it is understood that, in any compound described herein having one or more double bond(s) generating geometrical isomers that can be defined as E or Z, each double bond may independently be E or Z a mixture thereof. Likewise, all tautomeric forms are also intended to be included.

An embodiment disclosed herein relates to a compound of Formula (I), or a pharmaceutically acceptable salt or a prodrug thereof:

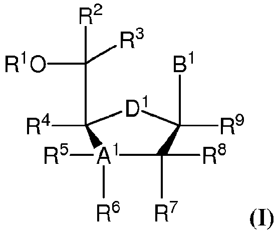

wherein: A¹ can be selected from C (carbon), O (oxygen) and S (sulfur); B¹ can be an optionally substituted heterocyclic base or a derivative thereof; D¹ can be selected from C=CH₂, CH₂, O (oxygen), S (sulfur), CHF, and CF₂; R¹ can be hydrogen, an optionally substituted alkyl, an optionally substituted cycloalkyl, an optionally substituted aralkyl, dialkylaminoalkylene, alkyl-C(=O)-, aryl-C(=O)-, alkoxyalkyl-C(=O)-, aryloxyalkyl-C(=O)-, alkylsulfonyl, arylsulfonyl, aralkylsulfonyl, 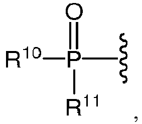 an -O-linked amino acid, diphosphate, triphosphate or derivatives thereof;
R² and R³ can be each independently selected from hydrogen, an optionally substituted C₁₋₆ alkyl, an optionally substituted C₂₋₆ alkenyl, an optionally substituted C₂₋₆ alkynyl and an optionally substituted C₁₋₆ haloalkyl, provided that at least one of R² and R³ is not hydrogen; or R² and R³ are taken together to form a group selected from among C₃₋₆ cycloalkyl, C₃₋₆ cycloalkenyl, C₃₋₆ aryl, and a C₃₋₆ heteroaryl; R⁴, R⁷ and R⁹ can be independently selected from hydrogen, halogen, -NH₂, -NHR^{a1}, NR^{a1}R^{b1}, ₋OR^{a1}, ₋SR^{a1}, - CN, -NC, -N₃, -NO₂, -N(R^{c1})-NR^{a1}R^{b1}, -N(R^{c1})-OR^{a1}, -S-SR^{a1}, -C(=O)R^{a1}, -C(=O)OR^{a1}, - C(=O)NR^{a1}R^{b1}, -O-(C=O)R^{a1}, -O-C(=O)OR^{a1}, -O-C(=O)NR^{a1}R^{b1}, -N(R^{c1})-C(=O)NR^{a1}R^{b1}, -S(=O)R^{a1}, S(=O)₂R^{a1}, -O-S(=O)₂NR^{a1}R^{b1}, -N(R^{c1})-S(=O)₂NR^{a1}R^{b1}, an optionally substituted C₁₋₆ alkyl, an optionally substituted C₂₋₆ alkenyl, an optionally substituted C₂₋₆ alkynyl, an optionally substituted aralkyl and an -O-linked amino acid; R⁵ and R⁶ can be independently absent or selected from hydrogen, halogen, -NH₂, -NHR^{a1}, NR^{a1}R^{b1}, ₋OR^{a1}, -SR^{a1}, -CN, -NC, -N₃, -NO₂, -N(R^{c1})-NR^{a1}R^{b1}, -N(R^{c1})-OR^{a1}, -S-SR^{a1}, - C(-O)R^{a1}, -C(=O)OR^{a1}, -C(=O)NR^{a1}R^{b1}, -O-C(=O)OR^{a1}, -O-C(=O)NR^{a1}R^{b1}, -N(R^{c1})-C(=O)NR^{a1}R^{b1}, -S(=O)R^{a1}, S(=O)₂R^{a1}, -O-S(=O)₂NR^{a1}R^{b1}, -N(R^{c1})-S(=O)₂NR^{a1}R^{b1}, an optionally substituted C₁₋₆ alkyl, an optionally substituted C₂₋₆ alkenyl, an optionally substituted C₂₋₆ alkynyl and an -O-linked amino acid; or R⁶ and R⁷ taken together form -O-C(=O)-O-; R⁸ can be halogen, -OR^{a1}, an optionally substituted C₁₋₆ alkyl, an optionally substituted C₂₋₆ alkenyl, an optionally substituted C₂₋₆ alkynyl and an optionally substituted C₁₋₆ haloalkyl; R^{a1}, R^{b1} and R^{c1} can be each independently selected from hydrogen, an optionally substituted alkyl, an optionally substituted alkenyl, an optionally substituted alkynyl, an optionally substituted aryl, an optionally substituted heteroaryl, an optionally substituted aralkyl and an optionally substituted heteroaryl(C₁₋₆ alkyl); R¹⁰ can be selected from O⁻, -OH, an optionally substituted aryloxy or aryl-O-, 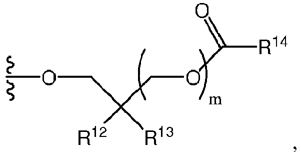 alkyl-C(=O)-O-CH₂-O-, alkyl-C(=O)-S-CH₂CH₂-O- and an -N-linked amino acid; R¹¹ can be selected from O⁻, -OH, an optionally substituted aryloxy or aryl-O-, 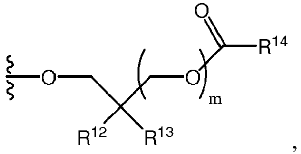 alkyl-C(=O)-O-CH₂-O-, alkyl-C(=O)-S-CH₂CH₂-O- and an -N-linked amino acid; each R¹² and each R¹³ can be independently -C=N or an optionally substituted substituent selected from C₁₋₈ organylcarbonyl, C₁₋₈ alkoxycarbonyl and C₁₋₈ organylaminocarbonyl; each R¹⁴ can be hydrogen or an optionally substituted C₁₋₆-alkyl;
each m can be independently 1 or 2, and if both R¹⁰ and R¹¹ are 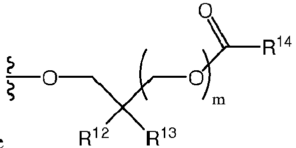 each R¹², each R¹³, each R¹⁴ and each m can be the same or different.

In an embodiment, m can be 1. In another embodiment, m can be 2. In some embodiments, A¹ can be carbon. In some embodiments, D¹ can be oxygen. In an embodiment, A¹ can be carbon and D¹ can be oxygen. In other embodiments, A¹ can be carbon, D¹ can be oxygen and m can be 1. In an embodiment, A¹ can be carbon, D¹ can be oxygen and m can be 2.

In some embodiments, the optionally substituted C₁₋₆ alkyl can be selected from methyl, ethyl, n-propyl, isopropyl, n-butyl, iso-butyl, tert-butyl, pentyl, and hexyl. In an embodiment, the optionally substituted C₁₋₆ alkyl can be methyl. In an embodiment, R² can be methyl and R³ can be hydrogen. In some embodiments, R² and R⁸ can both be methyl. In some embodiments, the optionally substituted C₁₋₆ alkoxy can be selected from methoxy, ethoxy, n-propoxy, isopropoxy, n-butoxy, iso-butoxy and tert-butoxy. In an embodiment, the optionally substituted C₁₋₆ haloalkyl can be trifluoromethyl. In some embodiments, R² can be trifluoromethyl and R³ can be hydrogen. In some embodiments, R² can be trifluoromethyl and R⁸ can be methyl.

In some embodiments, a compound of Formula (I) can be a nucleoside or nucleoside derivative. In an embodiment, R¹ can be hydrogen. In some embodiments, a compound of Formula (I) can be a nucleotide or nucleotide derivative. In an embodiment, R¹ can be monophosphate. In another embodiment, R¹ can be a diphosphate. In still another embodiment, R¹ can be a triphosphate. In yet still another embodiment, R¹ can be 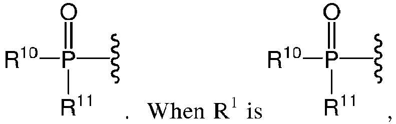 R¹⁰ and R¹¹ can both be O⁻. In some embodiments, to facilitate entry into a cell, the charge on the phosphate of the nucleotide or nucleotide derivative can be neutralized with an appropriate moiety. In some embodiments, the moiety can be 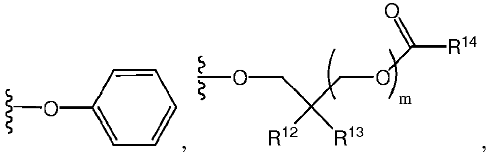 -O-naphthol and/or an -N-linked amino acid, such as those described herein.

In some embodiments, at least one of R¹⁰ and R¹¹ can be 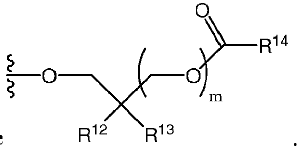 The substitutents on 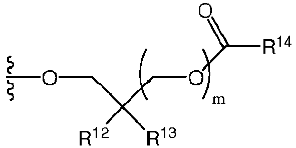 can vary. In some embodiments, R¹² can be -C=N and R¹³ can be an optionally substituted C₁₋₈ alkoxycarbonyl such as -C(=O)OCH₃. In other embodiments, R¹² can be -C≡N and R¹³ can be an optionally substituted C₁₋₈ organylaminocarbonyl, for example, - C(=O)NHCH₂CH₃ and -C(=O)NHCH₂CH₂phenyl. In still other embodiments, both R¹² and R¹³ can be an optionally substituted C₁₋₈ organylcarbonyl. In an embodiment, both R¹² and R¹³ can be -C(=O)CH₃. In yet still other embodiments, both R¹² and R¹³ can be an optionally substituted C₁₋₈ alkoxycarbonyl. In an embodiment, both R¹² and R¹³ can be -C(=O)OCH₃ or -C(=O)OCH₂CH₃. In an embodiment, both R¹² and R¹³ can be an optionally substituted C₁₋₈ alkoxycarbonyl, for example -C(=O)OCH₂CH₃, and m can be 2. In some embodiments, including those in this paragraph, R¹⁴ can be an optionally substituted C₁₋₆-alkyl. In an embodiment, including those in this paragraph, R¹⁴ can be methyl or tert-butyl.

Examples of suitable 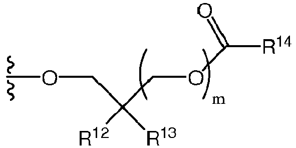 groups, include but are not limited to, the following:

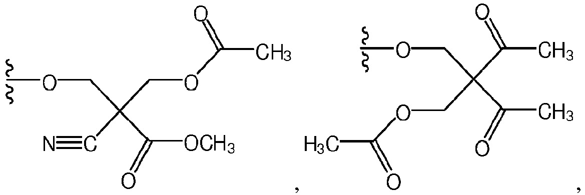

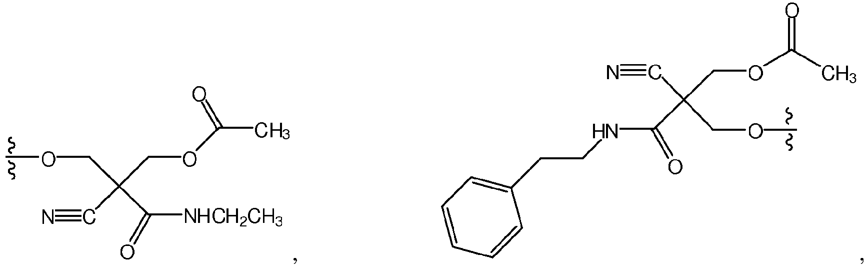

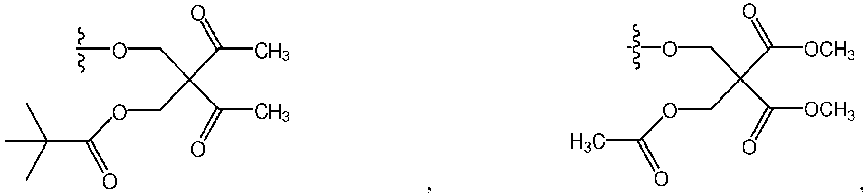

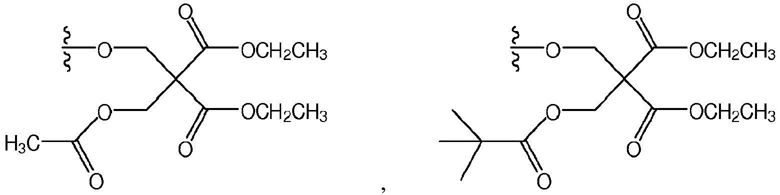

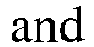

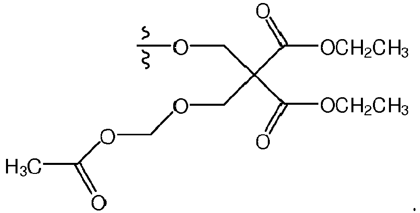

In an embodiment, R¹⁰ and/or R¹¹ can be 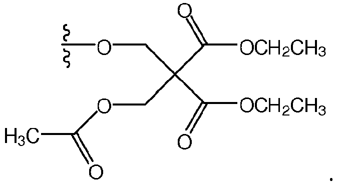 In another embodiment, R¹⁰ and/or R¹¹ can be 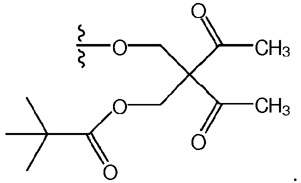 In still another embodiment, R¹⁰ and/or R¹¹ can be 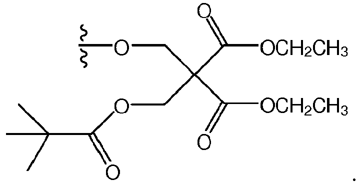 In yet still another embodiment, R¹⁰ and/or R¹¹ can be 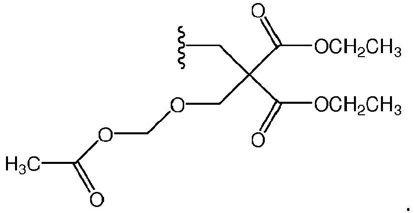 In an embodiment, R¹⁰ and/or R¹¹ can be

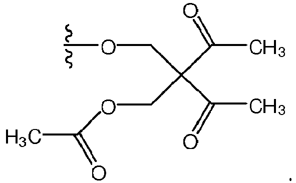

In some embodiments, both R¹⁰ and R¹¹ can be 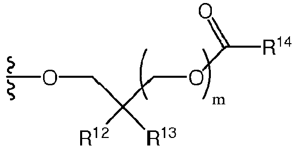 wherein each R¹², each R¹³, each R¹⁴ and each m can be the same or different. In some embodiments, when both R¹⁰ and R¹¹ are 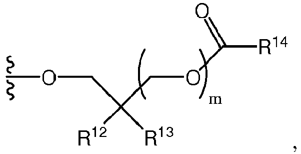 R¹⁰ and R¹¹ can be the same. In other embodiments, when both R¹⁰ and R¹¹ are 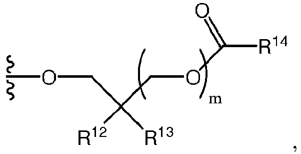 R¹⁰ and R¹¹ can be different.

In an embodiment, at least one of R¹⁰ and R¹¹ can be an -N-linked amino acid. Various amino acids can be utilized as a substituent for R¹⁰ or R¹¹. In some embodiments, R¹⁰ or R¹¹ can have the structure 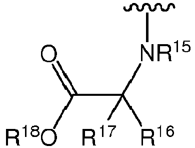 wherein: R¹⁵ can be hydrogen or an optionally substituted C₁₋₄-alkyl; R¹⁶ can be selected from hydrogen, an optionally substituted C₁₋₆-alkyl, an optionally substituted aryl, an optionally substituted aryl(C₁₋₆ alkyl) and haloalkyl; R¹⁷ can be hydrogen or an optionally substituted C₁₋₆-alkyl; and R¹⁸ can be selected from an optionally substituted C₁₋₆ alkyl, an optionally substituted C₆ aryl, an optionally substituted C₁₀ aryl, and an optionally substituted C₃₋₆ cycloalkyl. In an embodiment, R¹⁵ can be hydrogen. In some embodiments, R¹⁶ can be an optionally substituted C₁₋₆-alkyl, for example, methyl. In an embodiment, R¹⁷ can be hydrogen or an optionally substituted C₁₋₆-alkyl such as methyl. In some embodiment, R¹⁸ can be an optionally substituted C₁₋₆-alkyl. In an embodiment, R¹⁸ can be methyl. One example of a suitable 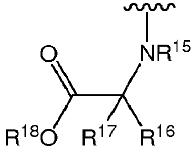 group includes, but are not limited to, 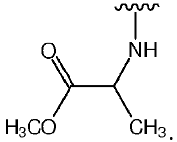 In some embodiments, the amino acid can be in the L-configuration. In other embodiments, the amino acid can be in the D-configuration. For example, 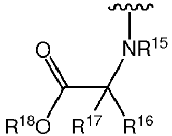 can be 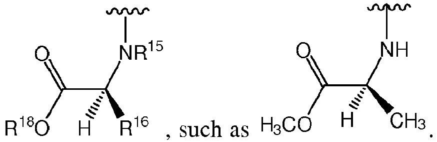 Additional suitable amino acids that can be used in embodiments disclosed herein are described in Cahard et al., Mini-Reviews in Medicinal Chemistry, 2004, 4:371-381 and McGuigan et al., J. Med. Chem., 2008, 51(18):5807-5812, which hereby incorporated by reference for the limited purpose of describing additional suitable amino acids.

In some embodiments, at least one of R¹⁰ and R¹¹ can be an -N-linked amino acid, such as those described herein, and the other of at least one of R¹⁰ and R¹¹ can be 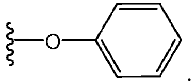 In other embodiments, at least one of R¹⁰ and R¹¹ can be an -N-linked amino acid, such as those described herein, and the other of at least one of R¹⁰ and R¹¹ can be 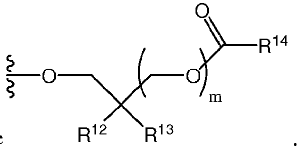 In some embodiments, at least one of R¹⁰ and R¹¹ can be 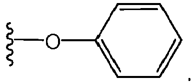 In an embodiment, R¹⁰ can be 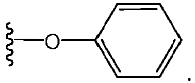 In some embodiments, at least one of R¹⁰ and R¹¹ can be an -N-linked amino acid. In an embodiment, R¹⁰ can be 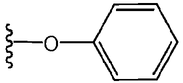 and R¹¹ can be an -N-linked amino acid.In another embodiment, R¹⁰ cannot be 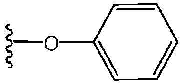 when R¹¹ is an -N-linked amino acid.

The substituent B¹ can also vary. In some embodiments, B¹ can be selected from: 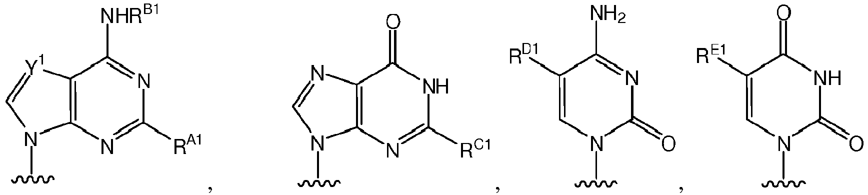 and 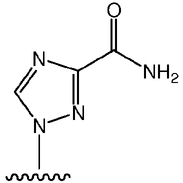 wherein: R^{A1} can be hydrogen or halogen; R^{B1} can be hydrogen, an optionally substituted C₁₋₆alkyl, or an optionally substituted C₃₋₈ cycloalkyl; R^{C1} can be hydrogen or amino; R^{D1} can be hydrogen, halogen, an optionally substituted C₁₋₆ alkyl, an optionally substituted C₂₋₆ alkenyl and an optionally substituted C₂₋₆ alkynyl; R^{E1} can be hydrogen, halogen, an optionally substituted C₁₋₆alkyl, an optionally substituted C₂₋₆ alkenyl and an optionally substituted C₂₋₆ alkynyl; and Y¹ can be N (nitrogen) or CR^{F1}, wherein R^{F1} can be selected from hydrogen, halogen, an optionally substituted C₁₋₆-alkyl, an optionally substituted C₂₋₆-alkenyl and an optionally substituted C₂-₆-alkynyl. In some embodiments, B¹ can be 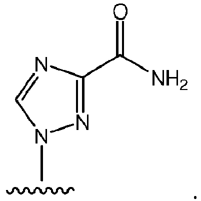 In other embodiments, B¹ can be 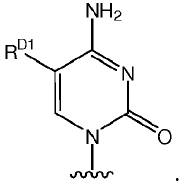 In yet other embodiments, B¹ can be 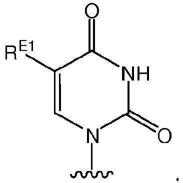 In an embodiment, R^{E} can be hydrogen. In yet still other embodiments, B¹ can be 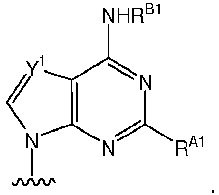 In an embodiment Y¹ can be nitrogen; R^{A1} can be hydrogen and R³¹ can be hydrogen. In another embodiment, Y¹ can be CR^{F1}, wherein R^{F1} can be selected from hydrogen, halogen, an optionally substituted C₁₋₆-alkyl, an optionally substituted C₂₋₆-alkenyl and an optionally substituted C₂₋₆-alkynyl; R^{A1} can be hydrogen and R^{B1} can be hydrogen. When B¹ is any of the aforementioned moieties shown above, in some embodiments, A¹ can be carbon. In an embodiment, B¹ can be any of the aforementioned moieties shown above, A¹ can be carbon and D¹ can be oxygen.

In some embodiments, R⁴ can be selected from hydrogen, halogen, - OR^{a1}, -CN, -N₃ and an optionally substituted C₁₋₆ alkyl. In some embodiments, R⁵ can be absent or selected from hydrogen, halogen, -OR^{a1} and an optionally substituted C₁₋₆ alkyl. In some embodiments, R⁶ can be absent or selected from hydrogen, halogen, -NH₂, - OR^{a1}, -N₃, an optionally substituted C₁₋₆ alkyl and an -O-linked amino acid. In some embodiments, R⁷ can be absent or selected from hydrogen, halogen, -OR^{a1}, -CN, -NC, an optionally substituted C₁₋₆ alkyl and an -O-linked amino acid. In an embodiment, R⁶ can be ₋OR^{a1}, wherein R^{a1} is hydrogen. In another embodiment, R⁶ can be an -O-linked amino acid. In some embodiments, R⁷ can be ₋OR^{a1}, wherein R^{a1} is hydrogen. In other embodiments, R⁷ can be a C₁₋₆ alkoxy such as methoxy. In still other embodiments, R⁷ can be an -O-linked amino acid. In some embodiments, both R⁶ and R⁷ can be hydroxy groups. In other embodiments, R⁷ can be a hydroxyl group and R⁶ can be -O-linked amino acid. A non-limiting list of suitable -O-linked amino acid include, but are not limited to the following: alanine, asparagine, aspartate, cysteine, glutamate, glutamine, glycine, proline, serine, tyrosine, arginine, histidine, isoleucine, leucine, lysine, methionine, phenylalanine, threonine, tryptophan and valine. In an embodiment, the -O-linked amino acid can be valine. In some embodiments, the -O-linked amino acid can be selected from-O-linked α-amino acid, -O-linked β-amino, acid, -O-linked γ-amino acid and -O-linked δ-amino acid. In an embodiment, the -O-linked amino acid can be in the L-configuration. In some embodiments, R⁹ can be selected from hydrogen, halogen and an optionally substituted C₁₋₆ alkyl.

In some embodiments, the compound of Formula (I) can be an antineoplastic agent. In other embodiments, the compound of Formula (I) can be an anti-viral agent. In still other embodiments, the compound of Formula (I) can be an anti-parasitic agent.

An embodiment disclosed herein relates to a compound of Formula (II), or a pharmaceutically acceptable salt or a prodrug thereof: 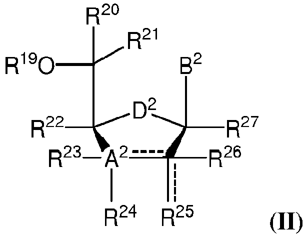 wherein: each 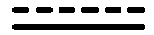 can be independently a double or single bond; A² can be selected from C (carbon), O (oxygen) and S (sulfur); B² can be an optionally substituted heterocyclic base or a derivative thereof; D² can be selected C=CH₂, CH₂, O (oxygen), S (sulfur), CHF, and CF₂; R¹⁹ can be hydrogen, an optionally substituted alkyl, an optionally substituted cycloalkyl, an optionally substituted aralkyl, dialkylaminoalkylene, alkyl-C(=O)-, aryl-C(=O)-, alkoxyalkyl-C(=O)-, aryloxyalkyl-C(=O)-, alkylsulfonyl, arylsulfonyl, aralkylsulfonyl, 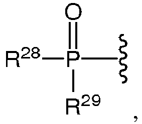 an -O-linked amino acid, diphosphate, triphosphate or derivatives thereof; R²⁰ and R²¹ can be each independently selected from hydrogen, an optionally substituted C₁₋₆ alkyl, an optionally substituted C₂₋₆ alkenyl, an optionally substituted C₂₋₆ alkynyl and an optionally substituted C₁₋₆ haloalkyl, provided that at least one of R²⁰ and R²¹ is not hydrogen; or R²⁰ and R²¹ are taken together to form a group selected from among C₃₋₆ cycloalkyl, C₃₋₆ cycloalkenyl, C₃₋₆ aryl, and a C₃₋₆ heteroaryl; R²² and R²⁷ can be independently selected from hydrogen, halogen, -NH₂, - NHR^{a2}, NR^{a2}R^{b2} -OR^{a2} -SR^{a2}, -CN, -NC -N₃, -NO₂, -N(R^{c2})-NR^{a2}R^{b2}, -N(RC²)-OR^{a2}, -S-SR^{a2}, -C(=O)R^{a2}, -C(=O)OR^{a2}, -C(=O)NR^{a2}R^{b2}, -O-C(=O)OR^{a2}, -O-C(=O)NR^{a2}R^{b2},-N(R^{c2})-C(=O)NR^{a2}Rb², -S(=O)R^{a2} , S(=O)₂R^{a2}, -O-S(=O)₂NR^{a2}R^{b2}, -N(R^{C2})-S(=O)₂NR^{a2}R^{b2}, an optionally substituted C₁₋₆ alkyl, an optionally substituted C₂₋₆ alkenyl, an optionally substituted C₂₋₆ alkynyl and an -O-linked amino acid; R²³, R²⁴ and R²⁵ can be independently absent or selected from the group consisting of hydrogen, halogen, - NH2, -NHR^{a2}, NR^{a2}R^{b2}, ₋OR^{a2}, -SR^{a2}, -CN, -NC, -N₃, -NO₂, -N(R^{c2})-NR^{a2}R^{b2} , -N(R^{c2})-OR^{a2}, -S-SR^{a2}, -C(=O)R^{a2}, -C(=O)OR^{a2}, -C(=O)NR^{a2}R^{b2}, -O-C(=O)R^{a2}, -O-C(=O)OR^{a2}, _ O-C(=O)NR^{a2}R^{b2} -N(R^{c2})-C(=O)NR^{a2}R^{b2}, -S(=O)R^{a2}, S(=O)R^{a2}, -O-S(=O)₂NR^{a2}R^{b2}, N(R^{c2})-S(=O)₂NR^{a2}R^{b2}, an optionally substituted C₁₋₆ alkyl, an optionally substituted C₂₋₆ alkenyl, an optionally substituted C₂₋₆ alkynyl, an optionally substituted aralkyl and an - O-linked amino acid; or R²⁴ and R²⁵ taken together form -O-C(=O)-O-; R²⁶ can be absent or selected from hydrogen, halogen, -NH₂, -NHR^{a2}, NR^{a2}R^{b2}, -OR^{a2}, -SR^{a2}, -CN,-NC, -N₃, -NO₂, -N(R^{c2})-NR^{a2}R^{b2}, -N(R^{c2})-OR^{a2}, -S-SR^{a2}, -C(=O)R^{a2}, -C(=O)OR^{a2}, - C(=O)NR^{a2}R^{b2}, -O-C(=O)OR^{a2}, -O-C(=O)NR^{a2}Rb² -N(RC²)-C(=O)NR^{a2}R^{b2}, -S(=O)R^{a2} S(=O)₂R^{a2}, -O-S(=O)₂NR^{a2}R^{b2}, -N(R^{c2})-S(=O)₂NR^{a2}R^{b2}, an optionally substituted C₁₋₆ alkyl, an optionally substituted C₂₋₆ alkenyl, an optionally substituted C₂₋₆ alkynyl, an optionally substituted haloalkyl, an optionally substituted hydroxyalkyl and an -O-linked amino acid, or when the bond to R²⁵ indicated by 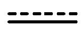 is a double bond, then R²⁵ is a C₂₋₆ alkylidene and R²⁶ is absent; R^{a2}, R^{b2} and R^{c2} can be each independently selected from hydrogen, an optionally substituted alkyl, an optionally substituted alkenyl, an optionally substituted alkynyl, an optionally substituted aryl, an optionally substituted heteroaryl, an optionally substituted aralkyl and an optionally substituted heteroaryl(C₁₋₆ alkyl);
R²⁸ can be selected from O⁻, -OH, an optionally substituted aryloxy or aryl-O-, 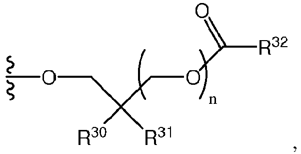 alkyl-C(=O)-O-CH₂-O-, alkyl-C(=O)-S-CH₂CH₂-O- and an -N-linked amino acid; R²⁹ can be selected from O⁻, -OH, an optionally substituted aryloxy or aryl-O-, 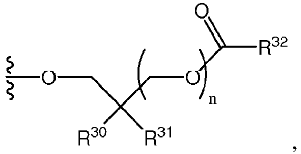 alkyl-C(=O)-O-CH₂-O-, alkyl-C(=O)-S-CH₂CH₂-O- and an -N-linked amino acid; each R³⁰ and each R³¹ can be independently -C=N or an optionally substituted substituent selected from C₁₋₈ organylcarbonyl, C₁₋₈ alkoxycarbonyl and C₁₋₈ organylaminocarbonyl; each R³² can be hydrogen or an optionally substituted C₁₋₆-alkyl; and each n can be independently 1 or 2, and if both R²⁸ and R²⁹ are 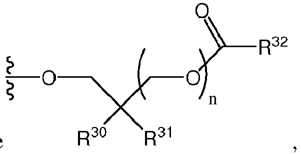 each R³⁰, each R³¹, each R³² and each n can be the same or different.

In an embodiment, n can be 1. In another embodiment, n can be 2. In some embodiments, A² can be carbon. In some embodiments, D² can be oxygen. In an embodiment, each 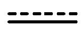 can be a single bond. In an embodiment, A² can be carbon, D² can be oxygen and each 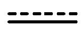 can be a single bond. In other embodiments, A² can be carbon, D² can be oxygen, each 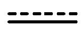 can be a single bond and n can be 1. In an embodiment, A² can be carbon, D² can be oxygen, each 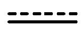 can be a single bond and n can be 2.

In some embodiments, the optionally substituted C₁₋₆ alkyl can be selected from methyl, ethyl, n-propyl, isopropyl, n-butyl, iso-butyl, tert-butyl, pentyl and hexyl. In an embodiment, the optionally substituted C₁₋₆ alkyl can be methyl. For example, in an embodiment, R²⁰ can be methyl and R²¹ can be hydrogen. In some embodiments, the optionally substituted C₁₋₆ alkoxy can be selected from methoxy, ethoxy, n-propoxy, isopropoxy, n-butoxy, iso-butoxy and tert-butoxy. In some embodiments, the optionally substituted C₁₋₆ haloalkyl can be trifluoromethyl. In an embodiment, R²⁰ can be trifluoromethyl and R²¹ can be hydrogen.

In some embodiments, a compound of Formula (II) can be a nucleoside or nucleoside derivative. In an embodiment, R¹⁹ can be hydrogen. In some embodiments, a compound of Formula (II) can be a nucleotide or nucleotide derivative. In an embodiment, R¹⁹ can be a monophosphate. In another embodiment, R¹⁹ can be a diphosphate. In yet another embodiment, R¹⁹ can be a triphosphate. In still yet another embodiment, R¹⁹ can be 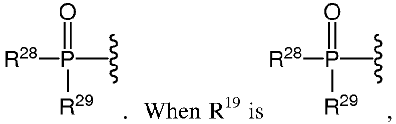 R²⁸ and R²⁹ can both be O⁻. In some embodiments, neutralizing the charge on the phosphate of the nucleotide or nucleotide derivative may facilitate the entry of the nucleotides and nucleotides analogs in a cell. In some embodiments, R²⁸ and R²⁹ can each be independently 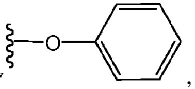 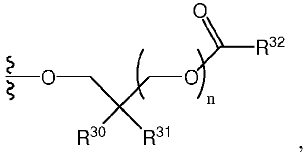 -O-naphthol and/or an -N-linked amino acid. In some embodiments, at least one of R²⁸ and R²⁹ can be 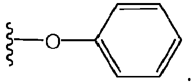 In an embodiment, R²⁸ can be 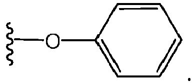 In some embodiments, at least one of R²⁸ and R²⁹ can be an -N-linked amino acid. In an embodiment, R²⁸ can be 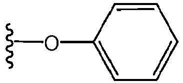 and R²⁹ can be an -N-linked amino acid, such as those described herein. In another embodiment, when R²⁸ is 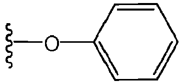 R²⁹ cannot be an -N-linked amino acid.

In an embodiment, at least one of R²⁸ and R²⁹ can be 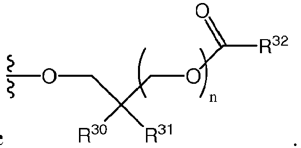 The substitutents on 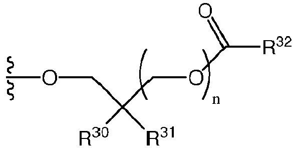 can vary. In some embodiments, R³⁰ can be -can and R³¹ can be an optionally substituted C₁₋₈ alkoxycarbonyl such as -C(=O)OCH₃. In other embodiments, R³⁰ can be -C=N and R³¹ can be an optionally substituted C₁₋₈ organylaminocarbonyl, for example, - C(=O)NHCH₂CH₃ and -C(=O)NHCH₂CH₂phenyl. In still other embodiments, both R³⁰ and R³¹ can be an optionally substituted C₁₋₈ organylcarbonyl. In an embodiment, both R³⁰ and R³¹ can be -C(=O)CH₃. In yet still other embodiments, both R³⁰ and R³¹ can be an optionally substituted C₁₋₈ alkoxycarbonyl. In an embodiment, both R³⁰ and R³¹ can be -C(=O)OCH₃ or -C(=O)OCH₂CH₃. In an embodiment, both R³⁰ and R³¹ can be an optionally substituted C₁₋₈ alkoxycarbonyl, for example -C(=O)OCH₂CH₃, and n can be 2. In some embodiments, including those in this paragraph, R³² can be an optionally substituted C₁₋₆-alkyl. In an embodiment, including those in this paragraph, R³² can be methyl or tert-butyl. Examples of 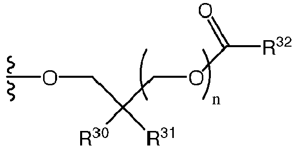 groups, include but are not limited to the following: 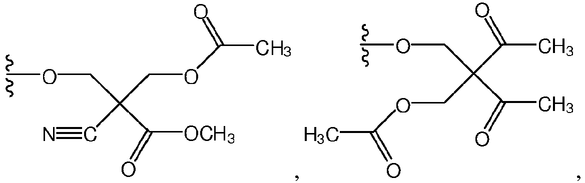 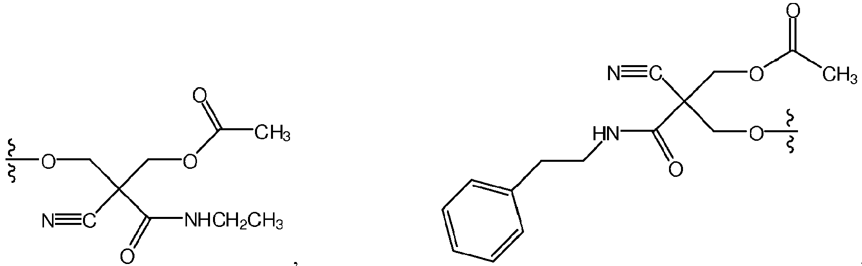 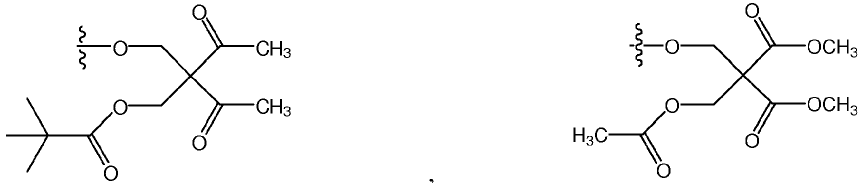 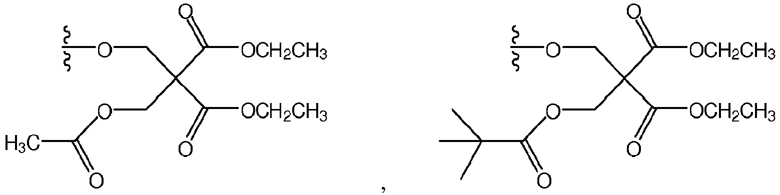 and

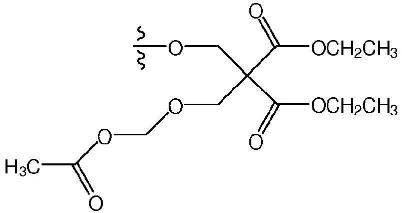

In an embodiment, at least one of R²⁸ and R²⁹ can be 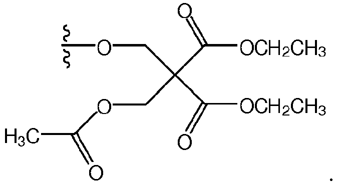 In another embodiment, at least one of R²⁸ and R²⁹ can be 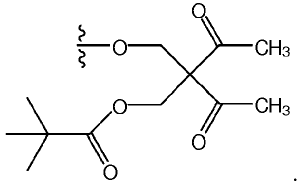 In still another embodiment, at least one of R²⁸ and R²⁹ can be 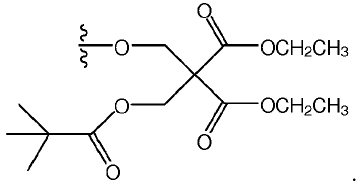 In yet still another embodiment, at least one of R²⁸ and R²⁹ can be 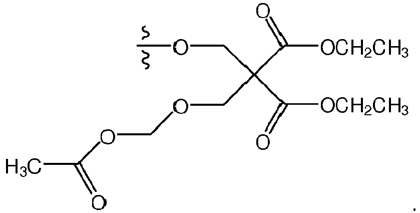 In some embodiments, at least one of R²⁸ and R²⁹ can be 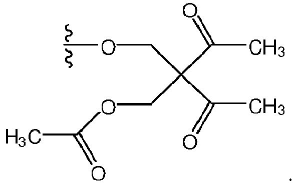 In some embodiments, both R²⁸ and R²⁹ can be 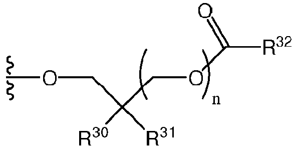 wherein each R³⁰, each R³¹, each R³² and each n can be the same or different. In an embodiment, when R²⁸ and R²⁹ are 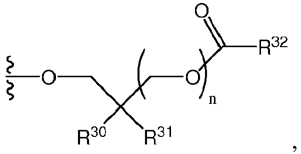 R²⁸ and R²⁹ can be the same. In another embodiment, when R²⁸ and R²⁹ are 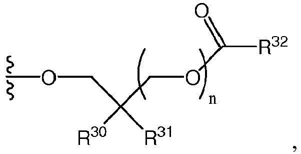 R²⁸ and R²⁹ can be different.

In some embodiments, at least one of R²⁸ and R²⁹ can be an -N-linked amino acid. Suitable amino acids include those described herein. In some embodiments, an -N-linked amino acid can have the structure 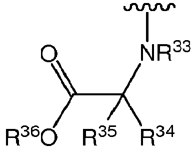 wherein: R³³ can be hydrogen or an optionally substituted C₁₋₄-alkyl; R³⁴ can be selected from hydrogen, an optionally substituted C₁₋₆-alkyl, an optionally substituted aryl, an optionally substituted aryl(C₁₋₆ alkyl) and an optionally substituted haloalkyl; R³⁵ can be hydrogen or an optionally substituted C₁₋₆-alkyl; and R³⁶ can be selected from an optionally substituted C₁₋₆ alkyl, an optionally substituted C₆ aryl, an optionally substituted C₁₀ aryl, and an optionally substituted C₃₋₆ cycloalkyl. In an embodiment, R³³ can be hydrogen. In some embodiments, R³⁴ can be an optionally substituted C₁₋₆-alkyl, for example, methyl. In an embodiment, R³⁵ can be hydrogen or an optionally substituted C₁₋₆-alkyl. In an embodiment, R³⁵ can be methyl. In some embodiment, R³⁶ can be an optionally substituted C₁₋₆-alkyl. One example of a suitable an -N-linked amino acid is 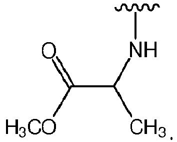 In some embodiments, the amino acid can be in the L-configuration. In other embodiments, the amino acid can be in the D-configuration. For example, 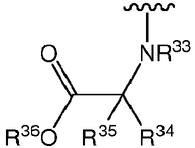 can be 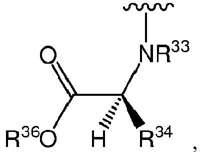 such as

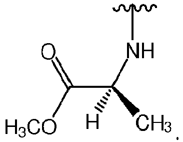

Various optionally substituted heterocyclic bases and optionally substituted heterocyclic base derivatives can be present in a compound of Formula (II). Examples of suitable optionally substituted heterocyclic bases and optionally substituted heterocyclic base derivatives are shown below. 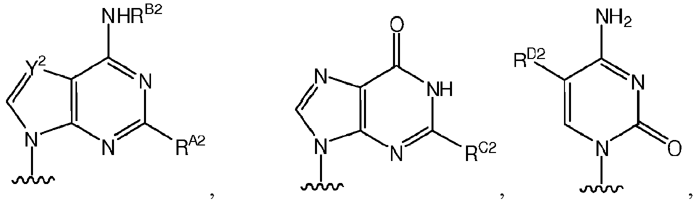 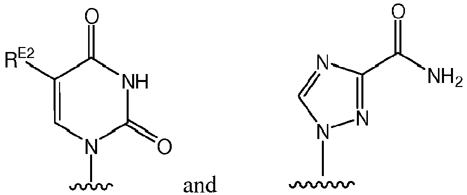 wherein: R^{A2} can be hydrogen or halogen; R^{B2} can be hydrogen, an optionally substituted C₁₋₆alkyl, or an optionally substituted C₃₋₈ cycloalkyl; R^{C2} can be hydrogen or amino; R^{D2} can be hydrogen, halogen, an optionally substituted C₁₋₆ alkyl, an optionally substituted C₂₋₆ alkenyl and an optionally substituted C₂₋₆ alkynyl,; R^{E2} can be hydrogen, halogen, an optionally substituted C₁₋₆alkyl, an optionally substituted C₂₋₆ alkenyl and an optionally substituted C₂₋₆ alkynyl; and Y² can be N (nitrogen) or CR^{F2}, wherein R^{F2} can be selected from hydrogen, halogen, an optionally substituted C₁₋₆-alkyl, an optionally substituted C₂₋₆-alkenyl and an optionally substituted C₂₋₆-alkynyl. In some embodiments, B² can be 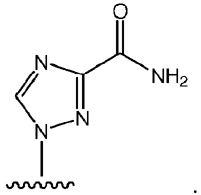 In other embodiments, B² can be 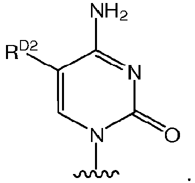 In yet other embodiments, B² can be 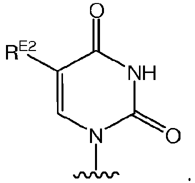 In yet still other embodiments, B² can be 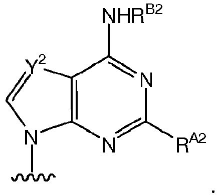 In an embodiment Y² can be nitrogen; R^{A2} can be hydrogen and R^{B2} can be hydrogen. In another embodiment, Y² can be CR^{F2}, wherein R^{F2} can be selected from hydrogen, halogen, an optionally substituted C₁₋₆-alkyl, an optionally substituted C₂₋₆-alkenyl and an optionally substituted C₂₋₆-alkynyl; R^{A2} can be hydrogen and R^{B2} can be hydrogen. When B² is any of the aforementioned moieties shown above, in some embodiments, A² can be carbon. In an embodiment, B² can be any of the aforementioned moieties shown above, A² can be carbon and D² can be oxygen. In some embodiments, B² can be any of the aforementioned moieties shown above, A² can be carbon, D² can be oxygen and each 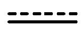 can be a single bond.

In some embodiments, R²² can be selected from hydrogen, halogen, - OR^{a2}, -CN, -N₃ and an optionally substituted C₁₋₆ alkyl. In some embodiments, R²³ can be absent or selected from hydrogen, halogen, -OR^{a2} and an optionally substituted C₁₋₆ alkyl. In some embodiments, R²⁴ can be absent or selected from hydrogen, halogen, - NH₂, -OR^{a2}, -N₃, an optionally substituted C₁₋₆ alkyl and an -O-linked amino acid. In some embodiments, R²⁴ can be -OR^{a2}, wherein R^{a2} is hydrogen. In other embodiments, R²⁴ can be an -O-linked amino acid. In some embodiments, R²⁵ can be selected from hydrogen, halogen, -OR^{a2}, -CN, -NC, an optionally substituted C₁₋₆ alkyl and an -O-linked amino acid. In some embodiments, R²⁵ can be -OR^{a2}, wherein R^{a2} is hydrogen. In other embodiments, R²⁵ can be a C₁₋₆ alkoxy such as methoxy. In still other embodiments, R²⁵ can be an -O-linked amino acid. In some embodiments, both R²⁴ and R²⁵ can be hydroxy groups. In other embodiments, R²⁵ can be a hydroxyl group and R²⁴ can be an -O-linked amino acid. Suitable -O-linked amino acids are described herein. In some embodiments, R²⁶ can be selected from hydrogen, halogen, an optionally substituted C₁₋₆ alkyl, an optionally substituted haloalkyl, an optionally substituted hydroxyalkyl, and the bond to R²⁵ indicated by 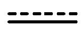 is a double bond, R²⁵ is a C₂₋₆ alkenyl and R²⁶ is absent. In some embodiments, R²⁷ can be selected from hydrogen, halogen and an optionally substituted C₁₋₆ alkyl.

In some embodiments, at least one of R²⁵ and R²⁶ can be a halogen. In other embodiments, both R²⁵ and R²⁶ can be a halogen.

Examples of compounds of Formula (II) are shown below.

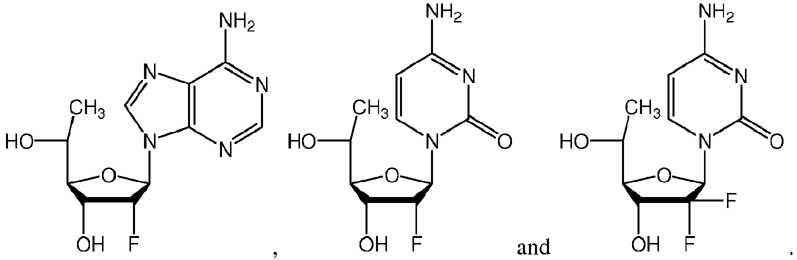

In some embodiments, B¹ and B² cannot be an optionally substituted pyridinyl group, an optionally substituted tricyclic heterocyclic group, an optionally substituted piperizinyl, an optionally substituted pyrrolo-pyrimidinone, a triazole substituted with an amidine, an optionally substituted pyrido-pyrimidine. In some embodiments, B¹ and B² cannot be any of moieties attached to the 1'-position disclosed in U.S. Application Nos. 2006-0229265 (filed March 30, 2006), 2005-0203044 (filed Jan. 26, 2005) and 2007-0258921 (filed April 30, 2007); U.S. Patent Nos. 7,268,119 (filed Feb. 14, 2007), 6,815,542 (filed Dec. 13, 2002), 6,495,677 (filed June 16, 2000), 7,081,449 (filed July 3, 2001), 6,130,326 (filed April 14, 1999), 6,552,183 (filed Aug. 7, 2000) 6,573,248 (Dec. 31, 2001) 6,642,206 (April 9, 2002), 5,767,097 (filed Jan. 23 1996); International Publication Nos. WO 2004/106356 (filed May 27, 2004), WO 2004/080466 (filed March 7, 2003), WO 03/039523 (filed Nov. 5, 2002); and Canadian Patent No. 02252144 (filed Oct. 26, 1998).

As stated previously, in some embodiments, neutralizing the charge on the phosphate group may facilitate the penetration of the cell membrane by compounds of Formulae (I) and (II) by making the compound more lipophilic. Furthermore, it is believed that the 2,2-disubstituted-acyl(oxyalkyl) groups, such as 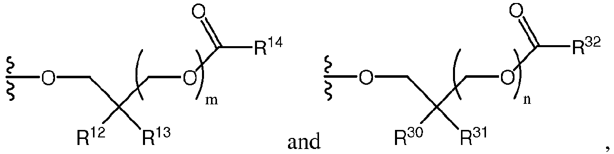 attached to the phosphate impart increased plasma stability to compounds of Formulae (I) and (II) by inhibiting the degradation of the compound. Once inside the cell, the 2,2-disubstituted-acyl(oxyalkyl) group attached to the phosphate can be easily removed by esterases via enzymatic hydrolysis of the acyl group. The remaining portions of the group on the phosphate can then be removed by elimination. The general reaction scheme is shown below in Scheme 1a.

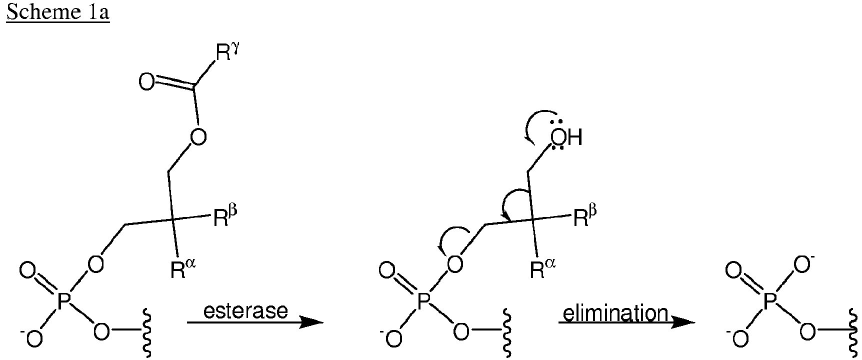

A further advantage of the 2,2-disubstituted-acyl(oxyalkyl) groups described herein is the rate of elimination of the remaining portion of the 2,2-disubstituted-acyl(oxyalkyl) group is modifiable. Depending upon the identity of the substituents on the 2-carbon, shown in Scheme 1a as R^{α} and R^{β}, the rate of elimination may be adjusted from several seconds to several hours. As a result, the removal of the remaining portion of the 2,2-disubstituted-acyl(oxyalkyl) group can be retarded, if necessary, to enhance cellular uptake but, readily eliminated upon entry into the cell. Upon removal of the groups on the oxygen atoms of the phosphate, the resulting nucleotide analog possesses a monophosphate. Thus, the necessity of an initial intracellular phosphorylation is no longer a prerequisite to obtaining the biologically active phosphorylated form.

### Synthesis

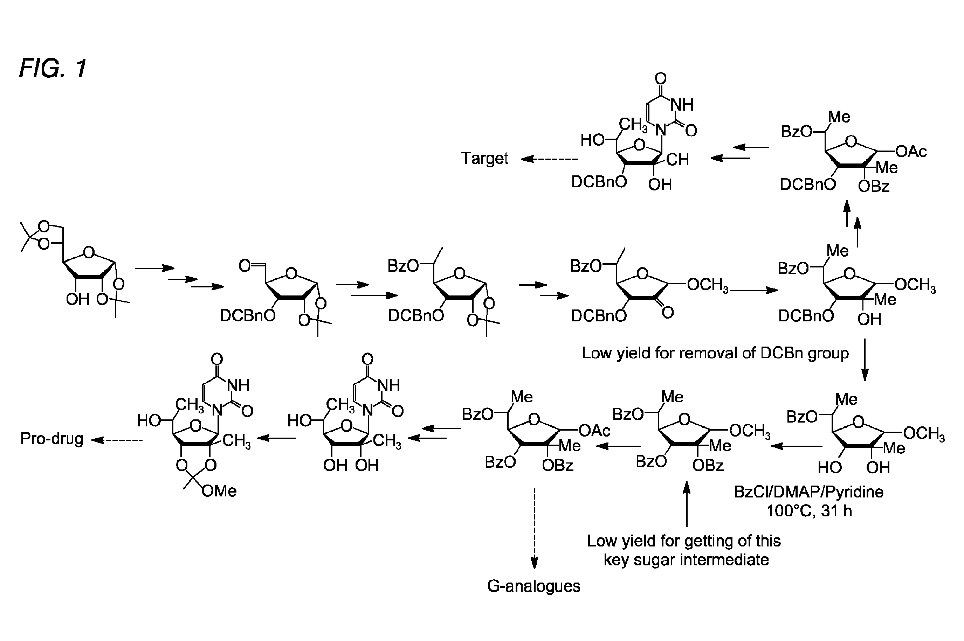

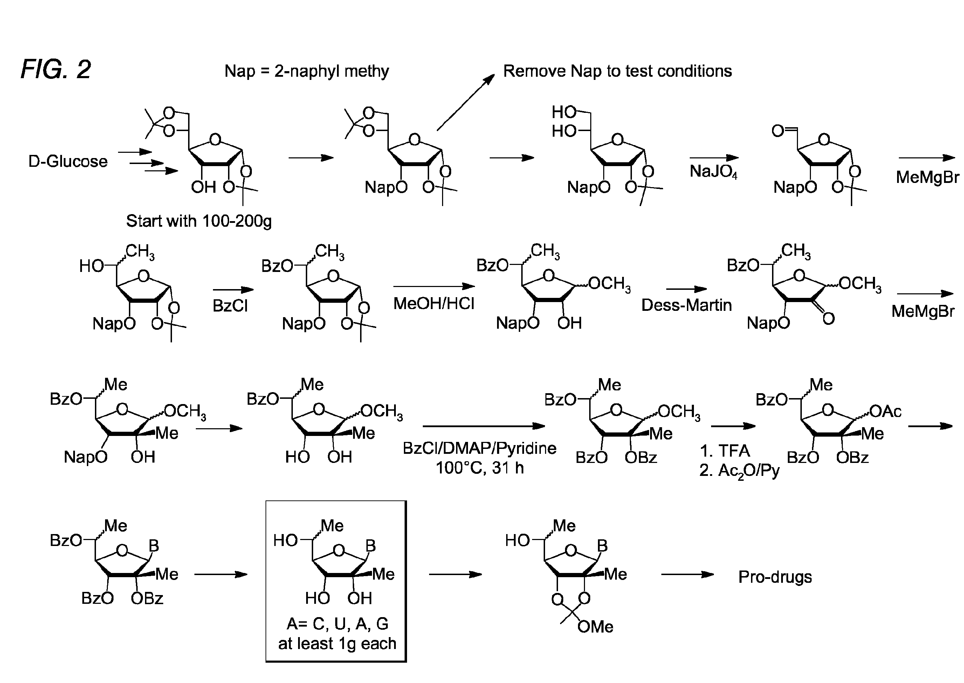

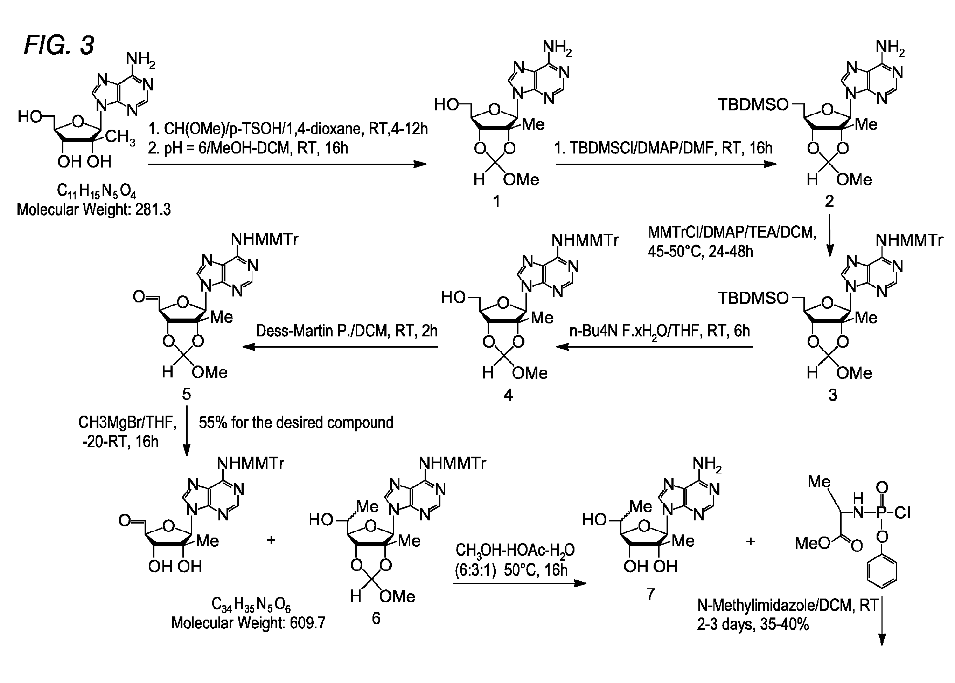

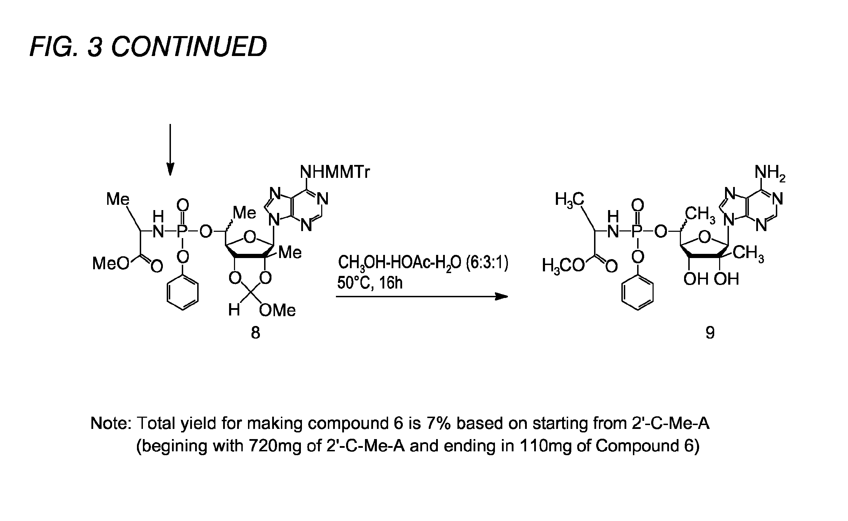

Compounds of Formulae (I) and (II), and those described herein may be prepared in various ways. General synthetic routes to the compounds of Formulae (I) and (II), and the starting materials used to synthesize the compounds of Formulae (I) and (II) are shown in Schemes 1-3 and Figures 1-3. The routes shown are illustrative only and are not intended, nor are they to be construed, to limit the scope of the claims in any manner whatsoever. Those skilled in the art will be able to recognize modifications of the disclosed synthesis and to devise alternate routes based on the disclosures herein; all such modifications and alternate routes are within the scope of the claims.

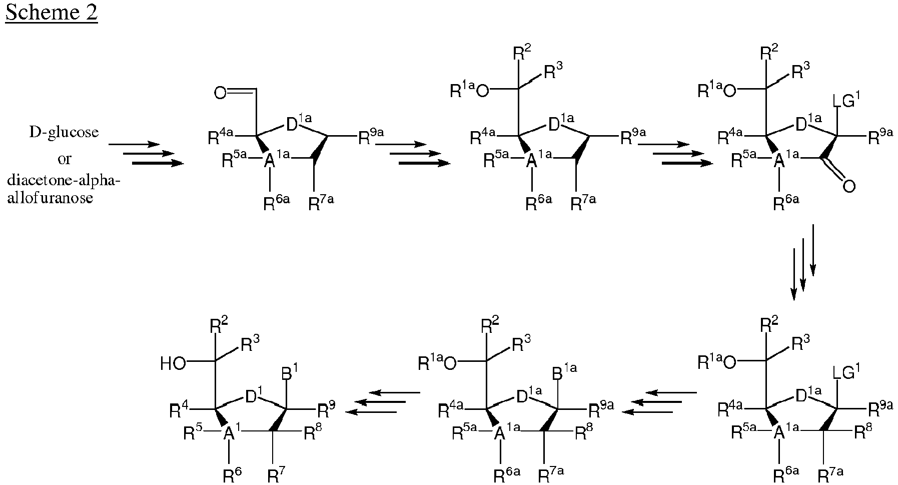

One method for forming a compound of Formula (I) is shown in Scheme 2 in which R², R³, R⁴, R⁵, R⁶, R⁷, R⁸, R⁹, A¹, B¹ and D¹ can be the same as disclosed herein, and R^{1a} can be hydrogen or a protecting group. Examples of suitable protecting groups include, but are not limited to, an optionally substituted benzoyl and silyl ethers such as trimethylsilyl (TMS), *tert*-butyldimethylsilyl (TBDMS), triisopropylsilyl (TIPS) and tert-butyldiphenylsilyl (TBDPS). Also, in Scheme 2, R^{4a}, R^{5a}, R^{6a}, R^{7a}, R^{9a}, A^{1a}, B^{1a} and D^{1a} can be the same as R⁴, R⁵, R⁶, R⁷, R⁹, A¹, B¹ and D¹, respectively, or can be each a protected version of R⁴, R⁵, R⁶, R⁷, R⁹, A¹, B¹ and D¹, respectively. By "protected versions, the substituents listed herein for R⁴, R⁵, R⁶, R⁷, R⁹, A¹, B¹ and D¹ may be altered to include one or more protecting groups. For example, the hydrogen of a hydroxy group may be exchanged for a protecting group, two hydroxy groups may be cyclized to form an acetal or an ortho-ester, the hydrogen on a NH group may be exchanged for a protecting group and/or one or both hydrogens on a -NH₂ group may be replaced for one or more protecting groups. Additionally, in Scheme 2, LG¹ can be a suitable leaving group, such as those described herein.

A five membered heterocyclic ring can be formed via an addition/cyclization reaction from D-glucose. In some embodiments, the five-membered heterocyclic ring can be an optionally substituted ribose sugar. In other embodiments, the five membered can be an optionally substituted deoxyribose sugar. Alternatively, diacetone-alpha-allofuranose, a commercially available reagent can be used.

The 5'-OH group can be oxidized to an aldehyde using methods known to those skilled in the art. Suitable oxidizing agents include, but are not limited to, Dess-Martin periodinane, TPAP/NMO (tetrapropylammonium perruthenate/N-methylmorpholine N-oxide), Swern oxidation reagent, PCC (pyridinium chlorochromate), and/or PDC (pyridinium dichromate), sodium periodate, Collin's reagent, ceric ammonium nitrate CAN, Na₂Cr₂O₇ in water, Ag₂CO₃ on celite, hot HNO₃ in aqueous glyme, O₂-pyridine CuCl, Pb(OAc)₄-pyridine and benzoyl peroxide-NiBr₂.

An optionally substituted C₁₋₆ alkyl, an optionally substituted C₂₋₆ alkenyl, an optionally substituted C₂₋₆ alkynyl or an optionally substituted C₁₋₆ haloalkyl can be added to the 5'-carbon using methods known to those skilled in the art. For example, an optionally substituted C₁₋₆ alkyl or an optionally substituted C₁₋₆ haloalkyl can be added to the 5'-carbon using alkylation methods are known to those skilled in the art, such as through the use of an organometallic moiety. A non-limiting list of suitable organometallic moieties include organomagnesium compounds, organolithium compounds, organotin compounds, organocuprates compounds, organozinc, and organopalladium compounds, metal carbonyls, metallocenes, carbine complexes, and organometalloids (e.g., organoboranes and organosilanes). In some embodiments, the organometallic moiety can be an organomagnesium compound. In an embodiment, the organomagnesium compound can be an optionally substituted C₁₋₆ alkyl or an optionally substituted C₁₋₆ haloalkyl-Mg-halo, for example, MeMgBr.

If not already present, addition of an optionally substituted C₁₋₆ alkyl to the 2'-position can also be accomplished using methods known to a person of ordinary skill in the art. When a hydroxy group is present on the 2'-position, in some embodiment, the hydroxy group can be oxidized to a ketone using one or more suitable methods. For example, the hydroxy group can be oxidized to a ketone using one or more oxidizing agents. Suitable oxidizing agent include, but are not limited to, acid dichromates, KMnO₄, Br₂, MnO₂, ruthenium tetraoxide, Jones reagent, Collin's reagent, Corey's reagent, pyridnium dichromate, Swern oxidation reagent, DMSO and trifluoroacetic anhydride (TFAA), and those previously described herein. In an embodiment, the oxidizing agent can be Dess-Martin periodinane or DMSO and TFAA.

An optionally substituted C₁₋₆ alkyl can be added to the 2'-carbon using methods known to those skilled in the art. In some embodiments, the 2'-carbon can be alkylated using a suitable organometallic moiety such as those described herein. In an embodiment, the organometallic moiety can be MeMgBr.

The substitutent at the 1'-position can be converted to an appropriate leaving group, for example a nucleofuge, using methods known to those skilled in the art. For example, the 1'-position can be converted to an appropriate leaving group via an hydrolysis reaction followed by acetylation using a suitable reagent such as acetic anhydride. As another example, the 1'-position can be converted to an appropriate leaving group by transforming the acetal to a hemiacetal under acid conditions followed by acetylation with an appropriate reagent (e.g., acetic anhydride).

An optionally substituted heterocyclic base or an optionally substituted heterocyclic base derivative can be added to the 1'-position using a catalyst. Suitable catalysts are known in the art. In an embodiment, the catalysts can be trimethylsilyl trifluoromethanesulfonate. To facilitate the reaction, in some embodiments, the addition of the optionally substituted heterocyclic base or the optionally substituted heterocyclic base derivative can take place in the presence of a base. Examples of suitable bases include amine-based bases such as triethylamine, 1,8-diazabicyclo[5.4.0]undec-7-ene (DBU) and 1,5-diazabicyclo[4.3.0]non-5-ene (DBN). After addition of the optionally substituted heterocyclic base or the optionally substituted heterocyclic base derivative, a compound of Formula (I) in which R¹ is H can be obtained after removal of any protecting groups that may be present.

If needed and/or desired, any hydroxy groups present on the 2', 3' and 4'-positions can be protected with one or more suitable protecting groups. The hydroxy groups can be protected with an individual protecting group. Alternatively, two adjacent hydroxy groups can be cyclized to form an acetal or an ortho ester. In some embodiments, some of the hydroxy groups can be protected with individual protecting groups and other hydroxy groups can be protected through the formation of an acetal or an ortho ester.

Alternatively, if an optionally substituted heterocyclic base or an optionally substituted heterocyclic base derivative is already present on the 5-membered heterocyclic ring, an optionally substituted C₁₋₆ alkyl or an optionally substituted C₁₋₆ haloalkyl (e.g., CF₃) can be added to the 5'-position as shown below in Scheme 3. The substituents R², R³, R⁴, R⁵, R⁶, R⁷, R⁸, R⁹, A¹, B¹ and D¹ can be the same as disclosed herein, and R^{4a}, R^{5a}, R^{6a}, R^{7a}, R^{9a}, A^{1a}, B^{1a} and D^{1a} can be the same as R⁴, R⁵, R⁶, R⁷, R⁹, A¹, B¹ and D¹, respectively, or can be each a protected version of R⁴, R⁵, R⁶, R⁷, R⁹, A¹, B¹ and D¹, respectively. R^{1a} can be hydrogen or a protecting group, including those described herein.

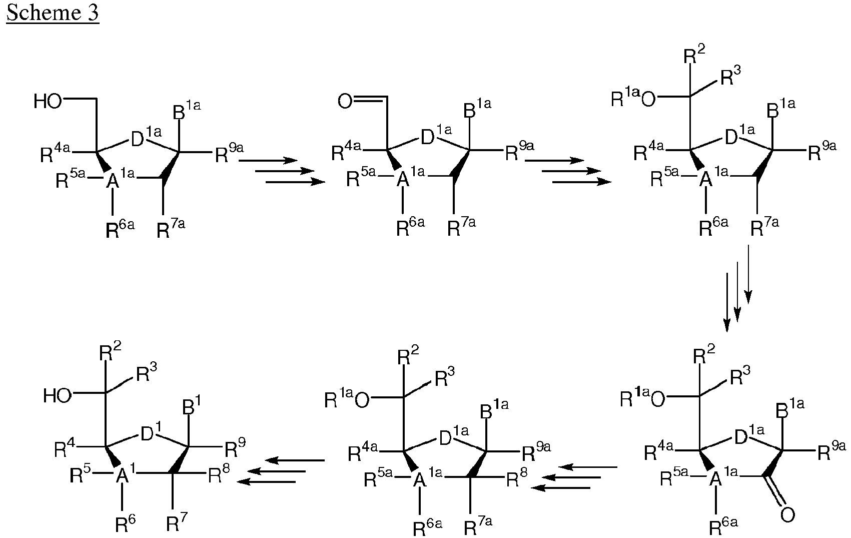

As described herein, the hydroxy group at the 5'-position can be oxidized to aldehyde using a suitable oxidizing reagent such as those described herein. An optionally substituted C₁₋₆ alkyl or an optionally substituted C₁₋₆ haloalkyl can be added the 5'-position using an appropriate alkylation method. Appropriate alkylation methods are described herein. In an embodiment, the 5'-position can be alkylated using an organometallic reagent, for example, an organomagnesium compound.

If an optionally substituted C₁₋₆ alkyl is not already present on the 2'-position, the optionally substituted C₁₋₆ alkyl can be added using known to those skilled in the art. For example, when a hydroxy group is present on the 2'-position, in some embodiment, the hydroxy group can be oxidized to a ketone using one or more suitable methods. In an embodiment, the hydroxy group can be oxidized to a ketone using one or more oxidizing agents disclosed herein. An optionally substituted C₁₋₆ alkyl can then be added to the 2'-carbon using methods known to those skilled in the art. In some embodiments, the 2'-carbon can be alkylated using a suitable organometallic moiety such as those described herein. In an embodiment, the organometallic moiety can be MeMgBr.

If needed and/or desired, the optionally substituted heterocyclic base or the optionally substituted heterocyclic base derivative can be protected with one or more suitable protecting groups during the formation of a compound of Formula (I). For example, one or more amino groups attached to a ring and/or any -NH groups present in a ring of the optionally substituted heterocyclic base and/or optionally substituted heterocyclic base derivative can be protected with one or more suitable protecting groups. In an embodiment, the optionally substituted heterocyclic base and/or optionally substituted heterocyclic base derivative can be protected with one or more triarylmethyl protecting groups. A non-limiting list of triarylmethyl protecting groups are trityl, monomethoxytrityl (MMTr), 4,4'-dimethoxytrityl (DMTr), 4,4',4"-trimethoxytrityl (TMTr),. 4,4',4"-tris- (benzoyloxy) trityl (TBTr), 4,4',4"-tris (4,5-dichlorophthalimido) trityl (CPTr), 4,4',4"-tris (levulinyloxy) trityl (TLTr), p-anisyl-1- naphthylphenylmethyl, di-o-anisyl-1-naphthylmethyl, p-tolyldipheylmethyl, 3-(imidazolylmethyl)-4,4'-dimethoxytrityl, 9-phenylxanthen-9-yl (Pixyl), 9-(p-methoxyphenyl) xanthen-9-yl (Mox), 4-decyloxytrityl, 4- hexadecyloxytrityl, 4,4'-dioctadecyltrityl, 9-(4- octadecyloxyphenyl) xanthen-9-yl, 1,1'-bis-(4-methoxyphenyl)-1'-pyrenylmethyl, 4,4',4"-tris- (tert-butylphenyl) methyl (TTTr) and 4,4'-di-3, 5-hexadienoxytrityl. Any protecting groups on the 5-membered heterocyclic ring can also be protected with one or more suitable protecting groups, including those described herein.

The protecting groups can be removed and other protecting groups can be added at different times during the general reaction schemes shown in Schemes 2 and 3, for example, before the formation of the aldehyde at the 5'-position, after the alkylation of the 5'-position, before the oxidation of the 2'-position, after alkylation of the 2'-position, before the addition of the optionally substituted heterocyclic base or optionally substituted heterocyclic base derivative and/or after the addition of the optionally substituted heterocyclic base or optionally substituted heterocyclic base derivative. Removal and replacement of a protecting group may be useful because of the reactions conditions. The protecting groups may assistant in preventing unwanted side reaction and/or make the separation of the desired product simpler

A phosphate group can be added to 5'-position as shown in Scheme 4. The substituents R², R³, R⁴, R⁵, R⁶, R⁷, R⁸, R⁹, A¹, B¹ and D¹ can be the same as disclosed herein, and R^{4a}, R^{5a}, R^{6a}, R^{7a}, R^{9a}, A^{1a}, B^{1a} and D^{1a} can be the same as R⁴, R⁵, R⁶, R⁷, R⁹, A¹, B¹ and D¹, respectively, or can be each a protected version of R⁴, R⁵, R⁶, R⁷, R⁹, A¹, B¹ and D¹, respectively.

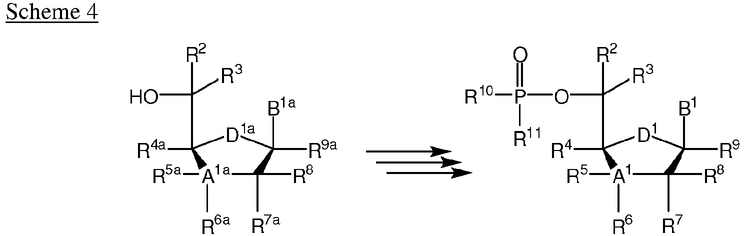

A variety of methods can be used to add a phosphate group to the 5'-position. Suitable methods are described in Current Protocals in Nucleic Acid Chemistry. Donald E. Bergstrom Nucleoside Phosphorylation and Related Modifications in Current Protocals in Nucleic Acid Chemistry, Chapter 1, (2008) John Wiley & Sons, Inc. For example, a phosphate at the 5'-position can be formed via a phosphoamidite and oxidation methods.

To add a 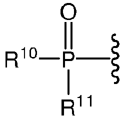 group wherein one of R¹⁰ and R¹¹ is 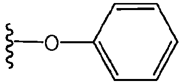 and the other of R¹⁰ and R¹¹ is an -N-linked amino acid, a (O-phenyl-N-linked amino acid))phosphoramidohalide can be reacted with the 5'-position of a nucleoside or a nucleoside derivative, such as 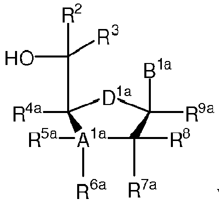 where R², R³ and R⁸ can be the same as previously defined herein, and R^{4a}, R^{5a}, R^{6a}, R^{7a}, R^{9a}, A^{1a}, B^{1a} and D^{1a} can be the same as R⁴, R⁵, R⁶, R⁷, R⁹, A¹, B¹ and D¹, respectively, or can be each a protected version of R⁴, R⁵, R⁶, R⁷, R⁹, A¹, B¹ and D¹, respectively. A variety of amino acids can be used to form the -N-linked amino acid. In some embodiments, the amino acid can have the following structure 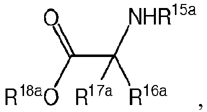 wherein R^{15a}, R^{16a}, R^{17a}, and R^{18a} can be the same as R¹⁵, R¹⁶, R¹⁷ and R¹⁸, as described herein with respect to Formula (I). If needed and/or desired, any hydroxy groups present on the 5-membered heterocyclic ring can be protected with one or more protecting groups such as those described herein. In some embodiments, any hydroxy groups on the 2'- and 3'-positions can be protected with one or more protecting groups. For example, when the 5'-membered heterocyclic ring has hydroxy groups at the 2'- and 3'-positions, the oxygens can be protected by forming an acetal or an ortho ester.

The hydroxy precursor, 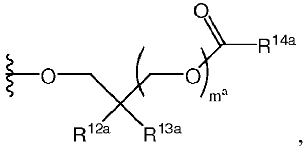 in which R^{12a}, R^{13a}, R^{14a} and m^{a} are the same as R¹², R¹³, R¹⁴ and m, respectively, as described herein, of the 2,2-disubstituted-acyl(oxyalkyl) groups can be synthesized according in a manner similar to those described in the following articles. Ora, et al., J. Chem. Soc. Perkin Trans. 2, 2001 6: 881-5; Poijärvi, P. et al., Helv. Chim. Acta. 2002 85:1859-76; Poijärvi, P. et al., Lett. Org. Chem., 2004, 1:183-88; and Poijärvi, P. et al., Bioconjugate Chem., 2005 16(6):1564-71, all of which are hereby incorporated by reference in their entireties.

Examples of hydroxy precursors can include the following: 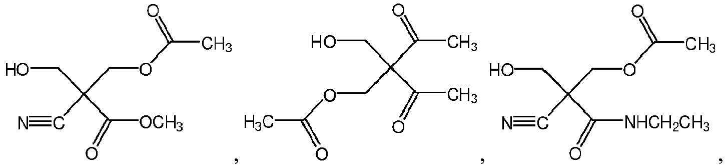 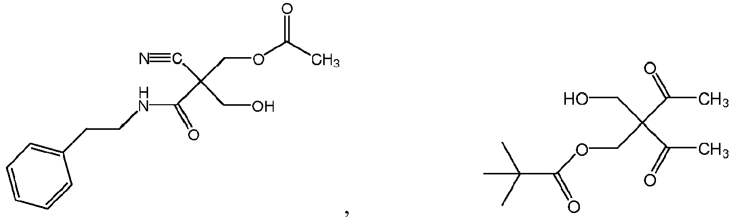 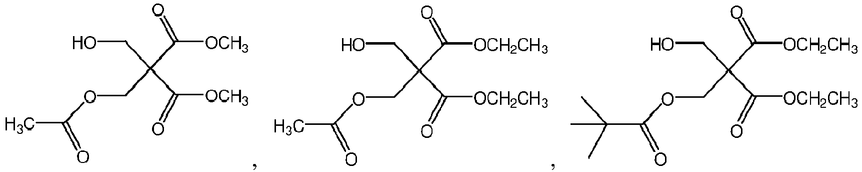 and

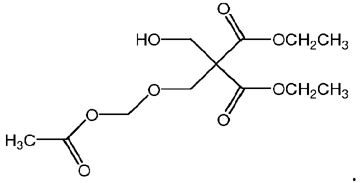

To add a 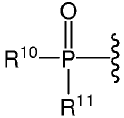 group wherein one of R¹⁰ and R¹¹ is 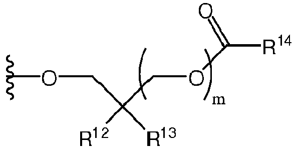 and the other R¹⁰ and R¹¹ is an -N-linked amino acid, diphenylphosphite can be reacted with one or more of the hydroxy precursors described herein, a nucleoside or nucleoside derivative (for example, 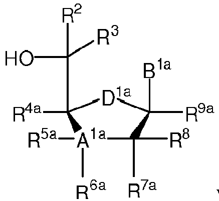 where R², R³ and R⁸ can be the same as previously defined herein, and R^{4a}, R^{5a}, R^{6a}, R^{7a}, R^{9a}, A^{1a}, B^{1a} and D^{1a} can be the same as R⁴, R⁵, R⁶, R⁷, R⁹, A¹, B¹ and D¹, respectively, or can be each a protected version of R⁴, R⁵, R⁶, R⁷, R⁹, A¹, B¹ and D¹, respectively), an amino acid, and a suitable oxidizing agent to form a compound of Formula (I). As previously discussed, various amino acids can be used, including those described herein. Likewise, any suitable oxidizing agent can be used. In an embodiment, the oxidizing agent can be carbon tetrachloride (CCl₄). In some embodiments, the oxidizing agent, such as CCl₄, oxidizes the phosphorus from (III) to (V).

Various methods can also be used to add a 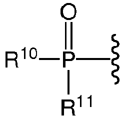 group wherein R¹⁰ and R¹¹ are 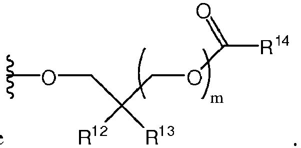 In some embodiments, diphenylphosphite can be reacted with one or more of the hydroxy precursors described herein, a nucleoside or nucleoside derivative (such as 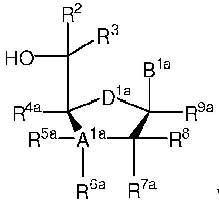 where R², R³ and R⁸ can be the same as previously defined herein, and R^{4a}, R^{5a}, R^{6a}, R^{7a}, R^{9a}, A^{1a}, B^{1a} and D^{1a} can be the same as R⁴, R⁵, R⁶, R⁷, R⁹, A¹, B¹ and D¹, respectively, or can be each a protected version of R⁴, R⁵, R⁶, R⁷, R⁹, A¹, B¹ and D¹, respectively) and a suitable oxidizing agent.

If desired and/or needed, one or more suitable protecting groups, including those described herein, can be used to protect the optionally substituted heterocyclic base, the optionally substituted heterocyclic base derivative, and/or any hydroxy groups presented on the 5-membered heterocyclic ring. For example, any hydroxy groups can be protected with individual protecting groups, as acetals and/or as ortho esters. Similarly, one or more amino groups attached to a ring and/or any -NH groups present in a ring of the optionally substituted heterocyclic base and/or optionally substituted heterocyclic base derivative can be protected with one or more suitable protecting groups, for example, one or more triarylmethyl protecting groups. As discussed herein, the protecting groups can be removed, replaced and exchanged at different times during the formation of a compound of Formula (I). For example, a variety of protecting groups can be used to protect the optionally substituted heterocyclic base and/or optionally substituted heterocyclic base derivative when the 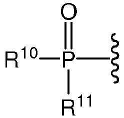 moiety is added to the 5'-position. Suitable protecting groups are known to those skilled in the art, including those described herein. The protecting groups present on the optionally substituted heterocyclic base and/or optionally substituted heterocyclic base derivative can be removed and other protecting groups can be added at different times during the addition of the phosphate groups. Likewise, any protecting groups present on the optionally substituted 5-membered heterocyclic ring can be removed and/or changed at different times during the addition of the 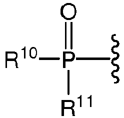 moiety. In some instances, removal and replacement of a protecting group may be useful because of the reactions conditions. The protecting groups can also assistant in preventing unwanted side reaction and/or make the separate of the desired product more facile.

In situations where the optionally substituted heterocyclic ring already has an optionally substituted C₁₋₆ alkyl at the 2'-position, the steps needed to add an optionally substituted C₁₋₆ alkyl at the 2'-position may be omitted.

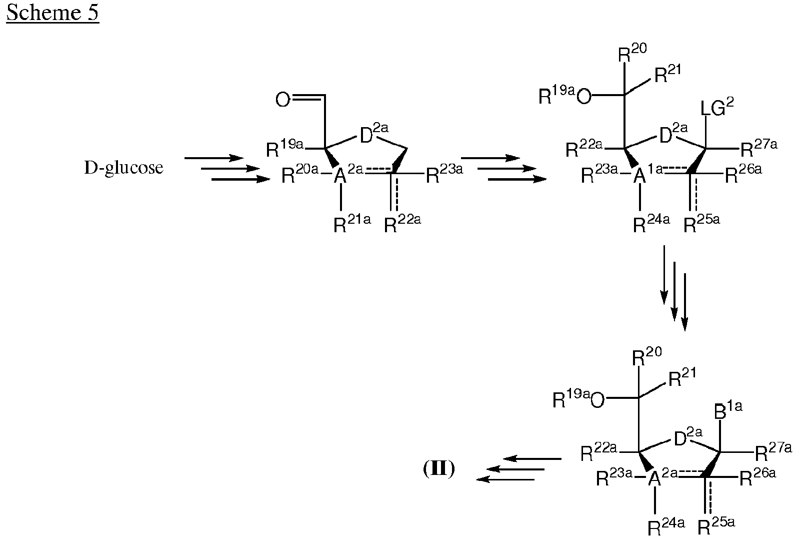

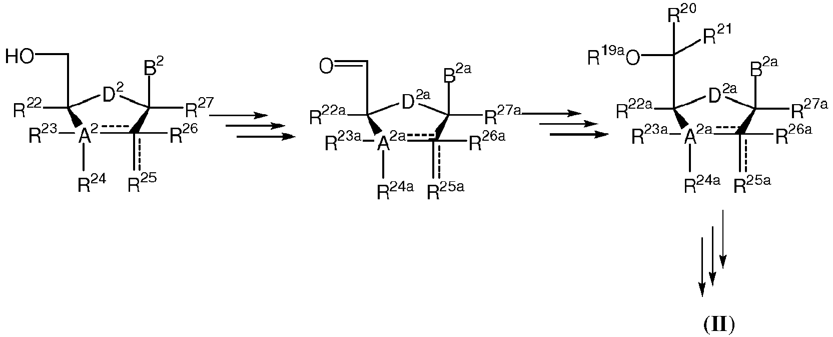

Compounds of Formula (II) can be formed using methods similar to those as described herein with respect to the preparation of compounds of Formula (I). As shown above in Scheme 5, an optionally substituted C₁₋₆ alkyl, an optionally substituted C₂₋₆alkenyl, an optionally substituted C₂₋₆ alkynyl or an optionally substituted C₁₋₆ haloalkyl can be added to the 5'-position after the 5'-position has been oxidized to aldehyde using one or more suitable reagents. The substituents R²², R²³, R²⁴, R²⁵, R²⁶, R²⁷, A², B² and D² can be the same as disclosed herein, and R^{22a}, R^{23a}, R^{24a}, R^{25a}, R^{26a}, R^{27a}, A^{2a}, B^{2a} and D^{2a} can be the same as R²², R²³, R²⁴, R²⁵, R²⁶, R²⁷, A², B² and D², respectively, or can be each a protected version of R²², R²³, R²⁴, R²⁵, R²⁶, R²⁷, A², B² and D², respectively. The substituent R^{19a} can be hydrogen or a protecting group, and LG² can be a suitable leaving group. Examples of suitable protecting groups include, but are not limited to, an optionally substituted benzoyl and silyl ethers such as trimethylsilyl (TMS), *tert*-butyldimethylsilyl (TBDMS), triisopropylsilyl (TIPS) and tert-butyldiphenylsilyl (TBDPS).

If an optionally substituted heterocyclic base or an optionally substituted heterocyclic base derivative is not already present on the 5-membered heterocyclic ring, the optionally substituted heterocyclic base or an optionally substituted heterocyclic base can added using methods known to those skilled in the art. For example, the substitutent at the 1'-position can be converted to an appropriate leaving group, for example a nucleofuge, using methods known to those skilled in the art. As an example, the 1'-position can be converted to an appropriate leaving group via an hydrolysis reaction followed by acetylation using a suitable reagent such as acetic anhydride. As another example, the 1'-position can be converted to an appropriate leaving group by transforming the acetal to a hemiacetal under acid conditions followed by acetylation with an appropriate reagent (e.g., acetic anhydride).

An optionally substituted heterocyclic base or an optionally substituted heterocyclic base derivative can be added to the 1'-position using a catalyst. Suitable catalysts are known in the art. In an embodiment, the catalysts can be trimethylsilyl trifluoromethanesulfonate. To facilitate the reaction, in some embodiments, the addition of the optionally substituted heterocyclic base or the optionally substituted heterocyclic base derivative can take place in the presence of a base. Examples of suitable bases include amine-based bases such as triethylamine, 1,8-diazabicyclo[5.4.0]undec-7-ene (DBU) and 1,5-diazabicyclo[4.3.0]non-5-ene (DBN). After addition of the optionally substituted heterocyclic base or the optionally substituted heterocyclic base derivative, a compound of Formula (II) in which R¹⁹ is H can be obtained after removal of any protecting groups that may be present.

A 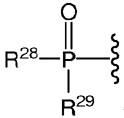 moiety can be added to the 5'-position using the same or similar methods for adding 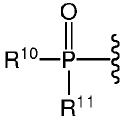 described herein. When one of R²⁸ and R²⁹ is an -N-linked amino acid, in some embodiments, the amino acid can have the structure, 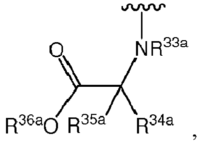 wherein R^{33a}, R^{34a}, R^{35a}, and R^{36a} can be the same as R³³, R³⁴, R³⁵ and R³⁶, as described herein with respect to Formula (II). In an embodiment, when one of R²⁸ and R²⁹ is 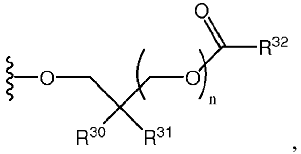 the hydroxy precursor can have the structure, 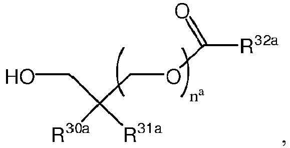 wherein R^{30a}, R^{31a}, R^{32a} and n^{a} are the same as R³⁰, R³¹, R³² and n, respectively, as described herein. Examples of suitable hydroxy precursors having the structure 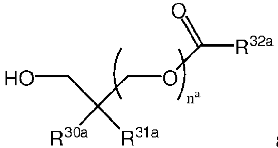 and method of obtaining the same are previously described herein.

If desired and/or needed, one or more suitable protecting groups, including those described herein, can be used to protect the optionally substituted heterocyclic base, the optionally substituted heterocyclic base derivative, and/or any hydroxy groups presented on the 5-membered heterocyclic ring during the synthesis of a compound of Formula (II). For example, any hydroxy groups can be protected with individual protecting groups, as acetals and/or as ortho esters. Similarly, one or more amino groups attached to a ring and/or any -NH groups present in a ring of the optionally substituted heterocyclic base and/or optionally substituted heterocyclic base derivative can be protected with one or more suitable protecting groups, for example, one or more triarylmethyl protecting groups. As discussed herein, the protecting groups can be removed, replaced and exchanged at different times during the formation of a compound of Formula (II), for example, during the addition of a 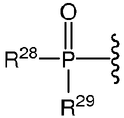 group.

### Pharmaceutical Compositions

An embodiment described herein relates to a pharmaceutical composition, that can include a therapeutically effective amount of one or more compounds described herein (e.g., a compound of Formula (I) and/or a compound of Formula (II)) and a pharmaceutically acceptable carrier, diluent, excipient or combination thereof.

The term "pharmaceutical composition" refers to a mixture of a compound disclosed herein with other chemical components, such as diluents or carriers. The pharmaceutical composition facilitates administration of the compound to an organism. Multiple techniques of administering a compound exist in the art including, but not limited to, oral, intramuscular, intraocular, intranasal, intravenous, injection, aerosol, parenteral, and topical administration. Pharmaceutical compositions can also be obtained by reacting compounds with inorganic or organic acids such as hydrochloric acid, hydrobromic acid, sulfuric acid, nitric acid, phosphoric acid, methanesulfonic acid, ethanesulfonic acid, p-toluenesulfonic acid, salicylic acid and the like. Pharmaceutical compositions will generally be tailored to the specific intended route of administration.

The term "physiologically acceptable" defines a carrier, diluent or excipient that does not abrogate the biological activity and properties of the compound.

As used herein, a "carrier" refers to a compound that facilitates the incorporation of a compound into cells or tissues. For example, without limitation, dimethyl sulfoxide (DMSO) is a commonly utilized carrier that facilitates the uptake of many organic compounds into cells or tissues of a subject.

As used herein, a "diluent" refers to an ingredient in a pharmaceutical composition that lacks pharmacological activity but may be pharmaceutically necessary or desirable. For example, a diluent may be used to increase the bulk of a potent drug whose mass is too small for manufacture or administration. It may also be a liquid for the dissolution of a drug to be administered by injection, ingestion or inhalation. A common form of diluent in the art is a buffered aqueous solution such as, without limitation, phosphate buffered saline that mimics the composition of human blood.

As used herein, an "excipient" refers to an inert substance that is added to a pharmaceutical composition to provide, without limitation, bulk, consistency, stability, binding ability, lubrication, disintegrating ability etc., to the composition. A "diluent" is a type of excipient.

The pharmaceutical compositions described herein can be administered to a human patient *per se,* or in pharmaceutical compositions where they are mixed with other active ingredients, as in combination therapy, or carriers, diluents, excipients or combinations thereof. Proper formulation is dependent upon the route of administration chosen. Techniques for formulation and administration of the compounds described herein are known to those skilled in the art.

The pharmaceutical compositions disclosed herein may be manufactured in a manner that is itself known, *e.g.,* by means of conventional mixing, dissolving, granulating, dragee-making, levigating, emulsifying, encapsulating, entrapping or tableting processes. Additionally, the active ingredients are contained in an amount effective to achieve its intended purpose. Many of the compounds used in the pharmaceutical combinations disclosed herein may be provided as salts with pharmaceutically compatible counterions.

Suitable routes of administration may, for example, include oral, rectal, topical transmucosal, or intestinal administration; parenteral delivery, including intramuscular, subcutaneous, intravenous, intramedullary injections, as well as intrathecal, direct intraventricular, intraperitoneal, intranasal, intraocular injections or as an aerosol inhalant.

One may also administer the compound in a local rather than systemic manner, for example, via injection of the compound directly into the infected area, often in a depot or sustained release formulation. Furthermore, one may administer the compound in a targeted drug delivery system, for example, in a liposome coated with a tissue-specific antibody. The liposomes will be targeted to and taken up selectively by the organ.

The compositions may, if desired, be presented in a pack or dispenser device which may contain one or more unit dosage forms containing the active ingredient. The pack may for example comprise metal or plastic foil, such as a blister pack. The pack or dispenser device may be accompanied by instructions for administration. The pack or dispenser may also be accompanied with a notice associated with the container in form prescribed by a governmental agency regulating the manufacture, use, or sale of pharmaceuticals, which notice is reflective of approval by the agency of the form of the drug for human or veterinary administration. Such notice, for example, may be the labeling approved by the U.S. Food and Drug Administration for prescription drugs, or the approved product insert. Compositions that include a compound disclosed herein formulated in a compatible pharmaceutical carrier may also be prepared, placed in an appropriate container, and labeled for treatment of an indicated condition.

### Methods of Use

One embodiment disclosed herein relates to a method of treating and/or ameliorating a disease or condition that can include administering to a subject a therapeutically effective amount of one or more compounds described herein, such as a compound of Formula (I) and/or a compound of Formula (II), or a pharmaceutical composition that includes a compound described herein.

Some embodiments disclosed herein relate to a method of ameliorating or treating a neoplastic disease that can include administering to a subject suffering from the neoplastic disease a therapeutically effective amount of one or more compounds described herein (e.g., a compound of Formula (I) and/or a compound of Formula (II)) or a pharmaceutical composition that includes one or more compounds described herein. In an embodiment, the neoplastic disease can be cancer. In some embodiments, the neoplastic disease can be a tumor such as a solid tumor. In an embodiment, the neoplastic disease can be leukemia. Examples of leukemias include, but are not limited to, acute lymphoblastic leukemia (ALL), acute myeloid leukemia (AML) and juvenile myelomonocytic leukemia (JMML).

An embodiment disclosed herein relates to a method of inhibiting the growth of a tumor that can include administering to a subject having the tumor a therapeutically effective amount of one or more compounds described herein or a pharmaceutical composition that includes one or more compounds described herein.

Other embodiments disclosed herein relates to a method of ameliorating or treating a viral infection that can include administering to a subject suffering from the viral infection a therapeutically effective amount of one or more compounds described herein or a pharmaceutical composition that includes one or more compounds described herein. In an embodiment, the viral infection can be caused by a virus selected from an adenovirus, an Alphaviridae, an Arbovirus, an Astrovirus, a Bunyaviridae, a Coronaviridae, a Filoviridae, a Flaviviridae, a Hepadnaviridae, a Herpesviridae, an Alphaherpesvirinae, a Betaherpesvirinae, a Gammaherpesvirinae, a Norwalk Virus, an Astroviridae, a Caliciviridae, an Orthomyxoviridae, a Paramyxoviridae, a Paramyxoviruses, a Rubulavirus, a Morbillivirus, a Papovaviridae, a Parvoviridae, a Picornaviridae, an Aphthoviridae, a Cardioviridae, an Enteroviridae, a Coxsackie virus, a Polio Virus, a Rhinoviridae, a Phycodnaviridae, a Poxviridae, a Reoviridae, a Rotavirus, a Retroviridae, an A-Type Retrovirus, an Immunodeficiency Virus, a Leukemia Viruses, an Avian Sarcoma Viruses, a Rhabdoviruses, a Rubiviridae and/or a Togaviridae. In an embodiment, the viral infection is a hepatitis C viral infection. In another embodiment, the viral infection is a HIV infection.

One embodiment disclosed herein relates to a method of ameliorating or treating a parasitic disease that can include administering to a subject suffering from the parasitic disease a therapeutically effective amount of one or more compounds described herein or a pharmaceutical composition that includes one or more compounds described herein. In an embodiment, the parasite disease can be Chagas' disease.

As used herein, a "subject" refers to an animal that is the object of treatment, observation or experiment. "Animal" includes cold- and warm-blooded vertebrates and invertebrates such as fish, shellfish, reptiles and, in particular, mammals. "Mammal" includes, without limitation, mice, rats, rabbits, guinea pigs, dogs, cats, sheep, goats, cows, horses, primates, such as monkeys, chimpanzees, and apes, and, in particular, humans.

As used herein, the terms "treating," "treatment," "therapeutic," or "therapy" do not necessarily mean total cure or abolition of the disease or condition. Any alleviation of any undesired signs or symptoms of a disease or condition, to any extent can be considered treatment and/or therapy. Furthermore, treatment may include acts that may worsen the patient's overall feeling of well-being or appearance.

The term "therapeutically effective amount" is used to indicate an amount of an active compound, or pharmaceutical agent, that elicits the biological or medicinal response indicated. For example, a therapeutically effective amount of compound can be the amount need to prevent, alleviate or ameliorate symptoms of disease or prolong the survival of the subject being treated This response may occur in a tissue, system, animal or human and includes alleviation of the symptoms of the disease being treated. Determination of a therapeutically effective amount is well within the capability of those skilled in the art, especially in light of the detailed disclosure provided herein. The therapeutically effective amount of the compounds disclosed herein required as a dose will depend on the route of administration, the type of animal, including human, being treated, and the physical characteristics of the specific animal under consideration. The dose can be tailored to achieve a desired effect, but will depend on such factors as weight, diet, concurrent medication and other factors which those skilled in the medical arts will recognize.

As will be readily apparent to one skilled in the art, the useful *in vivo* dosage to be administered and the particular mode of administration will vary depending upon the age, weight, the severity of the affliction, and mammalian species treated, the particular compounds employed, and the specific use for which these compounds are employed. (See *e.g.,* Fingl et al. 1975, in "The Pharmacological Basis of Therapeutics", which is hereby incorporated herein by reference in its entirety, with particular reference to Ch. 1, p. 1). The determination of effective dosage levels, that is the dosage levels necessary to achieve the desired result, can be accomplished by one skilled in the art using routine pharmacological methods. Typically, human clinical applications of products are commenced at lower dosage levels, with dosage level being increased until the desired effect is achieved. Alternatively, acceptable *in vitro* studies can be used to establish useful doses and routes of administration of the compositions identified by the present methods using established pharmacological methods.

Although the exact dosage will be determined on a drug-by-drug basis, in most cases, some generalizations regarding the dosage can be made. The daily dosage regimen for an adult human patient may be, for example, an oral dose of between 0.01 mg and 3000 mg of each active ingredient, preferably between 1 mg and 700 mg, e.g. 5 to 200 mg. The dosage may be a single one or a series of two or more given in the course of one or more days, as is needed by the patient. In some embodiments, the compounds will be administered for a period of continuous therapy, for example for a week or more, or for months or years.

In instances where human dosages for compounds have been established for at least some condition, those same dosages, or dosages that are between about 0.1% and 500%, more preferably between about 25% and 250% of the established human dosage will be used. Where no human dosage is established, as will be the case for newly-discovered pharmaceutical compositions, a suitable human dosage can be inferred from ED₅₀ or ID₅₀ values, or other appropriate values derived from *in vitro* or *in vivo* studies, as qualified by toxicity studies and efficacy studies in animals.

In cases of administration of a pharmaceutically acceptable salt, dosages may be calculated as the free base. As will be understood by those of skill in the art, in certain situations it may be necessary to administer the compounds disclosed herein in amounts that exceed, or even far exceed, the above-stated, preferred dosage range in order to effectively and aggressively treat particularly aggressive diseases or infections.

Dosage amount and interval may be adjusted individually to provide plasma levels of the active moiety which are sufficient to maintain the modulating effects, or minimal effective concentration (MEC). The MEC will vary for each compound but can be estimated from in vitro data. Dosages necessary to achieve the MEC will depend on individual characteristics and route of administration. However, HPLC assays or bioassays can be used to determine plasma concentrations.

Dosage intervals can also be determined using MEC value. Compositions should be administered using a regimen which maintains plasma levels above the MEC for 10-90% of the time, preferably between 30-90% and most preferably between 50-90%. In cases of local administration or selective uptake, the effective local concentration of the drug may not be related to plasma concentration.

It should be noted that the attending physician would know how to and when to terminate, interrupt, or adjust administration due to toxicity or organ dysfunctions. Conversely, the attending physician would also know to adjust treatment to higher levels if the clinical response were not adequate (precluding toxicity). The magnitude of an administrated dose in the management of the disorder of interest will vary with the severity of the condition to be treated and to the route of administration. The severity of the condition may, for example, be evaluated, in part, by standard prognostic evaluation methods. Further, the dose and perhaps dose frequency, will also vary according to the age, body weight, and response of the individual patient. A program comparable to that discussed above may be used in veterinary medicine.

In non-human animal studies, applications of potential products are commenced at higher dosage levels, with dosage being decreased until the desired effect is no longer achieved or adverse side effects disappear. The dosage may range broadly, depending upon the desired effects and the therapeutic indication. Alternatively dosages may be based and calculated upon the surface area of the patient, as understood by those of skill in the art.

Compounds disclosed herein can be evaluated for efficacy and toxicity using known methods. For example, the toxicology of a particular compound, or of a subset of the compounds, sharing certain chemical moieties, may be established by determining *in vitro* toxicity towards a cell line, such as a mammalian, and preferably human, cell line. The results of such studies are often predictive of toxicity in animals, such as mammals, or more specifically, humans. Alternatively, the toxicity of particular compounds in an animal model, such as mice, rats, rabbits, or monkeys, may be determined using known methods. The efficacy of a particular compound may be established using several recognized methods, such as *in vitro* methods, animal models, or human clinical trials. Recognized *in vitro* models exist for nearly every class of condition, including but not limited to cancer, cardiovascular disease, and various immune dysfunction. Similarly, acceptable animal models may be used to establish efficacy of chemicals to treat such conditions. When selecting a model to determine efficacy, the skilled artisan can be guided by the state of the art to choose an appropriate model, dose, and route of administration, and regime. Of course, human clinical trials can also be used to determine the efficacy of a compound in humans.

### EXAMPLES

Additional embodiments are disclosed in further detail in the following examples, which are not in any way intended to limit the scope of the claims.

### EXAMPLES

Additional embodiments are disclosed in further detail in the following examples, which are not in any way intended to limit the scope of the claims.

### Example 1

### 1-METHYL 3-ACETOXY-2-CYANO-2-(HYDROXYMETHYL)PROPANOATE (1)

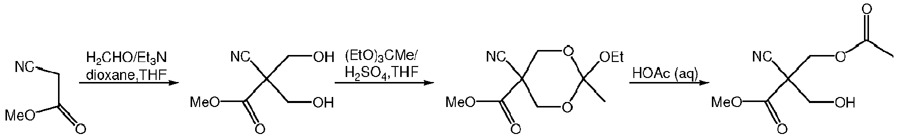

*Methyl 2-cyano-3-hydroxy-2-hydroxymethylpropanoate.* Formaldehyde (66.7 mmol, 2.0 g) was added as 20% aq solution (10 g) to 1,4-dioxane (30 mL) on an ice-bath. Methyl cyanoacetate (30.3 mmol, 2.12 mL) and Et₃N (0.61 mmol, 0.61 mL of 1 mol L⁻¹ solution in THF) were added and the mixture was stirred for 20 min. Another portion of Et₃N (0.61 mmol) was added and the ice-bath was removed. The mixture was stirred for 1.5 h at room temperature. The mixture was then diluted with water (200 mL) and extracted with benzene (3 x 50 mL) to remove side products. The aqueous phase was evaporated under reduced pressure at 30 °C to one fourth of the original volume and extracted 5 times with ethyl acetate. The combined extracts were dried over Na₂SO₄ and evaporated to a clear oil. The yield was 72% (4.82 g). The compound was used without characterization to the next step.

*Methyl 5-cyano-2-ethoxy-2-methyl-1,3-dioxane-5-carboxylate.* Methyl 2-cyano-3-hydroxy-2-hydroxymethylpropanoate (23.3 mmol, 3.7 g) was dissolved in dry THF (8 mL) and triethyl orthoacetate (34.9 mmol, 6.55 mL) was added. A catalytic amount of concentrated sulfuric acid (0.70 mmol, 37 µL) was added and the mixture was stirred over night at room temperature. The mixture was poured into a stirred ice-cold aq. NaHCO₃ (5%, 50 mL). The product was extracted into Et₂O (2 × 50 mL) and the extracts were washed with saturated aq. NaCl and dried over Na₂SO₄. The solvent was evaporated and purified by Silica gel chromatography applying a stepwise gradient from 5% ethyl acetate in dichloromethane to pure ethyl acetate. The product was obtained in 42% yield (5.33 g) as a clear oil that started to crystallize. ¹H NMR for the major diastereomer (CDCl₃) 4.34 (d, *J* = 7.0 Hz, 2H, -CH₂O-), 4.03 (d, *J =* 8.5 Hz, 2H, -CH₂O-), 3.84 (s, 3H, OMe), 3.54 (q, *J* = 7.2 Hz, 2H, -C*H*₂CH₃), 1.55 (s, 3H, -CH₃), 1.25 (t, *J* = 7.2, 3H, - CH₂C*H*₃). ¹³C NMR for the major diastereomer (CDCl₃) 164.8 (C=O), 117.0 (CN), 111.4 (C2), 62.3 (C4 and C6), 59.1 (-CH₂CH₃), 53.9 (-OCH₃), 42.4 (C5), 22.3 (2-CH₃), 15.0 (CH₂CH₃).

*Methyl 3-acetyloxy-2-cyano-2-(hydroxyrycethyl)propanoate.* Methyl 5-cyano-2-ethoxy-2-methyl-1,3-dioxane-5-carboxylate (2.18 mmol, 0.50 g) was dissolved in a mixture of acetic acid and water (4:1, v/v, 20 mL) and the mixture was stirred for 2 h at room temperature, after which the mixture was evaporated to dryness and the residue was coevaporated 3 times with water. The product was purified by Silica gel chromatography, eluting with dichloromethane containing 5% MeOH. The yield was 52% (0.23 g). ¹H NMR (CDCl₃) 4.53 (d, *J =* 11.0 Hz, 1H, -C*H*₂OA_{C}), 4.50 (d, *J =* 11.0 Hz, 1H, -CH₂OAc), 4.04 (d, *J* = 6.5 Hz, 2H, -C*H*₂OH), 3.91 (s, 3H, -OMe), 2.90 (t, *J =* 6.5 Hz, -O*H*), 2.16 (s, 3H, -C(O)C*H*₃). ¹³C NMR (CDCl₃) 170.4 (*C*=O), 166.0 (C=O), 116.0 (*C*N), 63.1 (-*C*H₂OH), 62.3 (-*C*H₂OAc), 54.1 (-OMe), 51.0 (C2), 20.6 (-C(O)*C*H₃).

### Example 2

### 2-CYANO-3-(ETHYLAMINO)-2-(HYDROXYMETHYL)-3-OXOPROPYL ACETATE (2)

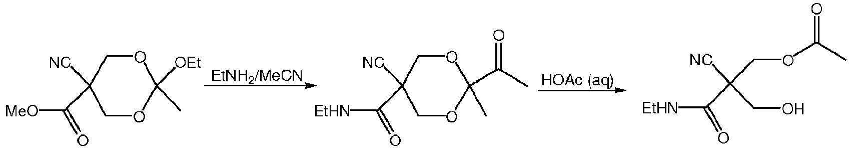

### 2-CYANO-3-(2-PHENYLETHYLAMINO)-2-(HYDROXYMETHYL)-3-OXOPROPYL ACETATE(2b)

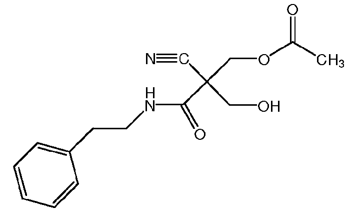

2-cyano-3-(2-phenylethylamino)-2-(hydroxymethyl)-3-oxopropyl acetate was prepared according to the procedure described in Poijärvi, P.; Mäki, E.; Tomperi, J.; Ora, M.; Oivanen, M.; Lönnberg, H., Helve. Chim. Acta. 2002 85:1869-1876, which is hereby incorporated by reference for the limited purpose of describing the method of synthesizing and purifying 2-cyano-3-(2-phenylethylamino)-2-(hydroxymethyl)-3-oxopropyl acetate.

### Example 3

### 2-ACETYL-2-(HYDROXYMETHYL)-3-OXOBUTYL ACETATE (3)

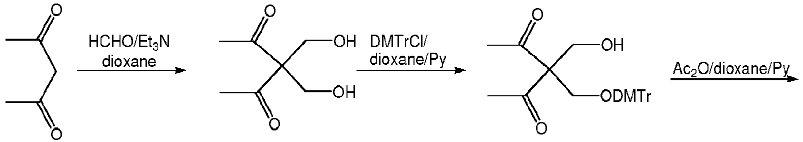

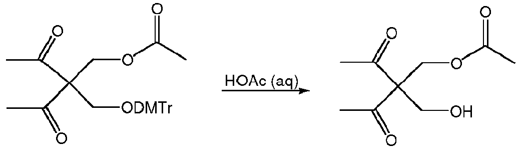

### Example 4

### 2-ACETYL-2-(HYDROXYMETHYL)-3-OXOBUTYL PIVALATE (4)

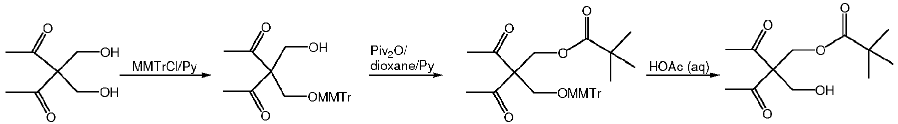

### Example 5

### 2-ACETYL-2-HYDROXYMETHYL-3-OXOBUTYL ACETATE (5)

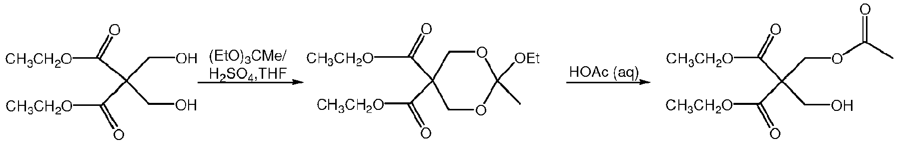

*Diethyl 2-ethoxy-2-methyl-1,3-dioxane-5,5-dicarboxylate.* Concentrated H₂SO₄ (1.3 mmol; 71 µL) was added to a mixture of diethyl 2,2-bis(hydroxymethyl)malonate (43.5 mmol, 9.6 g) and triethyl orthoacetate (65.2 mmol; 11.9 mL) in dry THF (15 mL). The reaction was allowed to proceed overnight and the mixture was the poured into an ice-cold solution of 5 % NaHCO₃ (50 mL). The product was extracted with diethyl ether (2 × 50 mL), washed with saturated aqueous NaCl (2 × 50 mL) and dried over Na₂SO₄. The solvent was evaporated and the crude product was purified on a silica gel column eluting with a mixture of dichloromethane and methanol (95:5, *v*/*v*). The product was obtained as clear oil in 89% yield (11.3 g). ¹H NMR *δ*_{H} (500 MHz, CDCl₃): 4.30-4.36 (m, 6H, 4-C*H*₂, 6-C*H*₂ and 5-COOC*H*₂Me), 4.18 (q, *J* = 7.1 Hz, 5-COOC*H*₂Me), 3.54 (q, *J* = 7.10 Hz, 2H, 2-OC*H*₂Me), 1.46 (s, 3H, 2-C*H₃*), 1.32 (t, *J* = 7.10 Hz, 3H, 2-OC*H*₂Me), 1.27 (t, *J* = 7.1 Hz 3H, 5-COOC*H*₂Me), 1.26 (t, *J* = 7.1 Hz 3H, 5-COOC*H*₂Me). ¹³C NMR (500 MHz, CDCl₃): *δ* = 168.0 and 167.0 (5-COOEt), 111.1 (C2), 62.0 and 61.9 (5-COOCH₂Me), 61.6 (C4 and C6), 58.7 (2-OCH₂Me), 52.3 (C5), 22.5 (2-Me), 15.1 (2-OCH₂CH₃), 14.0 and 13.9 (5-COOCH₂CH₃).

*Diethyl 2-(acetyloxymethyl)-2-(hydroxymethyl)malonate.* Diethyl 2-ethoxy-2-methyl-1,3-dioxane-5,5-dicarboxylate (17.9 mmol; 5.2 g) was dissolved in 80% aqueous acetic acid (30 mL) and left for 2h at room temperature. The solution was evaporated to dryness and the residue was coevaporated three times with water. The product was purified by silica gel column chromatography eluting with ethyl acetate in dichloromethane (8:92, *v*/*v*). The product was obtained as yellowish oil in 75% yield (3.6 g). ¹H NMR *δ*_{H} (500 MHz, CDCl₃): 4.76 (s, 2H, C*H*₂OAc), 4.26 (q, *J* = 7.10 Hz, 4H, OC*H*₂Me), 4.05 (d, *J* = 7.10 Hz, 2H, C*H*₂OH), 2.72 (t, *J* = 7.1 Hz, 1H, CH₂*OH*), 2.08 (s, 3H, Ac), 1.27 (t, *J* = 7.10 Hz, 6H, OCH₂CH₃). ¹³C NMR (500 MHz, CDCl₃): *δ* = 170.9 (C=O Ac), 168.1 (2xC=O malonate), 62.3 and 62.2 (CH₂OH and CH₂OAc), 61.9 (2×OCH₂CH₃) 59.6 (spiro C), 20.7 (CH₃ Ac), 14.0 (2×OCH₂CH₃).

### Example 6

### 2,2-BIS(ETHOXYCARBONYL)-3-HYDROXYPROPYL PIVALATE (6)

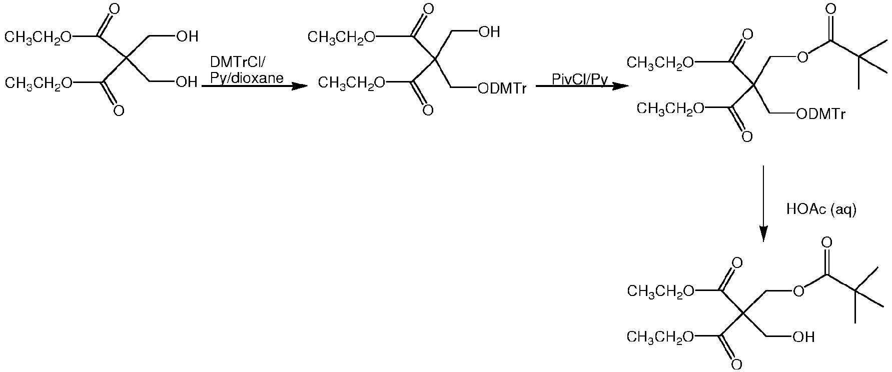

*2,2-Bis(ethoxycarbonyl)-3-(4,4'-dimethoxytrityloxy)propyl pivalate.* Diethyl 2,2-bis(hydroxymethyl)malonate was reacted with 1 equiv. of 4,4'-dimethoxytrityl chloride in 1,4-dioxane containing 1 equivalent of pyridine. Diethyl 2-(4,4'-dimethoxytrityloxymethyl)-2-(hydroxymethyl)malonate (2.35 g, 4.50 mmol) was acylated with pivaloyl chloride (0.83 mL, 6.75 mmol) in dry MeCN (10 mL) containing 3 equivalent pyridine (1.09 mL, 13.5 mmol). After 3 days at room temperature, the reaction was quenched with MeOH (20 mL) and a conventional CH₂Cl₂/aq HCO₃⁻ workup was carried out. Silica gel chromatography (EtOAc/hexane 1:1, *v*/*v*) gave 2.47 g (90 %) of the desired product as yellowish syrup. ¹H NMR (CDCl₃, 200 MHz): 7.13-7.39 [m, 9H, (MeO)₂ *Tr];* 6.81 (d, 4H, [MeO]₂ *Tr*); 4.71 (s, 2H, C*H*₂OPiv); 4.15 (q, *J=* 7.1, 4H, OC*H*₂CH₃); 3.78 [s, 6H, (C*H*₃O)₂Tr]; 3.67 (s, 2H, C*H*₂ODMTr); 1.27 (t, *J* = 7.1, 6H, OCH₂C*H*₃); 1.02 [s, 9H, COC(C*H*₃)₃].

*2,2-Bis(ethoxycarbonyl)-3-hydroxypropyl pivalate. 2,2-*Bis(ethoxycarbonyl)-3-(4,4'-dimethoxytrityloxy)propyl pivalate (2.47 g, 4.07 mmol) in a 4:1 mixture of CH₂Cl₂ and MeOH (20 mL) was treated for 4 h at room temperature with TFA (2.00 mL, 26.0 mmol) to remove the dimethoxytrityl group. The mixture was neutralized with pyridine (2.30 mL, 28.6 mmol), subjected to CH₂Cl₂/aq workup and purified by silica gel chromatography (EtOAc/hexane 3:7, v/v) to obtain 1.15 g (93 %) of the desired product. ¹H NMR (CDCl₃, 200 MHz): 4.59 (s, 2H, C*H*₂OPiv); 4.25 (q, *J* = 7.1, 4H, OC*H*₂CH₃); 4.01 (s, 2H, C*H*₂OH); 1.28 (t, *J* = 7.1, 6H, OCH₂C*H*₃); 1.18 [s, 9H, COC(C*H*₃)₃]. ESI- MS⁺: *m*/*z* 305.4 ([MH]⁺), 322.6 ([MNH₄]⁺), 327.6 ([MNa]⁺), 343.5 ([MK]⁺).

### Example 7

### DIETHYL 2-ACETYLOXYMETHYL-2-HYDROXYMETHYLMALONATE (7)

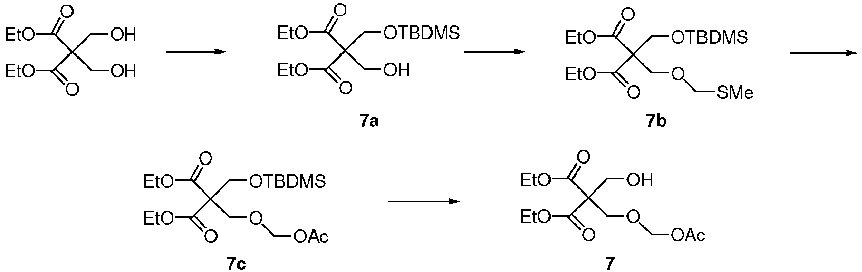

*Diethyl 2-(tert-butyldimethylsilyloxymethyl)-2-hydroxymethylmalonate* (7a). Diethyl 2,2-bis(hydroxymethyl)malonate (28.3 mmol; 6.23 g) was coevaporated twice from dry pyridine and dissolved in the same solvent (20 mL). *tert-*Butyldimethylsilyl chloride (25.5 mmol; 3.85 g) in dry pyridine (10 mL) was added portionwise. The reaction was allowed to proceed for 4 days. The mixture was evaporated to a solid foam, which was then equilibrated between water (200 mL) and DCM (4x100 mL). The organic phase was dried on Na₂SO₄. The product was purified by silica gel chromatography eluting with 10% ethyl acetate in DCM. The yield was 78%. ¹H NMR (CDCl₃) δ 4.18-4.25 (m, 4H, OC*H*₂Me), 4.10 (s, 2H, C*H*₂OSi), 4.06 (s, 2H, C*H*₂OH), 2.63 (br s, 1H, OH), 1.26 (t, *J* = 7.0 Hz, 6H, OCH₂C*H*₃), 0.85 (s, 9H, Si-SMe₃), 0.05 (s, 6H, Me-Si). ¹³C NMR (CDCl₃) δ 169.2 (C=O), 63.3 (CH₂OH), 62.8 (CH₂OSi), 61.6 (spiro C), 61.4 (OCH₂Me), 25.6 [C(CH₃)₃], 18.0 (Si-CMe₃), 14.0 (OCH₂CH₃), -3.6 (Si-*C*H₃). MS [M + H]⁺ obsd. 335.7, calcd. 335.2; [M + Na] obsd. 357.6, calcd. 357.2.

*Diethyl 2-(tert-butyldimethylsilyloxymethyl)-2-methylthiomethylmalonate* **(7b).** Compound **7a** (19.7 mmol; 6.59 g) was dissolved into a mixture of acetic anhydride (40 mL), acetic acid (12.5 mL) and DMSO (61 mL) and the mixture was stirred overnight. The reaction was stopped by dilution with cold aq. Na₂CO₃ (290 ml 10% aq. solution) and the product was extracted in diethyl ether (4x120 mL). The combined organic phase was dried on Na₂SO₄. The product was purified by silica gel chromatography using DCM as an eluent. The yield was 91%. ¹H NMR (CDCl₃) δ 4.61 (s, 2H, OC*H*₂S), 4.14-4.19 (m, 4H, OC*H*₂Me), 4.06 (s, 2H, C*H*₂OSi), 4.00 (s, 2H, C*H*₂OCH₂SMe), 2.06 (SC*H*₃), 1.22 (t, *J* = 7.0 Hz, 6H, OCH₂C*H*₃), 0.83 (s, 9H, Si-SMe₃), 0.02 (s, 6H, Me-Si). ¹³C NMR (CDCl₃) δ 168.3 (C=O), 75.6 (*C*H₂S), 65.7 (CH₂OCH₂SMe), 61.4 (CH₂OSi), 61.2 (spiro C), 60.9 (OCH₂Me), 25.6 [C(CH₃)₃], 18.0 (Si-*C*Me₃), 14.0 (OCH₂CH₃), 13.7 (S*C*H₃), -3.6 (Si-CH₃). MS [M + H]⁺ obsd. 395.4, calcd. 395.2; [M + Na]⁺ obsd. 417.6, calcd. 417.2.

*Diethyl 2-acetyloxymethyl-2-(tert-butyldimethylsilyloxymethyl)malonate* **(7c).** Compound **7b** (17.9 mmol; 7.08 g) was dissolved in dry DCM (96 mL) under nitrogen. Sulfurylchloride (21.5 mmol; 1.74 mL of 1.0 mol L⁻¹ solution in DCM) was added in three portions and the mixture was stirred for 70 min under nitrogen. The solvent was removed under reduced pressure and the residue was dissolved into dry DCM (53 mL). Potassium acetate (30.9 mmol; 3.03 g) and dibenzo-18-crown-6 (13.5 mmol; 4.85 g) in DCM (50 mL) were added and the mixture was stirred for one hour and a half. Ethyl acetate (140 mL) was added, the organic phase was washed with water (2x190 mL) and dried on Na₂SO₄. The product was purified by silica gel chromatography using DCM as an eluent. The yield was 71%. ¹H NMR (CDCl₃) δ 5.24 (s, 2H, OC*H*₂O), 4.15-4.22 (m, 4H, OC*H*₂Me), 4.13 (s, 2H, C*H*₂OSi), 4.08 (s, 2H, C*H*₂OAc), 2.08 (Ac), 1.26 (t, *J* = 8.0 Hz, 6H, OCH₂C*H*₃), 0.85 (s, 9H, Si-SMe₃), 0.04 (s, 6H, Me-Si). ¹³C NMR (CDCl₃) δ 170.2 (Ac), 168.0 (C=O), 89.3 (O*C*H₂O), 67.5 (CH₂OAc), 61.4 (O*C*H₂Me), 61.1 (*C*H₂OSi), 60.2 (spiro C), 25.6 [C(*C*H₃)₃], 21.0 (Ac), 18.1 (Si-*C*Me₃), 14.0 (OCH₂CH₃), -5.7 (Si-CH₃). MS [M + Na]⁺ obsd. 429.6, calcd. 429.2.

*Diethyl 2-acetyloxymethyl-2-hydroxymethylmalonate* (7). Compound 7c (7.2 mmol; 2.93 g) was dissolved in dry THF (23 mL) and triethylamine trihydrogenfluoride (8.64 mmol; 1.42 mL) was added. The mixture was stirred for one week. Aq. triethylammonium acetate (13 mL of 2.0 mol L⁻¹ solution) was added. The mixture was evaporated to dryness and the residue was purified by silica gel chromatography using DCM containing 2-5% MeOH as an eluent. The yield was 74%. ¹H NMR (CDCl₃) δ 5.25 (s, 2H, OC*H*₂O), 4.16-4.29 (m, 6H, OC*H*₂Me and C*H*₂OAc), 4.13 (s, 2H, C*H*₂OH), 2.10 (Ac), 1.81 (br s, 1H, OH), 1.26 (t, *J* = 9.0 Hz, 6H, OCH₂C*H*₃). MS [M + Na]⁺ obsd. 315.3, calcd. 315.1.

### Example 8

### 3'-O-LEVULINOYL-N⁴-(4-METHOXYTRITYL)-2'-O-METHYLCYTIDINE (8e)

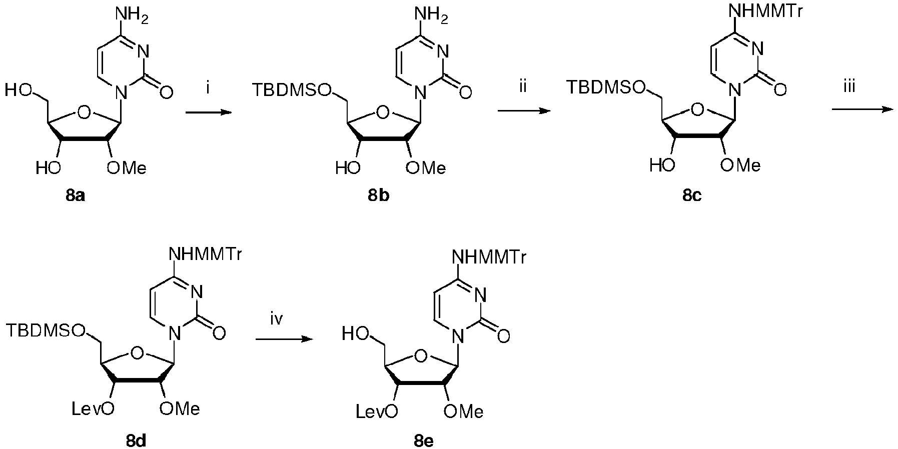

*5'-O-(tert-Butyldimethylsilyl)-2'-O-methylcytidine* **(8b).** 2'-*O-*methylcytidine **(8a;** 18.4 mmol; 4.74 g) was coevaporated twice from dry pyridine, dried over P₂O₅ (24 h) and dissolved in dry pyridine (20 mL). tert-Butyldimethylsilyl chloride (TBDMSCl; 20.2 mmol; 3.05 g) was added and the mixture was agitated at room temperature overnight. The unreacted TBDMSCl was quenched with MeOH, the mixture was evaporated to dryness and the residue was subjected to chloroform/aq. NaHCO₃ work-up. The yield of the crude product dried on Na₂SO₄ was nearly quantitative. It was used for 4-methoxytritylation of the amino group without further purification. ¹H NMR (CDCl₃): δ 8.14 (d, *J* = 7.5 Hz, 1H, H6), 6.00 (d, *J* = 1.1 Hz; 1H, H1'), 6.82 (d, *J* = 7.5 Hz, 1H, H5), 4.22 (dd, *J* = 8.0 and 5.1 Hz, 1H, H3'), 4.09 (dd, *J* = 11.8 and 1.8 Hz, 1H, H5'), 3.97 (m, 1H, H4'), 3.87 (dd, *J* = 11.8 and 1.6, 1H, H5"), 3.73 (dd, *J* = 5.1 and 1.0 Hz, 1H, H2'), 3.67 (s, 3H, 2'-OMe), 0.94 (s, 9H, Me₃C-Si), 0.13 (s, 3H, Me-Si), 0.13 (s, 3H, Me-Si).

*5'-O-(tert-Butyldimethylsityl)-N⁴-(4-methoxytrityl)-2'-O-methylcytidine* (8c). Compound **8b** (18.4 mmol; 6.84 g) was coevaporated twice from dry pyridine and dissolved in the same solvent (20 mL). 4-Methoxytrityl chloride (18.4 mmol; 5.69 g) was added and the mixture was agitated at 45 ^{c}o for 24 h. MeOH (20 mL) was added, the mixture was evaporated to dryness and the residue was subjected to chloroform/aq. NaHCO₃ work-up. Silica gel chromatography with DCM containing 2-5 % MeOH gave compound **8c** as a solid foam in 46% overall yield starting from 2'-O-methylcytidine. ¹H NMR (CDCl₃) δ 7.91 (d, *J =* 7.7 Hz, 1H, H6), 7.26-7.33 (m, 6H, MMTr), 7.21-7.23 (m, 4H, MMTr), 7.13-7.15 (m, 2H, MMTr), 6.82-6.85 (m, 2H, MMTr), 6.77 (br. s, 1H, NH), 5.99 (s, 1H, H1'), 5.00 (d, *J* = 7.7 Hz, 1H, H5), 4.12 (m, 1H, H3'), 4.02 (dd, *J* = 11.9 and 1.2 Hz, 1H, H5'), 3.86-3.88 (m, 1H, H4'), 3.81 (dd, *J* = 11.9 and 1.2 Hz, 1H, H5"), 3.81 (s, 3H, MeO-MMTr), 3.72-3.74 (m, 4H, H2' and 2'-OMe), 2.63 (br s, 1H, 3'-OH), 0.75 (s, 9H, Me₃C-Si), -0.03 (s, 3H, Me-Si), -0.05 (s, 3H, Me-Si). ¹³C NMR (CDCl₃) δ 165.6 (C4), 158.7 (MMTr), 155.1 (C2), 144.4 (MMTr), 144,3 (MMTr), 140.9 (C6), 136.0 (MMTr), 130.0 (MMTr), 128.6 (MMTr), 128.3 (MMTr), 127.5 (MMTr), 113.6 (MMTr), 94.2 (C5), 87.6 (C1') 83.9 (C2'), 83,7 (C4'), 70.5 (MMTr), 66.8 (C3'), 60.5 (C5'), 58.8 (2'-OMe), 55.2 (MMTr), 25.8 (TBDMS), 18.3 (TBDMS), -5.6 (TBDMS), -5.7 (TBDMS).

*5'-O-(tert-Butyldimethylsityl)-3'-O-levulinoyl-N⁴-(4-methoxytrityl)-2'-O-methylcytidine* **(8d).** Levulinic acid (21.6 mmol; 2.51 g) was dissolved in dry dioxane and dicyclohexylcarbodiimide (11.1 mmol; 2.28 g) was added portionwise during 1 h at 0 °C. The mixture was allowed to warm up to reduce its viscosity and it was then filtrated to a solution of compound **8c** (8.46 mmol; 5.45 g) in pyridine (18 mL). The mixture was agitated overnight, evaporated to dryness and the residue was subjected to DCM/NaHCO₃ work-up. The organic phase was dried on Na₂SO₄, evaporated to dryness and the residue was purified by Silica gel chromatography using DCM containing 1% MeOH as an eluent. Yield 86%. ¹H NMR (CDCl₃) δ 7.81 (d, *J* = 7.7 Hz, 1H, H6), 7.27-7.34 (m, 6H, MMTr), 7.22-7.23 (m, 4, MMTr), 7.14-7.15 (m, 2H, MMTr), 6.84-6.86 (m, 2H, MMTr), 6.80 (br. s, 1H, NH), 6.07 (d, *J =* 1.5 Hz, 1H, H1'), 4.99 (d, *J=* 7.7 Hz, 1H, H5), 4.97 (dd, *J* = 7.9 and 5.0 Hz, 1H, H3'), 4.21 (m, 1H, H2'), 3.99-4.01 (m, 2H, H4' and H5'), 3.81 (s, 3H, MeO-MMTr), 3.70 (dd, *J* = 12.0 and 1.3 Hz, 1H, H5"), 3.57 (s, 3H, 2'-OMe), 2.63-2.83 (m, 4H, Lev), 2.21 (s, 3H, Lev), 0.74 (s, 9H, Me₃C-Si), -0.05 (s, 3H, Me-Si), -0.07 (s, 3H, Me-Si). ¹³C NMR (CDCl₃) δ 206.1 (Lev), 172.0 (Lev), 165.5 (C4), 158.7 (MMTr), 155.1 (C2), 144.4 (MMTr), 144,3 (MMTr), 140.7 (C6), 136.0 (MMTr), 130.0 (MMTr), 128.6 (MMTr), 128.3 (MMTr), 127.5 (MMTr), 113.6 (MMTr), 94.4 (C5), 88.4 (C1'), 82.5 (C2'), 81,3 (C4'), 70.6 (MMTr), 69.1 (C3'), 60.8 (C5'), 58.9 (2'-OMe), 55.2 (MMTr), 37.8 (Lev), 29.8 (Lev), 27.8 (Lev), 25.7 (TBDMS), 18.2 (TBDMS), -5.7 (TBDMS), -5.8 (TBDMS).

*3'-O-Levulinoyl-N⁴-(4-methoxytrityl)-2'-O-methylcytidine* **(8e).** Compound 8d (3.40 mmol; 2.52 g) was dissolved into a mixture THF (48 mL) and AcOH (9 mL) containing tetrabutylammonium fluoride (6.85 mmol; 1.79 g). The mixture was agitated for 2 days and then evaporated to dryness. The residue was dissolved into EtOAc (50 mL), washed with water, aq. NaHCO₃ and brine, and dried on Na₂SO₄. The compound **8e** was obtained as a white foam in virtually quantitative yield. ¹H NMR (CDCl₃) δ 7.22-7.34 (m, 11H, H6 and MMTr), 7.12-7.15 (m, 2H, MMTr), 6.89 (br. s, 1H, NH), 6.83-6.85 (m, 2H, MMTr), 5.41 (d, *J* = 5.0 Hz, 1H, H1'), 5.31 (dd, *J* = 4.6 and 4.7, 1H, H4'), 5.07 (d, *J* = 7.6 Hz, 1H, H5), 4.58 (dd, *J* = 5.0 and 5.0 Hz, 1H, H3'), 4.18 (m, 1H, H2'), 3.90 (d, *J* = 12.7 Hz, 1H, H5'), 3.81 (s, 3H, MeO-MMTr), 3.71 (dd, *J* = 12.7 and 4.7 Hz, 1H, H5"), 3.45 (s, 3H, 2'-OMe), 2.75-2.80 (m, 2H, Lev), 2.63-2.66 (m, 2H, lev), 2.20 (s, 3H, Lev).

### 2'-O-METHYLCYTIDINE 5'-[O-PHENYL-N-(S-2-METHOXY-1-METHYL-2-OXOETHYL)]PHOSPHORAMIDATE (8)

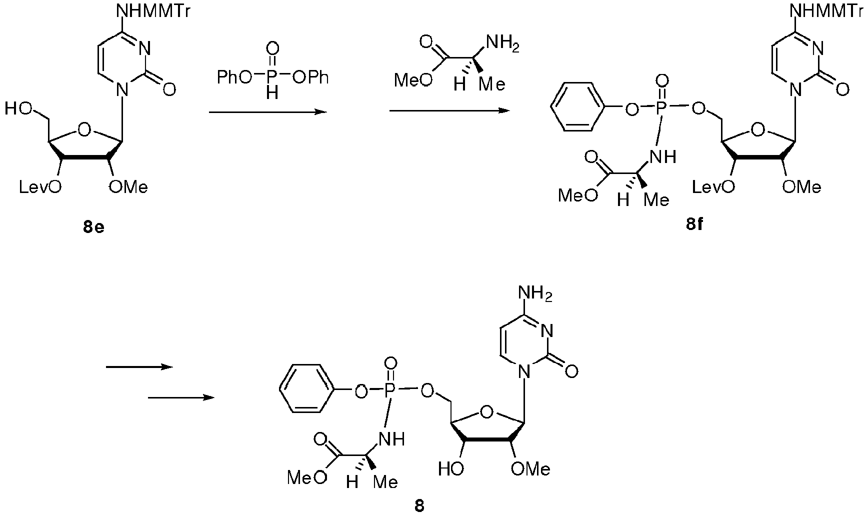

*3'-O-Levulinoyl-N⁴-(4-methoxytrityl)-2'-O-methylcytidine 5'-[O-phenyl-N-(S-2-methoxy-1-methyl-2-oxoethyl)]phosphoramidate* **(8f).** Compound **8e** (2.58 mmol; 1.62 g) dried on P₂O₅ for 2 days was dissolved in dry pyridine (5 mL) and diphenylphosphite (3.09 mmol; 595 µL) was added under nitrogen. After half an hour, carefully dried L-alanine methyl ester (3.94 mmol; 0.55 g) in a mixture of dry pyridine (1 mL) and MeCN (6 mL) was added. CCl₄ (15 mL) and triethylamine (18.1 mmol; 2.54 mL) was added and the reaction was allowed to proceed for 70 min. Volatiles were removed under reduced pressure and the residue was purified by silica gel chromatography increasing the MeOH content of DCM from 1 to 10% in a stepwise manner. Compound **8f** was obtained as a white foam in 70% yield. ¹H NMR (CDCl₃) mixture of *R*_{P} and *S*_{P} diastereomers δ 7.02-7.35 (m, 17H, MMTr and Ph), 6.80-6.85 (m, 3H, MMTr and N⁴*H*), 5.99 and 6.02 (2xd, *J* = 3.2 Hz, 1H, H1'), 4.90-5.00 (m, 2H, H3' and H4'), 3.88-4.43 (m, 4H, H5, H2', H5', H5"), 3.80 (s, 3H, MMTr), 3.68-3.75 (m, 1H, H^{α}-Ala, 3.63 and 3.64 (2xs, 3H, MeO-Ala), 3.46 and 3.52 (2xs, 3H, 2'-OMe), 2.74-2.81 (m, 2H, Lev), 2.59-2.64 (m, 2H, Lev), 2.19 and 2.20 (2xs, 3H, Lev), 1.88 (br s, 1H, NH-P), 1.27 and 1.31 (2xd, *J* = 7.1 Hz, Me Ala).

*2'-O-Methylcytidine 5'-(O-pheayl-N-(S-2-methoxy-1-methyl-2-oxoethyl)]phosphoramidate* **(8).** Compound **8f** (1.81 mmol; 1.57 g) was dissolved in a mixture of hydrazine hydrate (7.2 mmol; 350 µL), pyridine (11.5 mL) and AcOH (2.88 mL) and the reaction was allowed to proceed for 5 h. Volatiles were removed under reduced pressure and the residue was dissolved in DCM (50 mL) and washed with water, aq. NaHCO₃ and brine. The organic phase was dried on Na₂SO₄, evaporated to dryness and the residue was purified by silica gel chromatography using DCM containing 4-6% MeOH as an eluent.

The purified product was dissolved 80% aq. AcOH (8 mL) and the mixture was allowed to proceed at 55 °C for 2 h and additionally at 65 °C for 4.5 h. The mixture was evaporated to dryness and the residue was coevaporated twice from water and then purified by silica gel chromatography using gradient elution from 7 to 20% MeOH in DCM. The overall yield from **8** was 50 %. ¹H NMR (CDCl₃) mixture of two diastereomers δ 7.64 and 7.68 (2xd, *J* = 7.4, 1H, H6), 7.26-7.33 (m, 2H, Ph), 7.20-7.24 (m, 2H, Ph), 7.13-7.16 (m, 1H, Ph), 6.32 (br s, 2H, NH₂), 5.90 and 5.94 (2xs, 1 H, H1'), 5.69 and 5.82 (2xd, *J* = 7.4, 1H, H5), 4.35-4.55 (m, 2H, H5' and H5"), 4.12-4.18 (m, 2H, H3' and H4'), 3.98-4.08 (m, 2H, α-H-Ala and 3'-OH), 3.72-3.76 (m, 1H, 2'-OMe), 3.67 and 3.68 (2xs, 3H, MeO-Ala), 3.58 and 3.60 (2xs, 3H, 2'-OMe), 2.45 (br s, 1H, NH-P), 1.37 and 1.39 (2xd, *J* = 7.2 Hz, 3H, Me-Ala). ¹³C NMR (CDCl₃) δ174.2 (C=O Ala), 166.0 (C4), 155.9 (C2), 150.5 (Ph), 140.6 (C6), 129.8 (Ph), 125.1 (Ph), 120 (Ph), 95.1 (C5), 88.4 (C1'), 83.4 (C2'), 81.4 (C4'), 68.1 (C3'), 65.1 (C5'), 58.6 (2'-OMe), 52.5 (MeO-Ala), 50.3 (C^{α}-Ala), 20.7 (Me-Ala). ³¹P NMR δ 3.1 and 3.3. HRMS [M+H]⁺ obsd. 499.1590, calcd. 499.1583; [M+Na]⁺ obsd. 521.1438, calcd. 521.1408, [M+K]⁺ obsd. 537.1149, 537.1147.

### Example 9

### Preparation of 2', 5'-C-dimethyladenosine (9)

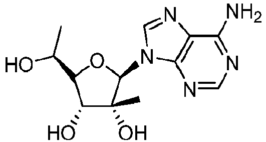

*Step 1. Preparation of 5-O-benzoyl-1,2-O-isopropylidene-5-C-methyl-*3-*O*-naphthalenyl-D-ribofuranose 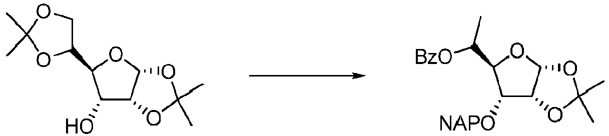 To a solution of dried 1,2 5,6-*O*-di(isopropylidene)-alpha-D-allofuranose (23.83 g, 91.55 mmol) in anhydrous THF (62 mL) was added powdered KOH (36 g, 642.86 mmol), and stirred at room temperature for 30-40 min, then followed by addition of 2-(bromomethyl)naphthalene (21 g), and stirred under nitrogen atmosphere for 4-6 h. The reaction mixture was then quenched with water and extracted with ethyl acetate (3x60 mL). The combined organic phase was dried with sodium sulfate and concentrated into a crude residue (43.38 g), which was treated with a mixture of acetic acid (187 mL) and water (84 mL) at room temperature for 14 h. The reaction mixture was concentrated under a good vacuum below 35 °C to give a crude residue, which was applied to a column of silica gel eluted with hexanes-ethyl acetate (4:1) and dichloromethane-methanol (10:1) to give a pure 3-*O*-naphthalenyl-1,2;5,6-*O*-di(isopropylidene)-alpha-D-allofuranose as syrup (36.57 g, 100%).
To a cold solution of dried 3-*O*-naphthalenyl-1,2;5,6-*O*-di(isopropylidene)-alpha-D-allofuranose (36.57 g, 101.3 mmol) in a mixture of 1,4-dioxane (214 mL) and water (534 mL) cooled with ice-bath was added sodium periodate (NaIO₄) (32 g, 149.61mmol) and stirred at the same temperature for 50 min. The reaction mixture was then extracted with ethyl acetate (4x50 mL), and the combined organic phase was dried with anhydrous sodium sulfate, and concentrated into a crude residue, which was dried under a good vacuum for a couple of hours and used in the next reaction without further purification. To a cold solution of the above dried crude residue (33.38 g, 101 mmol) in anhydrous ether (80 mL) cooled with dry ice-acetone to -78 °C was slowly added methylmagnium bromide (100 mL) (3M solution in ether) in portions, and stirred at -78 °C to room temperature overnight under nitrogen. The reaction mixture was then slowly quenched with sat. ammonium chloride solution, and extracted with acetyl acetate (4 x 60 mL). The combined organic phase was dried with anhydrous sodium sulfate and the filtrate was concentrated into a crude residue of 1,2-*O*-isopropylidene-5-*C*-methyl-3-*O*-naphthalenyl-D-ribofuranose (28.55 g, 83.21 mmol, 82.1%), which was dried under a good vacuum for 2-3 h and treated with benzoyl chloride (12.87 g, 91.53 mmoL) in the presence of DMAP (1.01 g, 8.32 mmol) in anhydrous pyridine (80-100 mL) at room temperature overnight. The reaction mixture was quenched with methanol and concentrated into a crude residue, which was poured into 10% sodium bicarbonate aq. solution and extracted with ethyl acetate (4x50 mL). The combined organic phase was concentrated and co-evaporated with toluene (3x50mL) into a crude residue, which was applied to a column of silica gel eluted with hexanes-ethyl acetate (100:1, 10:1, and 4:1) to give a pure 5-*O*-benzoyl-1,2-*O-*isopropylidene-5-*C*-methyl-3-*O*-naphthalenyl-D-ribofuranose (22.58 g, 50.50 mmol, 61 %).

### Step 2. preparation of 5-O-benzoyl-2-C,2-O-didehydro-1-O,5-C-dimethyl-3-O-naphthalenyl-D-ribofuranose

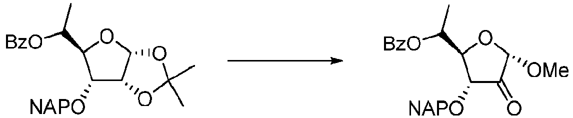

To a solution of dried 5-*O*-benzoyl-1,2-*O*-isopropylidene-5-*C*-methyl-3-*O-*naphthalenyl-D-ribofuranose (13.58 g, 30.37 mmol) in anhydrous methanol (100 mL) was added 4N HCl in 1,4-dioxane (4.9 mL) and stirred at room temperature for 12 h. The reaction mixture was neutralized with triethylamine to pH = 7.0 and concentrated into a crude residue, and poured to 10% sodium bicarbonate aq. solution and extracted with dichloromethane (4 x 20 mL). The combined organic phase was concentrated and co-evaporated with toluene into a crude residue, which was applied to a column of silica gel eluted with hexanes-ethyl acetate (4:1) to give a pure 5-*O*-benzoyl-1-*O*,5-*C*-dimethyl-3-*O-*naphthalenyl-D-ribofuranose (12.60 g, 29.93 mmol, 98.5 %). To a cold solution of DMSO (12.72 mL, 178.32 mmol) in anhydrous dichloromethane (50 mL) cooled with dry ice-acetone to -75 °C was added trifluoroacetic anhydride (TFAA) (7.6 mL, 53.87 mmol) and stirred at the same temperature for 30 min. 5-*O*-Benzoyl-1-*O*,5-C-dimethyl-3-*O-*naphthalenyl-D-ribofuranose (12.60 g, 29.93 mmol) in anhydrous dichloromethane (10 mL) was added in one portion, then warmed to -20 to -15 °C, and stirred at the same temperature for 2h, and followed by addition of triethylamine (20 mL), and warmed to RT, and stirred at room temperature for 1h. The reaction mixture was then quenched with water, and extracted with dichloromethane (3 x 50 mL). The combined organic phase was dried with sodium sulfate, and the filtrate was concentrated into a crude residue, which was applied to a short column of silica gel eluted with hexanes-ethyl acetate (20:1 and 1:1) to give 5-*O*-benzoyl-2-*C*,2-*O*-didehydro-1-*O*,5-*C*-dimethyl-3-*O*-naphthalenyl-D-ribofuranose as amorphous solid. (10.03 g, 23.90 mmol, 80 %).

### Step 3. Preparation of 2,3,5-O-tribenzoyl-1-O,2,5-C-trimethyl-D-ribofuranose.

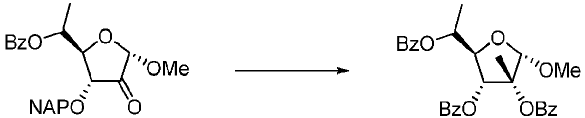

To a cold solution of dried 5-*O*-benzoyl-2-*C*,2-*O*-didehydro-1-*O*,5-*C*-dimethyl-3-O-naphthalenyl-D-ribofuranose (7.76 g, 18.52 mmol) in a mixture of anhydrous tetrahydrofuran (THF) (50 ml) and anhydrous ether (30 mL) cooled with dry ice-acetone to -30 to -15 °C was slowly added methylmagnium bromide (CH₃MgBr) (35 mL) (3.0 M in ether) and stirred at same temperature under nitrogen atmosphere for 6 h, and then at - 15 °C to room temperature overnight. The reaction mixture was carefully quenched with sat. ammonium chloride aq. solution, and extracted with ethyl acetate (4 x 60 mL). The combined organic phase was concentrated and co-evaporated with toluene (3 x 20 mL) into a crude residue, which was applied to column of silica gel eluted with hexanes-ethyl acetate (20:1) dichloromethane-methanol (10:1) to give a pure 5-*O*-benzoyl-3-*O-*naphthalenyl-1-*O*,2,5-*C*-trimethyl-D-ribofurano as syrup (5.32 g, 16.12 mmol, 87 %).

To a solution of dried 5-*O*-benzoyl-2-*C*,2-*O*-didehydro-3-*O-*naphthalenyl-1-*O*,2,5-*C*-trimethyl-D-ribofuranose (10.03 g, 30.39 mmol) and DMAP (1 g, 8.20 mmoL) in anhydrous pyridine (28 mL) was added benzoyl chloride (11.65 g, 9.62 mL, 82.88 mmol) and stirred at room temperature overnight under nitrogen. The reaction mixture was then quenched with methanol and concentrated into a crude residue, which was poured into 10% sodium bicarbonate aq. solution and extracted with ethyl acetate (3 x 20 mL). The combined organic phase was concentrated and co-evaporated with toluene into a crude residue that was applied to a short column of silica gel eluted with hexanes-ethyl acetate (50:1 and 10:1) to give a pure 2,5-*O*-dibenzoyl-3-*O*-naphthalenyl-1-*O*,2,5-C-trimethyl-D-ribofuranose as amorphous solid (9.17 g, 65 %).

To a solution of 2,5-*O*-dibenzoyl-3-*O*-naphthalenyl-1-*O*,2,5-C-trimethyl-D-ribofuranose (9.17 g, 17.04 mmol) in a mixture of dichloromethane (20 mL) and water (1 mL) was added DDQ (4.45 g, 19.60 mmol) and stirred at room temperature for 6h. The reaction mixture was diluted with dichloromethane (10 0 mL) poured into 10% sodium bicarbonate aq. solution, organic phase was separated and water phase was extracted with dichloromethane (3 x 50 mL). The combined organic phase was washed with sat. sodium bicarbonate aq. solution until all the DDQ was removed. The organic phase was concentrated and co-evaporated with toluene into a crude residue, which was further treated with BzCl (4.88 g, 34.69 mmol) in the presence of DMAP (650 mg) in anhydrous pyridine (20 mL) at room temperature overnight. The reaction mixture was then quenched with methanol and concentrated into a crude residue, which was poured into sat. sodium bicarbonate and extracted with ethyl acetate (4 x 50 mL). The combined organic phase was dried over anhydrous sodium sulfate and the filtrate was concentrated into a crude residue, which was applied to a short column of silica gel eluted with hexanes-ethyl acetate (30:1, and 10:1) to give a pure 2,3,5-*O*-tribenzoyl-1-*O*,2,5-C-trimethyl-D-ribofuranose as amorphous solid (4.21 g, 8.38 mmol, 49.20%).

### Step 4: Preparation of 2,3,5-O-tribenzoyl-1-O,2,5-C-trimethyl-D-ribofuranose

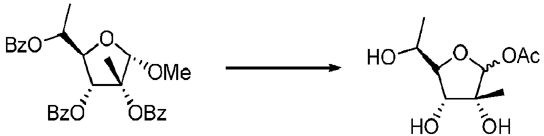

To a cold solution of dried 2,3,5-*O*-tribenzoyl-1-*O*,2,5-*C*-trimethyl-D-ribofuranose (2.43 g, 4.84 mmol) in acetic anhydride (10 mL) cooled with ice-bath was added a cold mixture of acetic anhydride (10 mL) and concentrated sulfuric acid (H₂SO₄) (95-98 %) (243 µL) and stirred at the same temperature for 1h. The reaction mixture was then poured into sat. sodium bicarbonate aq. solution stirred until pH of the mixture is 7 and extracted with ethyl acetate (3 x 30 mL). The combined organic phase was concentrated and co-evaporated with toluene (3 x 15 mL) into a crude residue, which was applied to a column of silica gel eluted with hexanes-ethyl acetate (20:1 and 10:1) to give a 1-*O-*acetyl--2,5-C-dimethyl-2,3,5-*O*-tribenzoyl-D-ribofuranose (1.6 g, 3.02 mmol, 62%).

### Step 5: Preparation of 2',5'-C-dimethyladenosine

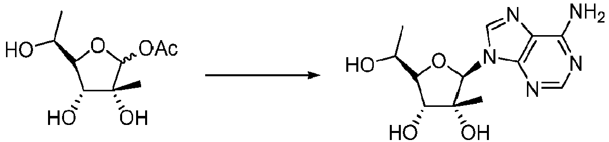

To a cold solution of *N*⁶-benzoyladenine (99 mg, 0.415mmol) and 1-*O*-acetyl--2,5-*C*-dimethyl-2,3,5-*O*-tribenzoyl-D-ribofuranose (220 mg, 0.415 mmol) in anhydrous ACN (5 mL) cooled with ice-bath was added TMSOTf (165 µL) and stirred at the same temperature for 1 h. The reaction mixture was then neutralized with triethylamine and concentrated into a crude residue, which was further treated with methanol-ammonia (7N) at room temperature for 4 days. The reaction mixture was then concentrated and co-evaporated with toluene into a crude residue, which was applied to a short column of silica gel eluted with dichloromethane-methanol (10:1 and 6:1) to give a pure 2',5'-*C-*dimethyladenosine as amorphous solid.

### Example 10

### Preparation of 2',5'-diMethylcytidine (10)

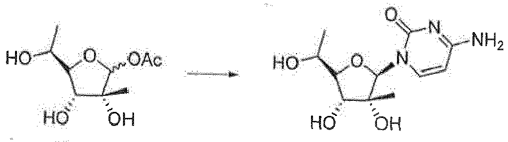

A stirred suspension of *N*⁴-acetylcytosine (576 mg, 3.76 mmol) and (NH₄)₂SO₄ (20 mg) in freshly distilled 1,1.1,3,3,3-hexamethyldisilazane (30 mL) was heated at reflux overnight under nitrogen atmosphere. The clear solution was evaporated under vacuum, and anhydrous toluene (20 mL) was added and subsequently distilled off. The crude bis-(trimethylsilyl) derivative obtained was dissolved in anhydrous acetonitrile (30 mL), and 1-*O*-acetyl--2,5-*C*-dimethyl-2,3,5-*O*-tribenzoyl-D-ribofuranose (1.0 g, 1.88 mmol) was added. The mixture was cold in an ice-water bath under an nitrogen atmosphere, and then TMSOTf (0.5 mL) was added dropwise with vigorous stirring. The resultant homogeneous pale yellow solution was stirred overnight. TLC showed there's still large mount of material. The mixture was cooled in an ice-water bath and another batch of TMSOTf (0.5 ml) was added dropwise. The resultant mixture was stirred overnight further. The reaction was quenched carefully by addition of 10% NaHCO₃ (20 mL) and stirred for an additional 15 min. The deposit was filtered and the filtrate was extracted with DCM (60mLx2). The combined organic phase was washed with brine and dried over anhydrous Na₂SO₄, After evaporation of the solvent, the residue was purified by silica gel chromatography eluting with PE:EA=2:1 to give *N*⁴-acetyl-2,5-*C*-dimethyl-2,3,5-*O-*tribenzoylcytidine (660 mg, 56.1%) as foam solid.
*N*⁴-Acetyl-2,5-*C*-dimethyl-2.3,5-*O*-tribetizoylcytidine (660 mg, 1.05 mmol) was dissolved in anhydrous MeOH which was saturated by NH₃. The mixture was heated to 60-70 °C with consistent stirring in a sealed tube for 2 days. The solvent was removed under vacuum and the residue was purified by prep-HPLC to give 2,5-*C*-dimethylcytidine (120 mg, 41.93% and 22mg, 7.7%). ¹H NMR of 2,5-*C*-dimethylcytidine (diastereomer 1): (MeOD): *δ* 7.73-7.75 (d, J = 8.0 Hz, 1H), 6.04 (s, 1H), 5.64-5.66 (d, J = 8.0 Hz, 1H), 4.05-4.07 (dd, J₁ = 2.4 Hz, J₂ = 4.8 Hz, 1H), 3.93-3.99 (m, 1H), 3.90 (d, J = 2.4 Hz, 1H), 1.23-1.24 (d, J = 6.4 Hz, 3H) □ 1.16 (s, 3H).

### Example 11

### Preparation of 2', 5'-dimethyluridine (11)

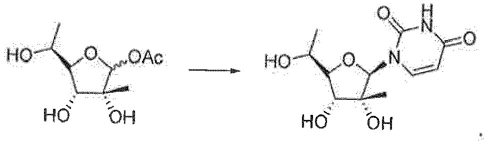

By a similar procedure as described in example 10, 2,5-*C*-dimethyluridine was prepared. ¹H NMR of 2,5-*C*-dimethyluridine (diastereomer 2): (MeOD): *δ* 7.73-7.75 (d, J = 8.0 Hz, 1H), 6.01 (s, 1H), 5.63-5.65 (d, J = 8.0 Hz,1H), 4.08-4.10 (dd, J₁ = 2.0 Hz, J₂ = 5.6 Hz, 1H), 4.06 (d, J = 2.0 Hz, 1H), 3.96-4.00 (m, 1H), 1.21-1.22 (d, J = 6.4 Hz, 3H)□1.18 (s, 3H).

### Example 12

### Preparation of 2'-deoxy-2'-fluoro-5'-C-methyladenosine (12)

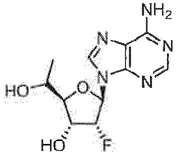

*Step 1*. *Preparation of 3',N⁶-bis(4,4'-dimethoxytrityl)-5'-O-*(*t-butyldimethylsilyl)-2'-deoxy-2'-fluoroadenosine.* 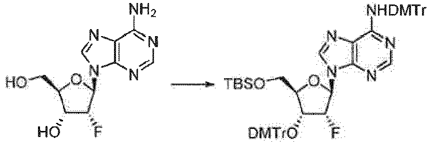 A mixture of 0.27 g (1.0 mmol) of 2'-deoxy-2'-fluoroadenosine, DMAP (244 mg, 2.0 mmol) and TBDMS-Cl (1.1 mmo), 181 mg) in anhydrous pyridine (15 mL) was stirred at room temperature overnight and then at 30 °C for 8 hours. DMTr-Cl (1.0 g, 3 mmol) was added and the mixture stirred at 56 °C for 3 days, cooled to 0 °C and quenched with water (1.5 mL). The resulting mixture was stirred at room temperature for 2 hours, diluted with ethyl acetate, washed with brine 3 times, and dried over sodium sulfate. Chromatography on silica gel with 20-35% ethyl acetate in hexane gave 746 mg of 3',N⁶--73-bis(4,4'-dimethoxytrityl)-5'-*O*-(*t*-butyldimethylsilyl)-2'-deoxy-2'-fluoroadenosine as white foam.

*Step 2. Preparation of 3',N⁶-bis(4,4'-dimethoxytrityl)-5'-dehydro-2'-deoxy-2'-fluoroadenosine.* 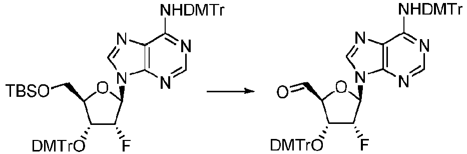 A solution of 3',N⁶-bis(4,4'-dimethoxytrityl)-5'-*O*-(*t*-butyldimethylsilyl)-2'-deoxy-2'-fluoroadenosine (0.73 g, 0.74 mmol) and TBAF (1.0 M in THF, 1.5 mL) in THF (6 mL) stood at room temperature overnight and then concentrated at room temperature. Chromatography on silica gel with acetone-hexane (2:3) gave the 5'-hydroxy product as white solid, which was dissolved in anhydrous DCM (12 mL). Pyridine (0.9 mL) and Dess-Martin periodinane (0.39 g) were added. The reaction mixture under argon was stirred at 25 °C for 2 hours, diluted with DCM, washed with 10% Na₂S₂O₃ 2 times and brine 1 time. Chromatography on silica gel with acetone-hexanes (1:3 to 2:3) gave 606 mg of 3',N⁶-bis(4,4'-dimethoxytrityl)-5'-dehydro-2'-deoxy-2'-fluoroadenosine.

*Step 3. Synthesis of 2'-deoxy-2'-fluoro-5'(R and S)-C-methyladenosine.* 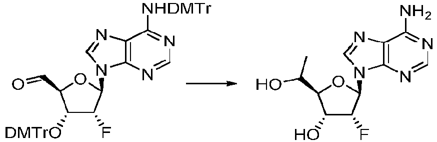 To a solution of 3',N⁶-bis(4,4'-dimethoxytrityl)-5'-dehydro-2'-deoxy-2'-fluoroadenosine (600 mg, 0.686 mmol) in THF (7 mL) at 0 °C under argon was added MeMgBr (1.4 M in THF, 2 mL). The reaction mixture was stirred at 0 °C under argon overnight. Additional MeMgBr (1.4 mL) was added and the reaction mixture was stirred at 0 °C for 2 hours and then at room temperature for 30 minutes. After cooling to 0 °C, the reaction mixture was quenched very slowly with 10% ammonium sulfate, diluted with ethyl acetate, and washed with 10% ammonium sulfate 2 times and 10% sodium bicarbonate 1 time. Chromatography on silica gel with acetone-hexane (1:3 to 2;3) gave 315 mg of 3',N⁶-bis(4,4'-dimethoxytrityl)-2'-deoxy-2'-fluoro-5'(*R* and S)-C-methyladenosine (216 mg of the upper isomer on TLC and 99 mg of the mixture of the two isomers, both as white solid).

3',N⁶-Bis(4,4'-dimethoxytrityl)-2'-deoxy-2'-fluoro-5'(*R* or S)-C-methyladenosine (upper isomer on TLC, 215 mg) was dissolved in 5 mL of THF, 8 mL of AcOH and 5 mL of water. The solution was stirred at 30 °C for 15 hours, concentrated to dryness and co-evaporated with toluene 3 times. Chromatography on silica with 10-12% MeOH in DCM gave 55 mg of 2'-deoxy-2'-fluoro-5'(R or S)-C-methyladenosine as white solid; ¹H NMR (DMSO) *δ*1.16 (d, J = 6.4 Hz, 1H), 3.79-3.85 (m, 2H, H4' and H5'), 4.45 (ddd, *J_{H,H}=* 6.4 and 3.2 Hz, *J_{H,F}* = 16.4 Hz, 1H, H3'), 5.26 (d, *J*= *6.0* Hz, 1H, OH), 5.40 (ddd, *J_{H,H}* = 4.0 and 3.2 Hz, *J_{HF}* = 53.2 Hz, 1H, H2'), 5.68 (d, *J* = 6.0 Hz, 1H, OH), 6.23 (dd, *J_{H,H}* = 3.2 Hz, *J_{H},_{F}* = 15.6 Hz, 1H, H1'), 7.38 (s, 2H, NH₂), 8.15 (s, 1H, H8), 8.41 (s, 1H, H2).

3',N⁶-Bis(4,4'-dimethoxytrityl)-2'-deoxy-2'-fluoro-5'(*R* and S)-C-methyladenosine (the upper isomer as the major and lower isomer as the minor, 99mg) was dissolved in 3 mL of THF, 3 mL of AcOH and 3 mL of water was stirred at room temperature overnight. THF was removed on a rotary evaporator and the remaining solution was heated at 45 °C for 45 minutes, concentrated, co-evaporated with toluene 3x. Chromatography on silica with 10-12% MeOH in DCM gave 22 mg of 2'-deoxy-2'-fluoro-5'(*R* and *S*)-*C*-methyladenosine.

### Example 13

### Preparation of 2'-deoxy-2'-fluoro-5'-C-methylcytidine (13)

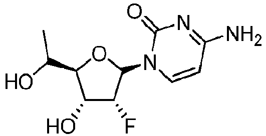

*Step 1*. *Preparation of 3-O-,N⁴-bis(4-methoxytrityl)-5'-O-(t-butyldimethylsilyl)-2'-deoxy-2'-fluorocytidine.* 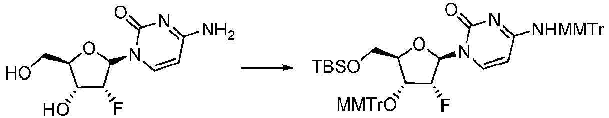 A solution of 2'-deoxy-2'-fluorocytidine (20.0 g, 81.6 mmol) and TBDMS-Cl (14.8 g, 97.9 mmol) in anhydrous pyridine (200 mL) was stirred at room temperature overnight and then concentrated. The residue was diluted with ethyl acetate, washed with brine, dried over anhydrous Na₂SO₄ and concentrated to give 24 g (82%) of *5'-O-(t-*butyldimethylsilyl)-2'-deoxy-2'-fluorocytidine as a white solid.

Silver nitrate (7 g, 41.7 mmol) was added to a solution of MMTr-Cl (13 g, 41.7 mmol), 5'-*O*-(*t*-butyldimethylsilyl)-2'-deoxy-2'-fluorocytidine (5 g, 13.9 mmol) and collidine (19 g, 153 mmol) in anhydrous DCM (50 mL). The reaction mixture was stirred at room temperature overnight, filtered, and washed with saturated NaHCO₃ and brine. The organic layer was dried over Na₂SO₄ and concentrated. Chromatography on silica gel with ethyl acetate-petroleum ether (1:2 to 1:1) gave 11 g (87%) of 3*'-O-,N⁴*-bis(4-methoxytrityl)-5'-O-(t-butyldimethylsilyl)-2'-deoxy-2'-fluorocytidine.

*Step 2. Preparation of 3-O-,N⁴-bis(4-methoxytrityl)-5'-C,5'-O-didehydro-2'-deoxy-2'-fluorocytidine.* 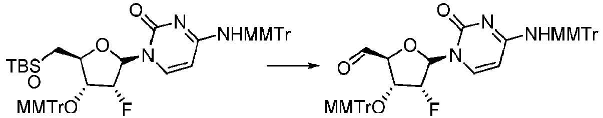 TBAF (24 mL, 1.0 M in THF) was added dropwise to a solution of 3'*-*O-,*N⁴*-bis(4-methoxytrityl)-5'-O-(t-butyldimethylsilyl)-2'-deoxy-2'-fluorocytidine (11 g, 12 mmol) in anhydrous THF (100 mL) at 0 °C. The solution was stirred at room temperature overnight and then solvent was removed *in vacuo* at room temperature. The residue was dissolved in ethyl acetate, washed with water and brine, dried over Na₂SO₄, and concentrated. Chromatography on silica gel with acetone/petroleum ether (1:3) gave 9 g (93%) of 3'-*O-*,*N⁴*-bis(4-methoxytrityl)- 2'-deoxy-2'-fluorocytidine. Pyridine (6 mL, 15 eq) and Dess-Martin periodinane (2.6 g, 6 mmol) was added to a solution of 3'-*O*-,*N⁴*-bis(4-methoxytrityl)- 2'-deoxy-2'-fluorocytidine (4 g, 5 mmol) in anhydrous DCM (30 mL) at 0 °C under N₂. The reaction mixture was stirred at room temperature for 2 hours, diluted with ethyl acetate, washed with 10% Na₂S₂O₃ twice and then with brine, dried over anhydrous Na₂SO₄ and concentrated. Chromatography on silica gel with acetone-petroleum ether (1:3 to 2:3) gave 3.5g (87%) of *3-O-,N⁴-bis(4-methoxytrityl)-5'-C,5'-O-didehydro-2'-deoxy-2'-fluorocytidine.*

### (13)

*Step 3. Preparartion of 2'-deoxy-2'-fluoro-5'(R and S)-C-methylcytidine.* 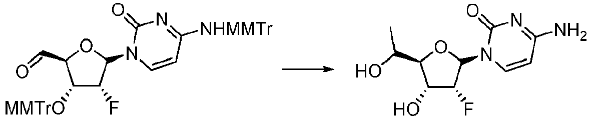 MeMgBr (3.0 M in ether, 15.2 mmol) was added dropwise to a solution of *3-O-,N⁴-bis(4-methoxytrityl)-5'-C,5'-O-didehydro-2'-deoxy-2'-fluorocytidine* (3 g, 3.8 mmol) in anhydrous THF (50 mL) in an ice-EtOH bath under N₂. The reaction mixture was stirred at room temperature for 5 hours, quenched with sat. NH₄Cl, diluted with ethyl acetate, washed with brine, dried over anhydrous Na₂SO₄ and concentrated to give the crude product. Chromatography on silica gel with acetone-petroleum ether (1:3 to 2:3) gave 1.8 g (58%) of pure 3-*O*-,*N⁴*-bis(4-methoxytrityl)-2'-deoxy-2'-fluoro-5'(*R* or *S)-C-*methylcytidine.

A solution of 3-*O*-,*N⁴*-bis(4-methoxytrityl)-2'-deoxy-2'-fluoro-5'(*R* or *S*)-*C*-methylcytidine (600 mg, 0.75 mmol) in AcOH/H₂O (v/v 4:1, 20 mL) was stirred at 50 °C overnight. The solution was concentrated, diluted with water, extracted with ethyl acetate twice and concentrated to dryness. Chromatography on a reverse-phase HPLC and then on a chiral HPLC gave 2'-deoxy-2'-fluoro-5'(R or *S*)-*C*-methylcytidine (30 mg, 16%); ¹H NMR (CD₃OD): *δ* 8.16 (d, *J* = 7.6 Hz, 1H, H6), 5.99 (dd, *J* = 17.6 Hz, 1.2 Hz, 1H, H1'), 5.92 (d, *J* = 7.6 Hz, 1H, H5), 5.06-4.92 (m, 1H, H2'), 4.28 (ddd, *J_{H,H}=* 8.4, 4.4 Hz, *J_{H,F} =* 21.6 Hz, 1H, H3'), 4.02 (dq, *J* = 4.0, 2.8 Hz, 1H, H5'), 3.87 (dd, *J* = 8.0, 2.0 Hz, 1H, H4'), 1.38 (d, *J* = 6.4 Hz, 3H, Me).

### Example 14

### Preparation of 2'-deoxy-2',2'-difluoro-5'(S)-C-methylcytidine (14)

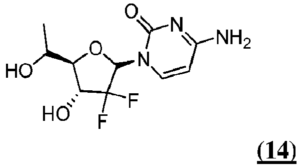

*Step 1*. *Preparation of 3-O-,N⁴-bis(4-methoxytrityl)-5'-O-(t-butyldimethylsilyl)-2'-deoxy-2',2'-difluorocytidine.* 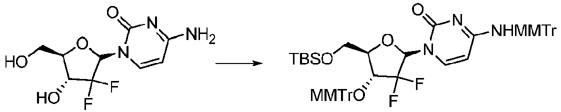 TBDMS-Cl (10.5 g, 69.3 mmol) was added to a solution of 2'-deoxy-2',2'-difluorocytidine hydrochloride (17.0 g, 57.7 mmol) in anhydrous pyridine (100 mL) at 0 °C under N₂. The reaction mixture was stirred at room temperature overnight, concentrated, diluted with ethyl acetate, washed with brine, dried over anhydrous Na₂SO₄ and concentrated to give 5'-*O*-(*t*-butyldimethylsilyl)-2'-deoxy-2',2'-difluorocytidine (21 g, 96%) as a white solid.

MMTr-Cl (13 g, 41 mmol, 3 eq) was added to a solution of *5'-O-(t-*butyldimethylsilyl)-2'-deoxy-2',2'-difluorocytidine (5 g, 13.5 mmol) in anhydrous DCM (50 mL), followed by addition of AgNO₃ (7 g, 41 mmol) and collidine (19 g, 153 mmol). The reaction mixture was stirred at room temperature overnight under N₂, filtered, washed with saturated NaHCO₃ and then with brine. The organic layer was dried over Na₂SO₄ and concentrated. Chromatography on silica gel with ethyl acetate-petroleum ether (1:3 to 1:2) gave 3-*O*-,*N⁴*-bis(4-methoxytrityl)-5'-*O*-(*t*-butyldimethylsilyl)-2'-deoxy-2',2'-difluorocytidine (11 g, 83%).

*Step 2. Preparation of 3-O-,N⁴-bis(4-methoxytrityl)-5'-C,5'-O-didehydro-2'-deoxy-2',2'-difluorocytidine.* 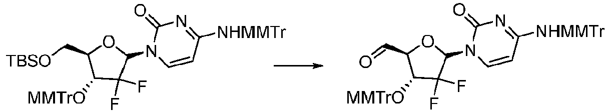 TBAF (1 M in THF,21.6 mL) was added dropwise to a solution of 3-*O*-,*N*⁴-bis(4-methoxytrityl)-5'-O-(t-butyldimethylsilyl)-2'-deoxy-2',2'-difluorocytidine (10.0 g, 10.8 mmol) in anhydrous THF (40 mL) at 0 °C. The resulting solution was stirred at room temperature overnight, concentrated, diluted with ethyl acetate, washed with brine, dried over anhydrous Na₂SO₄ and concentrated. Chromatography on silica gel with ethyl acetate-DCM (1:10 to 1:5) gave 3-*O*-,*N*⁴-bis(4-methoxytrityl)- 2'-deoxy-2',2'-difluorocytidine (4.4 g, 50%).

TFA (460 uL, 6 mmol) was added to a stirred solution of anhydrous pyridine (960 uL, 12 mmol) in anhydrous DMSO (10 mL) cooled with cold water under N₂, After addition, the TFA/pyridine solution was warmed to R.T. and added to a stirred solution of 3-*O*-,*N⁴*-bis(4-methoxytrityl)- 2'-deoxy-2',2'-difluorocytidine (8.1 g, 10 mmol) and DCC (6.2 g, 30 mmol) in anhydrous DMSO (30 mL) cooled with cold water under N₂. The reaction mixture was stirred at R.T. overnight. Cooled with cold water, quenched with water (20 mL) and stirred at R.T. for 1 h and diluted with EA. Precipitate was filtered and washed with EA. The combined EA solution was washed with brine, dried over anhydrous Na₂SO₄ and concentrated to give a residue which was purified by silica gel column (PE/EA=1/1 to 1/3) to give *3-O-,N⁴-bis(4-methoxytrityl)-5'-C,5'-O-didehydro-2'-deoxy-2',2'-difluorocytidine* (6.2 g, 76%).

*Step 3. Preparation of* 3-O-,N⁴-bis(4-methoxytrityl)-5'-dehydro-2'-deoxy-2',2'-difluorocytidine. 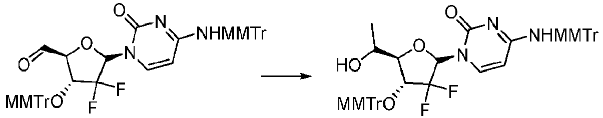 MeMgBr (3.0M in ether, 10 mL, 30 mmol) was added dropwise to a solution of the crude 3-*O*-,*N⁴*-bis(4-methoxytrityl)-5'-dehydro-2'-deoxy-2',2'-difluorocytidine (6.0 g, 7.4 mmol) in anhydrous THF (30 mL) in an ice-EtOH bath under N₂. The reaction mixture was stirred at room temperature overnight, quenched with saturated NH₄Cl, diluted with ethyl acetate, washed with brine, dried over anhydrous Na₂SO₄ and concentrated. Chromatography on silica gel with ethyl acetate-petroleum ether (1:3 to 1:1) gave 3.6 g of 3-*O*-,*N⁴*-bis(4-methoxytrityl)-2'-deoxy-2',2'-difluoro-5'-*C*-methylcytidine (59%).

### Step 4. Preparation of 2'-deoxy-2',2'-difluoro-5'(S)-C-methylcytidine.

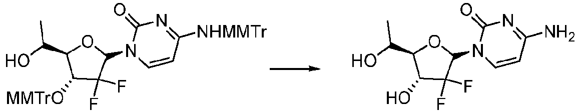

A solution of 3-*O*-,*N⁴*-bis(4-methoxytrityl)-2'-deoxy-2',2'-difluoro-5'(*R* and S)-C-methylcytidine (3 g, 3.65 mmol) in AcOH/H₂O (20 mL, v/v 4:1) was stirred at 50 °C overnight. After removal of solvents the residue was diluted with water, extracted with ethyl acetate twice and concentrated. Chromatography on a reverse-phase HPLC gave 0.3 g (30%) of **2'-deoxy-2',2'-difluoro-5'(*S*)-*C*-methylcytidine** as white solid; ¹H NMR (CD₃OD) *δ* 7.93 (d, *J* = 7.6 MHz, 1H, H6), 6.24 *(t, J_{H},_{F}* = 8.0 Hz, 1H, H1'), 5.95 (d, *J* = 7.6 MHz, 1H, H5), 4.26 (dt, *J_{H,H}=* 8.4 Hz, *J_{H,F} =* 12.4 Hz, 1H, H3'), 4.03 (dq, *J* = 4.0, 2.7 Hz, 1H, H5'), 3.74 (dd, *J* = 8.4, 2.8 Hz, 1H, H4'), 1.37 (d, *J* =6.4 MHz, 3H).

### Example 15

### 2'-DEOXY-2',2'-DIFLUORO-5'(R)-C-METHYLCYTIDINE (15)

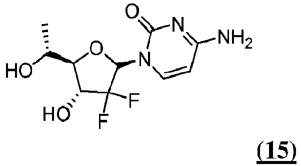

### Step 1. Preparation of 3-O,N⁴-bis(4-methoxytrityl)-2'-deoxy-2',2'-difluoro-5'(R)-methylcytidine.

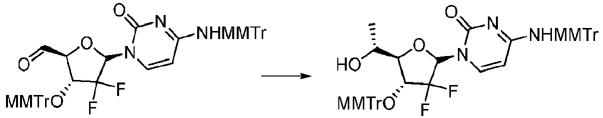

MeMgBr (1.4 M in THF, 2.6 mL, 3.6 mmol) was added dropwise to a solution of the crude 3-*O*-,*N*⁴-bis(4-methoxytrityl)-5'-dehydro-2'-deoxy-2',2'-difluorocytidine (580 mg, 0.72 mmol) in anhydrous THF (8 mL) at 0 °C under argon. The reaction mixture was stirred at room temperature for 3 h, cooled with ice, quenched with aqueous (NH₄)₂SO₄, diluted with ethyl acetate, washed with aqueous (NH₄)₂SO₄ solution four times and then with brine, dried over anhydrous Na₂SO₄ and concentrated. Chromatography on silica gel with ethyl acetate-hexanes (55:45 to 70:30) gave 317 mg of 3-*O*-,*N⁴*-bis(4-methoxytrityl)-**2'-deoxy-2',2'-difluoro-5'(*S*)-*C*-methylcytidine** and 44 mg of 3-*O*-,*N*⁴-bis(4-methoxytrityl)-2'-deoxy-2' ,2'-difluoro-5'(*R*)-*C*-methylcytidine.

### Step 2. Preparation of 2'-deoxy-2',2'-difluoro-5'(R)-C-methylcytidine.

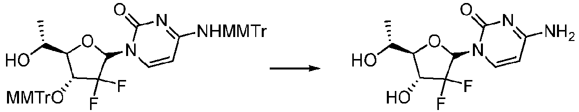

A solution of 3-*O*-,*N⁴*-bis(4-methoxytrityl)-2'-deoxy-2',2'-difluoro-5'(*R*)-*C-*methylcytidine (44 mg 0.53 mmol) in AcOH/H₂O (3 mL, v/v 4:1) was stirred at 40 °C overnight. After removal of solvents the residue was co-evaporated with toluene two times. Chromatography on silica gel with 10-15% MeOH in DCM gave 9 mg of 2'-deoxy-2',2'-difluoro-5'(*R*)-*C*-methylcytidine as white solid;

### Example 16

### Preparation of 2'-deoxy-2'-fluoro-5'(S)-C-methyladenosine (16)

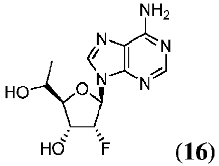

### Step 1. Preparation of 5'-O-(t-butyldimethylsilyl)-2'-deoxy-3'-O,N⁶-bis(4,4'-dimethoxytrityl)-2'-fluoroadenosine.

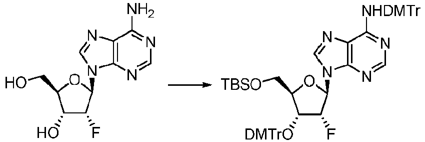

A mixture of 0.27 g (1.0 mmol) of 2'-deoxy-2'-fluoroadenosine, DMAP (244 mg, 2.0 mmol) and TBDMS-Cl (1.1 mmol, 181 mg) in anhydrous pyridine (15 mL) was stirred at RT overnight and then at 30 °C for 8 h. DMTr-Cl (1.0 g, 3 mmol) was added and the mixture stirred at 56 °C for 3 days, cooled to 0 °C and quenched with water (1.5 mL). The resulting mixture was stirred at RT for 2 h, diluted with ethyl acetate, washed with brine 3x, and dried over sodium sulfate. Chromatography on silica gel with 20-35% ethyl acetate in hexane gave 746 mg of 5'-*O*-(*t*-butyldimethylsilyl)-2'-deoxy-3'-*O*,*N*⁶-bis(4,4'-dimethoxytrityl)-2'-fluoroadenosine

### Step 2. Preparation of 2'-deoxy-5'-C,5'-O-didehydro-3'-O,N⁶-bis(4,4'-dimethoxytrityl)-2'-fluoroadenosine

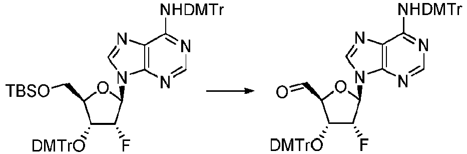

A solution of 5'-*O*-(*t*-butyldimethylsilyl)-2'-deoxy-3',N⁶-di(4,4'-dimethoxytrityl)-2'-fluoroadenosine (0.73 g, 0.74 mmol) and TBAF (1.0 M in THF, 1.5 mL) in THF (6 mL) stood at RT overnight and then concentrated at RT. Chromatography on silica gel with acetone-hexane (2:3) gave the 5'-hydroxy product as white solid, which was dissolved in anhydrous DCM (12 mL). Pyridine (0.9 mL) and Dess-Martin periodinane (0.39 g) were added. The reaction mixture under argon was stirred at 25 °C for 2 h, diluted with DCM, washed with 10% Na₂S₂O₃ 2x and brine 1x. Chromatography on silica gel with acetone-hexanes (1:3 to 2:3) gave 606 mg of 2'-deoxy-5'-*C*,5'-*O*-didehydro-3'-*O*,*N*⁶-bis(4,4'-dimethoxytrityl)-2'-fluoroadenosine as white foam.

### Step 3. Preparation of 2'-deoxy-2'-fluoro-5'(R and S)-C-methyladenosine

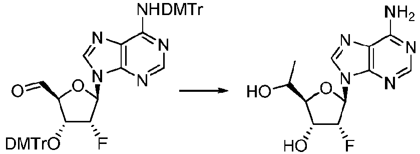

To a solution of 2'-deoxy-5'-*C*,5'-*O*-didehydro-3'-*O*,*N*⁶-bis(4,4'-dimethoxytrityl)-2'-fluoroadenosine (600 mg, 0.686 mmol) in THF (7 mL) at 0 °C under argon was added MeMgBr (1.4 M in THF, 2 mL). The reaction mixture was stirred at 0 °C under argon overnight. More MeMgBr (1.4 mL) was added and the reaction mixture was stirred at 0 °C for 2 h and then at RT for 30 min. After cooling to 0 °C, the reaction mixture was quenched very slowly with 10% ammonium sulfate, diluted with ethyl acetate, and washed with 10% ammonium sulfate 2x and 10% sodium bicarbonate 1x. Chromatography on silica gel with acetone-hexane (1:3 to 2:3) gave 315 mg of 3'-*O,*,*N*⁶-bis(4,4'-dimethoxytrityl)-2'-deoxy-2'-fluoro-5'(R and *S*)-*C*-methyladenosine (216 mg of 5'(*S*)-isomer and 99 mg of the mixture of the 5'(*S*)-isomer and 5'(*R*)-isomer, both as white foam.

3'-*O*,,*N*⁶-bis(4,4'-dimethoxytrityl)-2'-deoxy-2'-fluoro-5'(*S*)-*C*-methyladenosine (upper spot on TLC, 215 mg) was dissolved in 5 mL of THF, 8 mL of AcOH and 5 mL of water. The solution was stirred at 30 °C for 15 h, concentrated to dryness and co-evaporated with toluene 3x. Chromatography on silica with 10-12% MeOH in DCM gave 55 mg of 2'-deoxy-2'-fluoro-5'(*S*)-*C*-methyladenosine as white solid; ¹H NMR (DMSO) *δ* 1.16 (d, J = 6.4 Hz, 1H), 3.79-3.85 (m, 2H, H4' and H5'), 4.45 (ddd, *J_{H,H}=* 6.4 and 3.2 Hz, *J_{H},_{F}=* 16.4 Hz, 1H, H3'), 5.26 (d, *J* = *6.0* Hz, 1H, OH), 5.40 (ddd, *J_{H,H} =* 4.0 and 3.2 Hz, *J_{HF}* = 53.2 Hz, 1H, H2'), 5.68 (d, *J* = 6.0 Hz, 1H, OH), 6.23 (dd, *J_{H,H}* = 3.2 Hz, *J_{H,F}*= 15.6 Hz, 1H, H1'), 7.38 (s, 2H, NH₂), 8.15 (s, 1H, H8), 8.41 (s, 1H, H2).

2'-Deoxy-3',*N⁶*-di(4,4'-dimethoxytrityl)-2'-fluoro-5'(*R* and *S*)-*C*-methyladenosine (the upper isomer as the major and lower isomer as the minor, 99 mg) was dissolved in 3 mL of THF, 3 mL of AcOH and 3 mL of water was stirred at RT overnight. THF was removed on a rotary evaporator and the remaining solution was heated at 45 °C for 45 min, concentrated, co-evaporated with toluene 3x. Chromatography on silica with 10-12% MeOH in DCM gave 22 mg of 2'-deoxy-2'-fluoro-5'(R and S)-C-methyladenosine as white solid.

### Example 17

### Preparation of 2'-deoxy-2'-fluoro-5'-C-methylcytidine (17)

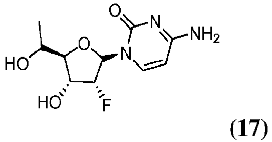

### Step 1. Preparation of 5'-O-(t-butyldimethylsilyl)-2'-deoxy-3-O,N⁴-di(4-methoxytrityl)-2'-fluorocytidine

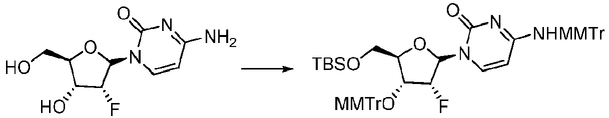

A solution of 2'-deoxy-2'-fluorocytidine (20.0 g, 81.6 mmol) and TBDMS-Cl (14.8 g, 98.2 mmol) in anhydrous pyridine (200 mL) was stirred at RT overnight and then concentrated. The residue was diluted with ethyl acetate, washed with brine, dried over anhydrous Na₂SO₄ and concentrated to give 24 g (82%) of 5'-O-(t-butyldimethylsilyl)-2'-deoxy-2'-fluorocytidine as a white solid.

Silver nitrate (7 g, 41.2 mmol) was added to a solution of MMTr-Cl (13 g, 42.2 mmol), 5'-*O*-(*t*-butyldimethylsilyl)-2'-deoxy-2'-fluorocytidine (5 g, 13.9 mmol) and collidine (19 g, 157 mmol) in anhydrous DCM (50 mL). The reaction mixture was stirred at RT overnight, filtered, and washed with sat. NaHCO₃ and brine. The organic layer was dried over Na₂SO₄ and concentrated. Chromatography on silica gel with ethyl acetate-petroleum ether (1:2 to 1:1) gave 11.5 g (91%) of 5'-O-(t-butyldimethylsilyl)-2'-deoxy-3'-*O*-,*N⁴*-di(4-methoxytrityl)-2'-fluorocytidine.

### Step 2. Preparation of 2'-deoxy-5'-C,5'-O-didehydro-3-O-,N⁴-di(4-methoxytrityl)-2'-fluorocytidine

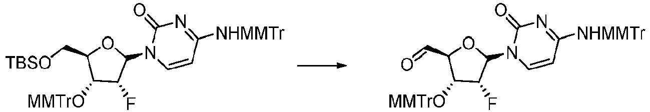

TBAF (24.4 mL, 1.0 M in THF) was added dropwise to a solution of *5'-O-(t-*butyldimethylsilyl)-2'-deoxy-3'-*O*-,*N⁴*-di(4-methoxytrityl)-2'-fluorocytidine (11 g, 12.2 mmol) in anhydrous THF (100 mL) at 0 °C. The solution was stirred at RT overnight and then solvent was removed *in vacuo* at RT. The residue was dissolved in ethyl acetate, washed with water and brine, dried over Na₂SO₄, and concentrated. Chromatography on silica gel with acetone/petroleum ether (1:3) gave 9 g (93%) of 2'-deoxy-3'-*O*-,*N*⁴-di(4-methoxytrityl)-2'-fluorocytidine.
Pyridine (6 mL) and Dess-Martin periodinane (2.6 g, 6.1 mmol) was added to a solution of 2'-deoxy-3'-*O*-,*N⁴*-di(4-methoxytrityl)-2'-fluorocytidine (4 g, 5.0 mmol) in anhydrous DCM (30 mL) at 0 °C under N₂. The reaction mixture was stirred at RT for 2h, diluted with ethyl acetate, washed with 10% Na₂S₂O₃ twice and then with brine, dried over anhydrous Na₂SO₄ and concentrated. Chromatography on silica gel with acetone-petroleum ether (1:3 to 2:3) gave 3.5 g (87%) of 2'-deoxy-5'-*C*,5'-*O*-didehydro-3-*O*-,*N⁴*-di(4-methoxytrityl)-2'-fluorocytidine.

### Step 3. Preparation of 2'-deoxy-2'-fluoro-5'(R and S)-C-methylcytidine

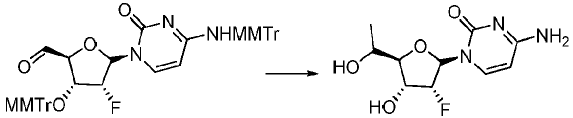

MeMgBr (3.0 M in ether, 5.1 mL) was added dropwise to a solution of 2'-deoxy-5'-*C*,5'-*O*-didehydro-3*-O*-,*N⁴*-di(4-methoxytrityl)-2'-fluorocytidine (3 g, 3.8 mmol) in anhydrous THF (50 mL) in an ice-EtOH bath under N₂. The reaction mixture was stirred at RT for 5 h, quenched with sat. NH₄Cl, diluted with ethyl acetate, washed with brine, dried over anhydrous Na₂SO₄ and concentrated to give a crude product (one isomer was dominant). Chromatography on silica gel with acetone-petroleum ether (1:3 to 2:3) gave 1.8 g (58%) of 2'-deoxy-3-*O*-,*N⁴*-di(4-methoxytrityl)-2'-fluoro-5'-*C*-methylcytidine.
A solution of 2'-deoxy-3-*O*-,*N⁴*-di(4-methoxytrityl)-2'-fluoro-5'-*C*-methylcytidine (600 mg, 0.75 mmol) in AcOH/H₂O (v/v 4:1, 20 mL) was stirred at 50 °C overnight. The solution was concentrated, diluted with water, extracted with ethyl acetate twice and concentrated to dryness. Chromatography on a reverse-phase HPLC and then by SFC separation gave 30 mg (16%) of 2'-deoxy-2'-fluoro-5'(*S*)-*C*-methylcytidine as white solid; ¹H NMR (CD₃OD): *δ* 8.16 (d, *J* = 7.6 Hz, 1H, H6), 5.99 (dd, *J* = 17.6 Hz, 1.2 Hz, 1H, H1'), 5.92 (d, *J* = 7.6 Hz, 1H, H5), 5.06-4.92 (m, 1H, H2'), 4.28 (ddd, *J_{H,H}=* 8.4, 4.4 Hz, *J_{U,F} =* 21.6 Hz, 1H, H3'), 4.02 (dq, *J* = 4.0, 2.8 Hz, 1H, H5'), 3.87 (dd, *J* = 8.0, 2.0 Hz, 1H, H4'), 1.38 (d, J = 6.4 Hz, 3H, Me).

### Example 18

### Preparation of 2'-deoxy-2'-fluoro-5'-C-methylarabinocytidine (18)

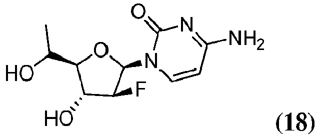

### Step 1. Preparation of 5'-O-(t-butyldimethylsilyl)-2'-deoxy-3'-O,N⁴-di(4-methoxytrityl)-2' -fluoroarabinocytidine

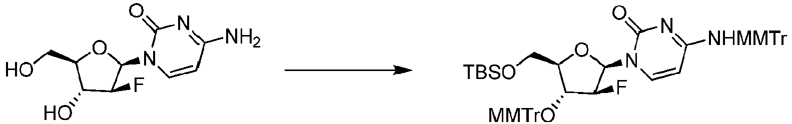

TBSCl (738 mg, 4.9 mmol) was added into a solution of 2'-deoxy-2'-fluoroarabinocytidine (1.0 g, 4.08 mmol) in anhydrous pyridine (10 mL) at 0 °C under N₂, and stirred at RT overnight. TLC showed the reaction was completed. Then the pyridine was evaporated under reduced pressure. The residue was diluted with EA, washed with water and followed by brine, dried over anhydrous Na₂SO₄ and concentrated in vacuo to give 5'-*O*-(*t*-butyldimethylsilyl)-2'-deoxy-2'-fluoroarabinocytidine (1.3 g, 89%) as a white solid.

MMTrCl (3.38 g, 10.8 mmol) was added into a solution of *5'-O-(t-*butyldimethylsilyl)-2'-deoxy-2'-fluoroarabinocytidine (1.3 g, 3.6 mmol) in anhydrous DCM (15 mL), AgNO₃ (1.82 g, 10.8 mmol) and collidine (5.4 ml, 39.6 mmol) was added thereto. The reaction mixture was stirred at RT overnight under N₂ and TLC showed the reaction was well. Then the reaction mixture was filtered and washed with sat. NaHCO₃ solution and followed by brine. The organic layer was dried over Na₂SO₄ and concentrated in vacuo to give the residue which was purified by silica gel (hexane/EA=2/1 to 1/1) to give 5'-*O*-(*t*-butyldimethylsilyl)-2'-deoxy-3'-*O*, *N*⁴-di(4-methoxytrityl)-2'-fluoroarabinocytidine (2.3 g, 71%).

### Step 2. Preparation of 2'-deoxy-5-C,5'-O-didehydro-3'-O,N⁴-di(4-methoxytrityl)-2'-fluoroarabinocytidine

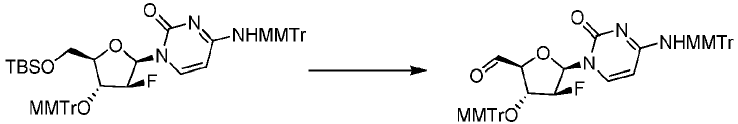

TBAF (5.08 ml, 1M in THF, 5.08 mmol) was added dropwise into a solution of 5'-*O*-(*t*-butyldimethylsilyl)-2'-deoxy-3'-*O*, *N*⁴-di(4-methoxytrityl)-2'-fluoroarabinocytidine (2.3 g, 2.54 mmol) in anhydrous THF (20 mL) at 0 °C and stirred at RT overnight. TLC showed the reaction was completed. Then the solvent was removed in vacuo at RT. EA was added to the residue and washed with water, followed by brine, dried over anhydrous Na₂SO₄ and concentrated in vacuo to give the residue which was purified by silica gel (heaxne/ EA=1:3) to give 2'-deoxy-3'-*O*, *N*⁴-di(4-methoxytrityl)-2'-fluoroarabinocytidine (1.7 g, 85%).
Pyridine (2.55 mL, 32.3 mmol) and Dess-Martin (1.1 g, 1.2 eq) was added into a solution of 2'-deoxy-3'-*O*, *N*⁴-di(4-methoxytrityl)-2'-fluoroarabinocytidine (1.7 g, 2.15 mmol) in anhydrous CH₂Cl₂ (15 mL) at 0 °C under N₂. The reaction mixture was stirred at RT for 2h and TLC showed the reaction was completed. Then the reaction mixture was diluted with EA. The organic layer was washed with 10% Na₂S₂O₃ twice, followed by water and brine, dried over anhydrous Na₂SO₄ and concentrated in vacuo to give the residue which was purified by silica gel (hexane/EA=1/3) to give 2'-deoxy-5-*C*,5'-*O-*didehydro-3'-*O*, *N*⁴-di(4-methoxytrityl)-2'-fluoroarabinocytidine (1.15 g, 68%).

### Step 3. Preparation of 2'-deoxy-2'-fluoro-5'-C-methylarabinocytidine

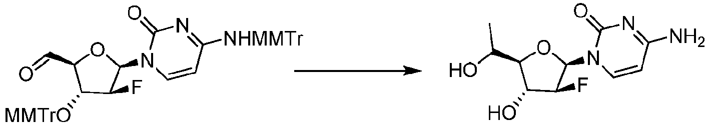

MeMgBr (4.17 mL, 5.84 mmol) was added dropwise into a solution of 2'-deoxy-5-C,5'-O-didehydro-3'-O, *N*⁴-di(4-methoxytrityl)-2'-fluoroarabinocytidine (1.15 g, 1.46 mmol, 1 eq) in anhydrous THF (25 mL) which was cooled by ice-EtOH bath under N₂. The reaction mixture was stirred at RT for 5h and TLC showed the reaction was completed. Then the reaction mixture was quenched with sat. NH₄Cl. EA was added to the mixture for extracting. The organic layer was washed with water and followed by brine, dried over anhydrous Na₂SO₄ and concentrated in vacuo to give the residue which was purified by silica gel (hexanes/EA=1/1 to 1/3) to give 2'-deoxy-3'-*O*, *N*⁴-di(4-methoxytrityl)-2'-fluoro-5'-C-methylarabinocytidine (1.0 g, 85%)
A solution of 2'-deoxy-3'-*O*,*N*⁴-di(4-methoxytrityl)-2'-fluoro-5'-*C-*methylarabinocytidine (200 mg, 0.24 mmol) in AcOH/H₂O (v/v = 4:1, 10 mL) was stirred at 50 °C overnight. TLC showed the reaction was completed. The solvent was evaporated in vacuo and the residue was diluted with water, extracted with EA twice to remove some impurity. The water layer was concentrated in vacuo to give the residue which was purified by Chromatography on silica with 5-12% MeOH in DCM gave give 2'-deoxy-2'-fluoro-5'-C-methylarabinocytidine (61mg). ¹H NMR (DMSO-d₆): 1.14 (d, J = 8.0 Hz, 3H), 3.53 (t, J = 5.2 Hz, 3H), 3.74 (br s, 1H), 4.11-4.35 (m, 1H), 4.79-5.00 (m, 2H), 5.71-5.82 (m, 2H), 6.01, 6.07 (each d, J = 3.6 Hz, 1H), 7.57 & 7.74 (each dd, J= 1.6, 7.6 Hz, 1H).

### Example 19

### Preparation of 2'-deoxy-2',2'-difluoro-5'(S)-C-methylcytidine (19)

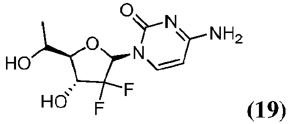

### Step 1. Preparation of 5'-O-(t-butyldimethylsilyl)-2'-deoxy-2',2'-difluoro-3'-O-,N⁴-di(4-methoxytrityl)cytidine

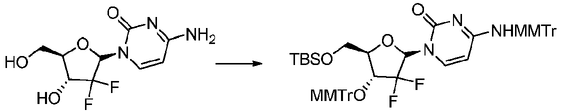

To an ice-cold solution of 2'-deoxy-2',2'-difluorocytidine (51.0 g, 170.7 mmol) in anhydrous pyridine (500 mL) was added TBSCl (32 g, 208 mmol) in small portions under N₂. The reaction mixture was stirred at RT overnight. The solvent was removed under vacuum and the residue was diluted with EA (1000 mL), washed with water and brine. The organic layer was separated, dried over anhydrous Na₂SO₄ and filtered. The filtrate was concentrated in vacuum to give crude 5'-O-(t-butyldimethylsilyl)-2'-deoxy-2',2'-difluorocytidine (63 g, 96%) as a white solid which was used without further purification.

To a mixture of 5'-*O*-(*t*-butyldimethylsilyl)-2'-deoxy-2',2'-difluorocytidine (60 g, 160 mmol), AgNO₃ (77.8 g, 510 mmol) and collidine (159.8 g, 1.32 mol) in anhydrous DCM (800 mL) was added MMTrCl (156.8g, 510 mmol) in small portions under N₂. The reaction mixture was stirred at RT overnight. The reaction mixture was filtered through a Buchner Funnel and the filtrate was washed with sat. NaHCO₃ solution and followed by brine. The organic layer was separated, dried over anhydrous Na₂SO₄ and filtered. The filtrate was concentrated in vacuum to give the residue which was purified by silica gel column (PE/EA = 3/1 to 2/1) to give crude 5'-*O*-(*t*-butyldimethylsilyl)-2'-deoxy-2',2'-difluoro-3'-*O*-,*N*⁴-di(4-methoxytrityl)cytidine (200 g).

### Step 2. Preparation of 2'-deoxy-5'-C,5'-O-didehydro-2',2'-difluoro-3'-O-,N⁴-bis(4-methoxytrityl)cytidine

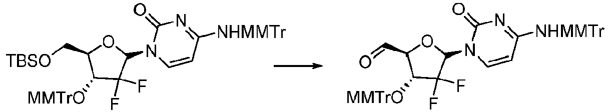

To an ice-cold solution of 5'-O-(t-butyldimethylsilyl)-2'-deoxy-2',2'-difluoro-3'-*O*-,*N*⁴-di(4-methoxytrityl)cytidine (200 g, 210 mmol) in anhydrous THF (322 mL) was added TBAF (1 M solution in THF, 330 mmol) dropwise under N₂. The reaction mixture was stirred at RT overnight. The solvent was removed and the residue was dissolved in EA (800 mL). The solution was washed with water and brine. The organic layer was separated, dried over anhydrous Na₂SO₄ and filtered. The filtrate was concentrated in vacuum to give a residue which was purified by silica gel column (CH₂Cl₂/EA = 10/1 to 5/1) to give the 2'-deoxy-2',2'-difluoro-3'-*O*-,*N⁴*-di(4-methoxytrityl)cytidine (128 g, 73%); ¹H NMR (400 MHz) (CDCl₃): δ 7.45-7.39 (m, 4H), 7.35-6.91 (m, 29H), 6.76 (dd, J = 8.8 Hz, 2.4 Hz, 4H), 6.24 (t, J = 8.0 Hz, 1H), 4.93 (d, J = 8.0 Hz, 1H), 4.20 (dd, J = 15.2 Hz, 9.2 Hz, 1H), 3.72 (d, J = 4.0 Hz, 6H), 3.27 (d, J = 13.2 Hz, 1H), 2.84 (d, J =12.4 Hz, 1H).

To a solution of pyridine (2.85 g, 36 mmol) in anhydrous DMSO (30 mL) at 10 °C was added dropwise TFA (2.05 g, 18 mmol). The mixture was stirred at RT until a clear solution formed. The solution was added dropwise into a solution of 2'-deoxy-2',2'-difluoro-3'-*O*-,*N⁴*-di(4-methoxytrityl)cytidine (24.2 g, 30 mmol) and DCC (18.6 g, 90 mmol) in anhydrous DMSO at 10 °C. The mixture was stirred at RT for 12 hours as checked by TLC. The mixture was quenched with water (200 mL) and stirred for 1hour at 10 °C. The precipitate was removed by filtration and the filtrate was extracted by EtOAc (1000 ml). The combined organic layer was washed by brine (200 mL) and dried by anhydrous Na₂SO₄. The solution was concentrated and the residue was purified by column (silica gel, EtOAc: Petro ether = 1/1 to 2/1) to give 2'-deoxy-5'-*C*,5'-*O-*didehydro-2',2'-difluoro-3'-*O*-,*N*⁴-bis(4-methoxytrityl)cytidine (21 g, 88%) which was used in the next step without any further purification.

### Step 3. Preparation of 2'-deoxy-2',2'-difluoro-5'-C-methylcytidine

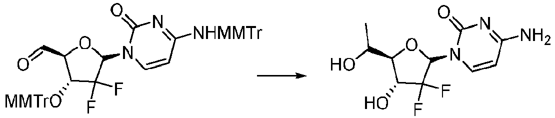

To an ice-EtOH bath cold solution of 2'-deoxy-5'-*C*,5'-*O*-didehydro-2',2'-difluoro-3'-*O*-,*N⁴*-di(4-methoxytrityl)cytidine (21 g, 26.08 mmol) in anhydrous THF (200 mL) was added MeMgBr (3 M solution in ether, 31.3 mL, 78.23 mmol) dropwise under N₂. The reaction mixture was stirred at RT overnight. The mixture was quenched by sat. NH₄Cl and extracted with EA (500 mLx3). The combined organic layer was dried over anhydrous Na₂SO₄ and concentrated. The resulting residue was purified by silica gel column (EA: PE =10/1 to 3/2) to give the 2'-deoxy-2',2'-difluoro-3'-*O*-,*N⁴*-di(4-methoxytrityl)-5'-C-methylcytidine (13 g, 61%, major : minor = 93:7); ¹H NMR (400 MHz) (CDCl₃): δ 7.41-7.05 (m, 27H), 6.77-6.74 (m, 4H), 6.22 (t, J = 8.8 Hz, 1H), 4.91 (d, J = 7.6 Hz, 1H), 4.20-4.15 (m, 1H), 3.74-3.69 (m, 6H), 3.03-3.00 (m, 1H), 0.98 (d, J =7.2 Hz, 3H).

2'-Deoxy-2',2'-difluoro-3'-*O*-,*N⁴*-di(4-methoxytrityl)-5'-*C*-methylcytidine (4.1 g, 5 mmol) was dissolved in 50 mL AcOH/H₂O (v/v = 4:1). The mixture was stirred at 50 °C overnight. The solvent was removed under vacuum and the residue was diluted with water (30 mL), extracted with EA (20 mLx2) to remove some impurity. 2'-Deoxy-2',2'-difluoro-5'(S)-C-methylcytidine (1.2 g, 87%) was obtained after column separation. ¹H NMR (400 Hz) (MeOD): δ 7.93 (d, J = 7.6 Hz, 1H), 6.24 (t, J = 7.6 Hz, 1H), 5.95 (d, J = 7.6 Hz, 1H), 4.30-4.22 (m, 1H), 4.05-4.00 (m, 1H), 3.74 (dd, J = 8.4 Hz, 2.8 Hz, 1H), 1.37 (d, J =6.4 Hz, 3H).

### Example 20

### Preparation of 2'-deoxy-2',2'-difluoro-5'(R)-C-methylcytidine (20)

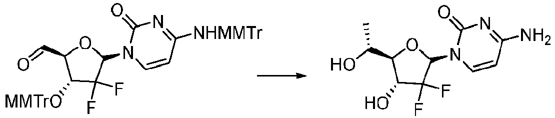

To an ice-EtOH bath cold solution of 2'-deoxy-5'-*C*,5'-*O*-didehydro-2',2'-difluoro-3'-*O*-,*N⁴*-di(4-methoxytrityl)cytidine (6.0 g, 7.4 mmol) in anhydrous THF (30 mL) was added MeMgBr (3M solution in ether) (10 mL, 30 mmol) dropwise under N₂. After addition, the reaction mixture was stirred at RT overnight. Then the reaction was quenched by sat. NH₄Cl. The mixture was extracted with EA (100 mLx2). The combined organic layer was dried over anhydrous Na₂SO₄ and concentrated to give a residue which was purified by silica gel column (PE/EA=3/1 to 1/1) to give 2'-deoxy-2',2'-difluoro-3'-*O*-,*N⁴*-di(4-methoxytrityl)-5'(*R* and *S*)-*C*-methylcytidine (3.6 g, 58.8%). ¹H NMR (400 MHz, CDCl₃): δ 7.48-7.08 (m, 26H), 6.80-6.84 (m, 4H), 6.28 (t, J = 8.8 Hz, 1H), 4.99 (d, J = 7.6 Hz, 1H), 4.25-4.20 (m, 1H), 3.81-3.79 (m, 7H), 3.77 (s, 3H), 3.12-3.07 (m, 1H), 1.05 (d, J = 6.8 Hz, 3H).
2'-Deoxy-2',2'-difluoro-3'-*O*-,*N⁴*-di(4-methoxytrityl)-5'(*R* and S)-C-methylcytidine (3 g, 3.65 mmol) was dissolved in 20 mL AcOH/H₂O (v/v = 4:1). The mixture was stirred at 50 °C overnight. The solvent was removed under vacuum and the residue was diluted with water (10 mL) and washed with EA (10 mLx2). The aqueous layer was lyophilized and the residue was purified by prep. SFC to give 2'-deoxy-2',2'-difluoro-5'(S)-C-methylcytidine (300 mg, 29.7%) and 2'-deoxy-2',2'-difluoro-5'(*R*)-*C-*methylcytidine (80 mg, 7.9%), both as white solid. 5'(*R*)-isomer: ¹H NMR (400 Hz, CD₃OD): δ 7.89 (d, J = 7.6 Hz, 1H), 6.19 (t, J = 7.6 Hz, 1H), 5.91 (d, J = 7.6 Hz, 1H), 4.17-4.25 (m, 1H), 3.97-3.99 (m, 1H), 3.69 (dd, J = 8.4 Hz, 2.8 Hz, 1H), 1.32 (d, J = 6.4 Hz, 3H). ESI-MS: m/z 555 [2M + H] ⁺, 278 [M + H]⁺.

### Example 21

### Preparation of 2'-deoxy-2',2'-difluoro-5'(S)-C-methyluridine (21)

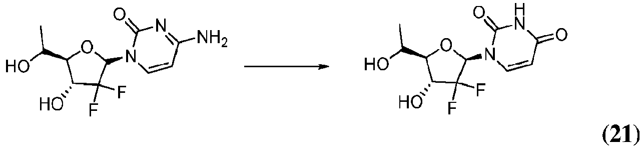

A solution of **2'-deoxy-2',2'-difluoro-5'(*S*)-*C*-methylcytidine** (1 g, 3.6 mmol), acetic anhydride (2.2 g, 21.6 mmol), 4-(dimethylamino) pyridine (DMAP, 12 mg, 0.1 mmol), and pyridine (20 mL) was stirred until disappearance of the starting material. The mixture was quenched with saturated aqueous NaHCO₃ solution. The aqueous layer was extracted with EA and the organic layer was washed with brine dried over MgSO₄, and concentrated under vacuum. The crude product was purified by flash chromatography to give 1.35 g of 2'-deoxy-3',5'-diacetyl-2',2'-difluoro-5'(*S*)-*C*-methylcytidine at 93% yield.
A solution of 2'-deoxy-3',5'-diacetyl-2',2'-difluoro-5'(*S*)-*C*-methylcytidine (1 g, 2.48 mmol) in DME (30 mL) and H₂O (20 mL) was heated in a sealed flask for 9 h at 125 °C. Volatiles were evaporated, and chromatography of the residue gave 600 mg of 2'-deoxy-3',5'-diacetyl-2',2'-difluoro-5'(*S*)-*C*-methyluridine (67%) as a colorless solid, which was dissolved in 20 mL saturated NH₃/MeOH solution. The mixture was stirred at 0 °C overnight. The solvent was removed under vacuum. Purification by flash chromatography on silica gel gave 440 mg (95%) of 2'-deoxy-2',2'-difluoro-5'(*S*)-*C-*methyluridine; ¹H NMR (400 Hz) (DMSO-d6): δ 11.55(s, 1H), 7.86(d, J = 8 Hz, 1H), 6.26 (d, J = 4.8 Hz, 1H), 6.03 (t, J = 7.8 Hz, 1H), 5.22 (d, J = 5.2 Hz, 1H), 4.17-4.13 (m, 1H), 3.85-3.81 (m, 1H), 3.65 (dd, J = 8.4 Hz, 2.8 Hz, 1H), 1.18 (d, J =6.8 Hz, 3H).

### Example 22

### Preparation of 2'-deoxy-2',2'-difluoro-5-ethynyl-5'(S)-C-methylcytidine (22)

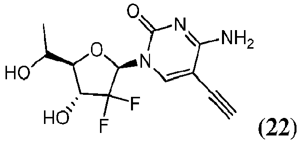

### Step 1. Preparation of 2'-deoxy-3',5'-O-diacetyl-2',2'-difluoro-5-iodo-5'(S)-C-methylcytidine

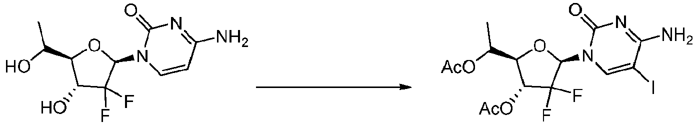

A solution of **2'-deoxy-2',2'-difluoro-5'(*S*)-*C*-methylcytidine** (1 g, 3.6 mmol), acetic anhydride (2.2 g, 21.6 mmol), 4-(dimethylamino) pyridine (DMAP, 12 mg, 0.1 mmol), and pyridine (20 mL) was stirred until disappearance of the starting material. The mixture was quenched with a saturated aqueous NaHCO₃ solution. The aqueous layer was extracted with diethyl ether and the organic layers were washed with water, dried over MgSO₄, and concentrated under vacuum. The crude product was purified by flash chromatography to give 1.35 g of 2'-deoxy-2',2'-difluoro-5'(*S*)-*C*-methyl-3',5'-*O*,*N*⁴-triacetylcytidine in 93% yield.
2'-Deoxy-2',2'-difluoro-5'(*S*)-*C*-methyl-3',5'-*O*,*N*⁴-triacetylcytidine (1.5 g, 3.7 mmol) was dissolved into a solution of I₂ (3 g, 11.8 mmol) in methanol (300 mL). The reaction was refluxed and monitored by TLC. Upon completion, a small quantity of sodium thiosulfate was added to quench the reaction. The solvent was removed and the residue was purified by column chromatography on silica gel to give 500 mg of 2'-deoxy-3',5'-O-diacetyl-2',2'-difluoro-5-iodo-5'(S)-C-methylcytidine in 27% yield. Step 2. Preparation of 2'-deoxy-2',2'-difluoro-5-ethynyl-5'-*C*-methylcytidine 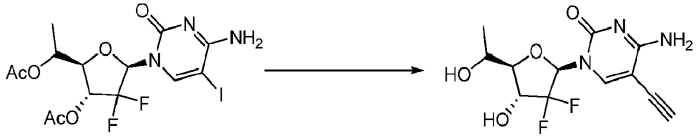 A solution of 2'-deoxy-3',5'-*O*-diacetyl-2',2'-difluoro-5-iodo-5'(*S*)-*C-*methylcytidine (500 mg, 1.03 mmol), acetic anhydride (648 mg, 6.1 mmol), 4-(dimethylamino) pyridine (DMAP, 12 mg, 0.1 mmol), and pyridine (20 mL) was stirred until disappearance of the starting material. The mixture was quenched with a saturated aqueous NaHCO₃ solution. The aqueous layer was extracted with diethyl ether and the organic layers were washed with water, dried over MgSO₄, and concentrated under vacuum. The crude product was purified by flash chromatography to give 500 mg of 2'-deoxy-2',2'-difluoro-5-iodo-5'(*S*)-*C*-methyl-3',5'-*O*-,*N⁴*-triacetylcytidine in 92% yield.
To a nitrogen degassed solution of triethylamine (303 mg, 3eq) in CH₃CN (30 mL) were added ethynyltrimethylsilane (196 mg, 2eq), 2'-deoxy-2'-deoxy-2',2'-difluoro-5-iodo-5'(*S*)-*C*-methyl-3',5'-*O*-,*N⁴*-triacetylcytidine (500 mg, 1eq), Pd (PPh₃)₂ Cl₂ (8.4 mg, 0.012 eq), and CuI (2.3 mg, 0.012 eq), and the mixture was stirred at 25 °C for 12 h. After removal of the solvent, the residue was filtered, concentrated, and purified by flash chromatography on silica gel eluting with PE:EtOAc (2:1) to give 200 mg (42%) of 2'-deoxy-2',2'-difluoro-5'(*S*)-*C*-methyl-3',5'-*O*-,*N⁴-*triacetyl-5-(trimethylsilylethynyl)cytidine as a white solid, which was dissolved in 20 mL saturated NH₃/MeOH solution. The mixture was stirred at RT overnight. The solvent was removed under vacuum. Purification by flash chromatography on silica gel gave 110 mg (91%) of 2'-deoxy-2',2'-difluoro-5-ethynyl-5'-*C*-methylcytidine; ¹H NMR (400 Hz) (MeOD-d4): δ 8.34 (s, 1H), 6.18 (t, J = 7.6 Hz, 1H), 4.26-4.19 (m, 1H), 4.00-3.98 (m, 1H), 3.84 (s, 1H), 3.72 (dd, J = 8.4 Hz, 2.8 Hz, 1H), 1.34 (d, J =6.8 Hz, 3H).

### Example 23

### Preparation of 2'-deoxy-2',2'-difluoro-5-ethyl-5'(S)-C-methylcytidine (23)

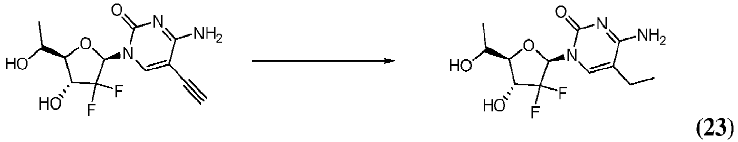

To a solution of 2'-deoxy-2',2'-difluoro-5-ethynyl-5'(*S*)-*C*-methylcytidine (50 mg) in EA (50 mL) was added Pd/C (50 mg) at 25 °C. Then the mixture was stirred under H₂ atmosphere at 1 atm for 4 h. The solvent was removed under vacuum. Purification by flash chromatography on silica gel gave 40 mg of 2'-deoxy-2',2'-difluoro-5-ethyl-5'-C-methylcytidine (79%); ¹H NMR (400 Hz) (MeOD-d4): δ 7.80 (s, 1H), 6.21 (t, J = 7.6 Hz, 1H), 4.29-4.21 (m, 1H), 4.03-3.95 (m, 1H), 3.71 (dd, J = 8.8 Hz, 2.8 Hz, 1H), 2.35(q, 2H), 1.34(d, J = 6.8 Hz, 3H), 1.17 (t, J = 7.4Hz, 3H).

### Example 24

### Preparation of 2'-deoxy-2',2'-difluoro-5'(S)-C-methylthymidine (24)

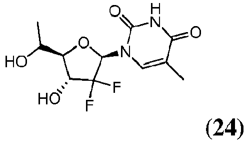

### Step 1. Preparation of 2'-deoxy-3',5'-diacetyl-2',2'-difluoro-5-iodo-5'-C-methyluridine

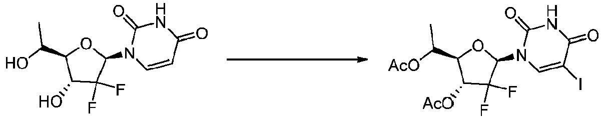

A solution of 2'-deoxy-2',2'-difluoro-5'(*S*)-*C*-methyluridine (200 mg, 0.72 mmol), acetic anhydride (466 mg, 4.3 mmol), 4-(dimethylamino) pyridine (DMAP, 12 mg, 0.1 mmol), and pyridine (20 mL) was stirred until disappearance of the starting material. The mixture was quenched with a saturated aqueous NaHCO₃ solution. The aqueous layer was extracted with diethyl ether and the organic layers were washed with water, dried over MgSO₄, and concentrated under vacuum. The crude product was purified by flash chromatography to give 236 mg of 2'-deoxy-3',5'-diacetyl-2',2'-difluoro-5'(S)-C-methyluridine in 91% yield.
A mixture of 230 mg (0.64 mmol) of 2'-deoxy-3',5'-diacetyl-2',2'-difluoro-5'(S)-C-methyluridine, 210 mg (0.83 mmol) of I₂, and 766 mg of CAN in 25 mL of MeCN was stirred at ambient temperature. When iodination was complete (as monitored by TLC), solvent was evaporated under reduced pressure. The resulting residue was treated with a cold mixture of ethyl acetate (15 mL), 5% NaHSO₃/H₂O (5 mL), and saturated NaCl/H₂O (5 mL). The organic layer was separated and the aqueous layer was extracted with EtOAc. The crude products were purified by silica gel column chromatogrphy to give 245 mg of 2'-deoxy-3',5'-diacetyl-2',2'-difluoro-5-iodo-5'(*S*)-*C*-methyluridine in 80% yield. Step 2. Preparation of **2'-deoxy-2',2'-difluoro-5'(*S*)-*C*-methylthymidine** 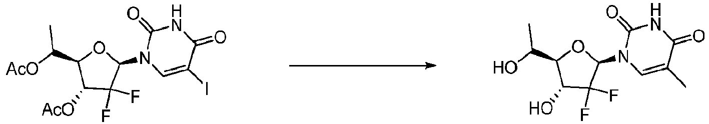 To a mixture of 2'-deoxy-3',5'-diacetyl-2',2'-difluoro-5-iodo-5'(*S*)-*C-*methyluridine (245 mg, 0.5 mmol) and Pd (PPh₃)Cl₂ (40 mg) in anhydrous THF (30 mL) was refluxed under Ar atmosphere for 10 min. Then AlMe₃ was added dropwise by a syringe through septum and the solution was refluxed overnight. After cooled to RT, water (20 mL) was added to the reaction and the mixture was extracted with DCM. The extract was dried and evaporated under reduced pressure. The residue was purified by Prep-TLC to give 2'-deoxy-3',5'-diacetyl-2',2'-difluoro-5(*S*)'-*C*-methylthymidine (50 mg) at 26% yield.
2'-Deoxy-3',5'-diacetyl-2',2'-difluoro-5'(*S*)-*C*-methylthymidine (50 mg, mmol) was dissolved in 20 mL of saturated NH₃/MeOH solution. The mixture was stirred at 0 °C overnight. The solvent was removed under vacuum. Purification by flash chromatography on silica gel gave 30 mg of **2'-deoxy-2',2'-difluoro-5'(*S*)-*C*-methylthymidine** (77%); ¹H NMR (400 Hz) (DMSO-d6): δ7.57(s 1H), 6.11 (t, J = 8 Hz, 1H), 4.30-4.22 (m, 1H), 4.02-3.96 (m, 1H), 3.70 (dd, J = 8.4 Hz, 2.8 Hz, 1H), 1.87 (s, 3H), 1.33 (d, J =6.4 Hz, 3H).

### Example 25

### Preparation of 2'-deoxy-2',2'-difluoro-5-vinyl-5'(S)-C-methylcytidine (25)

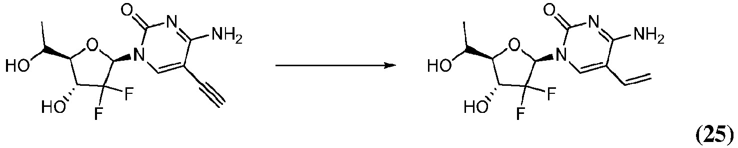

To a solution of 2'-deoxy-2',2'-difluoro-5-ethynyl-5'-*C*-methylcytidine (30 mg, 1 eq) in EA (50 mL) was added Lindlar Pd (30 mg) at 25 °C. Then the mixture was stirred under H₂ atmosphere at 1 atm for 4 h. The solvent was removed under vacuum. Purification by flash chromatography on silica gel gave 22 mg of 2'-deoxy-2',2'-difluoro-5-vinyl-5'(S)-C-methylcytidine (73%). ¹H NMR (400 Hz) (MeOD-d4): δ 8.24 (s, 1H), 6.51 (m, 1H), 6.21 (t, J = 14.5 Hz, 1H), 5.61 (dd, J = 17.2 Hz, 1.2 Hz, 1H), 5.27 (dd, J = 10.8 Hz, 1.2 Hz, 1H), 4.32-4.24 (m, 1H), 4.04-4.00 (m, 1H), 3.74 (dd, J = 8.8 Hz, 2.8 Hz, 1H), 1.35(d, J = 6.8 Hz, 3H).

### Example 26

### Preparation of 2'-deoxy-2',2'-difluoro-5'(S)-C-methyl-5-vinyluridine (26)

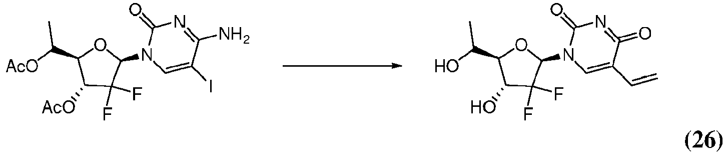

To a solution of PdCl₂(PPh₃)₂ (15.8 mg, 0.022 mmol) in acetonitrile (10 mL) was added 2'-deoxy-3',5'-*O*-diacetyl-2',2'-difluoro-5-iodo-5'(*S*)-*C*-methyluridine (110 mg, 0.23 mmol) and ethenyltributylstannane (143 mg, 0.45 mmol) . The mixture was heated up to reflux and stirred overnight, filtered through celite and evaporated under reduced pressure to remove solvent. The oily residue was purified by silica gel column chromatography to give 50 mg of 2'-deoxy-3',5'-*O*-diacetyl-2',2'-difluoro-5-vinyl-5'(*S*)-C-methyluridine in 50% yield, which was dissolved in 20 mLof saturated NH₃/MeOH solution. The mixture was stirred at 0 °C overnight. The solvent was removed under vacuum. Purification by flash chromatography on silica gel gave 20 mg of product, which was further purified by flash chromatography on silica gel to give 7 mg 2'-deoxy-2',2'-difluoro-5'(S)-C-methyl-5-vinyluridine (18%); ¹H NMR (400 Hz) (DMSO-d6): δ8.17(s, 1H), 6.50 (dd, J = 17.6 Hz, 11.2 Hz, 1H), 6.16 (t, J = 7 Hz, 1H), 6.16 (t, J = 7 Hz, 1H), 5.97 (d, J = 17.6 Hz, 1H), 5.20 (d, J = 11.2 Hz, 1H), 4.35-4.27 (m, 1H), 4.04-3.98 (m, 1H), 3.75 (d, J = 8.8 Hz, 1H), 1.34 (d, J =6.4 Hz, 3H).

### Example 27

### Preparation of 2'-deoxy-2',2'-difluoro-5-ethyl-5'(S)-C-methyluridine (27)

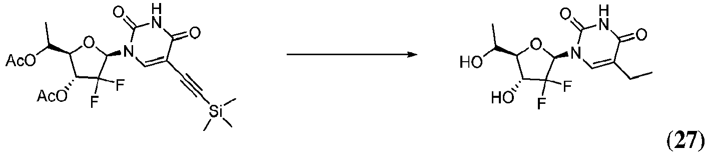

2' -Deoxy-3',5'- *O*-diacetyl-2' ,2' -difluoro-5-(trimethylsilylethynyl)-5'(*S*)-*C-*methyluridine (79 mg, 0.18 mmol) was dissolved in Sat. NH₃/MeOH (20 mL). The mixture was stirred at 25 °C for 20 h. The solvent was removed and the crude dissolved in MeOH (10 mL). Pd/C (5%, 10 mg) was added and the mixture was stirred at 25 °C under H₂ (1 atm) for 30 h. Then the catalyst was removed by filtration and the filtrate was evaporated to dryness. The residue was purified by columnon silica gel (DCM/MeOH = 1:10) to give 2'-deoxy-2',2'-difluoro-5-ethyl-5'(*S*)-*C*-methyluridine (19 mg, 36% over 2 steps); ¹H NMR (400 Hz, D₂O): δ 7.41 (s, 1H), 6.02 (t, J = 8 Hz, 1H), 4.15-4.23 (m, 1H), 3.93 (dd, J1 = 4.4 Hz, J2 = 6.4 Hz, 1H), 3.68 (dd, J1 = 4.4 Hz, J2 = 8.4 Hz, 1H), 2.15 (q, J = 7.2 Hz, 2H), 1.17 (d, J = 6.4 Hz, 3H), 0.91 (t, J = 7.2 Hz, 3H); LCMS (ESI): 307 [M + H]⁺.

### Example 28

### Preparation of 2'-deoxy-5'(S)-C-methyl-2',2',5-trifluorouridine (28)

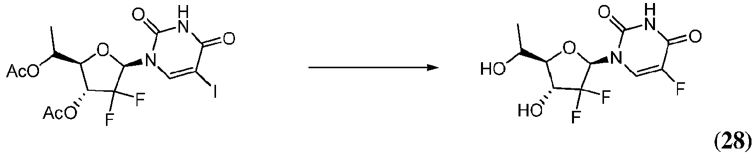

2'-Deoxy-3',5'-*O*-diacetyl-2',2'-difluoro-5-iodo -5'(S)-C-methyluridine (320 mg, 0.66 mmol), hexamethylditin (429 mg, 1.32 mmol), bis(triphenylphosphine)palladium dichloride (46 mg, 0.066 mmol), and 1,4-dioxane (20 mL) were stirred at 80 °C for 2 h. Upon completion, the solvent was removed at 45°C under reduced pressure and the residue was purified on Prep-TLC to provide 245 mg of 2'-deoxy-3',5'-*O*-diacetyl-2',2'-difluoro-5-(trimethylstannyl)-5'(*S*)-*C*-methyluridine in 71% yield.
To a dried round-bottomed flask (25 mL), 2'-deoxy-3',5'-*O*-diacetyl-2',2'-difluoro-5-(trimethylstannyl)-5'(S)-C-methyluridine (245 mg, 0.47 mmol), MeCN (15 mL) and Selectfluor (177 mg, 0.51 mmol) were added. The mixture was stirred at 55 °C for 10 h, during which the progress of the reaction was followed by TLC, until the starting material had been consumed (∼10 h). The solvent was removed and the crude mixture was purified by column chromatography to give 50 mg of 2'-deoxy-3',5'-*O*-diacetyl-5'(*S*)-*C-*methyl-2',2',5-trifluorouridine (30%).
2'-Deoxy-3',5'-*O*-diacetyl-5'(*S*)-C-methyl-2',2',5-trifluorouridine (50 mg, mmol) was dissolved in 20 mL saturated NH₃/MeOH solution. The mixture was stirred at 0 °C overnight. The solvent was removed under vacuum. Purification by flash chromatography on silica gel gave 30 mg of 2'-deoxy-5'(S)-C-methyl-2',2',5-trifluorouridine (77%); ¹H NMR (400 Hz) (MeOD-d4): δ8.25(d, J = 6.8 Hz 1H), 6.10 (t, J = 6.8 Hz, 1H), 4.31-4.22 (m, 1H), 4.02-3.97 (m, 1H), 3.72 (dd, J = 8.4 Hz, 2.4 Hz, 1H), 1.33 (d, J =6.4 Hz, 3H); ¹⁹F NMR (400 Hz) (MeOD-d4): δ-121.15 (t, J = 44.7 Hz, 2F), δ-169.89(1F).

### Example 29

### Preparation of 2'-deoxy-2',2'-difluoro-5'-O-isobutyryl-5'(S)-C-methylcytidine (29)

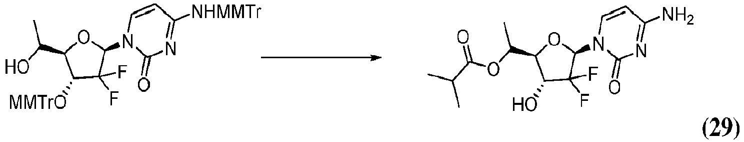

To a mixture of 2'-deoxy-2',2'-difluoro-3'-*O*,*N⁴-*di(4'-methoxytrityl)-5'(*S*)-*C-*methylcytidine (1.64 g, 2 mmol), isobutyric acid (211 mg, 2.4 mmol) and DMAP (0.12 g, 1 mmol) in DCM (20 mL) was added EDCI (1.15 g, 6 mmol). The mixture was stirred at RTfor 16 hours under N₂ as checked by TLC. Then the mixture was washed with Sat. NaHCO₃ aq. solution and followed by brine. The organic layer was dried over Na₂SO₄ and concentrated. The solvent was removed and the residue was purified by column (PE: EA = 1:1) to give 2'-deoxy-2',2'-difluoro-3'-*O*,*N⁴*-di(4'-methoxytrityl)-5'-*O*-isobutyryl-5'(S)-C-methylcytidine (1.2 g, 67%).
2'-Deoxy-2',2'-difluoro-3'-*O*,*N⁴*-di(4'-methoxytrityl)-5'-*O*-isobutyryl-5'(*S*)-*C-*methylcytidine (900 mg) was dissolved in 80% HOAc (20 mL). The mixture was stirred at 60 °C overnight as checked by TLC. The solvent was removed under reduced pressure and the residue was purified by prep. HPLC (HCOOH system) to give 2'-deoxy-2',2'-difluoro-5'-O-isobutyryl-5'(S)-C-methylcytidine as white solids (120 mg, 35%); 1H NMR (D₂O, 400 M Hz) δ 7.46 (d, J = 7.6 Hz, 1H), 6.24 (t, J = 8.0 Hz, 1H), 6.15 (d, J = 7.6 Hz, 1H), 5.28 (dt, J1 = 6.4 Hz, J2 = 11.2 Hz, 1H), 4.31 (dt. J1 = 8 Hz, J2 = 12.8 Hz, 1H), 4.15 (dd, J1 = 4.4 Hz, J2 = 8.0 Hz, 1H), 2.67-2.74 (m, 1 H), 1.42 (d, J = 6.8 Hz, 3H), 1.18-1.21 (m, 6H); ESI-LCMS: m/z 348 [M + H]⁺, 370 [M + Na]⁺, 717 [2M + Na]⁺.

### Example 30

### Preparation of 2'-deoxy-2',2'-difluoro-5'(S)-C-methyl-3'-O-(L-valinyl)cytidine (30)

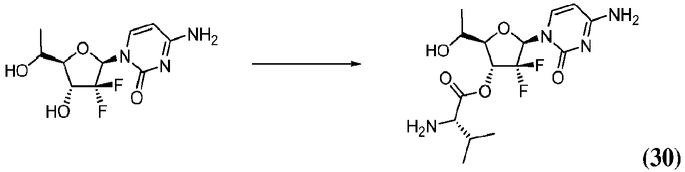

(*N-t*-Butoxycarbonyl)-*L*-valine (0.78 g, 3.6 mmol) and CDI (0.58 g, 3.6 mmol) were suspended in anhydrous THF (15 mL). The mixture was stirred at RT for 1 hour and then warmed to 50 °C. Stirring was continued for 30 mins. Then the mixture was cooled to RT and the solution was added into a solution of 2'-deoxy-2',2'-difluoro-5'(*S*)-*C-*methylcytidine (0.90 g, 3.25 mmol), DMAP (37 mg, 0.3 mmol) and TEA (10 mL) in anhydrous DMF (20 mL) dropwise at RT After addition the mixture was stirred at RT for 20 h and then concentrated under reduced pressure to remove THF and TEA. Then the solution was diluted in EA and washed with brine. The organic layer was dried over Na₂SO₄. The solvent was concentrated and the residue was purified by column (pure EA) to afford 3'-*O*-(*N*-*t*-butoxycarbonyl)-*L*-valinyl)-2'-deoxy-2',2'-difluoro-5'(*S*)-*C-*methylcytidine as a white foam (1.04 g, 67%), which was dissolved in a solution of HCl in EA (4 N, 150 mL). The mixture was stirred at RT for 10 hrs. The solvent was removed to afford crude product which was further purified by neutral prep. HPLC to afford 2'-deoxy-2',2'-difluoro-5'(*S*)-*C*-methyl-3'-*O*-(*L*-valinyl)cytidine as white solids (410 mg, 50%); 1H NMR (400MHz, D₂O) δ 7.80 (d, J = 7.6 Hz, 1H), 6.29 (t, J = 8.8 Hz, 1H), 6.13 (d, J = 7.6 Hz, 1H), 5.49-5.56 (m, 1H), 4.24-4.27 (m, 2H), 4.07-4.13 (m, 1H), 2.40-2.48 (m, 1H), 1.31 (d, J = 6.4 Hz, 3H), 1.07 (dd, J1 = 7.2 Hz, J2 = 11.2 Hz, 6H); ESI-MS: 753 [2M + H]⁺, 377 [M + H]⁺.

### Example 31

### Preparation of 2'-deoxy-2',2'-difluoro-5'(S)-C-methyl-5'-O-(L-valinyl)cytidine (31)

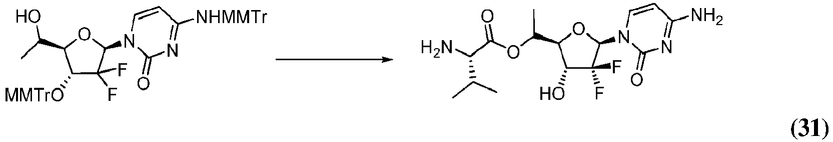

To a mixture of 2'-deoxy-2',2'-difluoro-3'-*O*,*N⁴*-di(4-methoxytrityl)-5'(*S*)-*C-*methylcytidine (2.0 g, 2.43 mmol), EDCI (933 mg, 4.86 mmol) and DMAP (179 mg, 1.46 mmol) in anhydrous DCM (20 mL) was added N-Boc-L-Val (529 mg, 2.43 mmol) under N₂. The reaction mixture was stirred at RT for 2h. The reaction mixture was washed with sat. NaHCO₃ solution and followed by brine. The organic layer was separated, dried over anhydrous Na₂SO₄ and filtered. The filtrate was concentrated in vacuum to give the residue which was purified by silica gel column (PE/EA=3/1 to 2/1) to give *5'-O-*(*N-*(*t-*butoxycarbonyl)-L-valinyl)-2'-deoxy-2',2'-difluoro-3'-*O,N*⁴-di(4-methoxytrityl)-5'(*S*)-*C-*methylcytidine (1.4 g, 56%).

To a solution of 5'-*O*-(*N*-(*t*-butoxycarbonyl)-L-valinyl)-2'-deoxy-2',2'-difluoro-3'-*O*,*N*⁴-di(4-methoxytrityl)-5'(*S*)-*C*-methylcytidine (1.1 g, 1.08 mmol) in EA (5 mL) was added 4 N HCl/EA (15 mL). The reaction mixture was stirred at RT overnight, concentrated into a residue which was purified by prep. HPLC (HCOOH system) to give 2'-deoxy-2',2'-difluoro-5'(*S*)-*C*-methyl-5'-*O*-(*L*-valinyl)cytidine (55 mg, 13.6%) as a white solid; ¹H NMR (400 Hz) (D₂O): δ 8.29 (S, 0.8H), 7.47 (d, J = 7.6 Hz, 1H), 5.99 (t, J = 8.0 Hz, 1H), 5.92 (d, J = 7.6 Hz, 1H), 5.32-5.25 (m, 1H), 4.25-4.17 (m, 1H), 3.98-3.93 (m, 2H), 2.25-2.17 (m, 1H), 1.29 (t, J = 6.4 Hz, 3H), 0.88 (d, J = 6.8 Hz, 1H), 0.85 (d, J = 6.8 Hz, 1H).

### Example 32

### Preparation of 2'-deoxy-2',2'-difluoro-3',5'-O-di(isobutyryl)-5'(S)-C-methylcytidine (32)

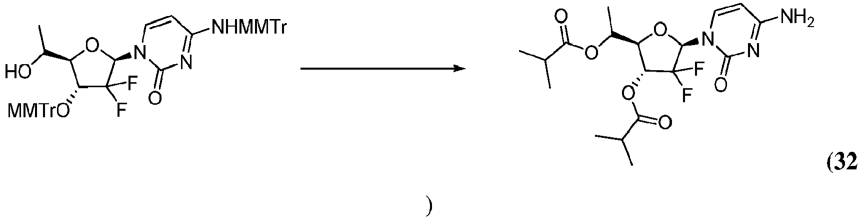

2'-Deoxy-2',2'-difluoro-3'-*O*-,*N⁴*-di(4-methoxytrityl)-5'-*C*-methylcytidine (5 g, 6.08 mmol) was dissolved in 30 mL of AcOH/H₂O (v/v = 4:1). The mixture was stirred at RT for 5h. The solvent was removed under vacuum and the residue was purified by silica gel column (PE/EA=1/1 to 1/5) to give 2'-deoxy-2',2'-difluoro- *N*⁴-(4-methoxytrityl)-5'(S)-C-methylcytidine (2.1g, 60%). ¹H NMR (400 Hz) (DMSO): δ 8.56 (S, 1H), 7.62 (d, J = 8.0 Hz, 1H), 7.26-7.09 (m, 13H), 6.80 (d, J = 8.8 Hz, 1H), 6.25 (d, J = 7.6 Hz, 1H), 6.13 (d, J = 6.8 Hz, 1H), 5.91 (t, J = 8.4 Hz, 1H), 5.05-5.03 (m, 1H), 4.08-4.04 (m, 1H), 3.77-3.75 (m, 1H), 3.68 (s, 3H), 3.51 (dd, J = 8.0, 2.8 Hz, 1H), 1.12 (d, J = 6.8 Hz, 1H).
To a mixture of 2'-deoxy-2',2'-difluoro- *N*⁴-(4-methoxytrityl)-5'(*S*)-*C-*methylcytidine (2.0 g, 3.64 mmol), EDCI (1.4 g, 7.28 mmol) and DMAP (270 mg, 2.18 mmol) in anhydrous DCM (20 mL) was added isobutyric acid (961 mg, 10.92 mmol) under N₂. The reaction mixture was stirred at RT for 3 h. The reaction mixture was washed with sat. NaHCO₃ solution and followed by brine. The organic layer was separated, dried over anhydrous Na₂SO₄ and filtered. The filtrate was concentrated in vacuum to give the residue which was purified by silica gel column (PE/EA=3/1 to 2/1) to give 2'-deoxy-2',2'-difluoro-3',5'-*O*-di(isobutyryl)- *N*⁴-(4-methoxytrityl)-5'(*S*)-*C-*methylcytidine (1.8 g, 71.7%), which was dissolved in 20 mL AcOH/H₂O (v/v = 4:1). The mixture was stirred at 50 °C overnight. The solvent was removed under vacuum and the residue was purified by prep. HPLC to give the 2'-deoxy-2',2'-difluoro-3',5'-*O-*di(isobutyryl-5'(*S*)-*C*-methylcytidine (480 mg, 48%). ¹H NMR (400 Hz) (MeOD): δ 7.84 (d, J = 7.6 Hz, 1H), 6.27 (t, J = 8.8 Hz, 1H), 6.15 (d, J = 7.6 Hz, 1H), 5.36-5.28 (m, 1H), 5.25-5.18 (m, 1H), 4.30 (dd, J = 6.8, 4.4 Hz, 1H), 2.75-2.67 (m, 1H), 2.65-2.54 (m, 1H), 1.33 (d, J = 6.8 Hz, 1H), 1.21-1.15 (m, 12H).

### Alternative method.

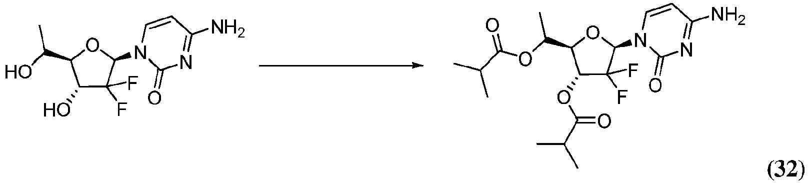

2'-Deoxy-2',2'-difluoro-5'(*S*)-*C*-methylcytidine (0.8 g, 2.89 mmol) was dissolved in 20 mL of DMF-DMA. The mixture was stirred at RT for 2h. The solvent was removed under vacuum to give the crude 2'-deoxy-2',2'-difluoro-*N*⁴-(*N,N-*dimethylaminomethylene)-5'-C-methylcytidine (998 mg) which was used for next step with no further purification.
To a mixture of 2'-deoxy-2',2'-difluoro-*N*⁴-(*N,N*-dimethylaminomethylene)-5'-C-methylcytidine (950 mg, 2.86 mmol), EDCI (1.1 g, 5.72 mmol) and DMAP (210 mg, 1.72 mmol) in anhydrous DMF (10 mL) was added isobutyric acid (756 mg, 8.58 mmol) under N₂. The reaction mixture was stirred at RT for 5h. The reaction was complex. Then EDCI (1.1 g, 5.72 mmol), DMAP (210 mg, 1.72 mmol) and isobutyric acid (756 mg, 8.58 mmol) was added into the solution and stirred at RT overnight. The reaction mixture was diluted with EA, washed with water and brine. The organic layer was separated, dried over anhydrous Na₂SO₄ and filtered. The filtrate was concentrated to give a crude 2'-deoxy-2',2'-difluoro-3',5'-*O*-di(isobutyryl)-*N*⁴-(*N,N*-dimethylaminomethylene)-5'(*S*)-*C-*methylcytidine (750 mg) which was used for the next step with no further purification.
2'-Deoxy-2',2'-difluoro-3',5'-*O*-di(isobutyryl)-*N*⁴-(*N,N-*dimethylaminomethylene)-5'(S)-C-methylcytidine (700 mg, 1.48 mmol) was dissolved in 10 mL AcOH/H₂O (v/v = 4:1). The mixture was stirred at 50°C overnight. The solvent was removed under vacuum and the residue was purified by prep. HPLC to give the 2'-deoxy-2',2'-difluoro-3',5'-*O*-di(isobutyryl)-5'(*S*)-*C*-methylcytidine (220 mg, 35.6%). ¹H NMR (400 Hz) (MeOD): δ 7.62 (d, J = 7.6 Hz, 1H), 6.32 (t, J = 8.8 Hz, 1H), 5.96 (d, J = 7.6 Hz, 1H), 5.30-5.25 (m, 1H), 5.24-5.17 (m, 1H), 4.25 (dd, J = 6.8, 4.0 Hz, 1H), 2.73-2.66 (m, 1H), 2.64-2.57 (m, 1H), 1.34 (d, J = 7.2 Hz, 1H), 1.22-1.17 (m, 12H).

### Example 33

### Preparation of 2'-deoxy-2',2'-difluoro-5'(S)-C-ethylcytidine (33)

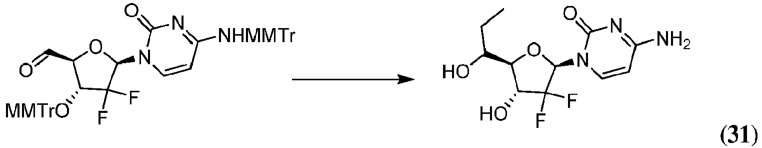

To an ice-EtOH bath cold solution of 2'-deoxy-5'-*C*,5'-*O*-didehydro-2',2'-difluoro-3'-*O*-,*N*⁴-di(4-methoxytrityl)cytidine ( 3 g, 3.72 mmol) in anhydrous THF (10 mL) was added EtMgBr (3 M solution in ether) (5 mL, 15 mmol) dropwise under N₂. The reaction mixture was stirred at RT overnight. The mixture was quenched by sat. NH₄Cl. The product was extracted with EA (50 mLx2). The combined organic layer was dried over anhydrous Na₂SO₄ and concentrated to give a residue which was purified by silica gel column (PE/EA=3/1 to 1/1) to give the 2'-deoxy-2',2'-difluoro-3'-*O*-,*N⁴*-di(4-methoxytrityl)-5'(S)-C-ethylcytidine (1.7 g, 54.6%).
2'-Deoxy-2',2'-difluoro-3'-O-,*N*⁴-di(4-methoxytrityl)-5'(*S*)-*C*-ethylcytidine (1.3 g, 1.56 mmol) was dissolved in 15 mL AcOH/H₂O (v/v = 4:1). The mixture was stirred at 50 °C overnight. The solvent was removed under vacuum and the residue was diluted with water (3 mL), extracted with EA (2 mLx2) to remove some impurity. The water layer was subjected to prep. HPLC separation to give 2'-deoxy-2',2'-difluoro-5'(*S*)-*C*-ethylcytidine (42 mg, 5%); ¹HNMR (400 Hz) (MeOD): δ 7.91 (d, J = 7.6 Hz, 1H), 6.18 (t, J = 8.0 Hz, 1H), 5.90 (d, J = 7.6 Hz, 1H), 4.28-4.22 (m, 1H), 3.78 (dd, J = 8.4 Hz, 2.4 Hz, 1H), 3.69-3.66 (m, 1H), 1.70-1.64 (m, 2H), 1.03 (t, J = 7.2 Hz, 3H).

### Example 34

### Preparation of 2'-deoxy-2',2'-difluoro-5'(R)-C-ethylcytidine (34)

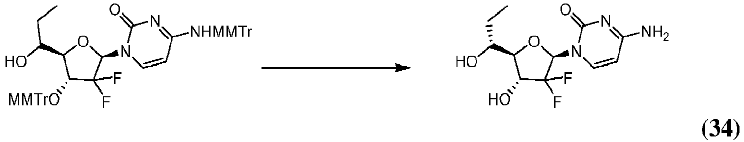

To an ice-cold suspension of CrO₃ (478 mg, 4.79 mmol) in anhydrous DCM (15 mL) was added anhydrous pyridine (0.77 mL, 9.57 mmol) and Ac₂O (0.45 mL, 4.79 mmol) under N₂. The mixture was stirred at RT for about 10 min until the mixture became homogeneous. The mixture was cooled to 0 °C and a solution of 2'-deoxy-2',2'-difluoro-3'-*O*-,*N⁴*-di(4-methoxytrityl)-5'(*S*)-*C*-ethylcytidine (1.0 g, 1.2 mmol) in anhydrous DCM (5 mL) was added. The resultant mixture was stirred at RT overnight. The reaction was complete detected by HPLC. The reaction mixture was diluted with EA (100 mL), washed with NaHCO₃ solution twice and brine. The organic layer was separated, dried over anhydrous Na₂SO₄ and filtered. The filtrate was concentrated in vacuum to give a residue which was purified by silica gel column (EA/PE= 1/2) to give the desired 2'-deoxy-5'-*C*,5'-*O*-didehydo-2',2'-difluoro-3'-*O*-,*N⁴*-bis(4-methoxytrityl)-5'-C-ethylcytidine (505 mg, 50.6%).
To an ice-cold solution of 2'-deoxy-5'-*C*,5'-*O*-didehydo-2',2'-difluoro-3'-*O*-,*N⁴*-bis(4-methoxytrityl)-5'-C-ethylcytidine (505 mg, 0.605 mmol) in 95% EtOH (10 mL) was added NaBH₄ (46 mg, 1.21 mmol) under N₂. The reaction mixture was stirred at RT for 7 h. The solvent was evaporated. The residue was diluted with EA (30 mL), washed with sat. NaHCO₃ and brine. The organic layer was separated, dried over anhydrous Na₂SO₄ and concentrated to give the residue which was purified by prep. TLC to give 2'-deoxy-2',2'-difluoro-3'-*O*-,*N⁴*-bis(4-methoxytrityl)-5'-*C*-ethylcytidine (320 mg, 63.1%), which was dissolved in 10 mL AcOH/H₂O (v/v = 4:1). The mixture was stirred at 50 °C overnight. The solvent was removed under vacuum and the residue was diluted with water (3 mL), extracted with EA (2 mLx2) to remove some impurity. Concentrated into a residue which was purified by silica gel column eluting with DCM/MeOH = 10:1 to give the product (80 mg, S:R = 3:7). 60 mg was subjected to SFC separation to give 2'-deoxy-2',2'-difluoro-5'(R)-C-ethylcytidine (17 mg, S:R = 7:93). ¹HNMR (400 Hz) (MeOD): δ 7.89 (d, J = 7.6 Hz, 0.07H), 7.91 (d, J = 7.6 Hz, 1H), 6.20 (t, J = 8.0 Hz, 1H), 5.89 (d, J = 7.6 Hz, 1H), 4.33-4.25 (m, 1H), 3.84-3.79 (m, 2H), 1.66-1.51 (m, 2H), 1.01 (t, J = 7.2 Hz, 3H).

### Example 35

### Preparation of 5'(S)-C-allyl-2'-deoxy-2',2'-difluorocytidine (35)

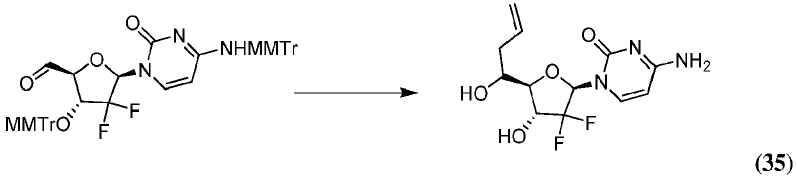

To an ice-EtOH bath cold solution of 2'-deoxy-5'-*C*,5'-*O*-didehydro-2',2'-difluoro-3'-*O*-,*N⁴*-di(4-methoxytrityl)cytidine (3 g, 3.72 mmol) in anhydrous THF (10 mL) was added AllylMgBr (1M solution in THF) (15 mL, 15 mmol) dropwise under N₂. The reaction mixture was stirred at RT overnight. The mixture was quenched by sat. NH₄Cl. The product was extracted with EA (50 mLx2). The combined organic layer was dried over anhydrous Na₂SO₄ and concentrated to give a residue which was purified by silica gel column (PE/EA=3/1 to 1/1) to give 5'-C-allyl-2'-deoxy-5'-C,5'-O-didehydro-2',2'-difluoro-3'-*O*-,*N⁴*-di(4-methoxytrityl)cytidine (1.1 g, 34.9%) which was dissolved in 15 mL AcOH/H₂O (v/v = 4:1). The mixture was stirred at 50 °C overnight. The solvent was removed under vacuum and the residue was diluted with water (3 mL), extracted with EA (2 mLx2) to remove some impurity. The aqueous layer was subjected to SFC separation to give 5'(S)-C-allyl-2'-deoxy-2',2'-difluorocytidine (5 mg, 1.3%); ¹H NMR (400 Hz) (D₂O): δ 7.50 (d, J = 7.6 Hz, 1H), 5.94 (t, J = 7.6 Hz, 1H), 5.81 (d, J = 7.6 Hz, 1H), 5.70-5.59 (m, 1H), 4.98-4.90 (m, 2H), 4.17-4.08 (m, 1H), 3.75-3.68 (m, 2H), 2.25-2.15 (m, 2H) and 3'-*O*-,*N⁴*-bis(4-methoxytrityl)-5'(*R*)-*C*-allyl-2'-deoxy-2',2'-difluorocytidine (5 mg, 1.3%).

### Example 36

### Preparation of 2'-deoxy-2',2'-difluoro-5'(S)-C-propylcytidine (36)

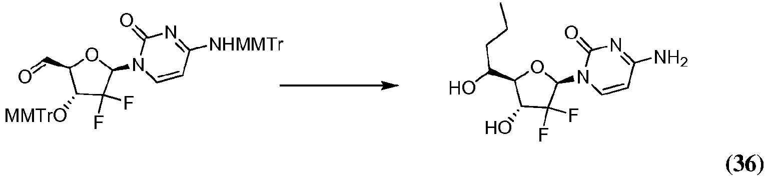

To an ice-EtOH bath cold solution of 2'-deoxy-5'-*C*,5'-*O*-didehydro-2',2'-difluoro-3'-*O*-,*N⁴*-di(4-methoxytrityl)cytidine (3 g, 3.72 mmol) in anhydrous THF (10 mL) was added PrMgBr (2M solution in THF) (8 mL, 16 mmol) dropwise under N₂. The reaction mixture was stirred at RT overnight. The mixture was quenched by sat. NH₄Cl. The product was extracted with EA (50 mLx2). The combined organic layer was dried over anhydrous Na₂SO₄ and concentrated to give a residue which was purified by silica gel column (PE/EA=3/1 to 1/1) to give 2'-deoxy-2',2'-difluoro-3'-*O*-,*N⁴*-di(4-methoxytrityl)-5'-propylcytidine (1.3 g, 41.1%) as a mixture.
2'-Deoxy-2',2'-difluoro-3'-*O*-,*N⁴*-di(4-methoxytrityl)-5'-propylcytidine (1.3 g, 1.53 mmol) was dissolved in 15 mL AcOH/H₂O (v/v = 4:1). The mixture was stirred at 50 °C overnight. The solvent was removed under vacuum and the residue was diluted with water (3 mL), extracted with EA (2 mLx2) to remove some impurity. The water layer was subjected to SFC separation to give 2'-deoxy-2',2'-difluoro-5'(*S*)-propylcytidine (30 mg, 6%) was obtained after HPLC separation. ¹H NMR (400 Hz) (MeOD): δ 7.58 (dd, J = 7.6 Hz, 3.6Hz, 1H), 6.04-5.99 (m, 1H), 5.89 (dd, J = 7.6 Hz, 3.6 Hz, 1H), 4.23-4.15 (m, 1H), 3.75-3.73 (m, 2H), 1.47-1.21 (m, 6H), 0.78-0.74 (m, 3H) and 3'-*O*-,*N⁴*-bis(4-methoxytrityl)-2'-deoxy-2',2'-difluoro-5'(R)-propylcytidine (5 mg, 1%).

### Example 37

### Preparation of 5'-O-acetyl-2'-deoxy-2',2'-difluoro-5'(S)-C-methyleytidine (37)

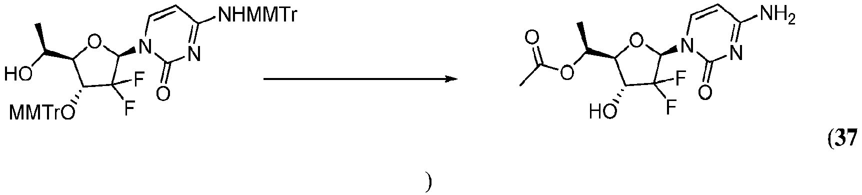

2'-Deoxy-2',2'-difluoro-3'-*O*-*,N⁴*-di(4-methoxytrityl)-5'(*S*)-*C*-ethylcytidine (1 g, 1.2 mmol), EDCI (1 g, 5.2 mmol) and DMAP (1 g, 8.2 mmol) in DCM (100 mL) was added acetic acid (0.5 g, 8.3 mmol) in portions at 0 °C under ice-water, then stirred at room temperature about 10 °C for 1 hour. Then the reaction mixture was washed with water (100 mL) and extracted with DCM (50 mL) twice. The organic layer was concentrated to afford the crude desired 5'-O-acetyl-2'-deoxy-2',2'-difluoro-3'-*O*-,*N⁴*-di(4-methoxytrityl)-5'(*S*)-*C*-methylcytidine which was used for next-step without purification.5'-O-Acetyl-2'-deoxy-2',2'-difluoro-3'-*O*-,*N⁴*-di(4-methoxytrityl)-5'(*S*)-*C-*methylcytidine was dissolved in AcOH:H₂O (50 mL, 80%). The reaction mixture was stirred at 60 °C overnight. Then concentrated and purified by Prep. HPLC to obtain 5'-*O-*acetyl-2'-deoxy-2',2'-difluoro-5'(S)-C-methylcytidine (210 mg) as white solid; ¹HNMR (CD₃OD, 400MHz) δppm: 7.65 (d, J = 7.6 Hz, 1H), 6.23 (t, J = 8.4 Hz, 1H), 5.95 (d, J = 7.6 Hz, 1H), 5.22 (m, 1H), 4.09 (m, 1H), 3.90 (dd, J1 = 4.8 Hz, J2 = 6.4 Hz, 1H), 2.08 (s, 3H), 1.37 (d, J = 6.4 Hz, 3H). **ESI-LCMS:** m/z 320 [M + H]⁺, 639 [2M +H]⁺.

### Example 38

### Preparation of 2'-deoxy-3',5'-O-diacetyl-2',2'-difluoro-5'(S)-C-methylcytidine (38)

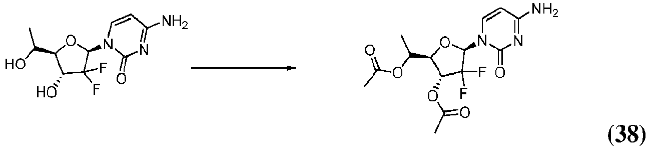

To a stirred solution of 2'-deoxy-2',2'-difluoro-5'(*S*)-*C*-methylcytidine (1 g, 3.6 mmol) in DMF (10 mL) was added DMF-DMA (10 mL). The mixture was stirred at 60 °C for 2 hours as checked with LCMS. The solvent was then removed under reduced pressure to give 2'-deoxy-2',2'-difluoro-*N*⁴-(*N,N*-dimethylaminomethylene)-5'(*S*)-*C-*methylcytidine (1.1 g).
To a stirred solution of 2'-deoxy-2',2'-difluoro-*N*⁴-(*N,N-*dimethylaminomethylene)-5'(*S*)-*C*-methylcytidine (0.5 g crude) in pyridine (20 mL) were added DMAP (122 mg, 1 mmol) and acetyl anhydride (1.02 g, 10 mmol). The mixture was stirred at RT for 4 hours as checked with LCMS. Then the solvent was removed under reduced pressure to give 2'-deoxy-3',5'-*O*-diacetyl-2',2'-difluoro-*N⁴*-(N,N-dimethylaminomethylene)-5'(S)-C-methylcytidine (1.8 g), which was dissolved in 50 mL 80% HOAc and stirred at 50 °C for 4 hours as checking with LCMS. The solvent was removed and the residue was purified by prep. HPLC (HCOOH system) to give 2'-deoxy-3',5'-O-diacetyl-2',2'-difluoro-5'(S)-C-methylcytidine as white solid (280 mg, 43% for 3 steps); 1H NMR (CD₃OD, 400 M Hz) δ 7.65 (d, J = 7.6 Hz, 1H), 6.32 (t, J = 8.8 Hz, 1H), 5.98 (d, J = 7.6 Hz, 1H), 5.23-5.31 (m, 1H), 5.18-5.22 (m, 1H), 4.22 (dd, J1 = 4Hz, J2 = 6.4 Hz, 1H), 2.16 (s, 3H), 2.09 (s, 3H), 1.34 (d, J = 6.4 Hz, 3H); ESI-LCMS: m/z 362 [M + H]⁺, 723 [2M + H]⁺.

### Example 39

### Preparation of 2'-deoxy-2',2'-difluoro-5'(S)-C-methyl-3',5'-O,N⁴-triacetylcytidine (39)

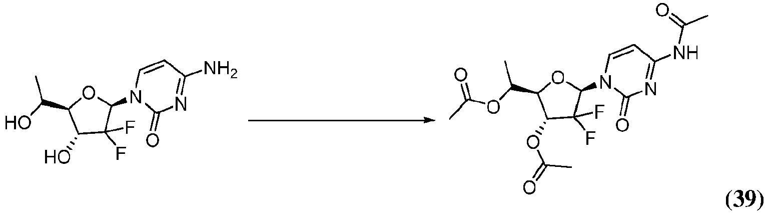

To a stirred solution of 2'-deoxy-2',2'-difluoro-5'(*S*)-*C*-methylcytidine (100 mg, 0.36 mmol) in pyridine (5 mL) were added Ac₂O (153 mg, 1.44 mmol) and DMAP (15 mg, 0.12 mmol). The mixture was stirred at RT for 3 hours as checked with LCMS. The mixture was then diluted with EA and washed with brine. The organic layer was dried over Na₂SO₄ and concentrated. The residue was purified by prep. TLC (PE:EA = 2:1) to afford 2'-deoxy-2',2'-difluoro-5'(*S*)-*C*-methyl-3',5'-*O*,*N*⁴-triacetylcytidine as white solid (80 mg, 55%). 1H NMR (CDCl₃, 400 M Hz) δ9.41 (br s, 1H), 7.90 (dd, J1 = 2.0 Hz, J2 = 7.2Hz, 1H), 7.53 (d, J = 7.2 Hz, 1H), 6.46 (dd, J1 = 6 Hz, J2 = 10.4 Hz, 1H), 5.17-5.24 (m, 2H), 4.14 (dd, J1 = 4.0 Hz, J2 = 5.6 Hz, 1H), 2.28 (s, 3H), 2.18 (s, 3H), 2.12 (s, 3H), 1.38 (d, J = 6.8 Hz, 3H); ESI-MS: m/z 404[M + H]⁺, 807 [2M + H]⁺.

### Example 40

### Preparation of 2'-deoxy-2',2'-difluoro-5'(S)-C-methyl-5'-O-propionylcytidine (40)

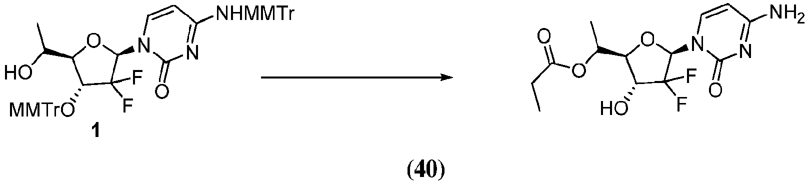

2'-Deoxy-2',2'-difluoro-3'-*O*-,*N*⁴-di(4-methoxytrityl)-5'(*S*)-*C*-methylcytidine (1 g, 1.2 mmol), EDCI (1 g, 5.2 mmol) and DMAP (1 g, 8.2 mmol) in DCM (100 mL) was added propionic acid (0.5 g, 6.8 mmol) in portions at 0 °C, then stirred at room temperature (about 10 °C) for 1 hour. Then the reaction mixture was washed with water (100 mL) and extracted with DCM (50 mL) twice. The organic layer was concentrated to afford the 2'-deoxy-2',2'-difluoro-3'-*O*-,*N*⁴-di(4-methoxytrityl)-5'(*S*)-*C*-methyl-5'-*O-*propionylcytidine which was used for next-step without purification.
2'-Deoxy-2\2'-difluoro-3'-*O*-,*N⁴*-di(4-methoxytrityl)-5'(*S*)-*C*-methyl-5'-*O-*propionylcytidine was dissolved in AcOH:H₂O (50 mL, 80%). The reaction mixture was stirred at 60 °C overnight. Then concentrated and purified by Prep. HPLC to obtain the desired 2'-deoxy-2',2'-difluoro-5'(*S*)-*C*-methyl-5'-*O*-propionylcytidine (180 mg) as white solids. ¹HNMR (CD₃OD, 400 MHz) δ ppm: 7.65 (d, J = 7.6 Hz, 1H), 6.23 (t, J = 8.4 Hz, 1H), 5.94 (d, J = 7.6 Hz, 1H), 5.19-5.25 (m, 1H), 4.04-4.12 (m, 1H), 3.91 (dd, J1 = 4.8 Hz, J2 = 7.6 Hz, 1H), 2.39 (q, J = 7.6 Hz, 2H), 1.36 (d, J = 6.4 Hz, 3H), 1.13 (m, J = 7.6 Hz, 3H). ESI-LCMS: m/z 334 [M + H]⁺, 667 [2M +H]⁺.

### Example 41

### Preparation of 2'-deoxy-2',2'-difluoro-3',5'-O-dipropionyl-5'(S)-C-methylcytidine (41)

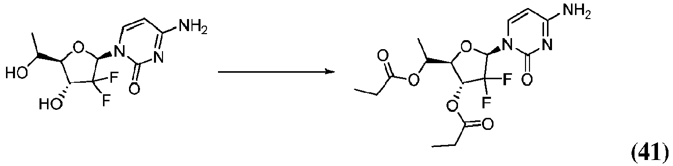

To a stirred solution of 2'-deoxy-2',2'-difluoro-5'(*S*)-*C*-methylcytidine (1 g, 3.6 mmol) in DMF (10 mL) was added DMF-DMA (10 mL). The mixture was stirred at 60 °C for 2 hours as checked with LCMS. The solvent was then removed under reduced pressure to give crude 2'-deoxy-2',2'-difluoro-*N*⁴-(*N,N*-dimethylaminomethylene)-5'(*S*)-C-methylcytidine (1.1 g).
To a stirred solution of 2'-deoxy-2',2'-difluoro-*N*⁴-(*N,N-*dimethylaminomethylene)-5'(*S*)-*C*-methylcytidine (0.5 g crude) in pyridine (20 mL) were added DMAP (12 mg, 0.1 mmol) and propionyl anhydride (1.3 g, 10 mmol). The mixture was stirred at RT for 4 hours as checked with LCMS. Then the solvent was removed under reduced pressure to give crude 2'-deoxy-2',2'-difluoro-*N*⁴-(*N,N-*dimethylaminomethylene)-3',5'-O-dipropionyl-5'(*S*)-*C*-methylcytidine (1.9 g).
Crude 2'-deoxy-2',2'-difluoro-*N*⁴-(*N,N*-dimethylaminomethylene)-3',5'-*O-*dipropionyl-5'(S)-C-methylcytidine (1.9 g) was dissolved in 50 mL 80% HOAc and stirred at 50 °C for 4 hours. The solvent was removed and the residue was purified by prep. HPLC (HCOOH system) to give 2'-deoxy-2',2'-difluoro-3',5'-*O*-dipropionyl-5'(*S*)-C-methylcytidine as white solid (205 mg, 29% for 3 steps); 1H NMR (CD₃OD, 400 M Hz) δ 7.65 (d, J = 7.6 Hz, 1H), 6.31 (t, J = 8.8 Hz, 1H), 5.97 (d, J = 7.6 Hz, 1H), 5.25-5.31 (m, 1H), 5.19-5.24 (m, 1H), 4.23 (dd, J1 = 4Hz, J2 = 6.8 Hz, 1H), 2.48 (q, J = 7.6 Hz, 2H), 2.40 (q, J = 7.6 Hz, 2H), 1.34 (d, J = 6.4 Hz, 3H), 1.15 (t, J = 7.6 Hz, 3H), 1.13 (t, J = 7.6 Hz, 3H); ESI-LCMS: m/z 390 [M + H]⁺, 412 [M +Na]⁺, 779 [2M + H]⁺.

### Example 42

### Preparation of 5'(S)-C-methylarabinocytidine (42)

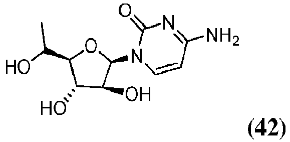

### Step 1. Preparation of 2',3'-O,N⁴-tri(4-methoxytrityl)arabinocytidine

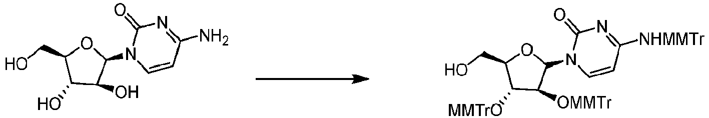

To an ice-cold solution of arabinocytidine (20.0 g, 82.2 mmol) in anhydrous pyridine (200 mL) was added TBSC1 (14.9 g, 98.7 mmol) in small portions under N₂. The reaction mixture was stirred at RT overnight. The solvent was removed under vacuum and the residue was diluted with EA (300 mL), washed with water and brine. The organic layer was separated, dried over anhydrous Na₂SO₄ and filtered. The filtrate was concentrated in vacuum to give 5'-*O*-(*t*-butydimethylsilyl)arabinocytidine (25.1 g, 85.4%) as a white solid which was used without further purification.

To a mixture of 5'-*O*-(*t*-butydimethylsilyl)arabinocytidine (15.0 g, 41.96 mmol), AgNO₃ (43.5 g, 252 mmol) and collidine (61 g, 503.5 mmol) in anhydrous DCM (300 mL) was added MMTrCl (77.7 g, 252 mmol) in small portion under N₂. The reaction mixture was stirred at RT for two days under N₂. The reaction mixture was filtered through a Buchner Funnel. The filtrate was washed with sat. NaHCO₃ solution and followed by brine. The organic layer was separated, dried over anhydrous Na₂SO₄ and filtered. The filtrate was concentrated in vacuum to give the residue which was purified by silica gel column (PE/EA= 2/1) to give 5'-*O*-(*t*-butydimethylsilyl)- 2',3'-*O*,*N*⁴-tri(4-methoxytrityl)arabinocytidine (33.5 g, 67.9%).

To an ice-cold solution of 5'-*O*-(*t*-butydimethylsilyl)- 2',3'-*O,N*⁴-tri(4-methoxytrityl)arabinocytidine (12.0 g, 10.2 mmol) in anhydrous THF (80 mL) was added TBAF (1 M solution in THF) (20.5 mL, 20.5 mmol) dropwise under N₂. The reaction mixture was stirred at RT overnight. The solvent was removed to give a residue. The residue was dissolved in EA (200 mL) and washed with water and brine. The organic layer was separated, dried over anhydrous Na₂SO₄ and filtered. The filtrate was concentrated in vacuum to give a residue which was purified by silica gel column (PE/EA= 6/1 to 2/1) to give 2',3'-*O*,*N*⁴-tri(4-methoxytrityl)arabinocytidine (9.8 g, 90.5%).

### Step 2. Preparation of 5'-dehydro-2',3'-O,N⁴-tri(4-methoxytrityl)arabinocytidine

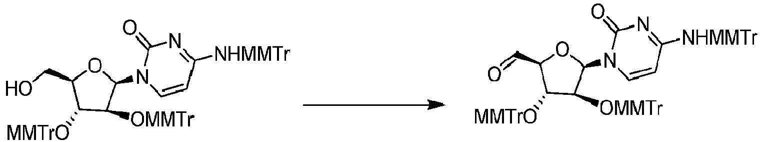

To an ice-cold mixture of anhydrous pyridine (2.0 mL) and Dess-Martin (3.2 g, 7.55 mmol) in anhydrous DCM (20 mL) was added a solution of 2',3'-*O*,*N*⁴-tri(4-methoxytrityl)arabinocytidine (4.0 g, 3.77 mmol) in 10 mL anhydrous DCM under N₂. The reaction mixture was stirred at RT overnight. The reaction mixture was diluted with EA (100 mL), washed with 10% Na₂S₂O₃ solution twice and brine. The organic layer was separated, dried over anhydrous Na₂SO₄ and filtered. The filtrate was concentrated in vacuum to give 5'-C,5'-*O*-didehydro-2',3'-*O*,*N*⁴-tri(4-methoxytrityl)arabinocytidine (3.8 g, 95%) which was used without further purification.

### Step 3. Preparation of 5'-C-methylarabinocytidine

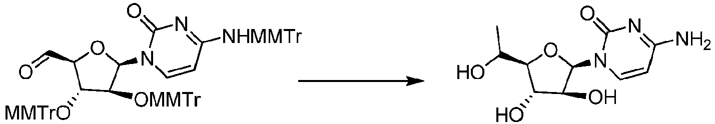

To an ice-EtOH cold solution of 5'-C,5'-*O*-didehydro-2',3'-*O*,*N*⁴-tri(4-methoxytrityl)arabinocytidine (2.0 g, 1.89 mmol) in anhydrous THF (10 mL) was added MeMgBr (3 M solution in ether) (3.2 mL, 9.43 mmol) dropwise under N₂. The reaction mixture was stirred at RT for 5h. The reaction was complete detected by HPLC. The mixture was cooled to 0 °C and quenched by sat. NH₄Cl. The product was extracted with EA (100 mLx2). The combined organic layer was dried over anhydrous Na₂SO₄ and concentrated to give the crude 5'(*S*)-*C*-methyl-2',3'-*O*,*N*⁴-tri(4-methoxytrityl)arabinocytidine (1.4 g, 68.9%).
5'-*C*-Methyl-2',3'-*O*,*N*⁴-tri(4-methoxytrityl)arabinocytidine (700 mg, 0.65 mmol) was dissolved in 10 mL of AcOH/H₂O (v/v = 4:1). The mixture was stirred at 50 °C overnight. The solvent was removed under vacuum and the residue was diluted with water (3 mL), extracted with EA (2 mLx2) to remove some impurity. The water layer was subjected to prep-HPLC separation. 5'(S)-C-Methylarabinocytidine (40 mg, 23.5%) was obtained after HPLC separation. ¹HNMR (400 Hz) (MeOD): δ 7.96 (d, J = 7.6 Hz, 1H), 6.20 (d, J = 4.0 Hz, 1H), 5.91 (d, J = 7.2 Hz, 1H), 4.20 (dd, J = 4.0 Hz, 2.4 Hz, 1H), 4.10 (t, J = 2.8 Hz, 1H), 4.01-4.07 (m, 1H), 3.75 (dd, J = 4.0 Hz, 3.2 Hz, 1H), 1.33 (d, J =6.4 Hz, 3H).

### Example 43

### Preparation of 5'(R)-C-methylarabinocytidine (43)

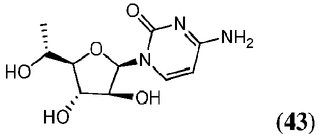

### Step 1. Preparation of 5'-C,5'-O-didehydro-5'-C-methyl-2',3'-O,N⁴-tri(4-methoxytrityl)arabinocytidine

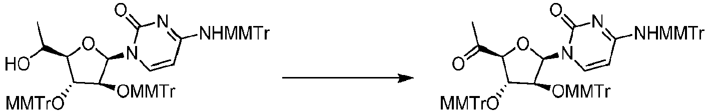

To an ice-cold suspension of CrO₃ (279 mg, 2.79 mmol) in anhydrous DCM (5 mL) was added anhydrous pyridine (0.45 mL, 5.59 mmol) and Ac₂O (0.28 mL, 2.79 mmol) under N₂. The mixture was stirred at RT for about 10 min until the mixture became homogeneous. The mixture was cooled to 0 °C and a solution of 5'(S)-C-methyl-2',3'-i,*N*⁴-tri(4-methoxytrityl)arabinocytidine (1.0 g, 0.93 mmol) in anhydrous DCM (5 mL) was added. The resultant mixture was stirred at RT overnight. The reaction was complete as detected by HPLC. The reaction mixture was diluted with EA (100 mL), washed with NaHCO₃ solution twice and brine. The organic layer was separated, dried over anhydrous Na₂SO₄ and filtered. The filtrate was concentrated in vacuum to give a residue which was purified by silica gel column (acetone/PE= 1/2) to give 5'-*C*,5'-*O-*didehydro-5'-C-methyl-2',3'-*O*,*N*⁴-tri(4-methoxytrityl)arabinocytidine (548 mg, 55%).

### Step 2. Preparation of 5'-dehydro-5'(R)-C-methylarabinocytidine

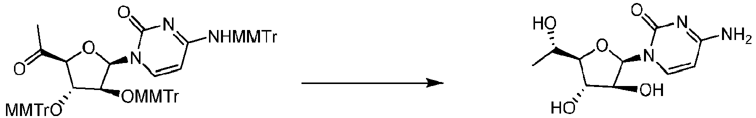

To an ice-cold solution of 5'-C,5'-i-didehydro-5'-C-methyl-2',3'-*O*,*N*⁴-tri(4-methoxytrityl)arabinocytidine (540 mg, 0.505 mmol) in 95% EtOH (10 mL) was added NaBH₄ (39 mg, 1.01 mmol) under N₂. The reaction mixture was stirred at RT for 7h. The reaction was complete detected by HPLC. The solvent was evaporated. The residue was diluted with EA (30 mL), washed with sat. NaHCO₃ and brine. The organic layer was separated, dried over anhydrous Na₂SO₄ and concentrated to give the crude 5'-C-methyl-2',3'-*O*,*N*⁴-tri(4-methoxytrityl)arabinocytidine (480 mg, 88%).
5'-C-methyl-2',3'-*O*,*N*⁴-tri(4-methoxytrityl)arabinocytidine (480 mg, 0.45 mmol) was dissolved in 10 mL AcOH/H₂O (v/v = 4:1). The mixture was stirred at 50 °C overnight. The solvent was removed under vacuum and the residue was diluted with water (3 mL), extracted with EA (2 mLx2) to remove some impurity. The water layer was sent to prep. HPLC separation. 5'(R)-C-methylarabinocytidine (30 mg, 26%) was obtained after HPLC separation. ¹HNMR (400 Hz) (MeOD): δ 7.84 (d, J = 7.6 Hz, 1H), 6.18 (d, J = 3.6 Hz, 1H), 5.91 (d, J = 7.6 Hz, 1H), 4.28 (t, J = 2.0 Hz, 1H), 4.17 (dd, J = 3.6 Hz, 1.6 Hz, 1H), 4.03-4.09 (m, 1H), 3.75 (dd, J = 5.2 Hz, 2.4 Hz, 1H), 1.32 (d, J =6.4 Hz, 3H).

### Example 44

### Preparation of 1-O-acetyl-2,5(S)-C-dimethyl-2,3,5-O-tribenzoyl-D-ribofuranose (44)

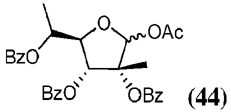

### Step 1. Preparation of 1(α)-O,2-C-dimethyl-D-ribofuranose

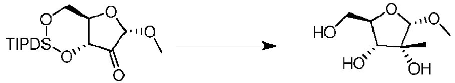

To a solution of 2-C,2-*O*-didehydro-1(*α*)-*O*-methyl-3,5-*O*-(1,1,3,3-tetraisopropyl-1,3-disiloxanediyl)-D-ribofuransone (prepared according to a published procedure, 46.0 g, 113.9 mmol) in THF (300 mL) cooled with dry ice was added CH₃MgBr in ether (3.0 M, 113.9 mL, 341.6 mmol) dropwise under N₂. The mixture was warmed to RT and stirred for 2 h. The mixture was quenched by saturated NH₄Cl. The product was extracted with EA (200x2). The combined organic layer was dried over anhydrous Na₂SO₄ and filtered. The filtrate was concentrated in vacuum to give 1(*α*)-*O*,5-*C*-dimethyl-3,5-*O*-(1,1,3,3-tetraisopropyl-1,3-disiloxanediyl)-D-ribofuransone as syrup (42.1 g, 88.0%) which was used without further purification.

To a solution of 1(*α*)-*O*,2-C-dimethyl-3,5-*O*-(1,1,3,3-tetraisopropyl-1,3-disiloxanediyl)-D-ribofuransone (42.1 g, 100.2 mmol) in anhydrous THF (200 mL) was added TBAF (52.6 g, 200.5 mmol) in small portion. The mixture was stirred at RT overnight. The solvent was removed and the residue was purified by silica gel column. (EA/MeOH = 100/1) to give 1(*α*)-*O*,2-C-dimethyl-D-ribofuransone as syrup (16.5 g, 92.4%); 1HNMR (400MHz) (MeOD):δ4.56 (s, 1H), 3.87-3.90 (m, 1H), 3.60-3.77 (m, 2H), 3.52 (d, J = 6.0 Hz), 3.43(s, 3H), 1.25 (s, 3H).

### Step 2. Preparation of 2,3-O-dibenzoyl-1(α)-O,2-C-dimethyl-D-ribofuranose

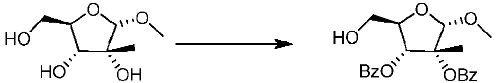

To an ice-cold of solution of 1(*α*)-*O*,2-C-dimethyl-*D*-ribofuransone (11.0 g, 61.8 mmol) in anhydrous pyridine (100 mL) was added TBSC1 (11.2 g, 74.2 mmol) in small portion under N₂. The reaction mixture was stirred at RT for 4h. The mixture was cooled in an ice bath and BzCl (17.4 g, 124 mmol) was added. The mixture was stirred at RT overnight. The solvent was diluted with EA (300 mL) and washed with saturated NaHCO₃. the organic layer was separated, dried over anhydrous Na₂SO₄ and filtered. The filtrate was concentrated in vacuum to give crude syrup. The cured syrup was purified by flash chromatography (PE/EA = 20/1 to 10/1) to give 3-*O*-benzoyl-5-*O*-(*t-*butyldimethylsilyl)-1(*α*)-*O*,2-*C*-dimethyl-*D*-ribofuransone (21.4 g, 87.2%); 1HNMR (400MHz) (CDCl₃):δ8.05 (d, J = 7.2 Hz, 2H), 7.56 (t, J = 7.2 Hz, 1H), 7.44 (t, J = 6.4 Hz, 2H), 4.98 (d, J = 4.0 Hz, 1H), 4.58 (s, 1H), 4.18 (m, 1H), 3.90-3.93 (m, 1H), 3.47 (s, 1H), 3.11 (s, 1H), 1.47 (s, 3H), 0.91 (s, 9H), 0.10 (d, J = 2.0 Hz, 3H).

To an ice-cold mixture of TEA (54.6 g, 540 mmol) and DMAP (6.6 g, 54.0 mmol) in anhydrous DCM (200 mL) was added BzCl ( 15.2 g, 108.0 mmol) followed by 3-*O-*benzoyl-5-*O*-(*t*-butyldimethylsilyl)-1(α)-*O*,2-*C*-dimethyl-*D*-ribofuransone (21.4 g, 54.0 mmol). The reaction mixture was stirred at RT for 2 days. The mixture was diluted with DCM (200 mL) and then washed with water and saturated NaHCO₃. The organic layer was separated, dried over anhydrous Na₂SO₄ and filtered. The filtrate was concentrated in vacuum to give crude syrup. The cured syrup was purified by flash chromatography (PE/EA= 50/1 to 20/1) to give 5-*O*-(*t*-butyldimethylsilyl)-2,3-*O*-dibenzoyl-1(*α*)-*O*,2-*C-*dimethyl-D-ribofuransone as syrup (24.5 g, 90.7%); 1HNMR (400MHz) (CDCl₃):δ8.11 (m, 2H), 7.75-7.77 (m, 2H), 7.54-7.57 (m, 1H), 7.38-7.45 (m, 3H), 7.13-7.15 (m ,1H), 5.35 (d, J = 2.8 Hz, 1H), 5.28 (s, 1H), 4.27 (m, 1H), 3.95-3.97 (m, 1H), 3.40 (s, 1H), 1.77 (s, 3H), 0.92 (s, 9H), 0.12 (d, J = 2.0 Hz, 3H).

To an ice-cold solution of 5-*O*-(*t*-butyldimethylsilyl)-2,3-*O*-dibenzoyl-1(*α*)-*O*,2-*C-*dimethyl-D-ribofuransone (24.5 g, 49.0 mmol) in THF (200 mL) was added a 1 M solution of TBAF in THF ( 58.8 mL, 58.8 mmol) dropwise. The mixture was stirred at this temperature for 2h. HOAc was added to the mixture to neutralize the solution to light acid. After that, the solvent was removed and the residue was purified by flash chromatography (PE/EA= 20/1 to 8/1) to give 2,3-*O*-dibenzoyl-1(α)-*O*,2-*C*-dimethyl-*D-*ribofuranose as syrup (15.2 g, 80.4%); 1HNMR (400MHz) (CDCl₃):δ8.11-8.13 (dd, J₁ = 5.2 Hz, J₂ = 7.2 Hz, 2H), 7.79-7.82 (dd, J₁ = 0.8 Hz, J₂ = 8.0 Hz, 2H), 7.57-7.61 (t, J = 7.2 Hz, 1H), 7.41-7.46 (m, 3H), 7.17-7.21 (t, J = 8.0 Hz, 2H), 5.30 (s, 1H), 5.28 (d, J = 4.4 Hz, 1H), 4.34 (q, J = 4.0 Hz, 1H), 3.94-4.04 (m, 2H), 3.43 (s, 3H), 2.29 (w, 1H), 1.79 (s,3H).

### Step 3. Preparation of 2,3-O-dibenzoyl-5-C-methyl-1(α)-O,2-C-dimethyl-D-ribofuranose

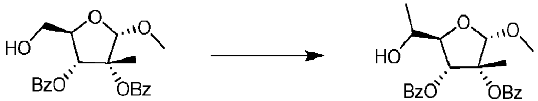

To a suspension of Dess-Martin reagent (15.7 g, 37.0 mmol) in anhydrous DCM (200 mL) cooled with an ice-bath was added a solution of 2,3-*O*-dibenzoyl-1(α)-0,2-*C-*dimethyl-D-ribofuranose (11.0 g, 28.5 mmol) in anhydrous DCM (50 mL) under N₂. The reaction mixture was stirred at RT overnight. The mixture was diluted with ether (500 mL) and washed with saturated Na₂S₂O₃ (22.51 g, 142.3 mmol). The organic layer was separated, dried over anhydrous MgSO₄ and filtered. The filtrate was concentrated in vacuum under a temperature lower than 30 °C to give 2,3-*O*-dibenzoyl-5-*C*,5-*O-*didehydro-1(α)-*O*,2-*C*-dimethyl-*D*-ribofuranose as a white foam (9.5 g, 96.4 %).
TiCl₄ (10.85 mL, 18.75 g, 98.86 mmol) was added dropwise to anhydrous ether (310 mL) cooled to -78 °C. To the resultant yellow etherate was slowly added 3.0 M CH₃MgBr in ether (33.0 mL, 32.9 mmol), the reaction mixture was then allowed to warmed to -30 °C, whereupon a solution of 2,3-*O*-dibenzoyl-5-C,5-*O*-didehydro-1(α)-*O*,2-*C*-dimethyl-D-ribofuranose (9.5 g, 24.7 mmol) in 30 mL of ether was added dropwise. After 4 h at -30 to -10 °C, TLC analysis showed complete conversion, and the reaction was separated and the aqueous phase extracted with 3x150 mL of ether. The combined organic layer was washed with water, dried over anhydrous MgSO₄, filtered and concentrated to a syrup. The syrup was purified by silica gel column (PE/EA=20/1 to10/1) to give 2,3-*O*-dibenzoyl-1(α)-*O*,2,5-*C*-trimethyl-*D*-ribofuranose as a foam solid (7.1 g, 71.7%); 1HNMR (400 MHz) (CDCl₃):δ8-13 (d, J = 4.4 Hz, 2H), 7.09 (d, J = 4.4 Hz, 2H), 7.36-7.47 (m, 5H), 7.11 (t, J = 1.6 Hz), 5.40 (d, J = 4.0 Hz, 1H), 5.28 (d, J = 8.4 Hz, 1H), 4.10-4.15 (m, 2H), 3.42 (s, 3H), 2.36 (w, 1H), 1.77 (s, 3H), 1.27 (d, J = 8.4 Hz, 3H).

### Step 4. Preparation of 1-O-acetyl-2,3,5-O-tribenzoyl-2,5-C-dimethyl-D-ribofuranose

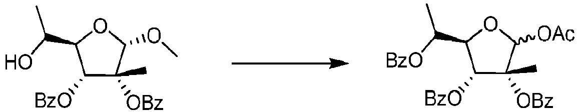

To an ice-cold solution of 2,3-*O*-dibenzoyl-1(α)-*O*,2,5-*C*-trimethyl-*D*-ribofuranose (2.0 g, 5.0 mmol) in anhydrous pyridine (20 mL) was added a BzCl (1.05 g , 7.5 mmol) under N₂. The mixture was stirred at RT overnight. The solvent was removed and the residue was purified by sillca gel column (PE/EA= 50/1 to 30/1). 2,3,5-*O*-Tribenzoyl-1(α)-*O*,2,5-*C*-trimethyl-*D*-ribofuranose was obtained as a white foam solid (1.5 g, 60 %); 1HNMR (400MHz) (CDCl₃):δ8.18 (d, J = 7.2 Hz, 2H), 8.06 (d, J = 7.2 Hz, 2H), 7.36-7.71 (m, 9H), 7.11 (t, J = 7.6 Hz, 2H), 5.60 (m, 1H), 5.56 (d, J = 5.2 Hz, 1H), 4.44 (t, J = 4.8 Hz, 1H), 3.43 (s, 1H), 1.79 (s, 1H), 1.48 (d, J = 6.4 Hz, 3H).
To a solution of 2,3,5-*O*-tribenzoyl-1(α)-*O*,2,5-*C*-trimethyl-*D*-ribofuranose (1.5 g, 3.0 mmol) in HOAc (10 mL) and Ac₂O (1 mL) cooled with a water bath was added 0.5 mL conc. H₂SO₄ dropwise. The mixtrure was stirred at RT for 3 h. The mixture was poured into ice-water and the precipitate was collected by filtration. The solid cake was dissolved in EA (50 mL) and washed with saturated NaHCO₃ (30 mLx2). The organic layer was separated, dried over anhydrous Na₂SO₄ and filtered. The filtrate was concentrated under vacuum to give 1-*O*-acetyl-2,5(*S*)-*C*-dimethyl-2,3,5-*O*-tribenzoyl-*D-*ribofuranose as white foam solid (1.4 g, 88.6%); 1HNMR (400MHz) (CDCl₃):67.87-8.02 (m, 5H), 7.06-7.63 (m, 10H), 6.73+6.55 (s, 1H), 5.84 (d, J = 8.0 Hz, 0.5 H), 5.64 (d, J = 3.6 Hz, 0.5 H), 5.30-5.51 (m, 1H), 4.41-4.50 (m, 1H), 1.94+1.86 (s, 3 H), 1.76+1.71 (s, 3H), 1.41+1.33 (d, J = 6.8 Hz, 3H).

### Example 45

### Preparation of 1-O-acetyl-2,5(R)-C-dimethyl-2,3,5-O-tribenzoyl-D-ribofuranose (45)

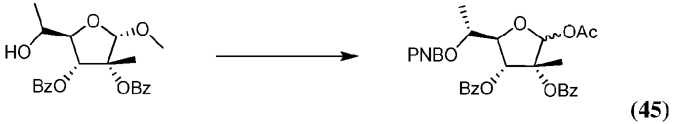

To an ice-cold solution of 2,3-*O*-dibenzoyl-1(α)-*O*,2,5-*C*-trimethyl-*D*-ribofuranose (2.0 g, 5.0 mmol), PNBA (3.3 g, 19.9 mmol) and Ph₃P (5.2 g, 19.9 mmol) in anhydrous THF (50 mL) was added DEAD (3.48 g, 19.9 mmol) dropwise under N₂. The resultant mixture was stirred at RT overnight. The solvent was removed and the residue was purified by silica gel column (PE/EA= 30/1 to 20/1). 2,3-*O*-dibenzoyl-1(α)-*O*,2,5-*C-*trimethyl-5-*O*-(4-nitrobenzoyl)-*D*-ribofuranose was obtained as a white foam solid (1.4 g, 51.0%); 1HNMR (400MHz) (CDCl₃): δ8.13-8.21 (m, 4H), 8.11-8.13 (dd, J₁ = 0.8 Hz, J₂ = 8.0 Hz, 2H), 7.70-7.73 (dd, J₁ = 0.8 Hz, J₂ = 8.0 Hz, 2H), 7.61 (t, J = 7.2 Hz, 1H), 7.38-7.45 (m, 3H), 7.13 (t, J = 8.0 Hz, 2H), 5.59 (m, 1H), 5.33 (s, 1H), 5.27 (d, J = 5.2 Hz, 1H), 4.47 (t, J = 5.2 Hz, 1H), 3.44 (s, 3H), 1.80 (s, 3H), 1.51-1.56 (dd, J₁ = 6.4 Hz, J₂ = 12.8 Hz, 3H).
To a water-cold solution of 2,3-*O*-dibenzoyl-1(α)-*O*,2,5-C-trimethyl-5-*O*-(4-nitrobenzoyl)-D-ribofuranose (1.4 g, 2.5 mmol) in HOAc (10 mL) and Ac₂O (1 mL) was added 0.5 mL conc. H₂SO₄ dropwise. The mixtrure was stirred at RT for 3 h. The mixture was poured into ice-water and the precipitate was collected by filtration. The solid cake was re-dissolved in EA (50 mL) and washed with saturated NaHCO₃ (30 mLx2). The organic layer was separated, dried over anhydrous Na₂SO₄ and filtered. The filtrate was concentrated under vacuum to give 1-*O*-acetyl-2,3-*O*-dibenzoyl-2,5(*R*)-*C*-dimethyl-5-*O-*(4-nitrobenzoyl)-D-ribofuranose as white foam solid (1.3 g, 89.6%); 1HNMR (400MHz) (CDCl₃):δ7.91-8.20 (m, 7H), 7.12-7.67 (m, 7H), 6.75+6.56 (s, 1H), 5.75 (d, J = 8.4 Hz, 0.5H), 5.57 (m, 0.5 H), 5.37 (m, 1H), 4.43-4.50 (m, 1H), 2.16+1.97 (s, 3 H), 1.78+1.73 (s, 3H), 1.48+1.38 (d, J = 6.8 Hz, 3H).

### Example 46

### Preparation of 1-O-acetyl-5-C-methyl-2,3,5-O-tribenzoyl-D-ribofuranose (46)

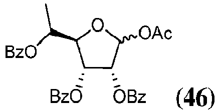

### Step 1. Preparation of 1-O,5-C-dimethyl-2,3-O-isopropylidene-D-ribofuranose

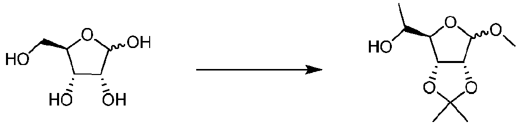

To *D*-ribose (200 g, 1.33 mol) in acetone (760 mL) and MeOH (760 mL) was added concentrated HCl (20 mL), and the solution was allowed to reflux for 18h. The reaction was cooled, neutralized with pyridine, poured into H₂O (2 L), and extracted with Et₂O (3x400 mL). The combined organic layers were washed with saturated aqueous CuSO₄ (300 mL), dried with MgSO₄ and then evaporated. The residue was distilled to afford 2,3-*O*-isopropylidene-1-*O*-methyl-*D*-ribofuranose as colorless oil (180.5 g, 56.5%).
To an ice-cold suspension of Dess-Martin preiodinane (487.3 g, 1.15 mol) in anhydrous DCM (800 L) was a solution of 2,3-*O*-isopropylidene-1-*O*-methyl-*D-*ribofuranose (180.5 g, 883.85 mmol) in anhydrous DCM (200 mL) dropwise under N₂. The resultant mixture was stirred at RT overnight and then diluted with Et₂O (2 L). The mixture was washed with saturated aqueous Na₂SO₃ (3x600 mL). The organic layer was separated, dried over anhydrous MgSO₄ and filtered. The filtrate was concentrated in vacuum to give 5-*C*,5-*O*-didehydro-2,3-*O*-isopropylidene-1-*O*-methyl-*D*-ribofuranose as syrup which was used for the next step without further purification (161.7 g, 90.48%).

To a solution of 5-C,5-*O*-didehydro-2,3-*O*-isopropylidene-1-*O*-methyl-*D-*ribofuranose (161.7 g, 799.70 mmol) in anhydrous THF (3.0 L) was added a 3M solution of MeMgBr in ether (800 mL, 2.40 mol) at 50 °C under N₂. After the addition, the reaction mixture was warmed to 0 °C during a 4 h period. The mixture was quenched with saturated aqueous NH₄Cl and the product was extracted with EA (2x2.0 L). The combined organic layer was dried over anhydrous MgSO₄ and filtered. The filtrate was concentrated in vacuum to give a syrup which was purified by silica gel column (PE/EA=30/1 to 10/1) to give 1-*O*,5-*C*-dimethyl-2,3-*O*-isopropylidene-*D*-ribofuranose as colorless syrup (120.3 g, 69.4%).

### Step 2. Preparation of 5-O-benzoyl-1-O,5-C-dimethyl-D-ribofuranose

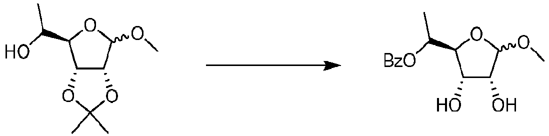

To an ice-cold solution of 1-*O*,5-*C*-dimethyl-2,3-*O*-isopropylidene-*D*-ribofuranose (20.0 g, 92.06 mmol) and DMAP (1.12 g, 9.21 mmol) in anhydrous pyridine (150 mL) was added BzCl (19.41 g, 138.1 mmol) dropwise under N₂. The reaction mixture was stirred at RT overnight. EA (300 mL) was added to the mixture and then washed with water (200 mL) and saturated aqueous NaHCO₃ (200 mL). The organic layer was separated, dried over anhydrous Na₂SO₄ and filtered. The filtrate was concentrated in vacuum to give a residue which was purified by column (PE/EA=30/1 to 10/1) to give 5-*O*-benzoyl-1-*O*,5-*C*-dimethyl-2,3-*O*-isopropylidene-*D*-ribofuranose as syrup (20.7 g, 69.7%).
5-*O*-Benzoyl-1-*O*,5-*C*-dimethyl-2,3-*O*-isopropylidene-i-ribofuranose (20.7 g, 67.14 mmol) was added to a solution of TFA (180 mL) and H₂O (20 mL) at 0 °C. The resultant mixture was stirred at 0 °C for 3h. TLC showed no starting material remained. The solvent was removed under vaccum at 0°C. The residue was dissolved in DCM (200 mL) and washed with saturated aqueous NaHCO₃ (2x150 mL). The organic layer was separated, dried over anhydrous Na₂SO₄ and filtered. The filtrate was concentrated in vacuum to give syrup 5-*O*-benzoyl-1-*O*,5-*C*-dimethyl-*D*-ribofuranose, which was used without further purification (12.0 g).

### Step 3. Preparation of 1-O-acetyl-2,3,5-O-tribenzoyl-5-C-methyl-D-ribofuranose

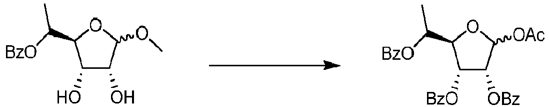

The crude 5-*O*-benzoyl-1-*O*,5-*C*-dimethyl-*D*-ribofuranose (12.0 g, 42.51 mmol) was dissolved in anhydrous pyridine and cooled with an ice-bath. DMAP (0.52, 4.25 mmol) and BzCl (14.9 g, 106.27 mmol) was added to the mixture and then stirred at RT overnight. EA (300 mL) was added to the mixture and then washed with water (200 mL) and saturated aqueous NaHCO₃ (200 mL). The organic layer was separated, dried over anhydrous Na₂SO₄ and filtered. The filtrate was concentrated in vacuum to give a residue which was purified by column (PE/EA=30/1 to 10/1) to give 1-*O*,5-*C*-dimethyl-2,3,5-*O-*tribenzoyl-*D*-ribofuranose as syrup (15.5 g, 74.34%).
To an water-cold (10°C) mixture of 1-*O*,5-C-dimethyl-2,3,5-*O*-tribenzoyl-*D-*ribofuranose (15.5 g, 31.6 mmol) in HOAc (50 mL) and Ac₂O (5 mL) was added concentrated H₂SO₄ (2.5 mL) dropwise. The resultant mixture was stirred at RT for 5h and then poured in ice-water. The precipitate was collected by filtration. The collected solid was dissolved in EA (100mL) and washed with saturated aqueous NaHCO₃ (100 mL). The organic layer was separated, dried over anhydrous Na₂SO₄ and filtered. The filtrate was concentrated in vacuum to give a residue which was purified by column (PE/EA=30/1 to 20/1) to give 1-*O*-acetyl-5-*C*-methyl-2,3,5-*O*-tribenzoyl-*D*-ribofuranose as foam solid (10.5 g, 64.02%).

### Example 47

### Preparation of 5'(S)-C-methyladenosine (47)

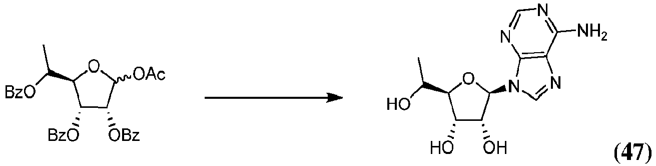

To an ice-cold solution of 1-*O*-acetyl-5(*S*)-*C*-methyl-2,3,5-*O*-tribenzoyl-*D-*ribofuranose (8.0 g, 15.43 mmol) and adenine (3.13 g, 23.14 mmmol) in anhydrous MeCN (100 mL) was added a 1 M solution of SnCl₄ in anhydrous MeCN (38.6 mL, 38.6 mmol) dropwise under N₂. The mixture was stirred at RT overnight and then quenched by aqueous NaHCO₃. The product was extracted by EA (2x100 mL). The combined organic layer was dried over anhydrous Na₂SO₄ and filtered. The filtrate was concentrated in vacuum to give a residue which was purified by silica gel column to give 8.0 g of 2',3',5'-*O*- tribenzoyl-5'-C-methyladenosine, which was dissolved in methanol (100 mL) and saturated with NH₃ for 1h at 0 °C, and then stirred at RT overnight. The solvent was removed under reduced pressure and the residue was re-dissolved in 400 mL saturated aqueous NH₃. The mixture was stirred at RT overnight and the solvent was removed. The residue was purified by prep-HPLC to give 5'(S)-C-methyladenosine (1.5 g, 34.5%).

### Example 48

### Preparation of 5'(S)-C-methylguanosine (48)

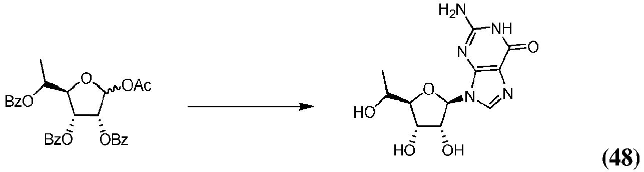

Under an argon atmosphere, a mixture of *N*²-acetylguanine (10.65 g, 81.00 mmol), dry pyridine (50 mL), and HMDS (300 mL) was heated to reflux for 2 h to obtain a clear solution. The solvent was removed carefully under vacuum, and the residue was dried under high vacuum for 1 h. To the flask containing persilylated *N*²-acetylguanosine was added anhydrous toluene (100 mL) and 1-*O*-acetyl-5(*S*)-*C*-methyl-2,3,5-*O*-tribenzoyl-*D-*ribofuranose (10.5 g, 20.25 mmol). To the resulting mixture was added TMSOTf (18.0 g, 81.00 mmol) slowly with vigorous stirring at room temperature. After stirring under argon atmosphere under reflux for 6 h, the reaction mixture was cooled to room temperature and quenched with saturated aqueous NaHCO₃. The organic layer was separated, and the aqueous layer was extracted with DCM (2x150 mL). The combined organic layers was washed with brine, and dried over anhydrous MgSO₄. The MgSO₄ was filtered off, and the solvent was removed by evaporation under vacuum to give light yellow foam (13.1 g). 8.0 g of the foam solid was purified by prep-HPLC to give 4.4 g (95 % purity) of *N*²-acetyl-5'(*S*)-*C*-methyl-2,3,5-*O*-tribenzoylguanosine, which was dissolved in methanol (100 mL) saturated with NH₃ and stirred at RT overnight. The solvent was removed under reduced pressure and the residue was re-dissolved in 400 mL of saturated aqueous NH₃. The mixture was stirred at RT overnight and the solvent was concentrated to about 150 mL. The precipitate was collected by filtration and dried under vacuum to give 5'(S)-C-methylguaonosine as a white solid (550 mg, 30.7%); ¹HNMR (400 MHz) (MeOD): δ 7.88 (s, 1H), 5.76 (d, J = 7.2Hz, 1H), 4.64 (t, J = 6.0Hz, 1H), 4.30 (d, J = 4.4Hz, 1H), 4.03-4.01 (m, 1H), 3.93 (s, 1H), 1.24 (d, J = 6.4Hz, 3H).

### Example 49

### Preparation of 1-O-acetyl-2,3-O-dibenzoyl-5(R)-C-methyl-5-O-(4-nitrobenzoyl)-D-ribofuranose (49)

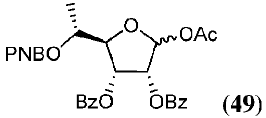

### Step 1. Preparation of 1-O,5-C-dimethyl-5-O-(4-nitrobenzoyl)-D-ribofuranose

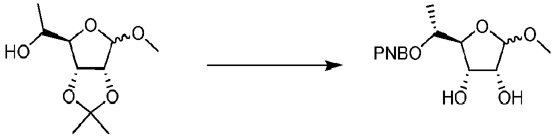

To an ice-cold solution of 1-*O*,5-*C*-dimethyl-2,3-*O*-isopropylidene-*D*-ribofuranose (25.0 g, 114.55 mmol), PNBA (76.57 g, 458.19 mmol) and Ph₃P (120.18 g, 458.19 mmol) in anhydrous THF (600 mL) was added DEAD (79.79 g, 458.19 mmol) dropwise under N₂. The reaction mixture was stirred at RT overnight. The solvent was removed and the residue was purified by silica gel column (PE/EA= 50/1 to 20/1) to give 1-*O*,5-*C-*dimethyl-2,3-*O*-isopropylidene-5-*O*-(4-nitrobenzoyl)-*D*-ribofuranose as a light yellow syrup (21.3 g, 50.62%).

1-*O*,5-*C*-Dimethyl-2,3-*O*-isopropylidene-5-*O*-(4-nitrobenzoyl)-*D*-ribofuranose (16.3 g, 44.37 mmol) was added to a solution of TFA (90 mL) and H₂O (10 mL) at 0°C. The resultant mixture was stirred at 0°C for 3h. TLC showed no starting material remained. The solvent was removed under vaccum at 0°C. The residue was dissolved in DCM (150 mL) and washed with saturated aqueous NaHCO₃ (2x150 mL). The organic layer was separated, dried over anhydrous Na₂SO₄ and filtered. The filtrate was concentrated in vacuum to give a syrup which was purified by silica gel column (PE/EA= 15/1 to 5/1). 1-*O*,5-*C*-dimethyl-5-*O*-(4-nitrobenzoyl)-*D*-ribofuranose was obtained as syrup (7.0 g, 48.2%).

### Step 2. Preparation of 1-O-acetyl-2,3-O-dibenzoyl-5-C-methyl-5-O-(4-nitrobenzoyl)-D-ribofuranose

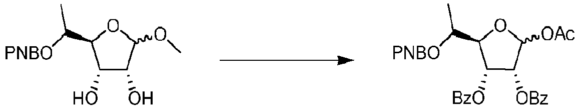

1-*O*,5-*C*-Dimethyl-5-*O*-(4-nitrobenzoyl)-*D-*ribofuranose (7.0 g, 21.39 mmol) was dissolved in anhydrous pyridine (50 mL)and cooled with an ice-bath. DMAP (0.26, 2.14 mmol) and BzCl (7.52 g, 53.47 mmol) was added to the mixture and then stirred at RT overnight. EA (200 mL) was added to the mixture and then washed with water (100 mL) and saturated aqueous NaHCO₃ (100 mL). The organic layer was separated, dried over anhydrous Na₂SO₄ and filtered. The filtrate was concentrated in vacuum to give a residue which was purified by column (PE/EA=30/1 to 10/1) to give 2,3-*O*-dibenzoyl-1-*O*,5-*C-*dimethyl-5-*O*-(4-nitrobenzoyl)-*D*-ribofuranose as syrup (9.2 g, 80.33%).
To a solution of 2,3-*O*-dibenzoyl-1-*O*,5-*C*-dimethyl-5-*O*-(4-nitrobenzoyl)-*D-*ribofuranose (9.2 g, 17.18 mmol) in HOAc (30 mL) and Ac₂O (3 mL) cooled with water bath was added 1.5 mL of concentrated H₂SO₄ dropwise. The mixture was stirred at RT for 3 h. The mixture was poured into ice-water and the precipitate was collected by filtration. The solid cake was re dissolved in EA (50 mL) and washed with saturated NaHCO₃ (30 mLx2). The organic layer was separated, dried over anhydrous Na₂SO₄ and filtered. The filtrate was concentrated under vacuum to give 1-*O*-acetyl-2,3-*O*-dibenzoyl-5(R)-*C*-methyl-5-*O*-(4-nitrobenzoyl)-*D*-ribofuranose as white foam solid (8.1 g, 83.7%). ¹HNMR (400 MHz) (CDCl₃): δ 8.83-8.18 (m, 4H), 8.05-7.96 (m, 2H), 7.86-7.82 (m, 2H), 7.86-7.82 (m, 2H), 7.59-7.49 (m, 2H), 7.45-7.40 (m, 2H), 6.75 (d, J = 4.4Hz, 0.3H), 6.42 (s, 0.7H), 5.86-5.83 (m, 0.7H), 5.74-70 (m, 1H), 5.54-4.92 (m, 1.3H), 4.64-4.61 (m, 1H), 2.20 (s, 2H), 2.16 (s, 1H), 1.54 (d, J = 6.8Hz, 1H),1.49 (d, J = 6.4Hz, 2H).

### Example 50

### Preparation of 5'(R)-C-methyladenosine (50)

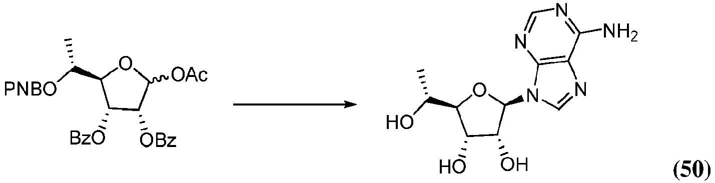

A mixture of *N⁶*-benzoyladenosine (2.39 g, 20 mmol) and *N,O-*bis(trimethylsilyl)acetamide (9.78 mL, 40 mmol) in anhydrous acetonitrile (50 mL) under argon was heated under reflux for 1 h and cooled to rt. A solution of 1-*O*-acetyl-2,3-*O-*dibenzoyl-5(*R*)-*C*-methyl-5-*O*-(4-nitrobenzoyl)-*D*-ribofuranose (1.5 g, 2.89 mmol) in anhydrous acetonitrile (50 mL) was added, followed by addition of trimethylsilyl trifluoromethanesulfonate (1.36 g, 7.5 mmol). The resulting mixture was heated under reflux overnight, cooled with ice, diluted with ethyl acetate, washed with aqueous sodium bicarbonate, dried over anhydrous Na₂SO₄ and concentrated. Chromatography on silica gel with10-15% ethyl acetate in DCM gave 3.62 g of 2',3'-O-dibenzoyl-5'(R)-C-methyl-5' -*O*-(4-nitrobenzoyl)adenosine.
2',3'-*O-*Dibenzoyl-5'(R)-*C*-methyl-5'-*O*-(4-nitrobenzoyl)adenosine (3.62 g) in methanol (300 mL) and 28% aqueous ammonia (30 mL) was stirred at RT overnight. The solvent was removed and the residue was re-dissolved in 28% aqueous NH₃ (250 mL). The mixture was stirred at rt for 2 days and the solvent was removed. Precipitation from MeOH/DCM gave 0.59 g of 5'(R)-C-methyladenosine as a white solid. The filtrate was concentrated and chromatographed on silica gel with 10-14% MeOH in DCM to give 0.68 g of 5'(*R*)-*C*-methyladenosine as a white solid. Total yield was 1.27 g. ¹HNMR (CD3OD): *δ* 8.31 (s, 1H), 8.18 (s, 1H), 5.95 (d, *J* = 6.4 Hz, 1H), 4.73 (q, *J₁* = 5.2 Hz, *J*₂ = 6.8 Hz, 1H), 4.27 (dd, *J₁* = 2.4 Hz, *J₂* = 5.2 Hz, 1H), 4.01 (t, *J* = 2.4 Hz, 1H), 3.97-3.91 (m, 1H), 1.25 (d, *J* = 6.4Hz, 3H).

### Example 51

### Preparation of 2',3'-methoxymethylidene-5'-O,N⁶-(4'-methoxytrityl)-5'(R)-methyladenosine (51)

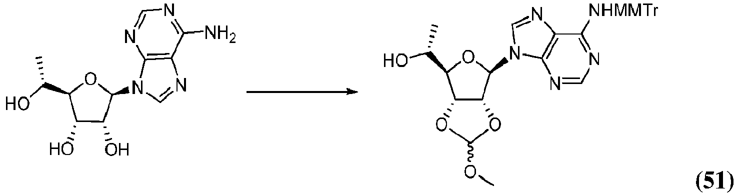

A mixture of **5'(*R*)-*C*-methyladenosine** (890 mg, 3.17 mmol), trimethyl orthoformate (9 mL) and p-toluenesulfonic acid monohydrate (904 mg, 4.75 mmol) in 1,4-dioxane (11.2 mL) was stirred at rt for 24 h, cooled with ice and quenched by adding triethylamine (1 mL) and concentrated. Chromatography on silica gel with 5-6% MeOH in DCM gave 716 mg of 2',3'-*O*-methoxymethylidene-5'(*R*)-*C*-methyladenosine.

A solution of 2',3'-*O*-Methoxymethylidene-5'(*R*)-*C*-methyladenosine (715 mg, 2.21 mmol) and 4-methoxytrityl chloride (1.03 g, 3.32 mmol) in pyridine (14 mL) was stirred at 50 °C for 20 h, diluted with ethyl acetate, washed with brine three times. Solvent was evaporated and the residue was chromatographed on silica gel with 25-55% ethyl acetate in hexanes to give 352 mg of 5'-*O*,*N*⁶-di(4'-methoxytrityl)-2',3'-methoxymethylidene-5'(*R*)-methyladenosine and 634 mg of 2',3'-methoxymethylidene-5'-*O*,*N*⁶-(4'-methoxytrityl)-5'(*R*)-methyladenosine as foam solid.

### Example 52

### Preparation of 2',3'-methoxymethylidene-5'-O,N⁶-(4'-methoxytrityl)-5'(S)-methyladenosine (52)

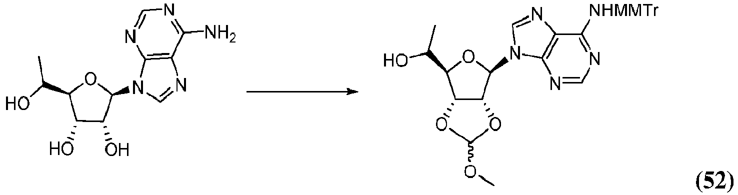

By a similar procedure as described for example **48-2,** 377 mg of 5'-*O*,*N*⁶-di(4'-methoxytrityl)-2',3'-methoxymethylidene-5'(S)-methyladenosine and 750 mg of 2',3'-methoxymethylidene-5'-*O*,*N*⁶-(4'-methoxytrityl)-5'(*S*)-methyladenosine as foam solid were prepared from 5'(*S*)-*C*-methyladenosine.

### Example 53

### Preparation of 2',5'(R and S)-C-dimethyladenosine (53)

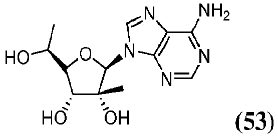

### Step 1. Preparation of 5'-O-(t-butyldimethylsilyl)-2',3'-O-(methoxymethylene)-2'-C-methyladenosine

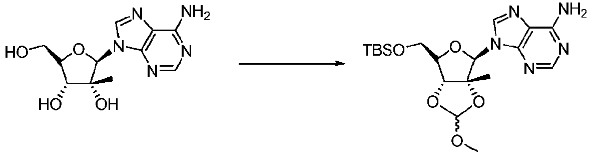

To a solution of dried 2'-C-methyladenosine (720 mg, 2.56 mmol) and trimethyl orthoformate (7.22 mL) in anhydrous 1,4-dioxane (9 mL) was added p-toluenesulfonic acid (374 mg), and stirred at room temperature under nitrogen atmosphere overnight. The reaction mixture was neutralized with methanol-ammonia (7N) to pH of 5-6 and concentrated into a crude residue, which was re-dissolved with methanol-dichloromethane (2:1, 10 mL) and stirred at room temperature overnight. The above reaction mixture was then concentrated into a crude residue, which was applied to a short column of silica gel eluted with dichloromethane-methanol (10:1) to give a pure compound 2',3'-*O-*(methoxymethylene)-2'-C-methyladenosine as amorphous solid (720 mg, 87%). *Two-isomer:* ¹H-NMR (DMSO-d₆, 500MHz): δ 8.36 (s, 1 H), 8.20 (s, 1 H), 8.15 (s, 1 H), 7.31 (s, 2 H, NH₂), 6.41 (s, 1 H), 6.22 (s, 0.3 H), 6.15 (s, 0.3 H), 5.40-5.37 (m, 1.05 H, H-1'), 4.64 (d, 0.3 H), 4.59 (d, 0.93 H, *J* = 4.10Hz), 4.28 (dd, 0.93 H), 4.20 (dd, 0.3 H), 3.80-3.76 (dt, 1.1 H), 3.72-3.67 (dt, 1.08 H), 3.39 (s, 2.7 H, OCH₃), 3.23 (s, 0.6 H), 1.14 (s, 0.4 H, 2'-CH₃), 1.03 (s, 2.84 H, 2'-CH₃).
To a solution of dried 2',3'-*O*-(methoxymethylene)-2'-*C*-methyladenosine (720 mg, 2.22 mmol) and imidazole (348 mg, 5.12 mmol) in anhydrous DMF (5 mL) was added tert-butyldimethylsilylchloride (579 mg, 3.84 mmol), and stirred at room temperature under nitrogen atmosphere overnight. The reaction mixture was then concentrated into a crude residue, and co-evaporated with toluene. The above crude residue was applied to a short column of silica gel eluted with dichloromethane-methanol (10:1) to give a pure 5'-*O*-(*t*-butyldimethylsilyl)-2',3'-*O*-(methoxymethylene)-2'-*C-*methyladenosine as amorphous solid (1.09 g, 100%).

### Step 2. Preparation of 2',3'-O-(methoxymethylene)-N⁶-(4-methoxytrityl)-2'-C-methyladenosine

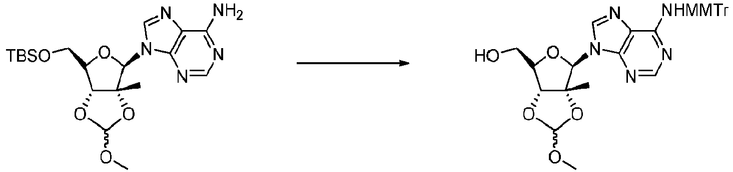

To a solution of dried 5'-*O*-(*t*-butyldimethylsilyl)-2',3'-*O*-(methoxymethylene)-2'-C-methyladenosine (1.09 g, 2.49 mmol), triethylamine (703 µL), and DMAP (290 mg) in anhydrous dichloromethane (5 mL) was added MMTrCl (1.15 g, 3.74 mmol), and stirred at 45-50 °C under nitrogen atmosphere overnight. Another portion of MMTrCl (1.15 g) was added after stirring at 45-50 °C for 16 h, and continued to be stirred at the same temperature for total of 48 h. The reaction mixture was then concentrated into a crude residue, and co-evaporated with toluene. The above crude residue was applied to a short column of silica gel eluted with hexanes-ethyl acetate (4: 1) to give a pure 5'*-O-*(*t-*butyldimethylsilyl)-2',3'-*O*-(methoxymethylene)-*N*⁶-(4-methoxytrityl)-2'-*C-*methyladenosine as amorphous solid (1.10 g, 62%).
To a solution of dried 5'-*O*-(*t*-butyldimethylsilyl)-2',3'-*O*-(methoxymethylene)-*N*⁶-(4-methoxytrityl)-2'-*C*-methyladenosine (1.1 g, 1.55 mmol) in tetrahydrofuran (THF) (6 mL) was added tetrabutylammonium fluoride hydrate (349 mg), and stirred at room temperature under nitrogen atmosphere overnight. The reaction mixture was then concentrated into a crude residue, which was applied to a short column of silica gel eluted with dichloromethane-methanol (10:1) to give a pure 2',3'-*O*-(methoxymethylene)-*N*⁶-(4-methoxytrityl)-2'-C-methyladenosine as an amorphous solid (610 mg, 66%).

### Step 3. Preparation of 2',5'-C-dimethyl-2',3'-O-(methoxymethylene)-N⁶-(4-methoxytrityl)adenosine

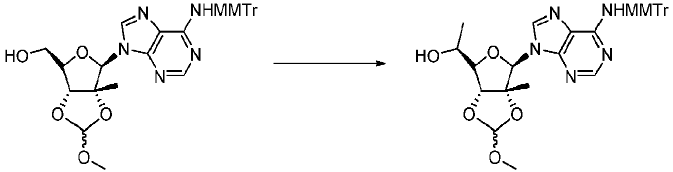

To a solution of dried 2',3'-*O*-(methoxymethylene)-*N*⁶-(4-methoxytrityl)-2'-*C-*methyladenosine (610 mg, 1.02 mmol) in a mixture of anhydrous dichloromethane (15 mL) and anhydrous pyridine (1.02 mL) was added Dess-Martin periodinane (647 mg, 1.53 mmol), and stirred at room temperature under nitrogen atmosphere for 2 h. The reaction mixture was quenched with a mixture of sat. sodium bicarbonate aq. solution and 10% Na₂S₂O₃ aq. The organic phase was separated and the aqueous phase was extracted with dichloromethane (3 x 20 mL). The combined organic phase was dried with anhydrous sodium sulfate and concentrated into a crude residue, which was applied to a short column of silica gel eluted with hexanes-ethyl acetate (1:1 and 1:2), then dichloromethane-methanol (10:1) to give a pure 5'-*C*,5'-*O*-didehysro-2',3'-*O-*(methoxymethylene)-*N*⁶-(4-methoxytrityl)-2'-*C*-methyladenosine as an amorphous solid (200 mg, 33%). *Two-isomer:* ¹H-NMR (CDCl₃, 500MHz): δ 9.5 (s, 1H, CH=O), 9.49 (s, 1 H, CH=O), 3.79 (s, 2OCH₃), 3.39 (s, 2.7 H, OCH₃), 1.14 (s, 0.4 H, 2'-CH₃), 1.03 (s, 2.84 H, 2'-CH₃).
To a cold solution of dried 5'-*C*,5'-*O*-didehysro-2',3'-*O*-(methoxymethylene)-*N*⁶-(4-methoxytrityl)-2'-C-methyladenosine (350 mg, 0.59 mmol) in anhydrous tetrahydrofuran (3-5 mL) cooled with an ice-sodium chloride bath to -20 °C was added methylmagnesium bromide (0.80 mL, 3.0 M in ether) and stirred at -20 to RT overnight under nitrogen. The reaction mixture was then quenched with sat ammonium chloride and concentrated to removal of tetrahydrofuran, and extracted with ethyl acetate (3 x 20 mL). The combined organic phase was concentrated and co-evaporated with toluene into a crude residue. The above crude residue was applied to a short column of silica gel eluted with hexanes-ethyl acetate (1:2) to give a pure 2',5'-C-dimethyl-2',3'-*O-*(methoxymethylene)-*N*⁶-(4-methoxytrityl)adenosine as amorphous solid (170 mg, 47%).

### Step 4. Preparation of 2',5'-C-dimethyladenosine

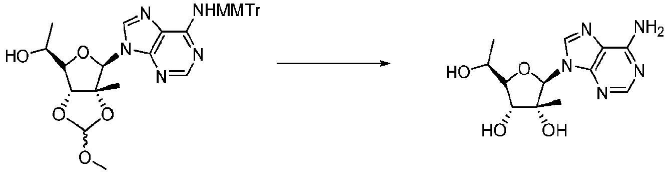

A solution of 2',5'-*C*-dimethyl-2',3'-*O*-(methoxymethylene)-*N*⁶-(4-methoxytrityl)adenosine (110 mg, 0.181 mmol) in a mixture of methanol (6 mL), acetic acid (3 mL), and water (1 mL) was stirred at 50 °C for 16 h, The reaction mixture was then concentrated and co-evaporated with toluene into a crude residue, which was applied to a short column of silica gel eluted with dichloromethane-methanol (10:1 and 6:1) to give a pure 2',5'-C-dimethyladenosine (40 mg, 75%) as amorphous solid. *Two-isomer A and B, ratio of A vs B is 1.77*: ¹H-NMR (CD₃OD, 500MHz): δ8.57 (s, 1 H, isomer-A), 8.55 (s, 1 H, isomer-B), 8.20 (s, 1 H, isomer-A), 8.19 (s, 1 H, isomer-B), 6.09 (s, 1 H, H'-1, isomer-A), 6.07 (s, 1 H, H'-1, isomer-B), 4.58 (s, 0.8 H), 4.30-4.28 (m, 4 H), 4.19 (d, 1 H), 4.08-4.07 (dq, 1 H), 3.97 (dd, 1 H), 3.88 (dd, 1 H), 1.36 (d, 3 H, 5'-CH₃, isomer-A, J = 6.6 Hz), 1.33 (d, 3 H, 5'-CH₃, isomer-B, *J* =6.9 Hz), 0.90 (s, 3 H, 2'-CH₃, isomer-A), 0.89 (s, 3 H, 2'-CH₃, isomer-B). ESI-MS (positive mode): 295 [M], 318 [M+Na].

### Example 54

### Preparation of 2',5'(S)-C-dimethyladenosine (54)

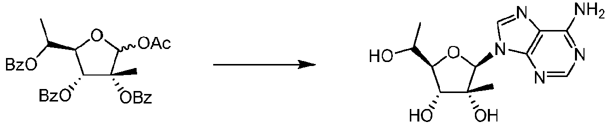

A mixture of *N⁶*-benzoyladenosine (1.46 g, 6.12 mmol) and *N,O-*bis(trimethylsilyl)acetamide (2.95 mL, 12.24 mmol) in anhydrous acetonitrile (15 mL) under argon was heated under reflux for 45 min and cooled to rt. A solution of 1-*O-*acetyl-5(*S*)-*C*-methyl-5-*O*-(4-nitrobenzoyl)- 2,3,5-*O*-tribenzoyl-*D*-ribofuranose (1.63 g, 3.06 mmol) in anhydrous acetonitrile (15 mL) was added, followed by addition of trimethylsilyl trifluoromethanesulfonate (0.86 mL, 4.59 mmol). The resulting mixture was heated under reflux overnight, cooled with ice, diluted with ethyl acetate, washed with aqueous sodium bicarbonate, dried over anhydrous Na₂SO₄ and concentrated. Chromatography on silica gel with10-15% ethyl acetate in DCM gave 1.60 g of 5'(S)-C-methyl-2',3',5'-*O*-tribenzoyladenosine.
5'(*S*)-*C*-Methyl-2',3',5'-*O*-tribenzoyladenosine (1.58 g) in methanol (150 mL) and 28% aqueous ammonia (50 mL) was stirred at RT overnight. The solvent was removed and the residue was re-dissolved in 28% aqueous NH₃ (130 mL). The mixture was stirred at rt for 3 days and the solvent was removed. Chromatography on silica gel with 12-14% MeOH in DCM gave 619 mg of 2',5'(*S*)-C-dimethyladenosine as a white solid; ¹H NMR (CD30D): *δ* 8.55 (s, 1H), 8.19 (s, 1H), 6.07 (s, 1H), 4.29 (d, *J*= 8.4 Hz, 1H), 4.26 (m, 1H), 3.97 (dd, *J*₁ = 8.4 Hz, *J*₂= 2.4 Hz,1H), 1.33 (d, *J* = 6.8 Hz, 3H), 0.89 (s, 3H).

### Example 55

### Preparation of 2',5'(R)-C-dimethyladenosine (55)

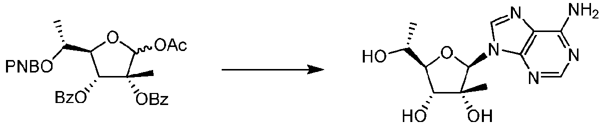

A mixture of *N⁶*-benzoyladenosine (1.75 g, 7.3 mmol) and *N,O-*bis(trimethylsilyl)acetamide (3.6 mL, 14.6 mmol) in anhydrous acetonitrile (18 mL) under argon was heated under reflux for 45 min and cooled to rt. A solution of 1-*O*-acetyl-5(*S*)-C-methyl-5-*O*-(4-nitrobenzoyl)-2,3,5-*O*-tribenzoyl-*D*-ribofuranose (2.11 g, 3.65 mmol) in anhydrous acetonitrile (18 mL) was added, followed by addition of trimethylsilyl trifluoromethanesulfonate (1.02 mL, 5.48 mmol). The resulting mixture was heated under reflux overnight, cooled with ice, diluted with ethyl acetate, washed with aqueous sodium bicarbonate, dried over anhydrous Na₂SO₄ and concentrated. Chromatography on silica gel with10-15% ethyl acetate in DCM gave 1.81 g of 5'(*R*)-C-methyl-5'-O-(4-nitrobenzoyl)- 2',3',5'-*O*-tribenzoyladenosine.
5'(R)-C-Methyl-5'-O-(4-nitrobenzoyl)- 2',3',5'-*O*-tribenzoyladenosine (2.04 g) in methanol (200 mL) and 28% aqueous ammonia (65 mL) was stirred at RT overnight. The solvent was removed and the residue was re-dissolved in 28% aqueous NH₃ (240 mL). The mixture was stirred at rt for 2 days and the solvent was removed. Precipitate was washed with 20% MeOH in DCM and then with MeOH to give 402 mg of 2',5'(R)-C-dimethyladenosine as white solid. Chromatography on silica gel with 10-14% MeOH in DCM gave 397 mg of 2',5'(*R*)-*C*-dimethyladenosine as a white solid. Total yield was 779 mg. ¹HNMR (CD₃OD): *δ*8.59 (s, 1H), 8.19 (s, 1H), 6.09 (s, 1H), 4.19 (d, *J* = 8.8 Hz, 1H), 4.06 (dq, 1H), 3.88 (dd, *J*₁ = 8.8 Hz, *J*₂= 2.4 Hz,1H), 1.36 (d, *J* = 6.8 Hz, 3H), 0.90 (s, 3H).

### Example 56

### Preparation of 5'(R)-C-methylguanosine (56)

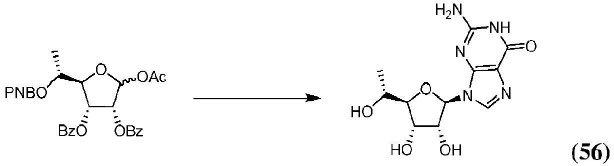

To a solution of 1-*O*-acetyl-2,3-*O*-dibenzoyl-5(*R*)-C-methyl-5-*O*-(4-nitrobenzoyl)-D-ribofuranose (969 mg, 1.72 mmol), 2-amino-6-chloropurine (0,32 g, 1.87 mmol) and DBU (0.77 mL, 5.10 mmol) in anhydrous acetonitrile (20 mL) was added dropwise trimethylsilyl trifluoromethanesulfonate (1.25 mL, 6.88 mmol). The resulting reaction mixture was stirred at 65 °C overnight, cooled, diluted with ethyl acetate, washed with 10% sodium bicarbonate and dried over sodium sulfate. Chromatography on silica gel with 10-15% ethyl acetate in DCM gave 0.62 g of 1-(2-amino-6-chloropurin-*N*⁹-yl)-2,3-*O*-dibenzoyl-5(*R*)-C-methyl-5-*O*-(4-nitrobenzoyl)-*β*-*D*-ribofuranose as a white solid, which was dissolved in 7 M NH₃ in MeOH and stood at RT overnight and concentrated. Chromatography on silica gel with 10-15% MeOH in DCM gave 260 mg of 1-(2-amino-6-chloropurin-*N*⁹-yl)-5(*R*)-*C*-methyl-*β*-*D*-ribofuranose as a white solid.
To a mixture of 1-(2-amino-6-chloropurin-*N*⁹-yl)-5(*R*)-*C*-methyl-*β*-*D*-ribofuranose (253 mg, 0.8 mmol) and mercaptoethnaol (2.28 mL, 4.0 mmol) in MeOH (5 mL) was added 0.5 M NaOMe in MeOH (8 mL, 4.0 mmol). The resulting mixture was refluxed overnight, cooled, neutralized with AcOH. Reverse phase HPLC with acetonitrile/water gave 5'(*R*)-*C*-methylguanosine as a white solid (167 mg); ¹HNMR (DMSO-d₆) *δ* 1.08 (d, J= 6.8 Hz, 3H), 3.16 (d, J= 4.8 Hz, 1H), 3.7 (t, J = 3.2 Hz, 1H), 3.73-3.79 (m, 1H), 4.06-4.10 (m, 2H), 4.30-4.34 (m, 1H), 5.34 (d, J= 5.2 Hz, 1H), 5.68 (d, J= 5.6 Hz, 1H), 6.47 (br s, 2H), 7.95 (s, 1H), 10.72 (br s, 1H).

### Example 57

### Preparation of 5'(R)-C-methylcytidine (57)

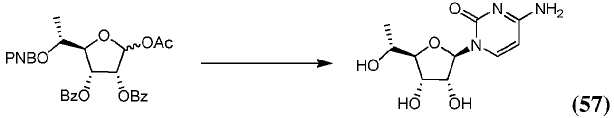

*N*⁴-Benzoylcytosine (215 mg, 1.0 mmol) and *N,O*-bis(trimethylsilyl)acetonitrile (0.49 mL, 2.0 mmol) in anhydrous acetonitrile (2 mL) was refluxed for 30 min and cooled. A solution of 1-*O*-acetyl-2,3-*O*-dibenzoyl-5(*R*)-*C*-methyl-5-*O*-(4-nitrobenzoyl)-*D-*ribofuranose (289 mg, 0.5 mmol) in acetonitrile (2 mL) was added, followed by addition of tin tetrachloride (0.24 mL, 2.0 mmol). The resulting reaction mixture was refluxed overnight, cooled, diluted with ethyl acetate, washed with 10% sodium bicarbonate and dried over sodium sulfate. Chromatography on silica gel with 10-20% ethyl acetate in DCM gave 2',3'-*O,N*⁴-tribenzoyl-5'(*R*)-*C*-methyl-5'-*O*-(4-nitrobenzoyl)cytidine, which was dissolved in 7.0 M NH₃/MeOH and stood at RT for 3 h. The solution was concentrated and the residue dissolved in 29% aqueous ammonia and stood at RT for 3 days. Volatile was evaporated and the residue was subjected to reverse-phase HPLC purification to give 122 mg of 5'(*R*)-*C*-methylcytidine.

### Example 58

### Preparation of 5'(S)-C-methyladenosine 5'-[phenyl(methoxy-L-alaninyl)]phosphate (58)

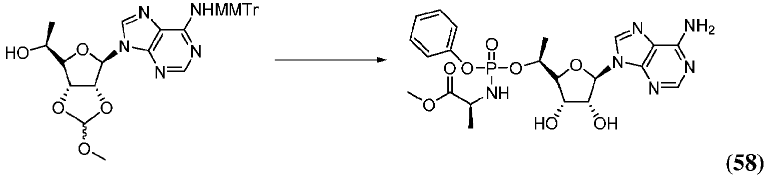

To a solution of 2',3'-*O*-methoxymethylene-*N*⁶-(4-methoxytrityl)-5'(*S*)-methyladenosine (60 mg, 0.1 mmol) in THF (1 mL) under argon was added 1.0 M t-BuMgBr in THF (0.25 mL, 0.25 mmol). The resulting solution was stirred at RT for 30 min and phenyl(methoxy-L-alaninyl) phosphorochloridate (85 mg, 0.3 mmol). The reaction mixture was stirred at RT for 3 days, cooled with ice, quenched with water, diluted with ethyl acetate, washed with brine three times. Chromatography on silica gel with ethyl acetate/hexanes (1:1 to 2:1) gave a mixture of four isomers as a white solid. The product was dissolved in 80% formic acid (5 mL) and stood at RT overnight. Solvent was evaporated at RT and co-evaporated with MeOH/toluene three times. Chromatography on silica gel with 10-15% MeOH in DCM, followed by re-purification by reverse-phase HPLC with acetonitrile/water, gave 9.5 mg of 5'(S)-C-methyladenosine 5'-[phenyl(methoxy-L-alaninyl)]phosphate as white solid; ¹HNMR (CD₃OD) *δ* 1.28 (d, J= 6.8 Hz, 3H), 1.44 (d, J= 6.4 Hz, 3H), 3.65 (s, 3H), 3.89-3.93 (m, 1H), 4.01-4.04 (m, 1H), 4.45-4.47 (m, 1H), 4.70 (t, J = 6.0 Hz, 1H), 4.58-5.98 (d, J= 6.8 Hz, 1H), 7.12-7.33 (m, 6H), 8.19 (s, 1H), 8.31 (s, 1H); ³¹P NMR (CD₃OD) *δ* 3.39.

### Example 59

### Preparation of 5'(R)-C-methyladenosine 5'-[phenyl(methoxy-L-alaninyl)]phosphate (59)

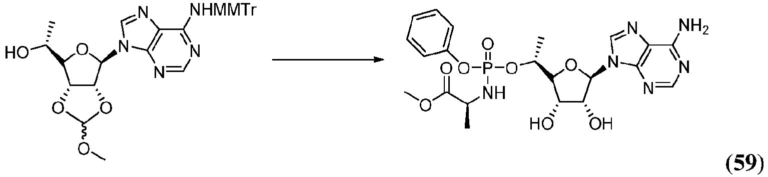

To a solution of 2',3'-*O*-methoxymethylene-*N*⁶-(4-methoxytrityl)-5'(*R*)-methyladenosine (60 mg, 0.1 mmol) in THF (1 mL) under argon was added 1.0 M t-BuMgBr in THF (0.25 mL, 0.25 mmol). The resulting solution was stirred at RT for 30 min and phenyl(methoxy-L-alaninyl) phosphorochloridate (85 mg, 0.3 mmol) was added. The reaction mixture was stirred at RT for 3 days, cooled with ice, quenched with water, diluted with ethyl acetate, washed with brine three times. Chromatography on silica gel with ethyl acetate/hexanes (1:1 to 2:1) gave a mixture of four isomers as a white solid. The product was dissolved in 80% formic acid (5 mL) and stood at RT overnight. Solvent was evaporated at RT and co-evaporated with MeOH/toluene three times. Chromatography on silica gel with 10-15% MeOH in DCM, followed by re-purification by reverse-phase HPLC with acetonitrile/water, gave 12 mg of 5'(*R*)-*C*-methyladenosine 5'-[phenyl(methoxy-L-alaninyl)]phosphate as white solid; ¹H NMR (CD₃OD) *δ* 1.24 (d, J= 6.8 Hz, 3H), 1.52 (d, J= 6.4 Hz, 3H), 3.66 (s, 3H), 3.91-3.97 (m, 1H), 4.05-4.08 (m, 1H), 4.35 (t, J = 4.4 Hz, 1H), 4.52 (t, J = 4.8 Hz, 1H), 4.82-4.85 (m, 1H), 6.04 (d, J= 5.6 Hz, 1H), 7.10-7.31 (m, 6H), 8.2 (s, 1H), 8.29 (s, 1H); ³¹P NMR (CD₃OD) *δ* 3.72.

### Example 60

### Preparation of 2',5'(S)-C-dimethyladenosine 5'-[phenyl(methoxy-L-alaninyl)]phosphate (60)

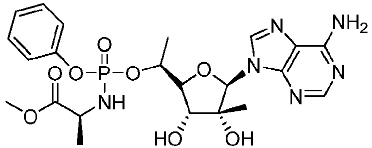

### Step 1. Preparation of 2',3'-O-methoxymethylidene-N⁶-(4'-methoxytrityl)-5'(S)-methyladenosine

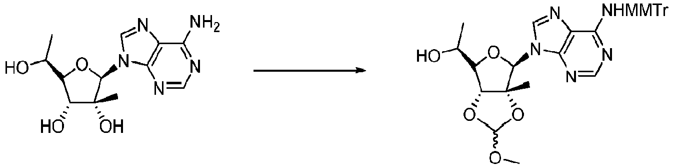

A mixture of 2,5'(*S*)-*C*-dimethyladenosine (585 mg, 1.98 mmol), trimethyl orthoformate (5.6 mL) and p-toluenesulfonic acid monohydrate (565 mg, 2.97 mmol) in 1,4-dioxane (7 mL) was stirred at 30 °C for 24 h, cooled with ice and quenched by adding triethylamine (1 mL) and concentrated. Chromatography on silica gel with 5-7% MeOH in DCM gave 716 mg of 2',3'-O-methoxymethylidene-2,5'(S)-C-dimethyladenosine.
A solution of 2',3'-*O*-methoxymethylidene-2,5'(*S*)-*C*-dimethyladenosine (575 mg, 1.71 mmol) and 4-methoxytrityl chloride (714 mg, 2.32 mmol) in pyridine (16 mL) was stirred at rt for 3 days. Additional 4-methoxytrityl chloride (72 mg) was added and the mixture was heated at 40 °C for 24 h. Additional 144 mg of 4-methoxytrityl was added the mixture was heated at 50 °C for 24 h, diluted with ethyl acetate, washed with brine three times. Solvent was evaporated and the residue was chromatographed on silica gel with 25-60% ethyl acetate in hexanes to give 151 mg of 5'-*O*,*N*⁶-di(4'-methoxytrityl)-2',3'-*O*-methoxymethylidene-5'(*S*)-methyladenosine and 489 mg of 2',3'-*O-*methoxymethylidene-*N*⁶-(4'-methoxytrityl)-5'(*S*)-methyladenosine as amophous solid.

### Step 2. Preparation of 2',5'(S)-C-dimethyladenosine 5'-[phenyl(methoxy-L-alaninyl)]phosphate

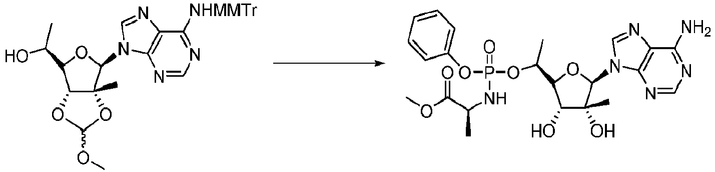

To a solution of 2',5'(*S*)-*C*-dimethyl-2',3'-*O*-methyomethylene-*N*⁶-(4-methoxytrityl)adenosine (60 mmg, 0.1 mmol) in THF (1 mL) under argon was added 1.0 M *t*-BuMgBr in THF (0.25 mL, 0.25 mmol). The resulting solution was stirred at RT for 30 min and phenyl(methoxy-L-alaninyl) phosphorochloridate (85 mg, 0.3 mmol). The reaction mixture was stirred at RT for 3 days, cooled with ice, quenched with water, diluted with ethyl acetate, washed with brine three times. Chromatography on silica gel with ethyl acetate/hexanes (1:1 to 2:1) gave a mixture of four isomers as a white solid. The product was dissolved in 80% formic acid (5 mL) and stood at RT overnight. Solvent was evaporated at RT and co-evaportaed with MeOH/toluene three times. Chromatography on silica gel with 10-15% MeOH in DCM, followed by re-purification by reverse-phase HPLC with acetonitrile/water, gave 6.8 mg of 2',5'(S)-C-dimethyladenosine 5'-[phenyl(methoxy-L-alaninyl)]phosphate as white solid; ¹HNMR (CD₃OD) *δ* 0.95 (d, J= 4.4 Hz, 3H), 1.21 (dd, J= 1.2, 7.2 Hz, 1H), 1.30 (dd, J= 0.8, 7.2 Hz, 1H), 1.55 (dd, J= 1.6, 6.8 Hz, 1H), 2.32 (s, 1H), 3.58 (s, 1H), 3.64 (s, 2H), 3.82-3.99 (m, 1H), 4.07-4.11 (m, 1H), 4.27 & 4.36 (each d, J= 8.8, 8.4 Hz, 1H), 4.99-5.05 (m, 1H), 6.10 & 6.13 (2 x s, 1H), 7.1-7.39 (m, 7H), 8.18 & 8.19 (2 x s, 1H), 8.29 & 8.31 (2 x s, 1H); ³¹P NMR (CD₃OD) *δ* 3.59, 3.74.

### Example 61

### Preparation of 2',5'(R)-C-dimethyladenosine 5'-[phenyl(methoxy-L-alaninyl)]phosphate (61)

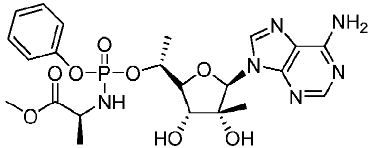

### Step 1. Preparation of 2',3'-O-methoxymethylidene-N⁶-(4'-methoxytrityl)-5'(R)-methyladenosine

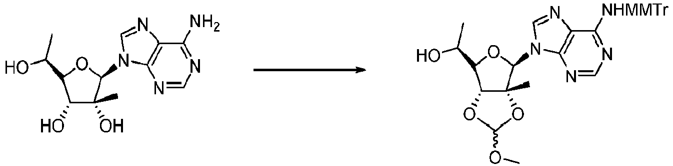

A mixture of 2,5'(*R*)-*C*-dimethyladenosine (395 mg, 1.34 mmol), trimethyl orthoformate (3.8 mL) and p-toluenesulfonic acid monohydrate (382 mg, 2.01 mmol) in 1,4-dioxane (4.8 mL) was stirred at 30 °C for 24 h, cooled with ice and quenched by adding triethylamine (1 mL) and concentrated. Chromatography on silica gel with 5-7% MeOH in DCM gave 360 mg of 2',3'-O-methoxymethylidene-2,5'(R)-C-dimethyladenosine.
A solution of 2',3'-*O*-methoxymethylidene-2,5'(*R*)-*C-*imethyladenosine (357 mg, 1.06 mmol) and 4-methoxytrityl chloride (444 mg, 1.44 mmol) in pyridine (10 mL) was stirred at rt for 3 days. Additional 222 mg of 4-methoxytrityl was added the mixture was heated at 50 °C for 24 h, diluted with ethyl acetate, washed with brine three times. Solvent was evaporated and the residue was chromatographed on silica gel with 25-60% ethyl acetate in hexanes to give 142 mg of 5'-O,*N*⁶-di(4'-methoxytrityl)-2',3'-*O-*methoxymethylidene-5'(*R*)-methyladenosine and 301 mg of 2',3'-*O-*methoxymethylidene-*N*⁶-(4'-methoxytrityl)-5'(*R*)-methyladenosine as amophous solid.

### Step 2. Preparation of 2',5'(R)-C-dimethyladenosine 5'-[phenyl(methoxy-L-alaninyl)]phosphate

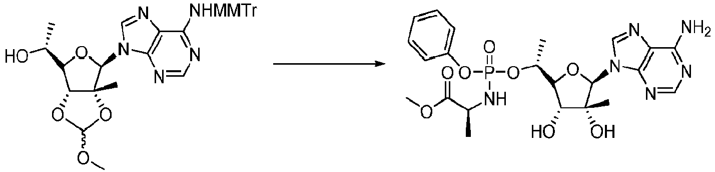

To a solution of 2',5'(*R*)-i-dimethyl-2',3'-*O*-methyomethylene-*N*⁶-(4-methoxytrityl)adenosine (61 mg, 0.1 mmol) in THF (1 mL) under argon was added 1.0 M *t*-BuMgBr in THF (0.25 mL, 0.25 mmol). The resulting solution was stirred at RT for 30 min and phenyl(methoxy-L-alaninyl) phosphorochloridate (85 mg, 0.3 mmol). The reaction mixture was stirred at RT for 3 days, cooled with ice, quenched with water, diluted with ethyl acetate, washed with brine three times. Chromatography on silica gel with ethyl acetate/hexanes (1:1 to 2:1) gave a mixture of four isomers as a white solid. The product was dissolved in 80% formic acid (5 mL) and stood at RT overnight. Solvent was evaporated at RT and co-evaportaed with MeOH/toluene three times. Reverse-phase HPLC with acetonitrile/water gave 16.1 mg of 2',5'(*R*)-*C*-dimethyladenosine 5'-[phenyl(methoxy-L-alaninyl)]phosphate as white solid. Isomer A: ¹HNMR (CD₃OD) *δ* 0.89 (s, 3H), 1.27 (dd, J= 1.2, 7.2 Hz, 3H), 1.61 (d, J= 6.4, 3H), 2.32 (s, 2H), 3.96-4.02 (m, 2H), 4.09 (d, J= 9.2 Hz, 1H), 4.96-5.00 (m, 1H), 3.65 (s, 3H), 3.94-4.02 (m, 2H), 4.09 (d, J= 9.2 Hz, 1H), 4.96 (m, 1H), 7.09-7.32 (m, 7H), 8.20 (s, 1H), 8.25 (s, 1H); ³¹P NMR (CD₃OD) *δ* 3.73; Isomer B: ¹H NMR (CD₃OD) *δ* 0.94 & 0.98 ( each s, 3H), 1.25 (d, J= 10.4 Hz, 3H), 1.50 (d, J= 6.8, 3H), 2.32 (s, 1H), 3.55 (s, 3H), 3.92-4.01 (m, 2H), 4.26 (d, J= 9.2 Hz, 1H), 6.09 (s, 1H), 7.08-7.36 (m, 7H), 8.21 (s, 1H), 8.2 (s, 1H); ³¹P NMR (CD₃OD) *δ* 3.61, 3.70.

### Example 62

### Preparation of 2'-O-(t-butyldimethysilyl)-3'-deoxy-5'(R,S)-O-methyl-N⁴-(4-methoxytrityl)cytidine (62)

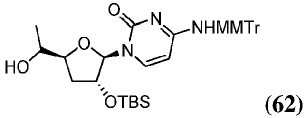

### Step 1. Preparation of N⁴-acetyl-2'-O-(t-butyldimethylsilyl)-5'-O-(4,4'-dimethoxytrityl)cytidine

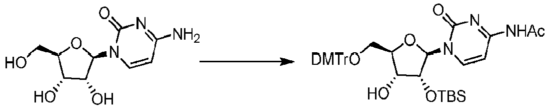

Cytidine (100.0g, 0.41 mol) was dissolved in DMF (500 ml), acetic anhydride (42.5 ml, 45.9 g, 0.45 mol) was added and the whole was left for 24 h. Solvent was evaporated, the residue boiled with methanol (40 ml) and cooled. Crystals were filtered and dried to furnish *N*⁴-acetylcytidine (102 g, 87.0%).
To a solution of *N*⁴-acetylcytidine (65.0 g, 0.228 mol) in anhydrous pyridine (600mL) cooled in an ice bath, DMTrCl (84.7 g, 0.251 mol) was added. The reaction mixture was stirred at room temperature overnight. To the reaction mixture cooled with an ice bath, THF (600 ml) and AgNO₃ (58.1 g, 0.342 mmol) were added. Then TBSC1 (51.5 g, 0.342 mmol) was added, and the reaction mixture was stirred at room temperature overnight. The reaction mixture was filtered, solvent was removed under vacuum to give a residue which was diluted with EtOAc (500 mL) and washed with water (200 ml) and brine (200 ml). The organic layer was separated and dried over anhydrous Na₂SO₄ and the filtrate was concentrated to a syrup which was purified by chromatography on silica gel (eluted with PE : EA = 5:1 to 3 : 1) to give *N*⁴-acetyl-2'-*O*-(*t*-butyldimethylsilyl)-5'-*O-*(4,4'-dimethoxytrityl)cytidine as yellow solid (80 g, 50%). ¹HNMR (CDCl₃) δ8.39 (d, J = 7.6 Hz, 1H), 7.34 (dd, J1 = 1.6 Hz, J2 = 8.4 Hz, 2H), 7.21 - 7.27 (m, 6H), 7.02 (d, J = 7.2 Hz, 1H), 6.80 (dd, J1 = 2.0 Hz, J2 = 6.8 Hz, 4H), 5.82 (d, J = 1.2 Hz, 1H), 4.26 - 4.32 (m, 1H), 4.20 (dd, J1 = 1.2 Hz, J2 = 4.4 Hz, 1H), 4.00 - 4.02 (m, 1H), 3.74 (d, J = 1.6 Hz, 6 H), 3.43 - 3.53 (m, 2H), 2.32 (d, J = 9.6 Hz, 1H), 2.18 (s, 3H), 0.86 (s, 9H), 0.22 (s, 3H), 0.11 (s, 3H).

### Step 2. Preparation of N⁴-acetyl-2'-O-(t-butyldimethylsilyl)-3'-deoxy-5'-O-(4,4'-dimethoxytrityl)cytidine

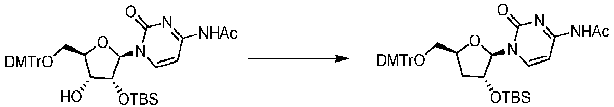

*N*⁴-Acetyl-2'-*O*-(*t*-butyldimethylsilyl)-5'-*O*-(4,4'-dimethoxytrityl)cytidine (50.0 g, 71.3 mmol) and DMAP (26.1 g, 213.9 mmol) was dissolved in ACN (2000 ml), and PTCCl (18.5 g, 106.9 mmol) was added dropwise under nitrogen atmosphere at room temperature, then the reaction mixture was stirred at room temperature overnight. Then solvent was removed under vacuum to give a residue which was diluted with EtOAc (500 mL) and washed with water (200 ml) and brine (200 ml). The organic layer was separated and dried over anhydrous Na₂SO₄ and the filtrate was concentrated to a syrup which was purified by chromatography on silica gel (eluted with PE : EA = 5:1 to 3 : 1) to give *N*⁴-Acetyl-2'-O-(t-butyldimethylsilyl)-5'-*O*-(4,4'-dimethoxytrityl)-3'-*O-*(phenoxythiono)cytidine as yellow solid (27.0 g, 45.2%).
To a solution of *N*⁴-acetyl-2'-*O*-(*t*-butyldimethylsilyl)-5'-*O*-(4,4'-dimethoxytrityl)-3'-O-(phenoxythiono)cytidine (24.0 g, 28.7 mmol) and AIBN (5.1 g, 31.6 mmol) in anhydrous toluene (1000 ml), (Bu)₃SnH (16.7 g, 57.3 mmol) was added dropwise under nitrogen atmosphere at room temperature, then the reaction mixture was refluxed at 120 °C for 10 h. The solvent was removed under vacuum to give a residue which was diluted with EtOAc (500 mL) and washed with water (200 ml) and brine (200 ml). The organic layer was separated and dried over anhydrous Na₂SO₄ and the filtrate was concentrated to a syrup which was purified by silica gel chromatography (eluted with PE : EA = 8:1 to 5 : 1) to give *N*⁴-acetyl-2'-*O*-(*t*-butyldimethylsilyl)-3'-deoxy-5'-*O*-(4,4'-dimethoxytrityl)cytidine as yellow solid (16.0 g, 81.6%).

### Step 3. Preparation of 2'-O-(t-butyldimethylsilyl)-3'-deoxy-5'-O-(4,4'-dimethoxytrityl)-N⁴-(4-methoxytrityl)cytidine

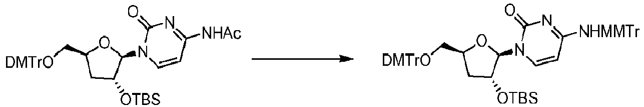

A solution of *N*⁴-acetyl-2'-*O*-(*t*-butyldimethylsilyl)-3'-deoxy-5'-*O*-(4,4'-dimethoxytrityl)cytidine (11.0 g, 16.0 mmol) in NH₃/MeOH (300 ml) was stirred at room temperature overnight. The solvent was removed under vacuum to give a residue which was purified by silica gel chromatography (eluted with PE : EA = 1:1 to 1 : 3) to give 2'-*O*-(*t*-butyldimethylsilyl)-3'-deoxy-5'-*O*-(4,4'-dimethoxytrityl)cytidine as yellow solid (6.0 g, 58.2%). 1HNMR (400MHz) (CDCl₃) δ 8.09 (d, J = 7.2 Hz, 1H), 7.35 (d, J = 8.8 Hz, 2H), 7.21 - 7.26 (m, 7H), 6.77 (dd, J₁ = 1.2 Hz, J₂ = 8.8 Hz, 4H), 5.70 (s, 1H), 5.18 (d, J = 7.2 Hz, 1H), 4.50 - 4.51 (m, 1H), 4.33 (d, J = 3.6 Hz, 1H), 3.72 (s, 6H), 3.55 (dd, J1 = 2.0 Hz, J2 = 11.2 Hz, 1H), 3.26 (dd, J1 = 3.6 Hz, J2 = 10.8 Hz, 1H), 1.97 (s, 1H), 1.94 (s, 1H), 1.63 (dd, J1 = 4.4 Hz, J2 = 12.4 Hz, 1H), 0.81 (s, 9H), 0.14 (s, 3H), 0.04 (s, 3H). To a solution of 2'-O-(t-butyldimethylsilyl)-3'-deoxy-5'-O-(4,4'-dimethoxytrityl)cytidine (6.0 g, 9.3 mmol), AgNO₃ (4.7 g, 28.0 mmol) and MMTrCl (8.6 g, 28.0 mmol) in anhydrous DCM (150 ml), collidine (16.9 g, 139.5 mmol) was added dropwise under nitrogen atmosphere at room temperature. Then the reaction mixture was refluxed at 50 °C for 12 h. The reaction mixture was filtered, solvent was removed under vacuum to give a residue which was purified by silica gel chromatography (eluted with PE : EA = 5:1 to 3 : 1) to give 2'-*O*-(*t*-butyldimethylsilyl)-3'-deoxy-5'-*O*-(4,4'-dimethoxytrityl)-*N*⁴-(4-methoxytrityl)cytidine as yellow solid (8.0 g, 93.7%). 1HNMR (400MHz) (CDCl₃):δ7.84 (dd, J = 2.8 Hz, 7.6 Hz, 1H), 6.62 - 7.20 (m, 27H), 5.20 (d, J = 4.8 Hz, 1H), 4.57 (dd, J₁ = 7.6 Hz, J₂ = 12.0 Hz, 1H), 4.40 (d, J = 8.8 Hz, 1H), 4.28 (d, J = 2.8 Hz, 1H), 3.64 - 3.68 (m, 9H), 3.41 - 3.45 (m, 1H), 3.23 (ddd, J = 3.2 Hz, 11.2 Hz, 1H), 1.88 - 1.91 (m, 1H), 1.55 - 1.61 (m, 1H), 1.19 (s, 9H), 0.14 (s, 3H), 0.05 (s, 3H).

### Step 4. Preparation of 2'-O-(t-butyldimethylsilyl)-3'-deoxy-5'C,5'-O-didehydro-N⁴-(4-methoxytrityl)cytidine

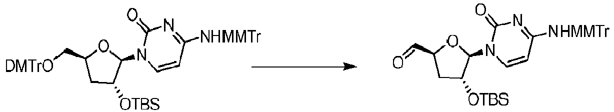

A solution of 2'-O-(t-butyldimethylsilyl)-3'-deoxy-5'-O-(4,4'-dimethoxytrityl)-*N*⁴-(4-methoxytrityl)cytidine (6.0 g, 6.6 mmol) in 80% AcOH (200 mL) was stirred at room temperature for 7 h. The reaction mixture was neutralized with NaHCO₃ to pH = 7, then was diluted with EtOAc (100 mL) and washed with water (100 ml) and brine (100 ml). The organic layer was separated and dried over anhydrous Na₂SO₄ and the filtrate was concentrated to a syrup which was purified by silica gel chromatography (eluted with PE : EA = 3:1 to 2 : 1) to give 2'-*O*-(*t*-butyldimethylsilyl)-3'-deoxy-*N*⁴-(4-methoxytrityl)cytidine (2.9 g, 72.5%); ¹HNMR (400MHz) (CDCl₃) δ 7.29 (d, J = 7.6 Hz, 1H),7.15 - 7.25 (m, 10H), 7.12 (d, J = 24 Hz, 2H), 6.75 (d, J = 8.8 Hz, 2H), 5.28 (d, J = 2.4 Hz, 1H), 4.99 (d, J = 7.6 Hz, 1H), 4.59 - 4.62 (m, 1H), 4.36 - 4.40 (m, 1H), 3.88 (dd, J = 2.0 Hz, 12.0 Hz, 1H), 3.73 (s, 3H), 3.54 (dd, J1 = 3.2 Hz, 12.0 Hz, 1H), 2.00 - 2.03 (m, 1H), 1.70 - 1.76 (m, 1H), 0.78 (s, 9H), 0.02 (s, 3H), 0.01 (s, 3H).
A mixture of 2'-*O*-(*t-*butyldimethylsilyl)-3'-deoxy-*N*⁴-(4-methoxytrityl)cytidine (2.9 g, 4.7 mmol), pyridine (1.9g, 23.8 mmol), anhydrous DCM (20 ml), and a solution of Dess-Martin reagents (3.0 g, 7.2 mmol) in anhydrous DCM (20 ml) was added dropwise under nitrogen atmosphere in an ice bath. Then the reaction mixture was stirred at room temperature overnight. The reaction mixture was filtered, and filtrate was washed with saturated Na₂S₂O₃ solution (20 ml). The organic layer washed with water brine (20 ml), dried over anhydrous Na₂SO₄ and the filtrate was concentrated to a syrup which was purified by silica gel chromatography (eluted with PE : EA = 3:1 then PE : EA = 1 : 1) to give 2' -*O*-(*t*-butyldimethylsilyl)-3'-deoxy-5'C,5'-O-didehydro-*N*⁴-(4-methoxytrityl)cytidine as yellow solid (1.5 g, 51.9%); ¹HNMR (400MHz) (CDCl₃) δ 9.68 (s, 1H), 7.35 (d, J = 7.6 Hz, 1H), 7.06 - 7.23 (m, 11H), 6.75 (d, J = 8.8 Hz, 4H), 5.57 (s, 1H), 4.99 (d, J = 7.6 Hz, 1H), 4.81 (dd, J = 6.4 Hz, 10.4 Hz, 1H), 4.53 (d, J = 2.0 Hz, 1H), 3.73 (s, 3H), 2.05 (dd, J = 2.0 Hz, 5.2 Hz, 1H), 1.71 - 1.78 (m, 1H), 0.82 (s, 9H), 0.11 (s, 3H), 0.05 (s, 3H).

### Step 5. Preparation of 2'-O-(t-butyldimethylsilyl)-3'-deoxy-5'-C-methyl-N⁴-(4-methoxytrityl)cytidine

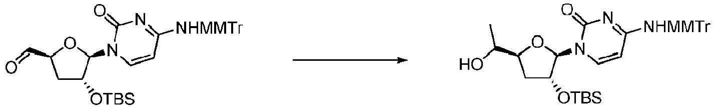

To a solution of 2'-*O*-(*t*-butyldimethylsilyl)-3'-deoxy-5'C,5'-*O*-didehydro-*N*⁴-(4-methoxytrityl)cytidine (0.85 g, 4.66 mmol) in anhydrous THF (10 ml), MeMgBr (2.8 ml, 8.50 mmol) was added dropwise under nitrogen atmosphere at -20 °C, then it was warmed up to room temperature and stirred overnight. The reaction mixture was slowly quenched with saturated NH₄Cl solution, and then extracted with EA (20 mLx3). The combined organic phase was dried with anhydrous Na₂SO₄ and the filtrate was concentrated to a syrup which was purified by silica gel chromatography (eluted with PE : EA = 5:1 then PE : EA = 3 : 1) to give 2'-*O*-(*t*-butyldimethylsilyl)-3'-deoxy-5'-*C*-methyl-*N*⁴-(4-methoxytrityl)cytidine as yellow solid (0.31 g, 35.6%), which was subjected to SFC separation to afford 2'-*O*-(*t*-butyldimethylsilyl)-3'-deoxy-5'(S)-*C*-methyl-*N*⁴-(4-methoxytrityl)cytidine and 2'-*O*-(*t*-butyldimethylsilyl)-3'-deoxy-5'(*R*)-*C*-methyl-*N*⁴-(4-methoxytrityl)cytidine. The isomer with shorter retention time in SFC was designated as 5'(S)-isomer; ¹H NMR (400MHz) (CDCl₃) δ 7.18 - 7.28 (m, 10H), 7.11 (d, J₁ = 8.8 Hz, 2H), 6.80, (d, J = 8.8 Hz, 3H), 5.15 (d, J = 3.6 Hz, 1H), 4.99 (d, J = 7.6 Hz, 1H), 4.75 - 4.79 (m, 1H), 4.17 - 4.20 (m, 1H), 4.05 - 4.07 (m, 1H), 3.88 (br, 1H), 3.78 (s, 3H), 2.18 - 2.25 (m, 1H), 1.72 - 1.78 (m, 1H), 1.09 (d, J = 6.4 Hz, 1H), 0.82 (s, 9H), 0.02 (s, 3H), 0.00 (s, 3H). The isomers with longer retention time in SFC was designated as 5'(R)-isomer; ¹H NMR (400MHz) (CDCl₃) δ 7.15 - 7.26 (m, 10H), 7.07 (d, J = 8.8 Hz, 2H), 6.25 (d, J = 8.8 Hz, 3H), 5.31 (d, J = 2.4 Hz, 1H), 4.93 (d, J = 6.8 Hz, 1H), 4.57 - 4.59 (m, 1H), 4.11 - 4.16 (m, 1H), 3.72 (s, 3H), 3.63 - 3.66 (m, 1H), 1.78 - 1.85 (m, 1H), 1.70 - 1.75 (m, 1H), 1.15 (d, J = 6.4 Hz, 3H), 0.79 (s, 9H), 0.02 (s, 3H), 0.00 (s, 3H).

### Example 63

### Preparation of 3'-deoxy-5'(R)-C-methylcytidine 5'-[phenyl(methoxy-L-alaninyl)]phosphate (63)

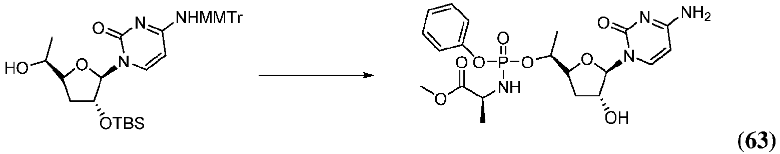

To a solution of 2'-(*t*-butyldimethysilyl)-3'-deoxy-5'(*R*)-methyl-*N*⁴-(4-methoxytrityl)cytidine (63 mmg, 0.1 mmol) in THF (1 mL) under argon was added 1.0 M *t*-BuMgBr in THF (0.25 mL). The resulting solution was stirred at RT for 30 min and phenyl(methoxy-L-alaninyl) phosphorochloridate (0.34 g, 1.2 mmol) was added. The reaction mixture was stirred at RT overnight, cooled with ice, quenched with water, diluted with ethyl acetate, washed with brine three times. Chromatography on silica gel with ethyl acetate/hexanes (1:1 to 2:1) gave a mixture of four isomers as a white solid. The product was dissolved in 80% formic acid (5 mL) and stood at RT overnight. Solvent was evaporated at RT and co-evaporated with MeOH/toluene three times. Reverse-phase HPLC purification with 1% formic acid in acetonitrile/water, followed by chromatography on silica gel with 10-15% MeOH in DCM, gave 13 mg of 3'-deoxy-5'(R)-C-methylcytidine 5'-[phenyl(methoxy-L-alaninyl)]phosphate as white solid; ¹H NMR (DMSO-d₆) *δ* 1.17-1.39 (m, 7H), 1.72-1.94 (m, 3H), 2.29 (s, 1H), 3.54, 3.59 (each s, 3H), 3.82-3.91 (m, 1H), 4.12 (br s, 1H), 4.22-4.32 (m, 2H), 4.54-4.59 (m, 1H), 5.57-5.76 (m, s, 1H4H), 5.97-6.04(m, 1H), 7.14-7.46 (m, 10H), 7.72 (d, J= 7.6 Hz, 1H), 8.13 (s, 1H), 12.84 (br s, 1H); ³¹P NMR (DMSO-d₆) *δ* 3.9, 4.08.

### Example 64

### Preparation of 3'-deoxy-5'(S)-C-methylcytidine 5'-[phenyl(methoxy-L-alaninyl)]phosphate (64)

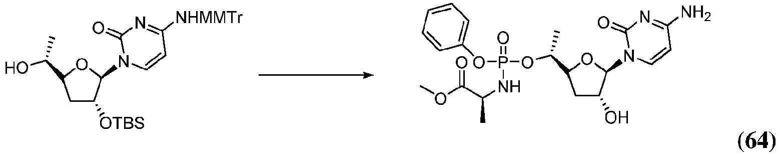

To a solution of 2'-(*t*-butyldimethylsilyl)-3'-deoxy-5'(*S*)-methyl-*N*⁴-(4-methoxytrityl)cytidine (94 mmg, 0.1 mmol) in THF (1 mL) under argon was added 1.0 M *t*-BuMgBr in THF (0.38 mL). The resulting solution was stirred at RT for 30 min and phenyl(methoxy-L-alaninyl) phosphorochloridate (0.51 g, 1.8 mmol). The reaction mixture was stirred at RT overnight, cooled with ice, quenched with water, diluted with ethyl acetate, washed with brine three times. Chromatography on silica gel with ethyl acetate/hexanes (1:1 to 2:1) gave a mixture of four isomers as a white solid. The product was dissolved in 80% formic acid (5 mL) and stood at RT overnight. Solvent was evaporated at RT and co-evaportaed with MeOH/toluene three times. Reverse-phase HPLC purification with 1% formic acid in acetonitrile/water, followed by chromatography on silica gel with 10-15% MeOH in DCM, gave 12 mg of 3'-deoxy-5'(S)-C-methylcytidine 5'-[phenyl(methoxy-L-alaninyl)]phosphate as white solid; ¹H NMR (DMSO-d₆) *δ* 1.20 (d, J= 6.0 Hz, 3H), 1.29 (d, J= 7.2 Hz, 3H), 1.77-1.93 (m, 2H), 2.29 (s, 2H), 3.05 (s, 3H), 3.79-3.86 (m, 1H), 4.12-4.15 (m, 2H), 4.64-4.67 (m, 1H), 5.55 (br s, 1H), 5.62-5.76 (m, 3H), 6.06 (dd, J = 10.4 Hz, 1H), 7.12-7.39 (m, 12H), 7.66 (d, J= 7.2 Hz, 1H); ³¹P NMR (DMSO-d₆) *δ* 3.32, 3.65.

### Example 65

### Preparation of 5'(S)-C-methylarabinocytidine 5'-[phenyl(methoxy-L-alaninyl)]phosphate (65)

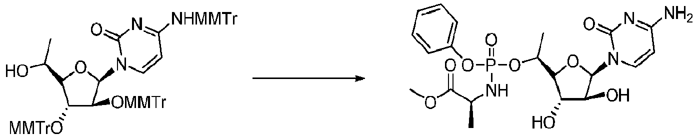

To a solution of 5'(*S*)-methyl-2',3'-O,*N*⁴-tris(4-methoxytrityl)arabinocytidine (212 mg, 0.2 mmol) in THF (2 mL) under argon was added 1.0 M *t*-BuMgBr in THF (0.45 mL). The resulting solution was stirred at RT for 15 min and phenyl(methoxy-L-alaninyl) phosphorochloridate (0.17 g, 0.6 mmol) was added. The reaction mixture was stirred at RT for 4 days, cooled with ice, quenched with water, diluted with ethyl acetate, washed with brine three times. Chromatography on silica gel with ethyl acetate/hexanes (1:1 to 3:1) gave a mixture of four isomers as a white solid (132 mg). The product was dissolved in 80% formic acid (5 mL) and stood at 40-50 °C for 4 h. Solvent was evaporated at RT and co-evaporated with MeOH/toluene three times. Reverse-phase HPLC purification with acetonitrile/water, followed by chromatography on silica gel with 15-30% MeOH in DCM, gave 31 mg of 5'(S)-C-methylarabinocytidine 5'-[phenyl(methoxy-*L-*alaninyl)]phosphate as white solid. Isomer A: ¹H NMR (CD₃OD) *δ* 1.29-1.33 (m, 5H), 1.51 (d, J= 6.4 Hz, 3H), 3.22 (m, 1H), 3.66 (s, 3H), 3.8-3.82 (m, 1H), 3.98-4.06 (m, 2H), 4.16-4.18 (m, 1H), 5.66 (d, J= 7.6 Hz, 1H), 6.18 (d, J= 3.6 Hz, 1H), 7.14-7.34 (m, 6H), 7.93 (d, J= 7.2 Hz, 1H), 8.32 (br s, 1H); ³¹P NMR (CD₃OD) *δ* 3.3; Isomer B: ¹H NMR (CD₃OD) *δ* 1.32-1.33 (m, 6H), 3.63 (s, 3H), 3.73-3.79 (m, 1H), 3.97-4.03 (m, 2H), 4.16-4.17 (m, 1H), 4.77-4.83 (m, 1H), 5.85 (d, J= 7.6 Hz, 1H), 6.21 (d, J= 3.6 Hz, 1H), 7.14-7.39 (m, 6H), 7.99 (d, J= 7.2 Hz, 1H), 8.24 (br s, 1H); ³¹P NMR (CD₃OD) *δ* 4.07.

### Example 66

### Preparation of 5'(S)-C-methyladenosin-5'-yl bis(S-pivaloyl-2-thioethyl) phosphate (66)

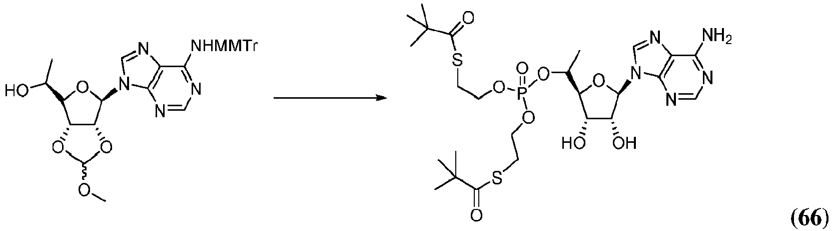

To a solution of 2',3'-O-methyomethylene-5'(*S*)-methyl-*N*⁶-(4-methoxytrityl)adenosine (120 mg, 0.2 mmol) in acetonitrile (0.4 mL) under argon was added bis(*S*-pivaloyl-2-thioethyl) *N,N*-diisopropylphosphoramidite (136 mg, 0.3 mmol), 0.25 mmol), followed by addition of 0.45 M tetrazole in acetonitrile (1.5 mL, 0.66 mmol). The resulting solution was stirred at RT for 1.5 h, cooled to -40 °C and a solution of mCPBA (69 mg, 0.4 mmol) in DCM (0.75 mL) was added. The mixture was warmed up to RT and stirred for 10 min, diluted with ethyl acetate, washed with 10% Na₂S₂O₃ two times and washed with brine. Chromatography on silica gel with 15-25% ethyl acetate in DCM gave 140 mg of purified product, which was dissolved in 80% AcOH (8 mL) and the solution was heated at 50 °C for 24 h. Solvent was evaporated and the residue was chromatographed on silica gel with 7-10% MeOH in DCM to give 5'(S)-C-methyladenosin-5'-yl bis(S-pivaloyl-2-thioethyl) phosphate (61 mg) as white solid; ¹H NMR (CDCl₃) *δ* 1.21 (s, 18H), 1.45 (d, J= 6.4 Hz, 3H), 3.02-3.11 (m, 4H), 3.94-4.07 (m, 4H), 4.16-4.18 (m, 1H), 4.58-4.75 (m, 3H), 5.91 (br s, 2H), 5.94 (d, J= 6.0 Hz, 1H), 6.05-6.15 (br s, 1H), 8.07 (s, 1H), 8.26 (s, 1H).

### Example 67

### Preparation of 5'(R)-C-methyladenosin-5'-yl bis(S-pivaloyl-2-thioethyl) phosphate (67)

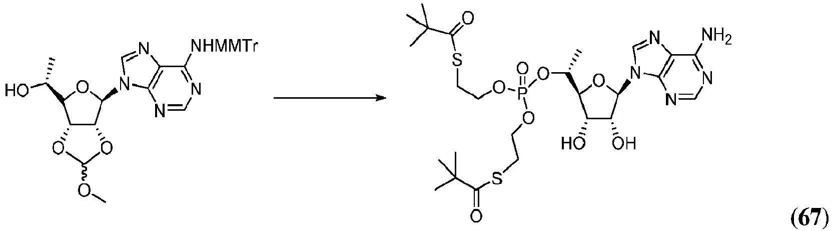

To a solution of 2',3'-*O*-methyomethylene-5'(*R*)-methyl-*N*⁶-(4-methoxytrityl)adenosine (238 mg, 0.4 mmol) in acetonitrile (0.8 mL) under argon was added bis(*S*-pivaloyl-2-thioethyl) *N,N*-diisopropylphosphoramidite (272 mg, 0.6 mmol), followed by addition of 0.45 M tetrazole in acetonitrile (3.0 mL, 1.32 mmol). The resulting solution was stirred at RT for 3 h, cooled to -40 °C and a solution of mCPBA (172 mg, 1.0 mmol) in DCM (2 mL) was added. The mixture was warmed up to RT and stirred for 10 min, diluted with ethyl acetate, washed with 10% Na₂S₂O₃ two times and washed with brine. Chromatography on silica gel with 25-35% ethyl acetate in DCM gave 326 mg of purified product, 207 mg of which was dissolved in 80% AcOH (12 mL) and the solution was heated at 50 °C for 24 h. Solvent was evaporated and the residue was chromatographed on silica gel with 5-7% MeOH in DCM to give 5'(R)-C-methyladenosin-5'-yl bis(*S*-pivaloyl-2-thioethyl) phosphate (105 mg) as a white solid; ¹H NMR (CDCl₃) *δ* 1.17 (s, 9H), 1.23 (s, 9H), 1.51 (d, J= 6.4 Hz, 3H), 3.04-3.13 (m, 4H), 4.02-4.10 (m, 4H), 4.21-4.22 (m, 1H), 4.46 (t, J= 3.6 Hz, 1H), 4.62 (t, J= 5.2 Hz, 1H), 4.74-4.78 (m, 1H), 6.00 (br s, 2H), 6.06 (d, J= 4.8 Hz, 1H), 8.17 (s, 1H), 8.26 (s, 1H).

### Example 68

### Preparation of 2',5'(S)-C-dimethyladenosin-5'-yl bis(S-pivaloyl-2-thioethyl) phosphate (68)

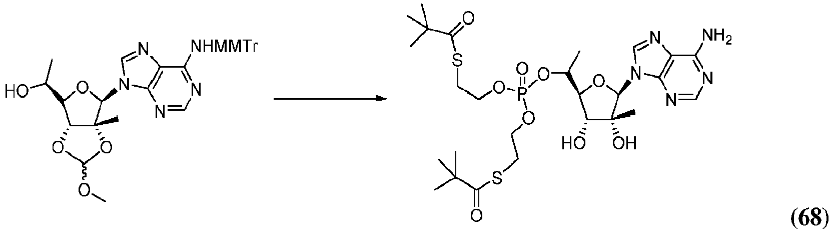

To a solution of 2',3'-O-methyomethylene-2',5'(*S*)-dimethyl-*N*⁶-(4-methoxytrityl)adenosine (122 mg, 0.2 mmol) in acetonitrile (0.4 mL) under argon was added bis(*S*-pivaloyl-2-thioethyl) *N,N*-diisopropylphosphoramidite (136 mg, 0.3 mmol), followed by addition of 0.45 M tetrazole in acetonitrile (1.5 mL, 0.66 mmol). The resulting solution was stirred at RT for 3 h, cooled to -40 °C and a solution of mCPBA (86 mg, 0.5 mmol) in DCM (1 mL) was added. The mixture was warmed up to RT and stirred for 10 min, diluted with ethyl acetate, washed with 10% Na₂S₂O₃ two times and washed with brine. Chromatography on silica gel with 25-35% ethyl acetate in DCM gave a purified product, which was dissolved in 80% AcOH (10 mL) and the solution was heated at 50 °C for 24 h. Solvent was evaporated and the residue was chromatographed on silica gel with 4-7% MeOH in DCM to give 2',5'(*S*)-C-dimethyladenosin-5'-yl bis(S-pivaloyl-2-thioethyl) phosphate (68 mg) as a white solid; ¹H NMR (CDCl₃) *δ* 1.02 (s, 3H), 1.22, 1.24 (2 x s, each 9H), 1.49 (d, J= 6.8 Hz, 3H), 3.14-3.20 (m, 4H), 4.01 (t, J= 5.6 Hz, 1H), 4.12-4.20 (m, 5H), 4.43-4.46 (m, 1H), 4.73 (br s, 1H), 4.83-4.88 (m, 1H), 5.64 (br s, 2H), 5.97 (s, 1H), 7.95 (s, 1H), 8.33 (s, 1H).

### Example 69

### Preparation of 2',5'(R)-C-dimethyladenosin-5'-yl bis(S-pivaloyl-2-thioethyl) phosphate (69)

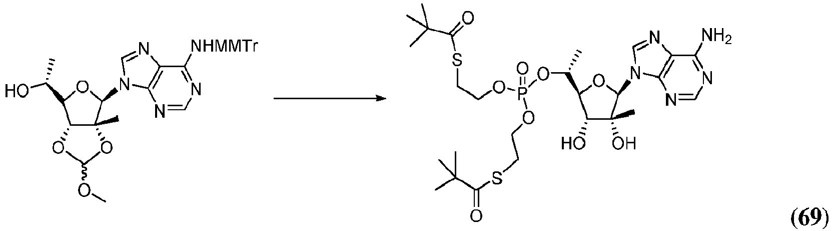

To a solution of 2',3'-*O*-methyomethylene-2',5'(*R*)-dimethyl-*N*⁶-(4-methoxytrityl)adenosine (183 mg, 0.3 mmol) in acetonitrile (0.6 mL) under argon was added bis(*S*-pivaloyl-2-thioethyl) *N,N*-diisopropylphosphoramidite (204 mg, 0.45 mmol), followed by addition of 0.45 M tetrazole in acetonitrile (2.2 mL, 0.99 mmol). The resulting solution was stirred at RT for 3 h, cooled to -40 °C and a solution of mCPBA (129 mg, 0.75 mmol) in DCM (1.5 mL) was added. The mixture was warmed up to RT and stirred for 10 min, diluted with ethyl acetate, washed with 10% Na₂S₂O₃ two times and washed with brine. Chromatography on silica gel with 25-35% ethyl acetate in DCM gave a purified product, which was dissolved in 80% AcOH (10 mL) and the solution was heated at 50 °C for 24 h. Solvent was evaporated and the residue was chromatographed on silica gel with 4-7% MeOH in DCM to give 2',5'(R)-C-dimethyladenosin-5'-yl bis(S-pivaloyl-2-thioethyl) phosphate (85 mg) as a white solid; ¹H NMR (CDCl₃) *δ* 1.01 (s, 3H), 1.20, 1.24 (2 x s, each 9H), 1.56 (d, = 6.8 Hz, 3H), 3.14-3.19 (m, 4H), 4.01-4.19 (m, 7H), 4.36 (s, 1H), 4.79-4.83 (m, 1H), 5.7 (br s, 2H), 6.15 (s, 1H), 8.13 (s, 1H), 8.35 (s, 1H).

### Example 70

### General procedure for synthesis of 5'-alkylated nucleoside 5'-triphosphates

1,2,3-Triazol (41 mg, 0.6 mmol) was dissolved in the mixture of 1 ml of dry CH₃CN and 88 ul of dry triethylamine in 1.5 ml centrifuge tube. The solution was cooled down to 0 °C and POCl₃ (19 ul, 0.2 mmol) was added. The mixture was vortexed and left at 5 °C for 20 min. The white precipitate was centrifugated and supernatant was added to 0.1 mmol of dry nucleoside in 10 ml flask. Reaction mixture was kept at +5 °C for 2 hours, then tris(tetrabutylammonium)pyrophosphate was added (360 mg, 0.4 mmol). The reaction was left for 2 hours more at room temperature and solvents were evaporated. The residue was dissolved in 80% HCOOH and left for 2 hours more at ambient temperature. Formic acid was evaporated, the residue distributed between 6 ml of water and 3 ml of DCM. Organic fraction was separated and the aqueous fraction was extracted with DCM (2 x 3 ml). Aqueous fraction containing target NTP was loaded on ion-exchange column HiLoad 16/10 Q Sepharose High Performance. Target NTP was eluted by gradient of NaCl from 0 to 1 M in 50 mmol TRIS-buffer (pH 8). Corresponding fractions were collected and desalted by RP Chromatography on Synergi 4u Hydro-RP 80A 100 X 21 in linear gradient of methanol in TEAB-buffer (pH 8.5) from 0 to 40%. Fraction containing target NTP was lyophilized from water (3 x 5 ml).
The general procedure was used for synthesis of following nucleoside 5'-triphosphates.

### 2',5'(S)-C-Dimethyladenosine 5'-triphosphate

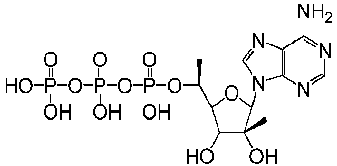

MS: 534.1 (M-1). H¹ NMR (D₂O): 0.83 (s, 3H, methyl); 1.13 (t, 34H, Et₃N-salt); 1.36-1.37 (d, 3H, methyl); 3.02-3.08 (dd, 22H, Et₃N-salt); 3.96-3.99 (m, 1H, 4'-H), 4.20-4.22 (d, 1H, 5'-H); 4.60-4.63 (m, 1H, H-3'); 6.10 (s, 1H, H-1'); 8.10 and 8.31 (s, 1H, adenine). ³¹P NMR (D₂O): -8.75 (d, 1P); -11.45 (d, 1P), -22.48(t, 1P).

### 2',5'(R)-C-Dimethyladenosine 5'-triphosphate

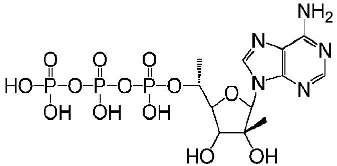

MS: 534.4 (M-1). H¹ NMR (D₂O): 0.85 (s, 3H, methyl); 1.15 (t, 29H, Et₃N-salt); 1.39-1.40 (s, 3H, methyl); 3.04-3.10 (dd, 18H, Et₃N-salt); 3.92-3.94 (m, 1H, 4'-H), 4.29-4.27 (d, 1H, 5'-H, J= 9.2 Hz); 6.04 (s, 1H, H-1'); 8.12 and 8.46 (s, 1H, adenine). ³¹P NMR (D₂O): -8.58 (bs, 1P); -11.09 (d, 1P), -22.15 (t, 1P).

### 2'-Deoxy-2'2'-difluoro-5'(S)-ethynylcytidine 5'-triphosphate

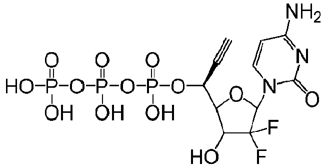

MS: 526.2 (M-1). H¹ NMR (D₂O): 1.15 (t, 16H, Et₃N-salt); 2.89-3.05 (dd, 10H, Et₃N-salt); 4.10-4.12 (d, 1H, 4'-H), 4.68-4.90 (m, 1H, 5'-H, J= 9.2 Hz); 5.14-5.16 (d, 1H, H-3'); 6.03-6.05 (d, 1H, H-5); 6.16-6.20 (t, 1H, H-1'); 7.74-7.76 (d, 1H, H-6) ³¹P NMR (D₂O): -9.58 (bs, 1P); -11.65 (d, 1P), -21.92 (bs, 1P)

### 2'-Deoxy-2'2'-difluoro-5'(S)-ethylcytidine 5'-triphosphate

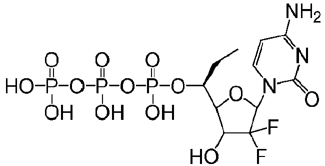

MS: 529.9 (M-1). H¹ NMR (D₂O): 0.84-0.88 (t, 3H, CH₂CH₃); 1.14 (t, 16H, Et₃N-salt); 1.71-1.84 (m, 2H, CH₂CH₃); 3.03-3.09 (dd, 12H, Et₃N-salt); 3.97-4.00 (d, 1H, 4'-H), 4.30-4.36 (m, 1H, 5'-H); 4.45-4.50 (m, 1H, H-3'); 6.05-6.07 (d, 1H, H-5); 6.10-6.14 (m, 1H, H-1'); 7.81-7.83 (d, 1H, H-6). ³¹P NMR (D₂O): -10.15 (d, 1P); -11.20 (d, 1P), -22.45 (t, 1P).

### 5'(S)-methylarabinocytidine 5'-triphosphate

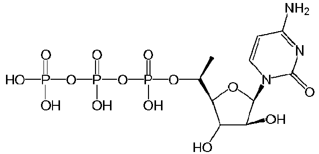

MS: 496.0 (M-1). H¹ NMR (D₂O): 1.14 (t, 15H, Et₃N-salt); 1.33 (d, 1H, methyl), 3.04-3.10 (dd, 12H, Et₃N-salt); 3.67-3.70 (m, 1H, 4'-H), 4.12-4.16 (m, 1H, 5'-H); 4.29-4.32 (m, 1H, H-3'); 4.50-4.60 (m, 1H, H-2'); 6.03-6.05 (d, 1H, H-5); 6.10-6.11(d, 1H, H-1'); 7.90-7.92 (d, 1H, H-6). ³¹P NMR (D₂O): -10.15 (d, 1P); -11.30 (d, 1P), -22.54 (t, 1P).

### 5'(S)-methyladenosine 5'-triphosphate

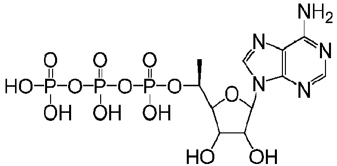

MS: 520.1 (M-1). H¹ NMR (D₂O): 1.23-1.24 (s, 3H, methyl); 1.11 (t, 30H, Et₃N-salt); 3.01-3.07 (dd, 18H, Et₃N-salt); 4.06 (bs, 1H, 4'-H), 4.45-4.53 (m, 2H); 6.00 (d, 1H, H-1'); 8.09 and 8.45 (s, 1H, adenine). ³¹P NMR (D₂O): -9.75 (d, 1P); -11.41 (d, 1P), -22.54 (t, 1P).

### 2'-Deoxy-2'2'-difluoro -5'(S)-methylcytidine 5'-triphosphate

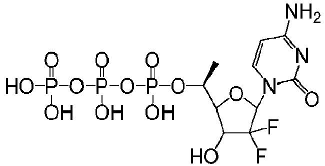

MS: 516.0 (M-1). H¹ NMR (D₂O): 1.15 (t, 25H, Et₃N-salt); 1.36-1.37 (d, 3H, CH₃); 3.04-3.10 (dd, 16H, Et₃N-salt); 3.83-3.85 (d, 1H, 4'-H), 4.35-4.58 (m, 2H), 6.02-6.04 (d, 1H, H-5); 6.11-6.14 (m, 1H, H-1'); 7.81-7.83 (d, 1H, H-6). ³¹P NMR (D₂O): -9.50 (bs, 1P); - 11.30 (d, 1P), -22.33 (t, 1P).

### ADDITIONAL EXEMPLARY COMPOUNDS

Compounds prepared by similar protocols and procedures to the preceding examples include, for example, the compounds shown in Table 1. The compounds show in Table 1 are illustrative only and are not intended, or are they to be construed, to limit the scope of the claims in any manner whatsoever.

**Table 1: Exemplary Compounds**

| **Structure** | **Structure** |
|---|---|
| 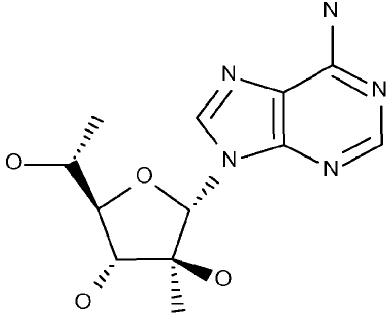 | 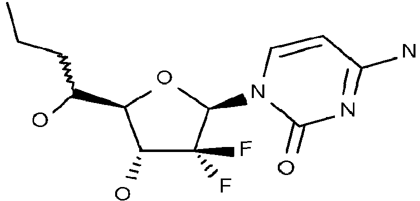 |
| 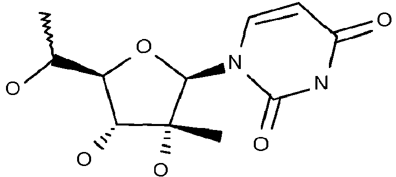 | 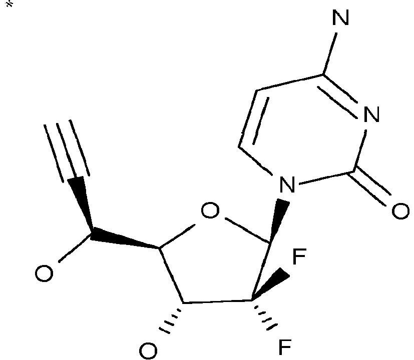 |
| 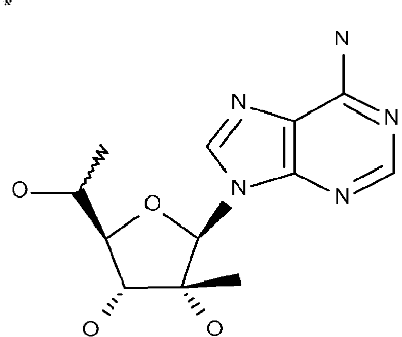 | 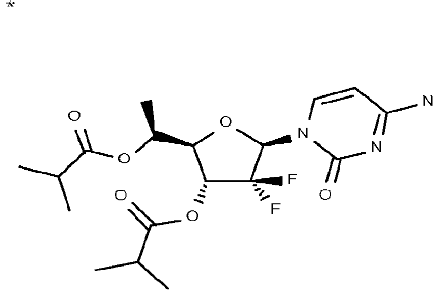 |
| 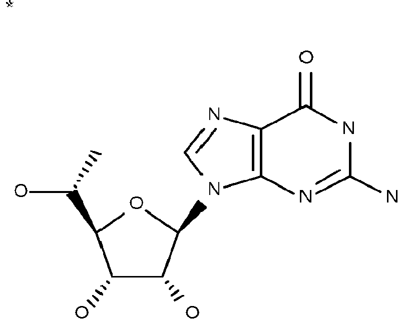 | 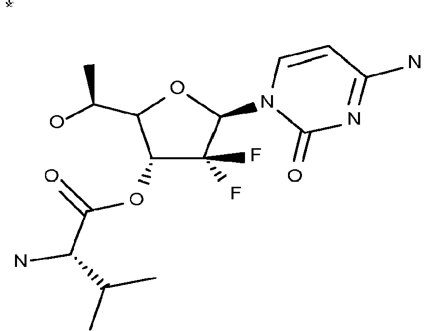 |
| 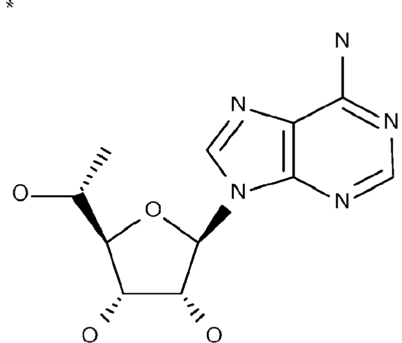 | 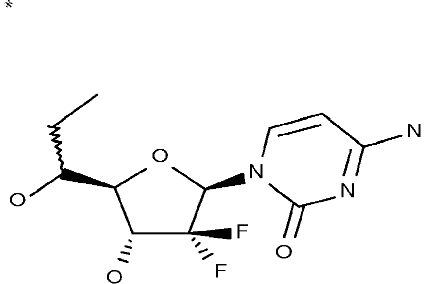 |
| 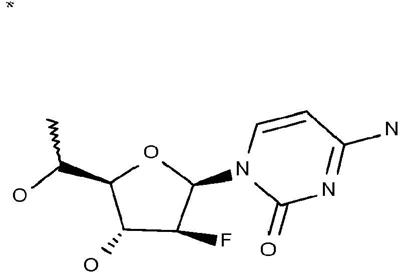 | 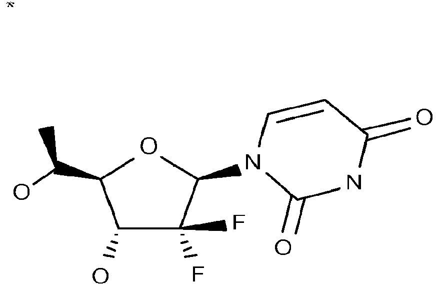 |
| 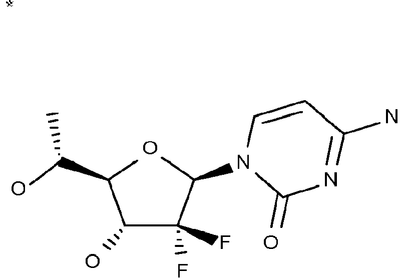 | 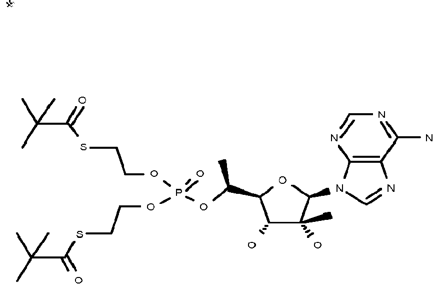 |
| 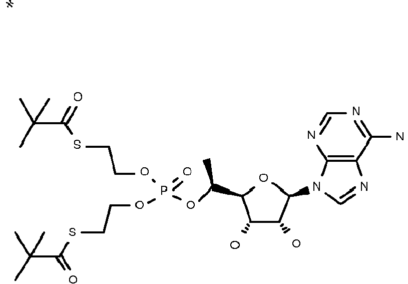 | 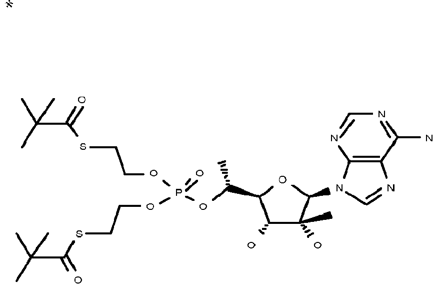 |
| 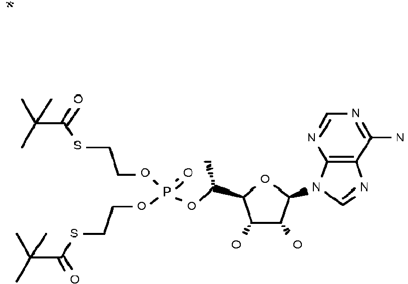 | 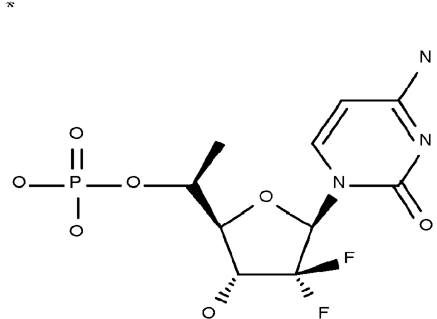 |
| 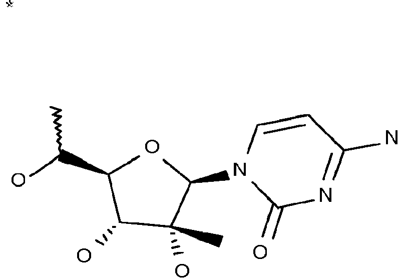 | 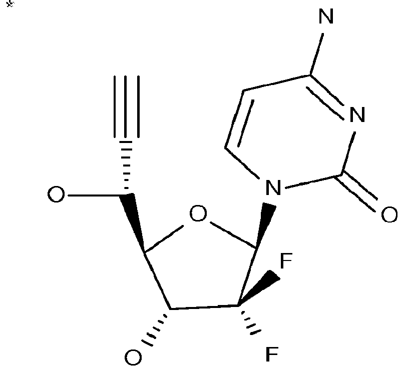 |
| 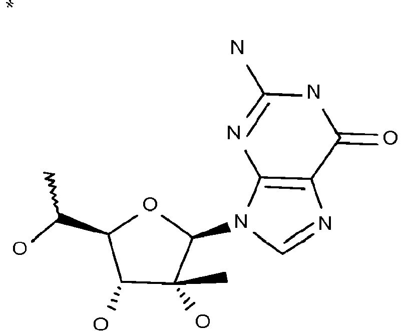 | 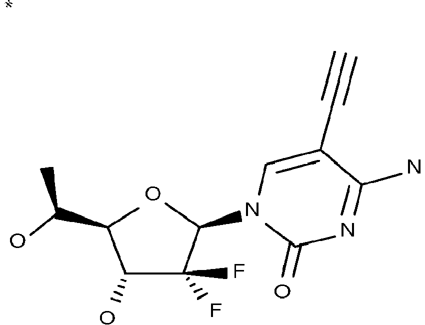 |
| 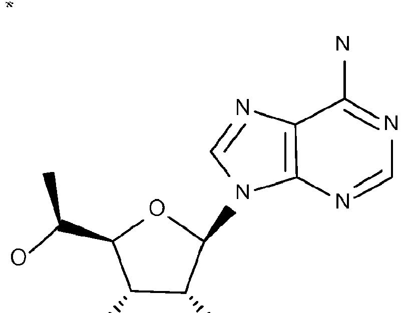 | 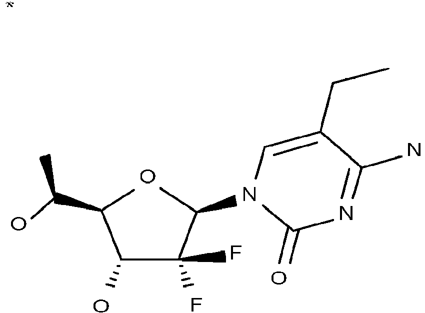 |
| 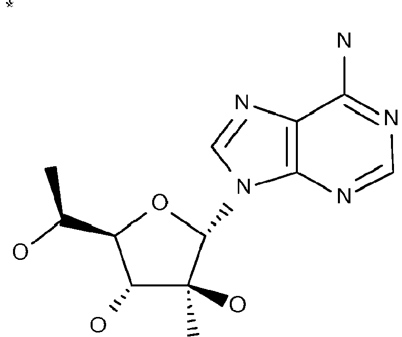 | 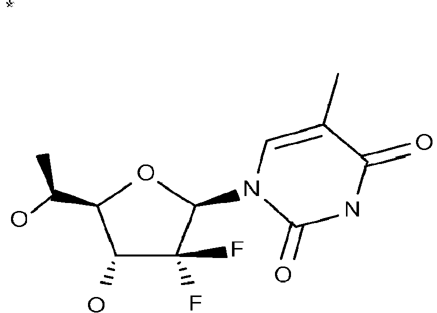 |
| 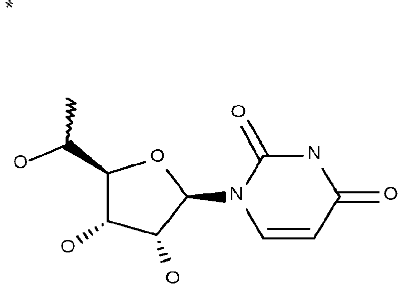 | 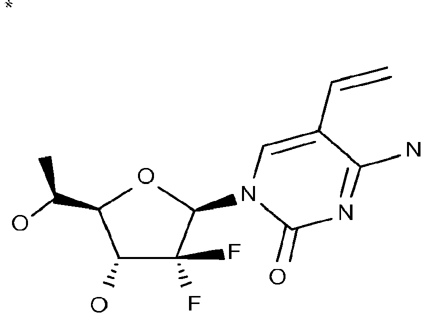 |
| 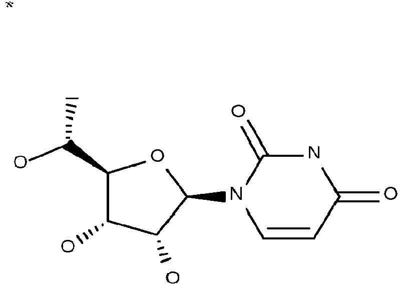 | 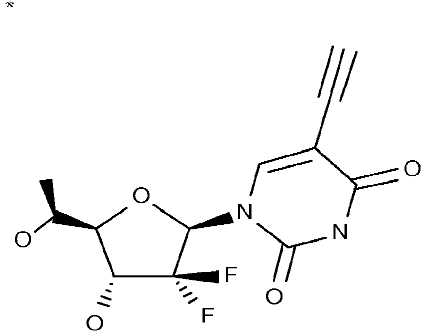 |
| 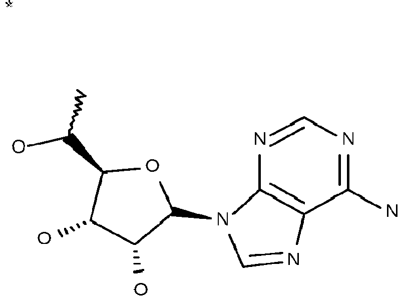 | 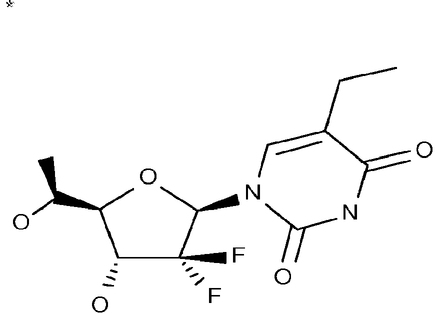 |
| 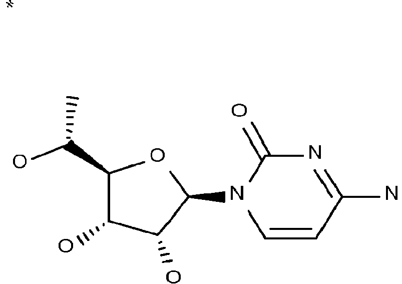 | 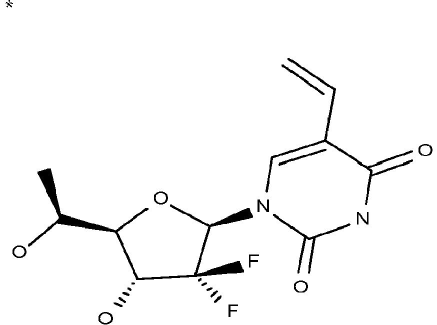 |
| 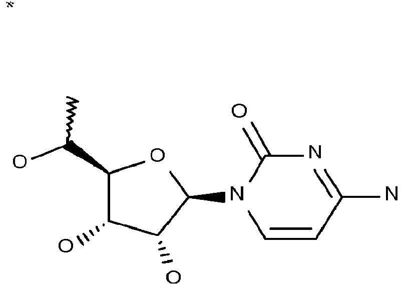 | 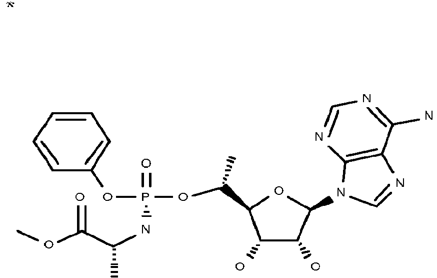 |
| 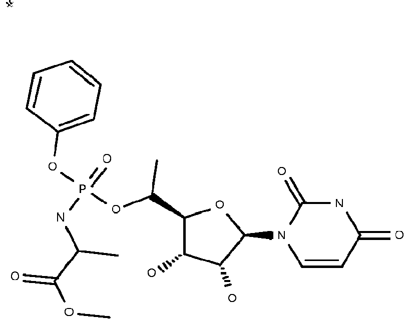 | 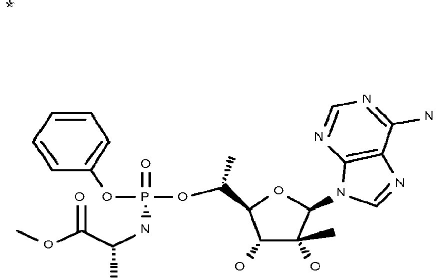 |
| 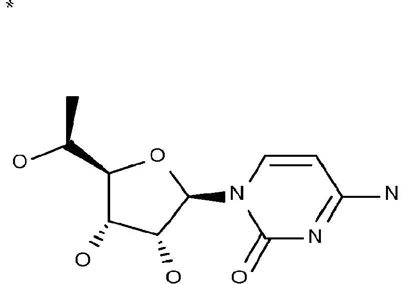 | 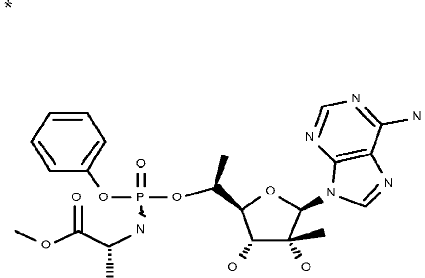 |
| 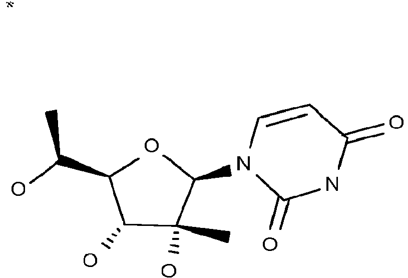 | 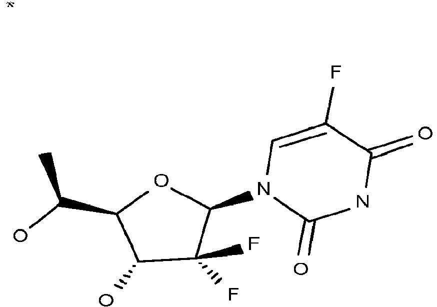 |
| 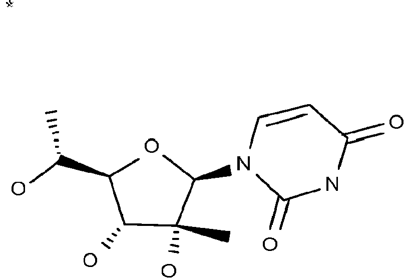 | 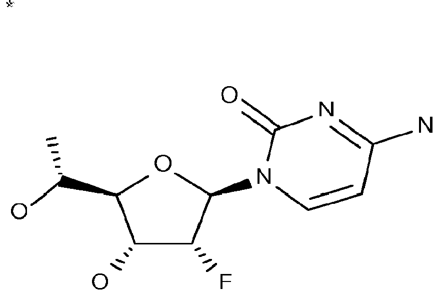 |
| 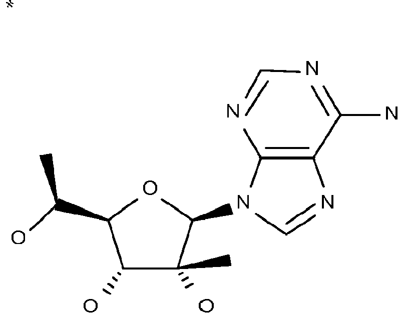 | 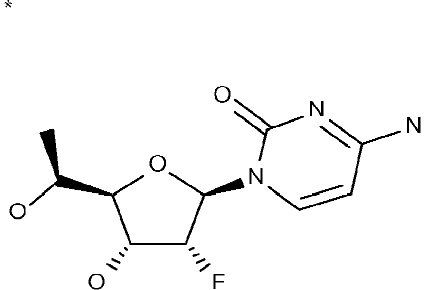 |
| 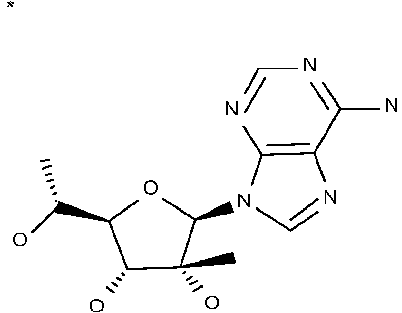 | 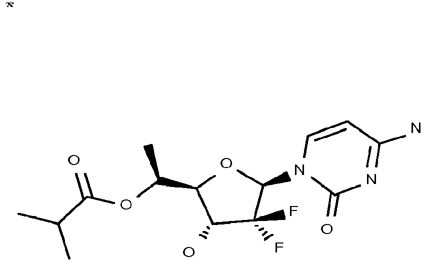 |
| 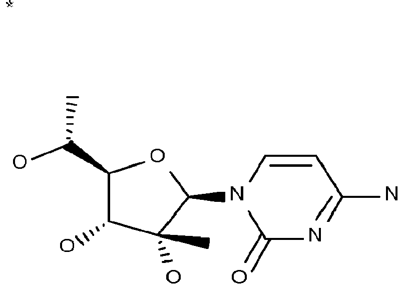 | 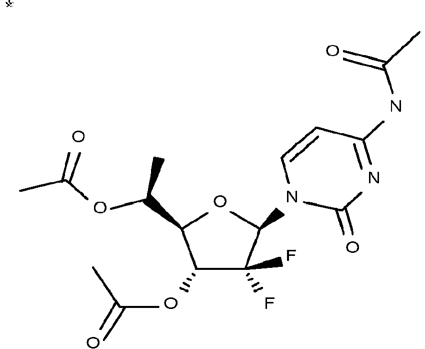 |
| 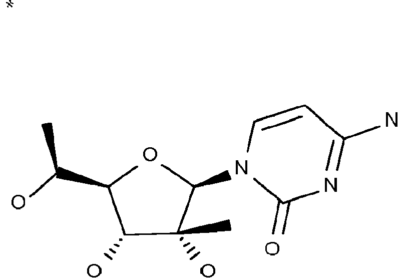 | 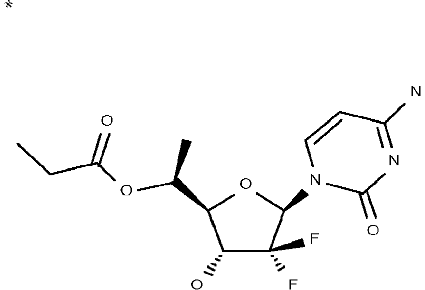 |
| 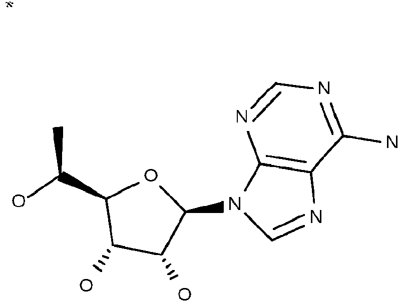 | 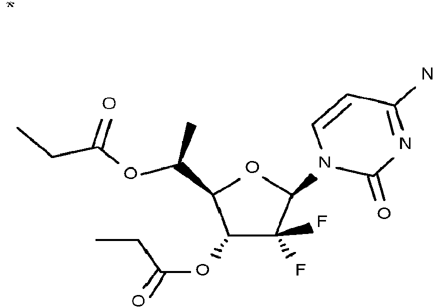 |
| 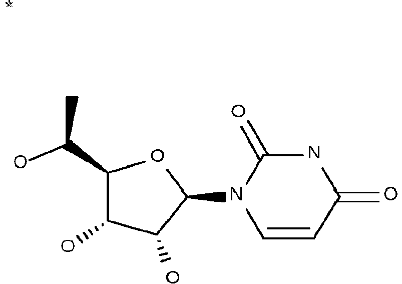 | 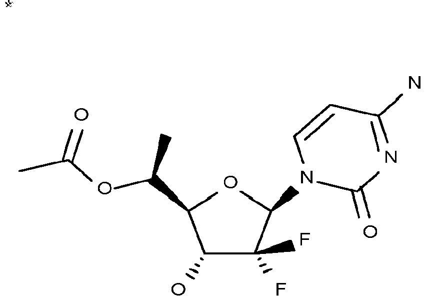 |
| 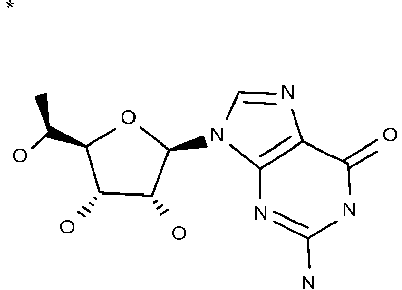 | 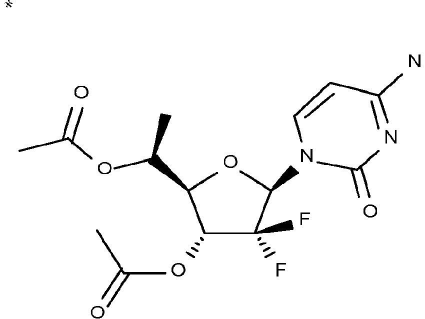 |
| 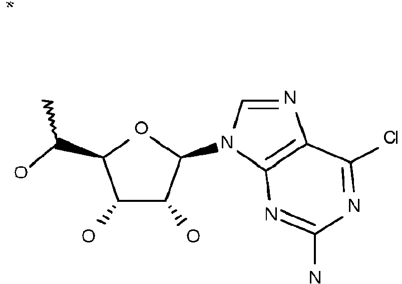 | 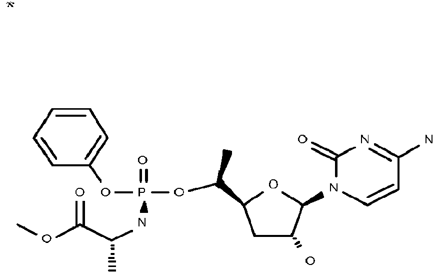 |
| 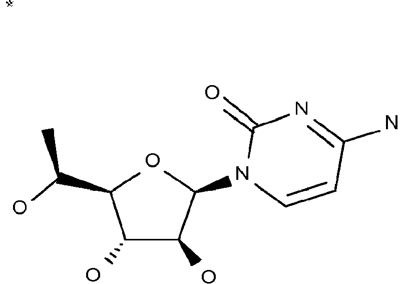 | 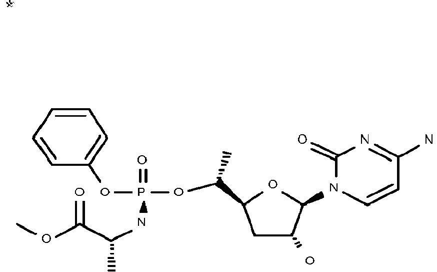 |
| 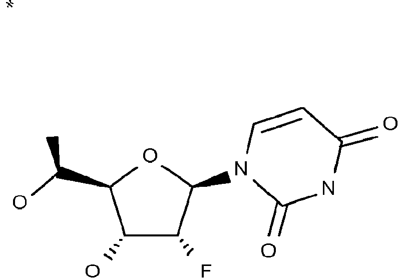 | 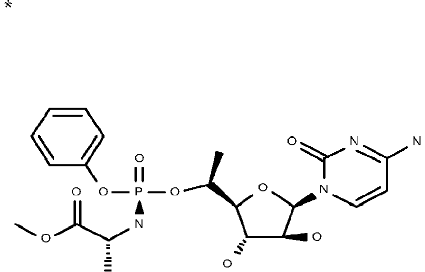 |
| 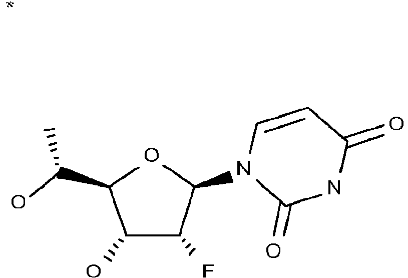 | 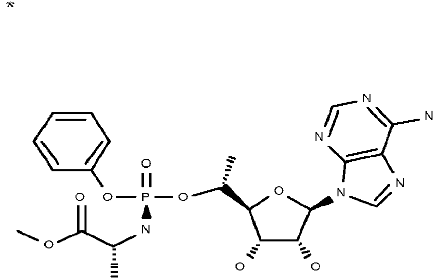 |
| 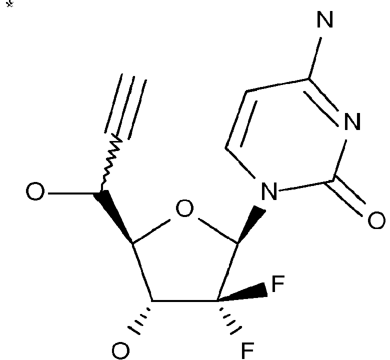 | 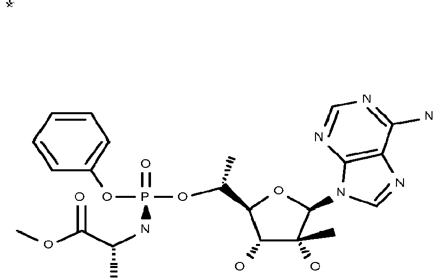 |
| 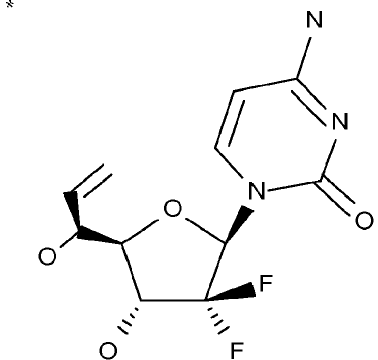 | 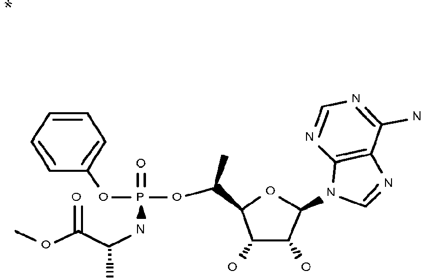 |
| 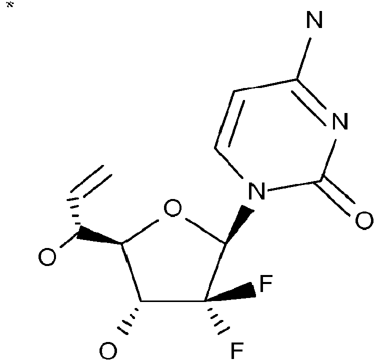 | 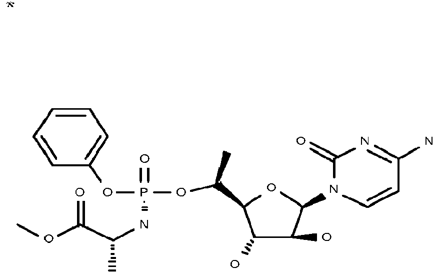 |
| 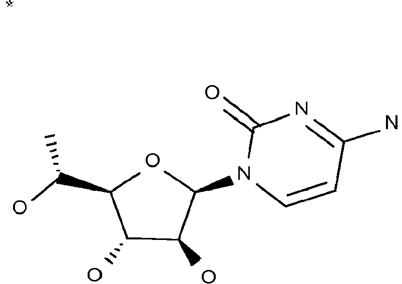 | 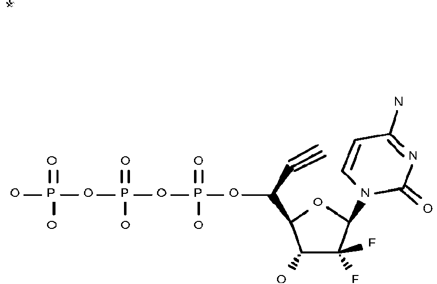 |
| 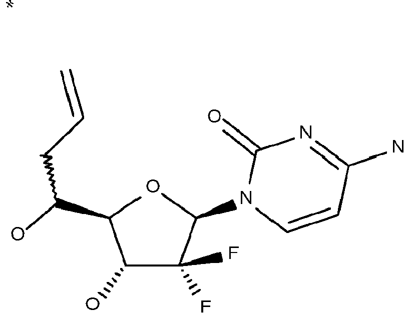 | 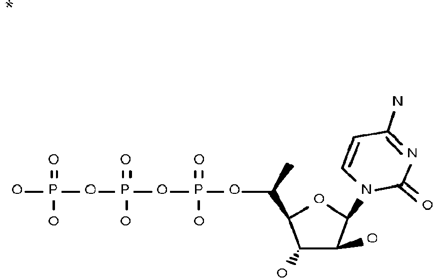 |
| 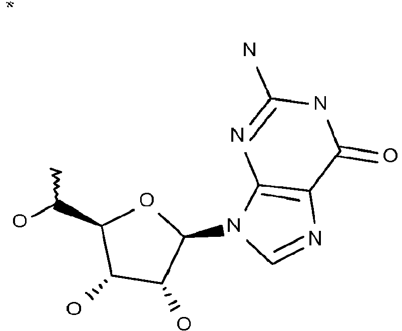 | 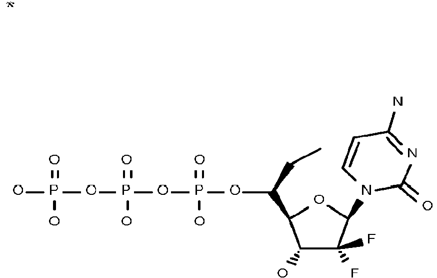 |
| 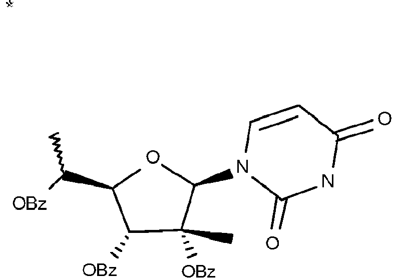 | 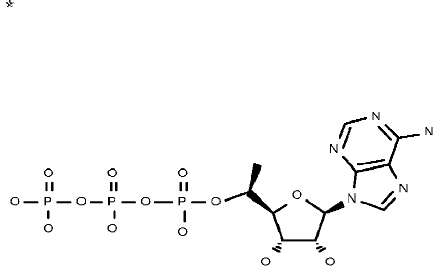 |
| 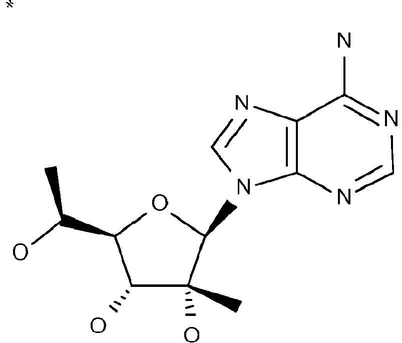 | 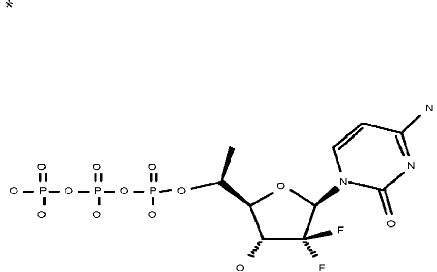 |
| 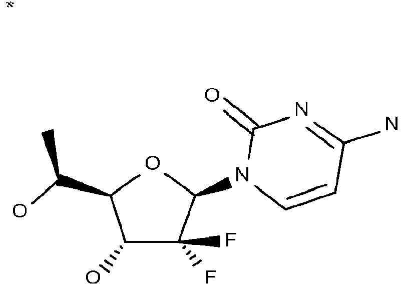 | 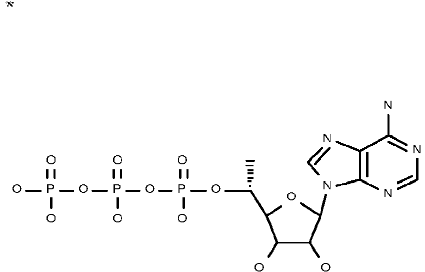 |
| 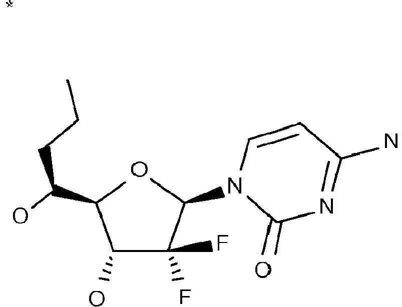 | 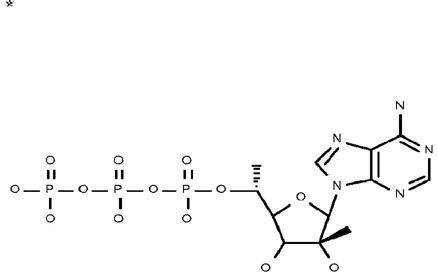 |
| 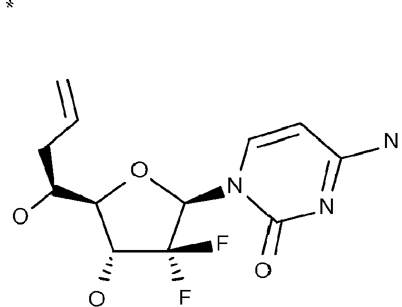 | 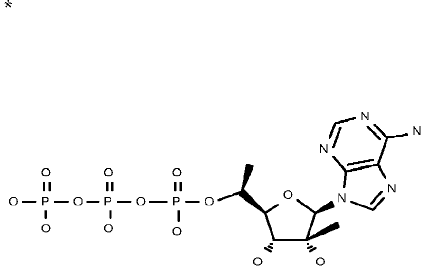 |
| 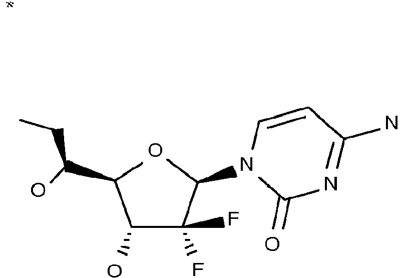 | 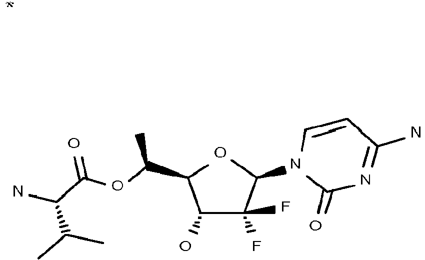 |

### Example 71

### HCV Replicon Assay

[0225] Antiviral activity of test compounds was assessed (Okuse, et al., *Antivir. Res.* **2005** 65:23) in the stably HCV RNA-replicating cell line, AVA5 (genotype 1b, subgenomic replicon, Blight, et al., *Sci.* **2000** 290:1972). Compounds were added to dividing cultures daily for three days. Cultures generally start the assay at 30-50% confluence and reach confluence during the last day of treatment. Intracellular HCV RNA levels and cytotoxicity were assessed 72 hours after treatment.

[0226] Quadruplicate cultures for HCV RNA levels and cytoxicity (on 96-well plates) were used. A total of 12 untreated control cultures, and triplicate cultures treated with α-interferon (concentrations of: 10 IU/mL, 3.3 IU/mL, 1.1 IU/mL and 0.37 IU/mL) and 2'C-Me-C (concentrations of: 30 µM, 10 µM, 3.3 µM and 1.1 µM) served as assay controls.

[0227] Intracellular HCV RNA levels were measured using a conventional blot hybridization method, in which HCV RNA levels are normalized to the levels of β-actin RNA in each individual culture (Okuse, et al., *Antivir. Res.* **2005** 65:23). Cytotoxicity was measured using an established neutral red dye uptake assay (Korba and Gerin, *Antivir. Res.* **1992** 19:55; Okuse, et al., *Antivir. Res.* **2005** 65:23). HCV RNA levels in the treated cultures are expressed as a percentage of the mean levels of RNA detected in untreated cultures. The absorbance of the internalized dye at 510 nM (A₅₁₀) was used for quantitative analysis.

[0228] Compounds were dissolved in 100% tissue culture grade DMSO (Sigma, Inc.) at 10 mM. Aliquots of test compounds sufficient for one daily treatment were made in individual tubes and all material was stored at -20°C. For the test, the compounds were suspended into culture medium at room temperature, and immediately added to the cell cultures. Compounds were analyzed separately in two groups with separate assay controls. The concentrations of the test compounds were run at concentrations of 10 µM, 3.3 µM, 1.1 µM and 0.37 µM. CC ₅₀, EC₅₀ and EC90 were determined using the concentration response curve.

[0229] The results demonstrate that compounds 8a and 9 are active and have an EC₅₀ (µM) between 1.0 and 10. The antiviral activity of additional exemplary compounds is shown in Table 2, wherein 'A' represents an EC₅₀ of less than 5 µM, 'B' represents an EC₅₀ of less than 30 µM, and 'C' represents an EC₅₀ of less than 200 µM.

**Table 2: Activity of Exemplary Compounds (C < 200 µM, B < 30 µM, A < 5 µM)**

| **Structure** | **Activity** |
|---|---|
| 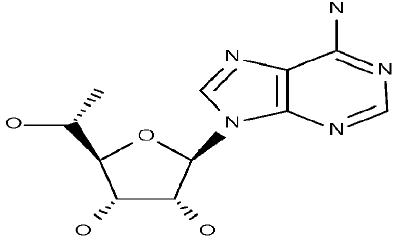 | B |
| 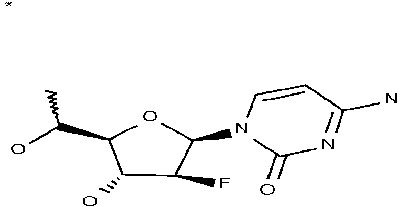 | B |
| 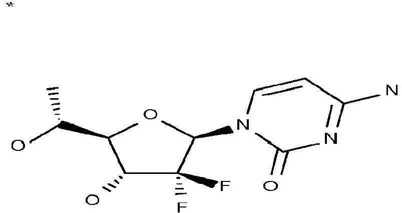 | A |
| 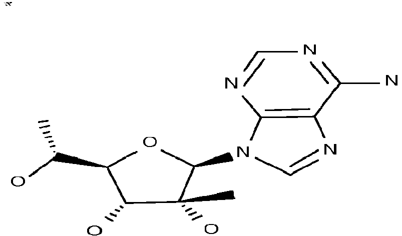 | B |
| 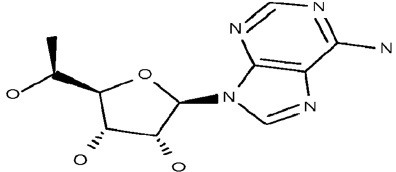 | C |
| 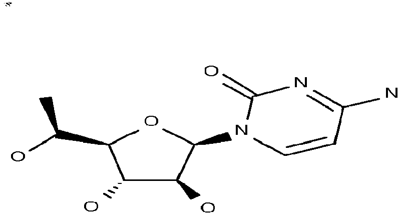 | C |
| 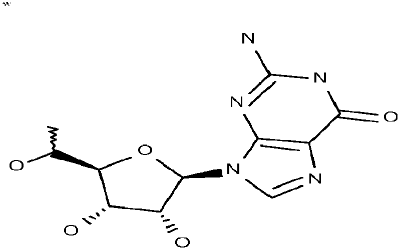 | C |
| 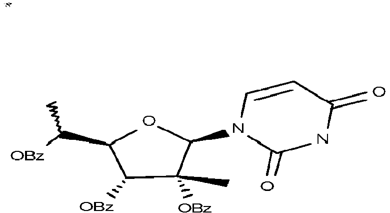 | B |
| 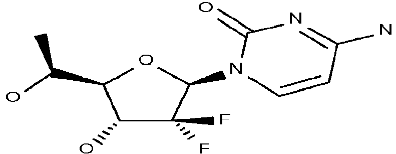 | A |
| 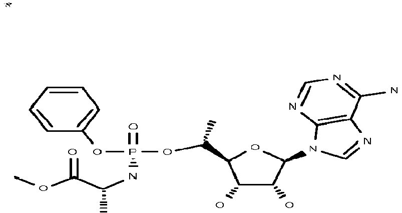 | A |
| 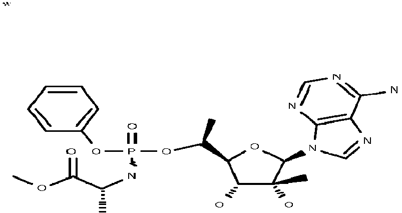 | C |
| 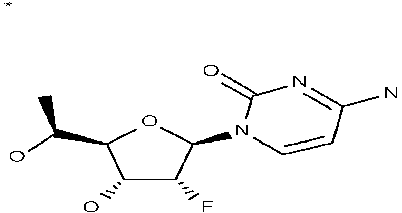 | B |
| 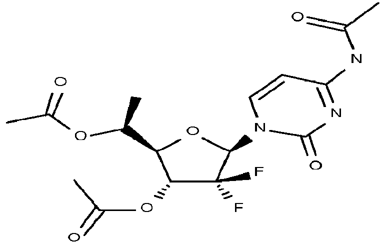 | A |
| 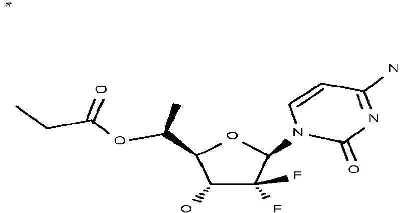 | A |
| 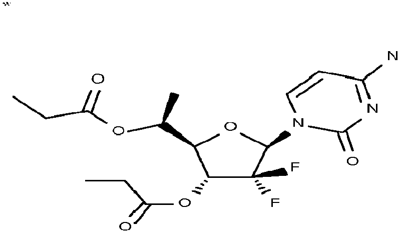 | A |
| 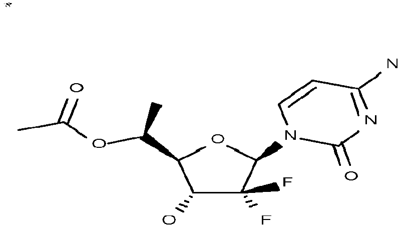 | A |
| 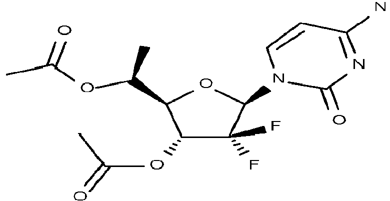 | A |
| 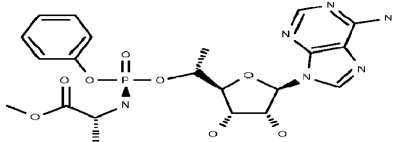 | A |
| 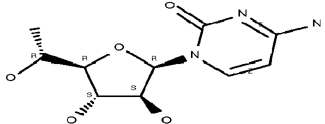 | A |

### Example 72

### STABILITY STUDIES

[0230] **Preparation of the cell extract.** 10 x 10⁶ of human prostate carcinoma cells (PC3) are treated with 10 mL of RIPA-buffer [15 mM Tris-HCl pH 7.5, 120 mM NaCl, 25 mM KCl, 2 mM EDTA, 2 mM EGTA, 0,1% Deoxycholic acid, 0,5% Triton X-100, 0,5% PMSF supplemented with Complete Protease Inhibitor Cocktail (Roche Diagnostics GmBH, Germany)] at 0 °C for 10 min. Most of the cells are disrupted by this hypotonic treatment and the remaining ones are disrupted mechanically. The cell extract obtained is centrifuged (900 rpm, 10 min) and the pellet is discarded. The extract is stored at -20 °C.

[0231] **Stability of nucleotides and nucleotides analogs in the cell extract.** The cell extract is prepared as described above (1 mL), and is diluted with a 9-fold volume of HEPES buffer (0.02 mol L⁻¹, pH 7.5, *I* = 0.1 mol L⁻¹ with NaCl). A nucleoside analog or a nucleotide analog (0.1 mg) is added into 3 mL of this HEPES buffered cell extract and the mixture is kept at 22 ± 1 °C. Aliquots of 150 µL are withdrawn at appropriate intervals, filtered with SPARTAN 13A (0.2 µm) and cooled in an ice bath. The aliquots are analyzed immediately by HPLC-ESI mass spectroscopy (Hypersil RP 18, 4.6 x 20 cm, 5µm). For the first 10 min, 0.1% aq. formic acid containing 4% MeCN is used for elution and then the MeCN content is increased to 50% by a linear gradient during 40 min.

[0232] **Stability of nucleoside and nucleotide analogs towards Porcine Liver Esterase.** A nucleoside analog or a nucleotide analog (1 mg) and 3mg (48 units) of Sigma Porcine Liver Esterase (66H7075) are dissolved in 3 mL of HEPES buffer (0.02 mol L⁻¹, pH 7.5, *I* = 0.1 mol L⁻¹ with NaCl). The stability test is carried out as described above for the cell extract

[0233] **Stability tests in human serum.** Stability tests in human serum are carried out as described for the whole cell extract. The measurements are carried out in serum diluted 1:1 with HEPES buffer (0.02 mol L⁻¹, pH 7.5, *I* = 0.1 mol L⁻¹ with NaCl).

[0234] It will be understood by those of skill in the art that numerous and various modifications can be made without departing from the spirit of the present disclosure. Therefore, it should be clearly understood that the forms disclosed herein are illustrative only and are not intended to limit the scope of the present disclosure.

### FEATURES OF THE INVENTION

1. A compound of Formula (II) or a pharmaceutically acceptable salt or a prodrug thereof: 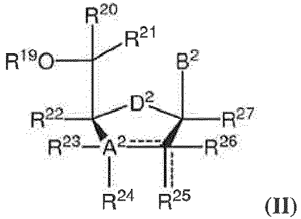 wherein:
   each 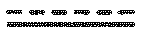 is independently a double or single bond;
   A² is selected from the group consisting of C (carbon), O (oxygen) and S (sulfur);
   B² is an optionally substituted heterocyclic base or a derivative thereof;
   D² is selected from the group consisting of C=CH₂, CH₂, O (oxygen), S (sulfur), CHF, and CF₂;
   R¹⁹ is selected from the group consisting of hydrogen, an optionally substituted alkyl, an optionally substituted cycloalkyl, an optionally substituted aralkyl, dialkylaminoalkylene, alkyl-C(=O)-, aryl-C(=O)-, alkoxyalkyl-C(=O)-, aryloxyalkyl-C(=O)-, alkylsulfonyl, arylsulfonyl,
      aralkylsulfonyl, 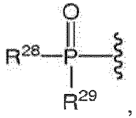 an -O-linked amino acid, diphosphate, triphosphate or derivatives thereof;
   R²⁰ and R²¹ are independently selected from the group consisting of hydrogen, an optionally substituted C₁₋₆ alkyl, an optionally substituted C₂₋₆ alkenyl, an optionally substituted C₂₋₆ alkynyl and an optionally substituted C₁₋₆ haloalkyl, provided that at least one of R²⁰ and R²¹ is not hydrogen; or R²⁰ and R²¹ are taken together to form a group selected from among C₃₋₆ cycloalkyl, C₃₋₆ cycloalkenyl, C₃₋₆ aryl, and a C₃₋₆ heteroaryl;
   R²² and R²⁷ is independently selected from the group consisting of hydrogen, halogen, -NH₂, -NHR^{a2}, NR^{a2}R^{b2}, -OR^{a2}, -SR^{a2}, -CN, -NC, -N₃, -NO₂, - N(R^{c2})-NR^{a2}R^{b2}, -N(R^{c2})-OR^{a2}, -S-SR^{a2}, -C(=O)R^{a2}, -C(=O)OR^{a2}, - C(=O)NR^{a2}R^{b2}, -O-C(=O)OR^{a2}, -O-C(=O)NR^{a2}R^{b2}, -N(R^{c2})-C(=O)NR^{a2}R^{b2},-S(=O)R^{a2}, S(=O)₂R^{a2}, -O-S(=O)₂NR^{a2}R^{b2}, -N(R^{c2})-S(=O)₂NR^{a2}R^{b2}, an optionally substituted C₁₋₆ alkyl, an optionally substituted C₂₋₆ alkenyl, an optionally substituted C₂₋₆ alkynyl and an -O-linked amino acid;
   R²³, R²⁴ and R²⁵ are independently absent or selected from the group consisting of hydrogen, halogen, -NH₂, -NHR^{a2}, NR^{a2}R^{b2}, -OR^{a2}, -SR^{a2}, -CN, -NC, -N₃, -NO₂, -N(R^{c2})-NR^{a2}R^{b2}, -N(R^{c2})-OR^{a2}, -S-SR^{a2}, -C(=O)R^{a2}, -C(=O)OR^{a2}, - C(=O)NR^{a2}R^{b2}, -O-(C=O)R^{a2}, -O-C(=O)OR^{a2}, -O-C(=O)NR^{a2}R^{b2}, -N(R^{c2})-C(=O)NR^{a2}R^{b2}, -S(=O)R^{a2}, S(=O)₂R^{a2}, -O-S(=O)₂NR^{a2}R^{b2}, -N(R^{c2})-S(=O)₂NR^{a2}R^{b2}, an optionally substituted C₁₋₆ alkyl, an optionally substituted C₂₋₆ alkenyl, an optionally substituted C₂₋₆ alkynyl, an optionally substituted aralkyl and an -O-linked amino acid; or R²⁴ and R²⁵ taken together form -O-C(=O)-O-;
   R²⁶ is absent or selected from the group consisting of hydrogen, halogen, - NH₂, -NHR^{a2}, NR^{a2}R^{b2}, -OR^{a2}, -SR^{a2}, -CN, -NC, -N₃, -NO₂, -N(R^{c2})-NR^{a2}R^{b2}, - N(R^{c2})-OR^{a2}, -S-SR^{a2}, -C(=O)R^{a2}, -C(=O)OR^{a2}, -C(=O)NR^{a2}R^{b2}, -O-C(=O)OR^{a2}, - O-C(=O)NR^{a2}R^{b2}, -N(R^{c2})-C(=O)NR^{a2}R^{b2}, -S(=O)R^{a2}, S(=O)₂R^{a2}, -O-S(=O)₂NR^{a2}R^{b2}, -N(R^{c2})-S(=O)₂NR^{a2}R^{b2}, an optionally substituted C₁₋₆ alkyl, an optionally substituted C₂₋₆ alkenyl, an optionally substituted C₂₋₆ alkynyl, an optionally substituted haloalkyl, an optionally substituted hydroxyalkyl and an-O-linked amino acid, or when the bond to R²⁵ indicated by 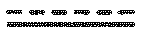 is a double bond, then R²⁵ is a C₂₋₆ alkylidene and R²⁶ is absent;
   R^{a2}, R^{b2} and R^{c2} are each independently selected from the group consisting of hydrogen, an optionally substituted alkyl, an optionally substituted alkenyl, an optionally substituted alkynyl, an optionally substituted aryl, an optionally substituted heteroaryl, an optionally substituted aralkyl and an optionally substituted heteroaryl(C₁₋₆ alkyl);
   R²⁸ is selected from the group consisting of O⁻, -OH, an optionally substituted aryloxy or aryl-O-, 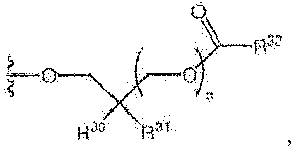 alkyl-C(=O)-O-CH₂-O-, alkyl-C(=O)-S-CH₂CH₂-O- and an -N-linked amino acid;
   R²⁹ is selected from the group consisting of O⁻, -OH, aryloxy or aryl-O-, 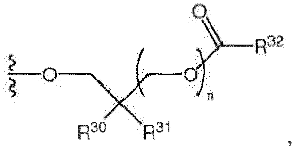 alkyl-C(=O)-O-CH₂-O-, alkyl-C(=O)-S-CH₂CH₂-O- and an -N-linked amino acid; each R³⁰ and each R³¹ are independently -C≡N or an optionally substituted substituent selected from the group consisting of C₁₋₈ organylcarbonyl, C₁₋₈ alkoxycarbonyl and C₁₋₈ organylaminocarbonyl; each R³² is hydrogen or an optionally substituted C₁₋₆-alkyl; each n is independently 1 or 2; and if both R²⁸ is and R²⁹ are 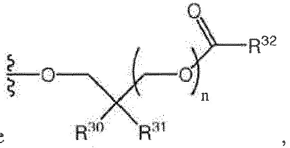 each R³⁰, each R³¹, each R³² and each n can be the same or different.
2. The compound of feature 1, wherein A² is C (carbon), D² is O (oxygen),
   and
   both bonds indicated by 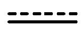 are single bonds.
3. The compound of any one of features 1 to 2, wherein R²² is selected from the group consisting of hydrogen, halogen, -OR^{a2}, -CN, -N₃, and an optionally substituted C₁₋₆,alkyl;
   R²³ is absent or selected from the group consisting of hydrogen, halogen, -OR^{a2} and an optionally substituted C₁₋₆ alkyl;
   R²⁴ is absent or selected from the group consisting of hydrogen, halogen, -NH₂, - OR^{a2}, -N₃, an optionally substituted C₁₋₆alkyl and an -O-linked amino acid;
   R²⁵ is selected from the group consisting of hydrogen, halogen, -OR^{a2}, -CN, -NC, an optionally substituted C₁₋₆ alkyl and an -O-linked amino acid; and
   R²⁶ is selected from the group consisting of hydrogen, halogen, an optionally substituted C₁₋₆ alkyl, an optionally substituted haloalkyl, an optionally substituted hydroxyalkyl.
4. The compound of any one of features 1 to 3, wherein at least one of R²⁵ and R²⁶ is halogen.
5. The compound of any one of features 1 to 3, wherein both R²⁵ and R²⁶ are halogen.
6. The compound of any one of features 1 to 5, wherein R²⁷ is selected from the group consisting of hydrogen, halogen, and an optionally substituted C₁₋₆ alkyl.
7. The compound of any one of features 1 to 6, wherein R¹⁹ is selected from the group consisting of hydrogen, a monophosphate, a diphosphate, and a triphosphate.
8. The compound of any one of features 1 to 6, wherein R¹⁹ is 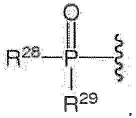
9. The compound of feature 8, wherein at least one of R²⁸ and R²⁹
   is 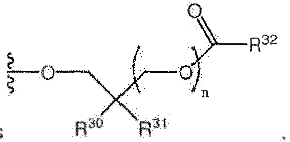 10. The compound of feature 9, wherein R³⁰ is -C≡N, and R³¹ is an optionally substituted C₁₋₈ alkoxycarbonyl or an optionally substituted C₁₋₈ organylaminocarbonyl.
11. The compound of feature 9, wherein both R³⁰ and R³¹ are an optionally substituted C₁₋₈ organylcarbonyl or an optionally substituted C₁₋₈ alkoxycarbonyl.
12. The compound of feature wherein n is 2, both R³⁰ and R³¹ are an optionally substituted C₁₋₈ alkoxycarbonyl, and R³² is an optionally substituted C₁₋₆-alkyl,
13. The compound of any one of features 9 to 12, wherein 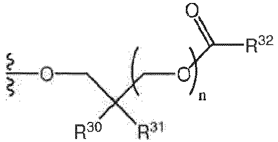 is selected from the group consisting of: 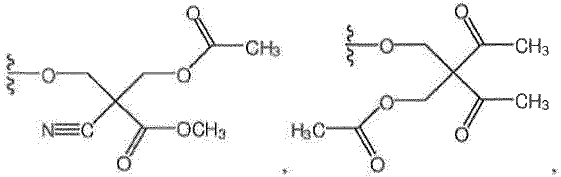 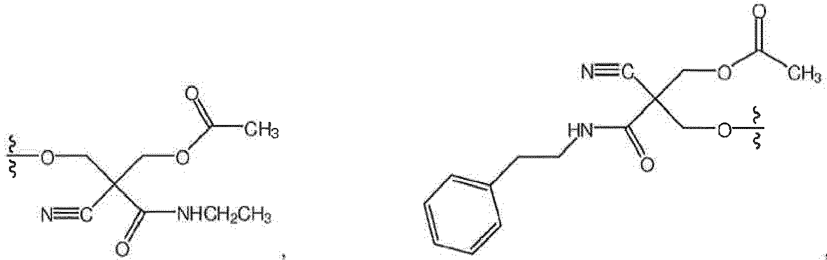 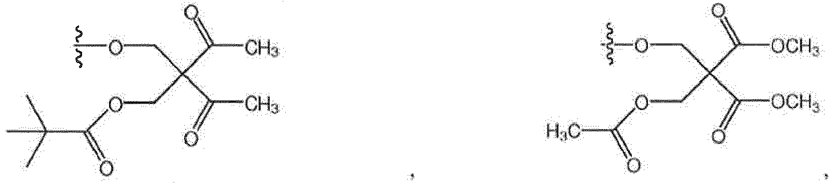 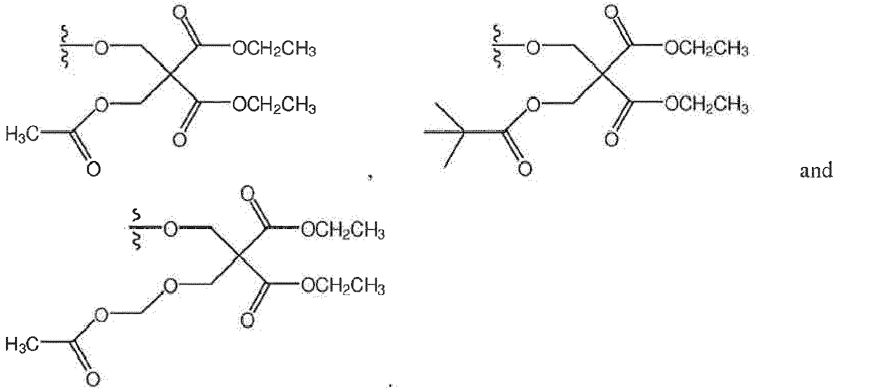 and
14. The compound of any one of features 8 to 13, wherein at least one of R²⁸
   and R²⁹ is 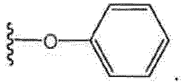 15. The compound of any one of features 8 to 13, wherein at least one of R²⁸ and R²⁹ is an -N-linked amino acid.
16. The compound of feature 15, wherein the -N-linked amino acid has the structure: 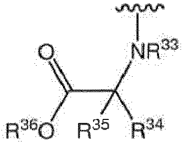 R³³ is hydrogen or an optionally substituted C₁₋₄-alkyl;
   R³⁴ is selected from the group consisting of hydrogen, an optionally substituted C₁₋₆-alkyl, an optionally substituted aryl, an optionally substituted aryl(C₁₋₆ alkyl) and an optionally substituted haloalkyl;
   R³⁵ is hydrogen or an optionally substituted C₁₋₆-alkyl; and
   R³⁶ is selected from the group consisting of an optionally substituted C₁₋₆ alkyl, an optionally substituted C₆ aryl, an optionally substituted C₁₀ aryl, and an optionally substituted C₃₋₆ cycloalkyl,
17. The compound of feature 16, wherein R³³ is hydrogen, and R³⁶ is an optionally substituted C₁₋₆ alkyl.
18. The compound feature 16, wherein at least one of R²⁸ and R²⁹ is: 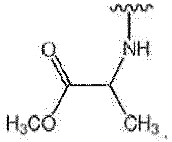
19. The compound of feature 8, wherein both R²⁸ and R²⁹ are
   both 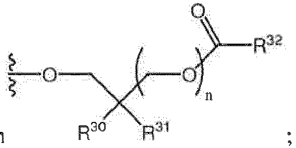 and wherein each R³⁰, each R³¹, each R³² and each n can be the same or different.
20. The compound of feature 8, wherein when R²⁸ and R²⁹ are both O⁻.
21. The compound of any one of features 1 to 20, wherein at least one of R²⁴ and R²⁵ is -OR^{a2} or an -O-linked amino acid, and wherein R^{a2} is hydrogen.
22. The compound of feature 21, wherein the -O-linked amino acid is selected from the group consisting of alanine, asparagine, aspartate, cysteine, glutamate, glutamine, glycine, proline, serine, tyrosine, arginine, histidine, isoleucine, leucine, lysine, methionine, phenylalanine, threonine, tryptophan and valine.
23. The compound of feature 21, wherein the -O-linked amino acid is selected from the group consisting of -O-linked α-amino acid, -O-linked β-amino acid, -O-linked γ-amino acid and -O-linked δ-amino acid.
24. The compound of any one of features 1 to 23, wherein B² is selected from the group consisting of: 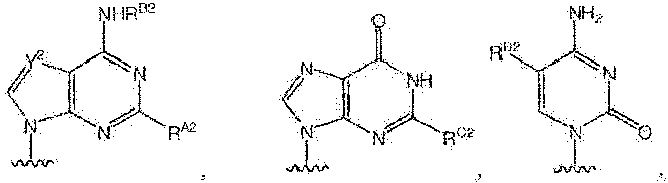 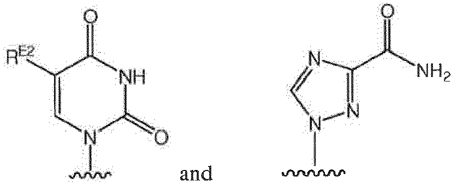 wherein:
   R^{A2} is hydrogen or halogen;
   R^{B2} is hydrogen, an optionally substituted C₁₋₆ alkyl, or an optionally substituted C₃₋₈ cycloalkyl;
   R^{C2} is hydrogen or amino;
   R^{D2} is selected from the group consisting of hydrogen, halogen, an optionally substituted C₁₋₆ alkyl, an optionally substituted C₂₋₆ alkenyl and an optionally substituted C₂₋₆ alkynyl;
   R^{E2} is selected from the group consisting of hydrogen, halogen, an optionally substituted C₁₋₆alkyl, an optionally substituted C₂₋₆ alkenyl and an optionally substituted C₂₋₆ alkynyl; and
   Y² is N or CR^{F2}, wherein R^{F2} can be selected from the group consisting of hydrogen, halogen, an optionally substituted C₁₋₆-alkyl, an optionally substituted C₂₋₆-alkenyl and an optionally substituted C₂₋₆-alkynyl.
25. The compound of any one of features 1 to 24, wherein R²⁰ is methyl or CF₃; and R²¹ is hydrogen,
26. The compound of feature 1, wherein the compound of Formula (II) is selected from the group consisting of: 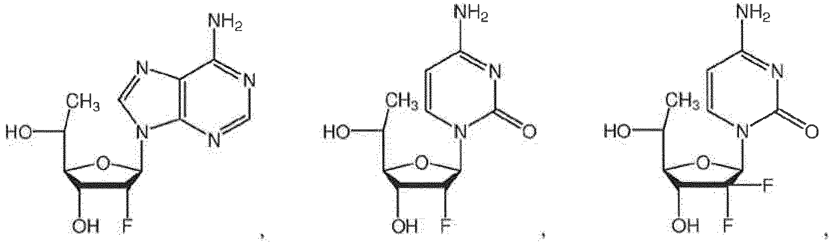 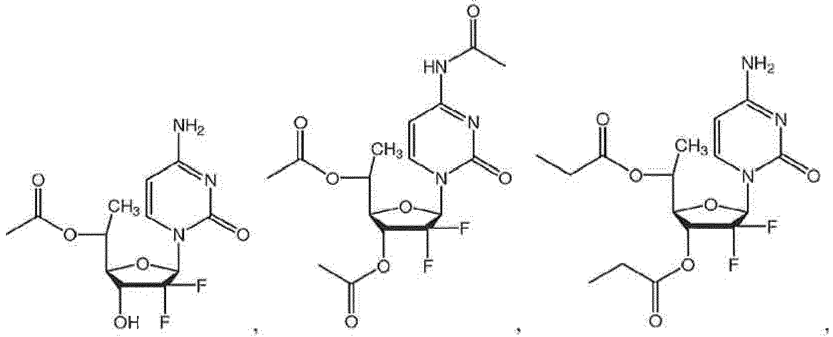 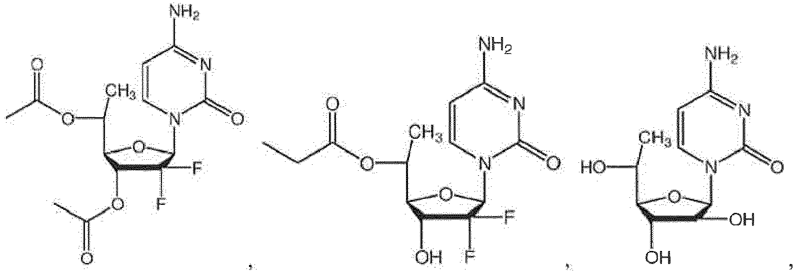 and 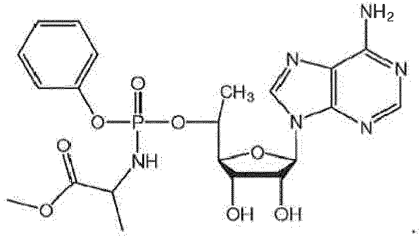
27. A compound of Formula (I) or a pharmaceutically acceptable salt or a prodrug thereof: 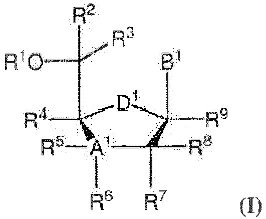 wherein:
   A¹ is selected from the group consisting of C (carbon), O (oxygen) and S (sulfur);
   B¹ is an optionally substituted heterocyclic base or a derivative thereof;
   D¹ is selected from the group consisting of C=CH₂, CH₂, O (oxygen), S (sulfur), CHF, and CF₂;
   R¹ is selected from the group consisting of hydrogen, an optionally substituted alkyl, an optionally substituted cycloalkyl, an optionally substituted aralkyl, dialkylaminoalkylene, alkyl-C(=O)-, aryl-C(=O)-, alkoxyalkyl-C(=O)-, aryloxyalkyl-C(=O)-, alkylsulfonyl, arylsulfonyl,
      aralkylsulfonyl, 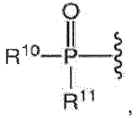 an -O-linked amino acid, diphosphate, triphosphate or derivatives thereof;
   R² and R³ are each independently selected from the group consisting of hydrogen, an optionally substituted C₁₋₆ alkyl, an optionally substituted C₂₋₆ alkenyl. an optionally substituted C₂₋₆ alkynyl and an optionally substituted C₁₋₆ haloalkyl, provided that at least one of R² and R³ is not hydrogen; or R² and R³ are taken together to form a group selected from among C₃₋₆ cycloalkyl, C₃₋₆ cycloalkenyl, C₃₋₆ aryl, and a C₃₋₆ heteroaryl;
   R⁴, R⁷ and R⁹ is independently selected from the group consisting of hydrogen, halogen, -NH₂, -NHR^{a1}, NR^{a1}R^{b1}, -OR^{a1}, -SR^{a1}, -CN, -NC, -N₃, -NO₂, - N(R^{c1})-NR^{a1}R^{b1}, -N(R^{c1})-OR^{a1}, -S-SR^{a1}, -C(=O)R^{a1}, -C(=O)OR^{a1}, - C(=O)NR^{a1}R^{b1}, -O-(C=O)R^{a1}, -O-C(=O)OR^{a1}, -O-C(=O)NR^{a1}R^{b1}, -N(R^{c1})-C(=O)NR^{a1}R^{b1}, -S(=O)R^{a1}, S(=O)₂R^{a1}, -O-S(=O)₂NR^{a1}R^{b1}, -N(R^{c1})-S(=O)₂NR^{a1}R^{b1}, an optionally substituted C₁₋₆ alkyl, an optionally substituted C₂₋₆ alkenyl, an optionally substituted C₂₋₆ alkynyl, an optionally substituted aralkyl and an -O-linked amino acid;
   R⁵ and R⁶ is independently absent or selected from the group consisting of hydrogen, halogen, -NH₂, -NHR^{a1}, NR^{a1}R^{b1}, -OR^{a1}, -SR^{a1}, -CN, -NC, -N₃, -NO₂, - N(R^{c1})-NR^{a1}R^{b1}, -N(R^{c1})-OR^{a1}, -S-SR^{a1}, -C(=O)R^{a1}, -C(=O)OR^{a1}, - C(=O)NR^{a1}R^{b1}, -O-C(=O)OR^{a1}, -O-C(=O)NR^{a1}R^{b1}, -N(R^{c1})-C(=O)NR^{a1}R^{b1},-S(=O)R^{a1}, S(=O)₂R^{a1}, -O-S(=O)₂NR^{a1}R^{b1}, -N(R^{c1})-S(=O)₂NR^{a1}R^{b1}, an optionally substituted C₁₋₆ alkyl, an optionally substituted C₂₋₆ alkenyl, an optionally substituted C₂₋₆ alkynyl and an -O-linked amino acid; or R⁶ and R⁷ taken together form -O-C(=O)-O-;
   R⁸ is halogen, -OR^{a1}, an optionally substituted C₁₋₆ alkyl, an optionally substituted C₂₋₆ alkenyl, an optionally substituted C₂₋₆ alkynyl and an optionally substituted C₁₋₆ haloalkyl;
   R^{a1}, R^{b1} and R^{c1} are each independently selected from the group consisting of hydrogen, an optionally substituted alkyl, an optionally substituted alkenyl, an optionally substituted alkynyl, an optionally substituted aryl, an optionally substituted heteroaryl, an optionally substituted aralkyl and an optionally substituted heteroaryl(C₁₋₆ alkyl);
   R¹⁰ is selected from the group consisting of O⁻, -OH, an optionally substituted aryloxy or aryl-O-, 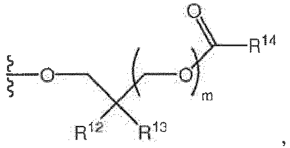 alkyl-C(=O)-O-CH₂-O-, alkyl-C(=O)-S-CH₂CH₂-O- and an -N-linked amino acid;
   R¹¹ is selected from the group consisting of O⁻, -OH, aryloxy or aryl-O- 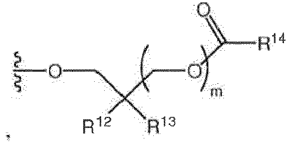 alkyl-C(=O)-O-CH₂-O-, alkyl-C(=O)-S-CH₂CH₂-O- and an -N-linked amino acid;
   each R¹² and each R13 are independently -C≡N or an optionally substituted substituent selected from the group consisting of C₁₋₈ organylcarbonyl, C₁₋₈ alkoxycarbonyl and C₁₋₈organylaminocarbonyl; -183-
   each R¹⁴ is hydrogen or an optionally substituted C₁₋₆-alkyl; and
   each m is independently 1 or 2;
   and if both R¹⁰ and R¹¹ are 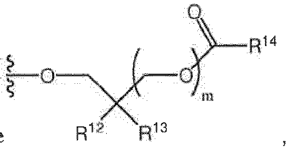 each R¹², each
   R¹³, each R¹⁴ and each m can be the same or different.
28. The compound of feature 27, wherein A¹ is C (carbon), and D¹ is O (oxygen).
29. The compound of any one of features 27 to 28, wherein R⁴ is selected from the group consisting of hydrogen, halogen, -OR^{a1}, -CN, -N₃, and an optionally substituted C₁₋₆ alkyl;
   R⁵ is absent or selected from the group consisting of hydrogen, halogen, -OR^{a1} and an optionally substituted C₁₋₆ alkyl;
   R⁶ is absent or selected from the group consisting of hydrogen, halogen, -NH₂, - OR^{a1}, -N₃, an optionally substituted C₁₋₆alky! and an -O-linked amino acid;
   R⁷ is selected from the group consisting of hydrogen, halogen, -OR^{a1}, -CN, -NC, an optionally substituted C₁₋₆alkyl and an -O-linked amino acid; and
   R⁹ is selected from the group consisting of hydrogen, halogen, and an optionally substituted C₁₋₆ alkyl.
30. The compound of any one of features 27 to 29, wherein R¹ is selected from the group consisting of hydrogen, a monophosphate, a diphosphate, and a triphosphate.
31. The compound of any one of features 27 to 29, wherein R¹ is 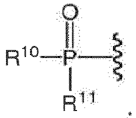
32. The compound of feature 31, wherein at least one of R¹⁰ and R¹¹ is 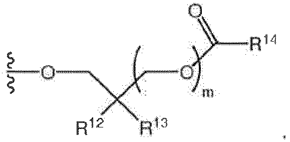
33. The compound of feature 32, wherein R¹² is -C≡N, and R¹³ is an optionally substituted C₁₋₈ alkoxycarbonyl or an optionally substituted C₁₋₈organylaminocarbonyl. -184-
34. The compound of feature 32, wherein both R¹² and R¹³ are an optionally substituted C₁₋₈ organylcarbonyl or an optionally substituted C₁₋₈ alkoxycarbonyl.
35. The compound of feature 32, wherein m is 2, both R¹² and R¹³ are an optionally substituted C₁₋₈ alkoxycarbonyl, and R¹⁴ is an optionally substituted C₁₋₆-alkyl.
36. The compound of any one of features 32 to 35, wherein 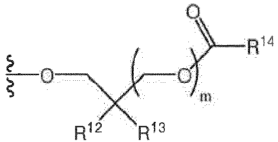 is selected from the group consisting of: 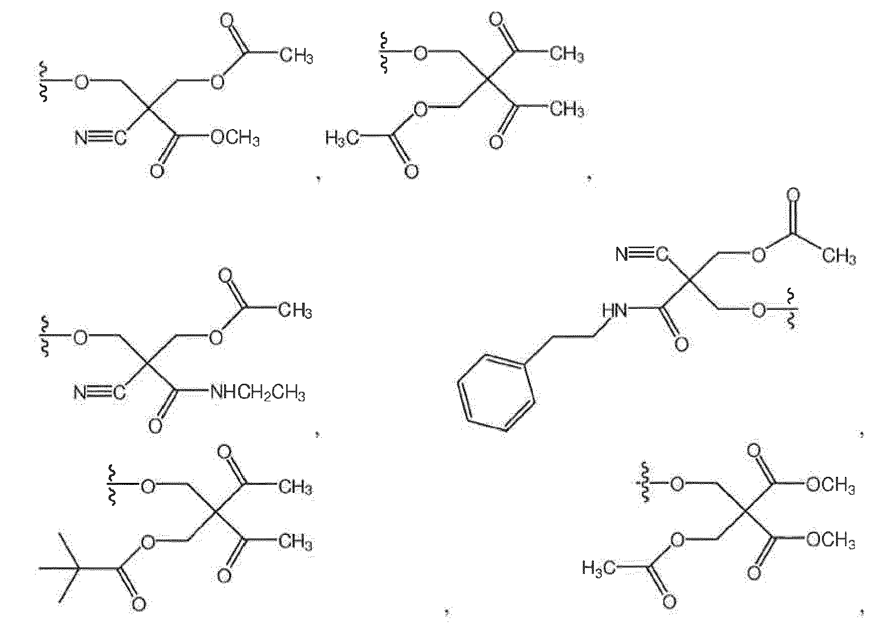 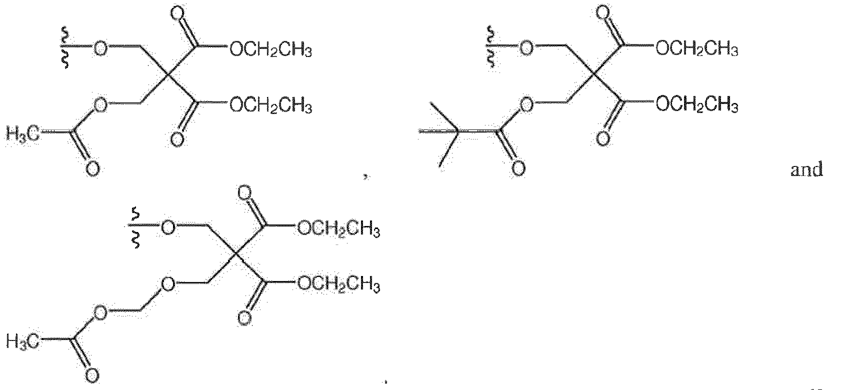
37. The compound of any one of features 31 to 36, wherein at least one of R¹⁰ and R¹¹ is 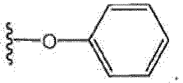
38. The compound of any one of features 27 to 37, wherein the -N-linked amino acid has the structure: 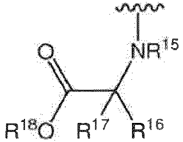 R¹⁵ is hydrogen or an optionally substituted C₁₋₄-alkyl;
   R¹⁶ is selected from the group consisting of hydrogen, an optionally substituted C₁₋₆-alkyl, an optionally substituted aryl, an optionally substituted aryl(C₁₋₆ alkyl) and an optionally substituted haloalkyl;
   R¹⁷ is hydrogen or an optionally substituted C₁₋₆-alkyl; and
   R¹⁸ is selected from the group consisting of an optionally substituted C₁₋₆ alkyl, an optionally substituted C₆ aryl, an optionally substituted C₁₀ aryl, and an optionally substituted C₃₋₆ cycloalkyl.
39. The compound of feature 38, wherein R¹⁵ is hydrogen, and R¹⁸ is an optionally substituted C₁₋₆ alkyl.
40. The compound of feature 38, at least one of R¹⁰ and R¹¹ is: 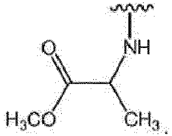
41. The compound of feature 31, wherein when R¹⁰ and R¹¹ are
   both 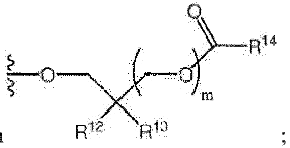 and wherein each R¹², each R¹³, each R¹⁴ and each m can be the same or different.
42. The compound of feature 31, R¹⁰ and R¹¹ are both O⁻.
43. The compound of any one of 27 to 42, wherein at least one of R⁶ and R⁷ is -OR^{a1} or an -O-linked amino acid, and wherein R^{a1} is hydrogen.
44. The compound of feature 43, wherein the -O-linked amino acid is selected from the group consisting of alanine, asparagine, aspartate, cysteine, glutamate, glutamine, glycine, proline, serine, tyrosine, arginine, histidine, isoleucine, leucine, lysine, methionine, phenylalanine, threonine, tryptophan and valine.
45. The compound of feature 43, wherein the -O-linked amino acid is selected from the group consisting of -O-linked α-amino acid, -O-linked β-amino acid, -O-linked γ-amino acid and -O-linked δ-amino acid.
46. The compound of any one of features 27 to 45, wherein B¹ is selected from the group consisting of: 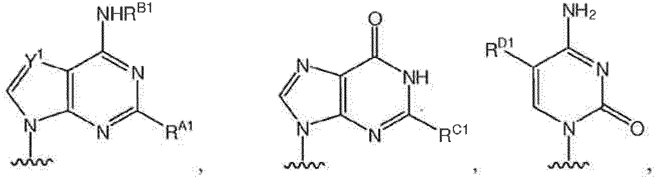 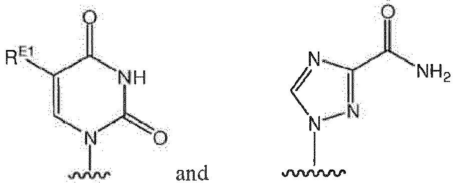 wherein:
   R^{A1} is hydrogen or halogen;
   R^{B1} is hydrogen, an optionally substituted C₁₋₆ alkyl, or an optionally substituted C₃₋₈ cycloalkyl;
   R^{C1} is hydrogen or amino;
   R^{D1} is selected from the group consisting of hydrogen, halogen, an optionally substituted C₁₋₆ alkyl, an optionally substituted C₂₋₆ alkenyl and an optionally substituted C₂₋₆ alkynyl;
   R^{E1} is selected from the group consisting of hydrogen, halogen, an optionally substituted C₁₋₆alkyl, an optionally substituted C₂₋₆ alkenyl and an optionally substituted C₂₋₆ alkynyl; and
   Y¹ is N or CR^{F1}, wherein R^{F1} can be selected from the group consisting of hydrogen, halogen, an optionally substituted C₁₋₆-alkyl, an optionally substituted C₂₋₆-alkenyl and an optionally substituted C₂₋₆-alkynyl.
47. The compound of any one of features 27 to 46, wherein R² is methyl or CF₃; and R³ is hydrogen.
48. The compound of any one of features 27 to 47, wherein R⁸ is methyl.
49. A pharmaceutical composition comprising a compound of any one of 1 to 48, and a pharmaceutically acceptable carrier, diluent, excipient or combination thereof.
50. A compound of any one of features 1 to 48 or a pharmaceutical composition of Claim 49 for use in treating a neoplastic disease, viral disease, or parasitic disease.
51. The compound or composition of features 50, wherein the use is treatment of cancer.
52. The compound or composition of feature 50, wherein the use is treatment of leukemia.
53. The compound or composition of feature 50, wherein the use is treating a viral infection.
54. The compound or composition of feature 53, wherein the viral infection is caused by a virus selected from the group consisting of an adenovirus, an Alphaviridae, an Arbovirus, an Astrovirus, a Bunyaviridae, a Coronaviridae, a Filoviridae, a Flaviviridae, a Hepadnaviridae, a Herpesviridae, an Alphaherpesvirinae, a Betaherpesvirinae, a Gammaherpesvirinae, a Norwalk Virus, an Astroviridae, a Caliciviridae, an Orthomyxoviridae, a Paramyxovindae, a Paramyxoviruses, a Rubulavirus, a Morbillivirus, a Papovaviridae, a Parvoviridae, a Picornaviridae, an Aphthoviridae, a Cardioviridae, an Enteroviridae, a Coxsackie virus, a Polio Virus, a Rhinoviridae, a Phycodnaviridae, a Poxviridae, a Reoviridae, a Rotavims, a Retroviridae, an A-Type Retrovirus, an Immunodeficiency Virus, a Leukemia Viruses, an Avian Sarcoma Viruses, a Rhabdoviruses, a Rubiviridae and a Togaviridae.
55. The compound or composition of feature 53, wherein the use is treatment of a hepatitis C viral infection or a HIV viral infection.
56. The compound or composition of feature 50, wherein the use is treating a parasitic disease.
57. The compound or composition of feature 56, wherein the use is treatment of Chagas' disease.

## Claims

1. A compound of Formula (II) or a pharmaceutically acceptable salt or a prodrug thereof: 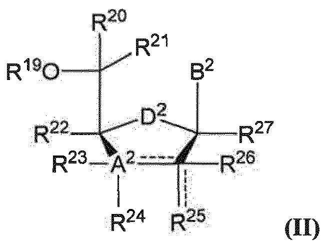 wherein:
each 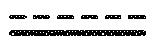 is independently a double or single bond;
A² is selected from the group consisting of C (carbon), O (oxygen) and S (sulfur);
B² is an optionally substituted heterocyclic base or a derivative thereof;
D² is selected from the group consisting of C=CH₂, CH₂, O (oxygen), S (sulfur), CHF, and CF₂;
R¹⁹ is selected from the group consisting of hydrogen, an optionally substituted alkyl, an optionally substituted cycloalkyl, an optionally substituted aralkyl, dialkylaminoalkylene, alkyl-C(=O)-, aryl-C(=O)-, alkoxyalkyl-C(=O)-, aryloxyalkyl-C(=O)-, alkylsulfonyl, arylsulfonyl,
aralkylsulfonyl, 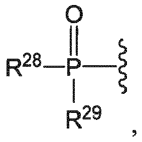 an -O-linked amino acid, diphosphate, triphosphate or derivatives thereof;
R²⁰ and R²¹ are independently selected from the group consisting of hydrogen, an optionally substituted C₁₋₆ alkyl, an optionally substituted C₂₋₆ alkenyl, an optionally substituted C₂₋₆ alkynyl and an optionally substituted C₁₋₆ haloalkyl, provided that at least one of R²⁰ and R²¹ is not hydrogen; or R²⁰ and R²¹ are taken together to form a group selected from among C₃₋₆ cycloalkyl, C₃₋₆ cycloalkenyl, C₃₋₆ aryl, and a C₃₋₆ heteroaryl;
R²² and R²⁷ is independently selected from the group consisting of hydrogen, halogen, -NH₂, -NHR^{a2}, NR^{a2}R^{b2}, -OR^{a2}, -SR^{a2}, -CN, -NC, -N₃, -NO₂, - N(R^{c2})-NR^{a2}R^{b2}, -N(R^{c2})-OR^{a2}, -S-SR^{a2}, -C(=O)R^{a2}, -C(=O)OR^{a2}, - C(=O)NR^{a2}R^{b2}, -O-C(=O)OR^{a2}, -O-C(=O)NR^{a2}R^{b2}, -N(R^{c2})-C(=O)NR^{a2}R^{b2}, - S(=O)R^{a2}, S(=O)₂R^{a2}, -O-S(=O)₂NR^{a2}R^{b2}, -N(R^{c2})-S(=O)₂NR^{a2}R^{b2}, an optionally substituted C₁₋₆ alkyl, an optionally substituted C₂₋₆ alkenyl, an optionally substituted C₂₋₆ alkynyl and an -O-linked amino acid;
R²³, R²⁴ and R²⁵ are independently absent or selected from the group consisting of hydrogen, halogen, -NH₂, -NHR^{a2}, NR^{a2}R^{b2}, -OR^{a2}, -SR^{a2}, -CN, - NC, -N₃, -NO₂, -N(R^{c2})-NR^{a2}R^{b2}, -N(R^{c2})-OR^{a2}, -S-SR^{a2}, -C(=O)R^{a2}, - C(=O)OR^{a2}, -C(=O)NR^{a2}R^{b2}, -O-(C=O)R^{a2}, -O-C(=O)OR^{a2}, -O-C(=O)NR^{a2}R^{b2}, - N(R^{c2})-C(=O)NR^{a2}R^{b2}, -S(=O)R^{a2}, S(=O)₂R^{a2}, -O-S(=O)₂NR^{a2}R^{b2}, -N(R^{c2})-S(=O)₂NR^{a2}R^{b2}, an optionally substituted C₁₋₆ alkyl, an optionally substituted C₂₋₆ alkenyl, an optionally substituted C₂₋₆ alkynyl, an optionally substituted aralkyl and an -O-linked amino acid; or R²⁴ and R²⁵ taken together form -O-C(=O)-O-;
R²⁶ is selected from the group consisting of halogen, -NH₂, -NHR^{a2}, NR^{a2}R^{b2}, -SR^{a2}, -CN, -NC, -N₃, -NO₂, -N(R^{c2})-NR^{a2}R^{b2}, -N(R^{c2})-OR^{a2}, -S-SR^{a2},-C(=O)R^{a2}, -C(=O)OR^{a2}, -C(=O)NR^{a2}R^{b2}, -O-C(=O)OR^{a2}, -O-C(=O)NR^{a2}R^{b2}, - N(R^{c2})-C(=O)NR^{a2}R^{b2}, -S(=O)R^{a2}, S(=O)₂R^{a2}, -O-S(=O)₂NR^{a2}R^{b2}, -N(R^{c2})-S(=O)₂NR^{a2}R^{b2}, an optionally substituted C₁₋₆ alkyl, an optionally substituted C₂₋₆ alkenyl, an optionally substituted C₂₋₆ alkynyl, an optionally substituted haloalkyl, an optionally substituted hydroxyalkyl and an -O-linked amino acid;
R^{a2}, R^{b2} and R^{c2} are each independently selected from the group consisting of hydrogen, an optionally substituted alkyl, an optionally substituted alkenyl, an optionally substituted alkynyl, an optionally substituted aryl, an optionally substituted heteroaryl, an optionally substituted aralkyl and an optionally substituted heteroaryl(C₁₋₆ alkyl);
R²⁸ is selected from the group consisting of O⁻, -OH, an optionally
substituted aryloxy or aryl-O-, 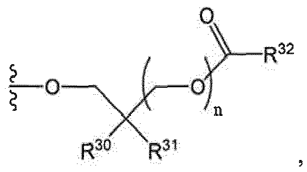 alkyl-C(=O)-O-CH₂-O-, alkyl-C(=O)-S-CH₂CH₂-O- and an -N-linked amino acid;
R²⁹ is selected from the group consisting of O⁻, -OH, aryloxy or aryl-O-, 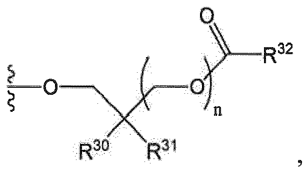 alkyl-C(=O)-O-CH₂-O-, alkyl-C(=O)-S-CH₂CH₂-O- and an -N-linked amino acid;
each R³⁰ and each R³¹ are independently -C≡N or an optionally substituted substituent selected from the group consisting of C₁₋₈ organylcarbonyl, C₁₋₈ alkoxycarbonyl and C₁₋₈ organylaminocarbonyl;
each R³² is hydrogen or an optionally substituted C₁₋₆-alkyl;
each n is independently 1 or 2; and
if both R²⁸ and R²⁹ are 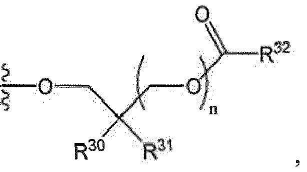 each R³⁰, each R³¹, each R³² and each n can be the same or different.

2. The compound of Claim 1, wherein A² is C (carbon), D² is O (oxygen), both bonds indicated by 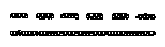 are single bonds, and B² is selected from the group consisting of: 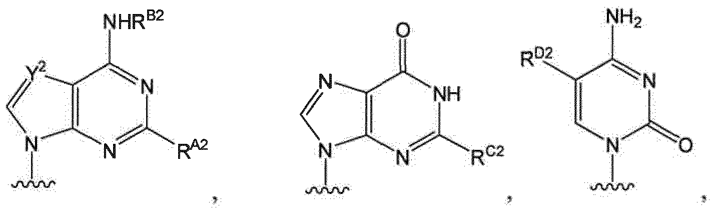 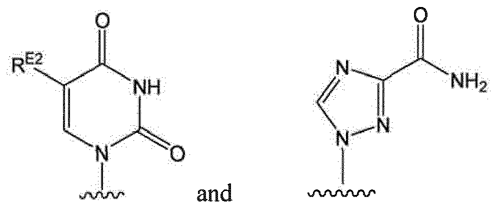 wherein:
R^{A2} is hydrogen or halogen;
R^{B2} is hydrogen, an optionally substituted C₁₋₆ alkyl, or an optionally substituted C₃₋₈ cycloalkyl;
R^{C2} is hydrogen or amino;
R^{D2} is selected from the group consisting of hydrogen, halogen, an optionally substituted C₁₋₆ alkyl, an optionally substituted C₂₋₆alkenyl and an optionally substituted C₂₋₆ alkynyl;
R^{E2} is selected from the group consisting of hydrogen, halogen, an optionally substituted C₁₋₆alkyl, an optionally substituted C₂₋₆alkenyl and an optionally substituted C₂₋₆alkynyl; and
Y² is N or CR^{F2}, wherein R^{F2} can be selected from the group consisting of hydrogen, halogen, an optionally substituted C₁₋₆-alkyl, an optionally substituted C₂₋₆-alkenyl and an optionally substituted C₂₋₆-alkynyl.

3. The compound of Claims 1 or 2, wherein B² is

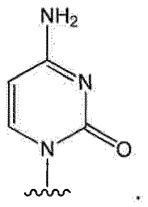

4. The compound of any one of Claims 1 to 3, wherein R²⁰ is methyl; and R²¹ is hydrogen.

5. The compound of any one of Claims 1 to 4, wherein:
R²² is selected from the group consisting of hydrogen, halogen, -OR^{a2}, - CN, -N₃, and an optionally substituted C₁₋₆ alkyl;
R²³ is absent or selected from the group consisting of hydrogen, halogen, - OR^{a2} and an optionally substituted C₁₋₆ alkyl;
R²⁴ is absent or selected from the group consisting of hydrogen, halogen, - NH₂, -OR^{a2}, -N₃, an optionally substituted C₁₋₆ alkyl and an -O-linked amino acid;
R²⁵ is selected from the group consisting of hydrogen, halogen, -OR^{a2}, - CN, -NC, an optionally substituted C₁₋₆ alkyl and an -O-linked amino acid; and
R²⁶ is selected from the group consisting of halogen, an optionally substituted C₁₋₆ alkyl, an optionally substituted haloalkyl, an optionally substituted hydroxyalkyl.

6. The compound of any one of Claims 1 to 5, wherein R²² is hydrogen.

7. The compound of any one of Claims 1 to 6, wherein R²³ is hydrogen.

8. The compound of any one of Claims 1 to 7, wherein R²⁷ is hydrogen.

9. The compound of any one of Claims 1 to 4 or 6 to 8, wherein R²⁴ is selected from the group consisting of -OR^{a2}, wherein R^{a2} is hydrogen; -O-C(=O)R^{a2} and an -O-linked amino acid.

10. The compound of any one of Claims 1 to 4 or 6 to 9, wherein R²⁵ is selected from the group consisting of halogen, -CN, -NC, -N₃, -NO₂, and an optionally substituted C₁₋₆ alkyl.

11. The compound of any one of Claims 1 to 10, wherein R²⁵ is halogen, preferably fluoro.

12. The compound of any one of Claims 1 to 11, wherein R²⁶ is selected from the group consisting of halogen, an optionally substituted C₁₋₆ alkyl, and an optionally substituted haloalkyl.

13. The compound of any one of Claims 1 to 12, wherein R²⁶ is halogen, preferably fluoro.

14. The compound of any one of Claims 1 to 13, wherein R¹⁹ is selected from the group consisting of alkyl-C(=O)-, aryl-(=O)-, alkoxyalkyl-C(=O)-, aryloxyalkyl-C=O)- and an -O-linked amino acid.

15. The compound of any one of Claims 1 to 13, wherein R¹⁹ is selected from the group consisting of a monophosphate, a diphosphate, and a triphosphate.

16. The compound of any one of Claims 1 to 13, wherein R¹⁹ is hydrogen.

17. The compound of any one of Claims 1-13, wherein R¹⁹ is 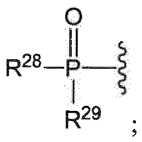 wherein R²⁸ and R²⁹ are each alkyl-C(=O)-O-CH₂-O-; or
wherein R²⁸ and R²⁹ are each alkyl-C(=O)-S-CH₂CH₂-O-; or
wherein R²⁸ is an optionally substituted aryloxy and R²⁹ is an -N-linked amino acid; or
wherein R²⁸ is an -N-linked amino acid and R²⁹ is an -N-linked amino acid; and
preferably, each -N-linked amino acid is independently selected from a methyl ester, an ethyl ester, a propyl ester, a benzyl ester and a tert-butyl ester of an amino acid selected from alanine, asparagine, aspartate, cysteine, glutamate, glutamine, glycine, proline, serine, tyrosine, arginine, histidine, isoleucine, leucine, lysine, methionine, phenylalanine, threonine, tryptophan and valine; or
preferably, each -N-linked amino acid independently has the following structure: 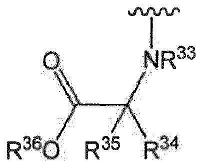 R³³ is hydrogen or an optionally substituted C₁₋₄-alkyl, preferably hydrogen;
R³⁴ is selected from the group consisting of hydrogen, an optionally substituted C₁₋₆-alkyl, an optionally substituted aryl, an optionally substituted aryl(C₁₋₆ alkyl) and an optionally substituted haloalkyl, preferably an optionally substituted C₁₋₆-alkyl;
R³⁵ is hydrogen or an optionally substituted C₁₋₆-alkyl, preferably hydrogen; and
R³⁶ is selected from the group consisting of an optionally substituted C₁₋₆ alkyl, an optionally substituted C₆ aryl, an optionally substituted C₁₀ aryl, and an optionally substituted C₃₋₆ cycloalkyl.

18. The compound of Claim 1, wherein the compound has the following structure: 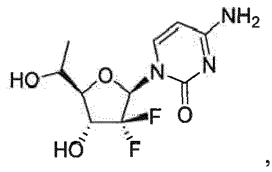 or a pharmaceutically acceptable salt thereof.

19. The compound of Claim 1, wherein the compound has the following structure: 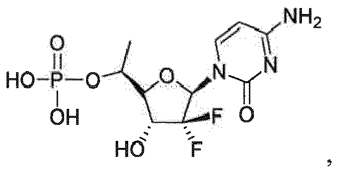 or a pharmaceutically acceptable salt thereof.

20. The compound of Claim 19, wherein the compound is the diphosphate, or a pharmaceutically acceptable salt thereof.

21. The compound of Claim 1, wherein the compound has the following structure: 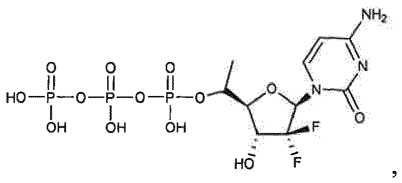 or a pharmaceutically acceptable salt thereof.

22. A pharmaceutical composition comprising a compound of any one of Claims 1 to 21, and a pharmaceutically acceptable carrier, diluent, excipient or combination thereof.

23. A compound of any one of Claims I to 21 or a pharmaceutical composition of Claim 22 for use in treating a neoplastic disease.

24. The compound or composition of Claim 23, wherein the use is treatment of cancer.

25. The compound of Claim 23 or 24, wherein the compound is selected from
the group consisting of 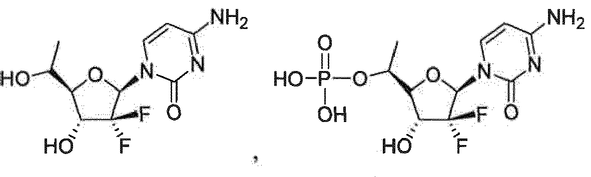 or a
diphosphate thereof, and 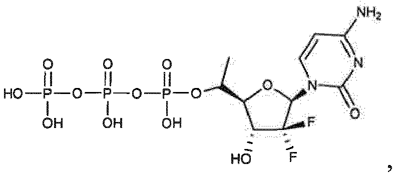 or a pharmaceutically acceptable salt of any of the foregoing.
